# EUROPÄISCHE PATENTSCHRIFT

(11) **EP 1 963 289 B1**
(45) Veröffentlichungstag und Bekanntmachung des Hinweises auf die Patenterteilung: **15.05.2013**
(21) Anmeldenummer: 06841031.5
(22) Anmeldetag: 19.12.2006
(51) Int. Cl.: C07D 271/06, C07D 413/04, C07D 413/06, C07D 413/08, C07D 413/14, A61K 31/4245

(54) **SUBSTITUIERTE OXADIAZOL-DERIVATE UND IHRE VERWENDUNG ALS OPIOID-REZEPTOR LIGANDEN**
SUBSTITUTED OXADIAZOLE DERIVATIVES AND THEIR USE AS OPIOID RECEPTOR LIGANDS
DERIVES OXADIAZOL SUBSTITUES ET UTILISATION EN TANT QUE LIGANDS DE RECEPTEURS OPIOIDES

(30) Priorität: 22.12.2005 DE 102005061427
(43) Veröffentlichungstag der Anmeldung: 03.09.2008
(73) Patentinhaber: Grünenthal GmbH, 52078 Aachen (DE)
(72) Erfinder: MERLA, Beatrix, 52078 Aachen (DE); OBERBÖRSCH, Stefan, 52078 Aachen (DE); SUNDERMANN, Bernd, 61381 Friedrichsdorf (DE); ENGLBERGER, Werner, 52223 Stolberg (DE); HENNIES, Hagen-Heinrich, 52152 Simmerath (DE); GRAUBAUM, Heinz, 12557 Berlin (DE)
(74) Vertreter: Brosch, Oliver
(86) Internationale Anmeldenummer: PCT/EP2006/012225
(87) Internationale Veröffentlichungsnummer: WO 2007/079931

(56) Entgegenhaltungen:
- WO-A-01/49654

## Beschreibung

Die vorliegende Erfindung betrifft substituierte Oxadiazol-Derivate, Verfahren zu deren Herstellung, Arzneimittel enthaltend diese Verbindungen und die Verwendung von substituierten Oxadiazol-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

J. Med. Chem 1993, 36, 1529-1538 offenbart 5-HT_{1D}-Agonisten, die eine Wirkung gegen Migräne aufweisen. Diese Verbindungen sind am Oxadiazol-Ring mit einem Indolylrest substituiert und tragen keinen Cyclohexylaminomethylrest.

Aus der WO 01/49654 sind substituierte Aminomethyl-Phenyl-Cyclohexanderivate sowie Verfahren zu deren Herstellung, deren Verwendung zur Herstellung von Arzneimitteln und Arzneimittel enthaltend diese Verbindungen bekannt.

Eine der Erfindung zugrundeliegende Aufgabe bestand darin, neue analgetisch wirksame Substanzen zur Verfügung zu stellen, die sich zur Schmerztherapie - insbesondere auch chronischer und neuropathischer Schmerzen - eignen.

Gegenstand der Erfindung sind daher substituierte Oxadiazol-Derivate der allgemeinen Formel I, 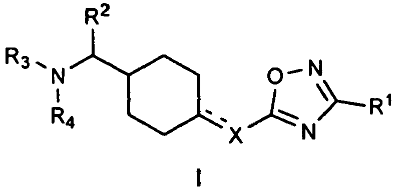 , worin
X CH, CH₂, CH=CH, CH₂CH₂, CH₂CH=CH oder CH₂CH₂CH₂ bedeutet;
R¹ Aryl, Heteroaryl, jeweils unsubstituiert oder einfach oder mehrfach substituiert; über eine C₁₋₃-Alkylgruppe verknüpften Aryl oder Heteroarylrest, jeweils unsubstituiert oder einfach oder mehrfach substituiert; bedeutet;
R² Aryl oder Heteroaryl, jeweils unsubstituiert oder einfach oder mehrfach substituiert; einen über eine C₁₋₃-Alkylkette gebundenen Arylrest, jeweils unsubstituiert oder einfach oder mehrfach substituiert, bedeutet;
R³ und R⁴ unabhängig voneinander H; C₁₋₆-Alkyl, jeweils gesättigt oder ungesättigt, verzweigt oder unverzweigt, bedeutet, wobei R³ und R⁴ nicht gleichzeitig H bedeuten,
oder die Reste R³ und R⁴ zusammen CH₂CH₂OCH₂CH₂, oder (CH₂)₃₋₆ bedeuten,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren.

Die Verbindungen weisen eine Affinität zum µ-Opioid-Rezeptor auf.

Die Ausdrücke "C₁₋₃-Alkyl" und "C₁₋₆-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 3 C-Atomen bzw. 1-6 C-Atomen, d.h. C₁₋₃-Alkanyle, C₂₋₃-Alkenyle und C₂₋₃-Alkinyle bzw. C₁₋₆-Alkanyle, C₂₋₆-Alkenyle und C₂₋₆-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, n-Hexyl, 2-Hexyl, Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl und Hexinyl umfasst. Besonders vorteihaft sind Methyl und *tert*-Butyl.

Der Ausdruck "Aryl" bedeutet im Sinne dieser Erfindung aromatische Kohlenwasserstoffe, u.a. Phenyle und Naphthyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl, welche jeweils unsubstituiert oder ein- oder mehrfach substituiert sein können, enthält. Besonders vorteilhaft ist der Phenyl-Rest.

Der Ausdruch "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxadiazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann. Besonders bevorzugt sind Pyridyl, Pyrrolyl und Thienyl.

Der Ausdruck "über C₁₋₃-Alkyl gebundenes Aryl oder Heteroaryl" bedeuten für die Zwecke der vorliegenden Erfindung, daß C₁₋₃-Alkyl und Aryl bzw. Heteroaryl die oben definierten Bedeutungen haben und der Aryl- bzw. Heteroaryl-Rest über eine C₁₋₃-Alkyl-Gruppe an die Verbindung der allgemeinen Struktur I gebunden ist. Besonders vorteilhaft im Sinne dieser Erfindung ist Benzyl und Phenethyl.

Im Zusammenhang mit "Alkyl" versteht man unter dem Begriff "substituiert" im Sinne dieser Erfindung die Substitution eines Wasserstoffrestes durch F, Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-Alkyl-OH)₂, NO₂, SH, S-C₁₋₆-Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl oder Benzyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen.

In Bezug auf "Aryl" und "Heteroaryl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, NH₂, NH-C₁₋₆-Alkyl, NH- C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, C₁₋₆-Alkyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" und "Heteroaryl" sind dabei bevorzugte Substituenten -F, -Cl, -CF₃, -O-CH₃, Methyl, O-CF₃, und *tert*.-Butyl.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure, Phosphorsäure und/oder Asparaginsäure. Besonders bevorzugt ist die Salzsäure.

Unter dem Begriff (CH₂)₃₋₆ bzw. (CH₂)₄₋₅ ist -CH₂-CH₂-CH₂-, -CH₂-CH₂-CH₂-CH₂-, - CH₂-CH₂-CH₂-CH₂-CH₂- und CH₂-CH₂-CH₂-CH₂-CH₂-CH₂- bzw -CH₂-CH₂-CH₂-CH₂-, -CH₂-CH₂-CH₂-CH₂-CH₂ zu verstehen.

Bevorzugt im Sinne dieser Erfindung sind Oxadiazol-Denvate, worin X CH, CH₂, CH=CH oder CH₂CH₂ bedeutet.

Bevorzugt im Sinne dieser Erfindung sind substituierte Oxadiazol-Derivate, worin
R¹ Phenyl, Naphthyl, Pyrrolyl, Indolyl, Furyl, Benzofuranyl, Thienyl, Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxadiazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, jeweils unsubstituiert oder einfach oder mehrfach substituiert mit F, Cl, Br, I, CN, NH₂, NH-C₁₋₆-Alkyl, NH- C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, OCF₃, C₁₋₆-Alkyl; über eine C₁₋₃-Alkylgruppe verknüpftes Phenyl, Naphthyl, Pyrrolyl, Indolyl, Furyl, Benzofuranyl, Thienyl, Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxadiazolyl, Isoxazoyl, Pyridyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl, jeweils unsubstituiert oder einfach oder mehrfach substituiert mit F, Cl, Br, I, CN, OCF₃, NH₂, NH-C₁₋₆-Alkyl, NH- C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, C₁₋₆-Alkyl; bedeutet;
insbesondere
R¹ Phenyl, Naphthyl, Thienyl, Pyridyl oder Pyrrolyl, Benzyl, Methylindolyl, Methylthienyl oder Phenethyl, unsubstituiert oder einfach oder mehrfach substituiert mit F, Cl, Br, CN, OCF₃, NH₂, NO₂, SH, S-C₁₋₆-Alkyl, OH, O-C₁₋₆-Alkyl, CO₂H, CO₂-C₁₋₆-Alkyl, CF₃ oder C₁₋₆-Alkyl bedeutet.

Besonders bevorzugt sind substituierte Oxadiazol-Derivate, worin R¹ Phenyl, Thienyl, Benzyl, Methylindolyl, Methylthienyl oder Pyrrolyl, unsubstituiert oder einfach oder mehrfach substituiert mit Cl, Br, OCH₃, CH₃, F, OCF₃, CF₃ oder *tert*.-Butyl bedeutet.

Bevorzugt im Sinne dieser Erfindung sind auch substituierte Oxadiazol-Derivate, worin
R² Phenyl, Thienyl oder Pyridyl, jeweils unsubstituiert oder einfach oder mehrfach substituiert mit F, Cl, CN, NH-C₁₋₆-Alkyl, NH-C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, C₁₋₆-Alkyl; einen über eine C₁₋₃-Alkylkette gebundenen Arylrest, jeweils unsubstituiert oder einfach oder mehrfach substituiert mit F, Cl, CN, NH-C₁₋₆-Alkyl, NH-C₁₋₆-Alkyl-OH, N(C₁₋₆Alkyl)₂, N(C₁₋₆-Alkyl-OH)₂, NO₂, SH, S-C₁₋₆-Alkyl, OH, O-C₁₋₆-Alkyl, O-C₁₋₆Alkyl-OH, C(=O)C₁₋₆-Alkyl, CO₂H, CO₂-C₁₋₆-Alkyl, CF₃, C₁₋₆-Alkyl, bedeutet;
vorzugsweise
R² Phenyl oder Thienyl, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl; einen über eine C₁₋₃-Alkylkette gebundenen Phenylrest, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆Alkyl, bedeutet;
insbesondere
R² Phenyl, unsubstituiert oder einfach oder mehrfach substituiert mit F, Cl, OH, OCH₃, CF₃ oder CH₃; Thienyl; oder einen über eine C₁₋₃-Alkylkette gebundenen Phenylrest, unsubstituiert oder einfach oder mehrfach substituiert mit F, Cl, CN, OH, OCH₃, CF₃ oder CH₃; bedeutet.

Besonders bevorzugt sind Oxadiazol Derivate, worin R² Phenyl, unsubstituiert oder einfach substituiert mit Cl oder F, Phenethyl oder Thienyl bedeutet.

Außerdem bevorzugt im Sinne dieser Erfindung sind Oxadiazol-Derivate, worin R³ und R⁴ unabhängig voneinander H oder C₁₋₆-Alkyl bedeuten, wobei R³ und R⁴ nicht gleichzeitig H bedeuten, oder R³ und R⁴ zusammen CH₂CH₂OCH₂CH₂, oder (CH₂)₄₋₅ bedeuten.

Besonders bevorzugt sind Oxadiazol-Derivate, worin R³ und R⁴ CH₃ bedeuten.

Es ist bevorzugt, dass X CH, CH₂, CH=CH oder CH₂CH₂ bedeutet.

Ganz besonders bevorzugt sind substituierte Oxadiazol-Derivate aus der Gruppe
43. (4-((3-(4-chlorbenzyl)-1,2,4-oxadiazol-5-yl)methyl)cyclohexyl)-N,N-dimethyl(phenyl)methanamin
44. (4-((3-(4-methoxybenzyl)-1,2,4-oxadiazol-5-yl)methyl)cyclohexyl)-N,N-dimethyl(phenyl)methanamin
45. ((4-Chlor-phenyl)-{4-[3-(4-chlor-phenyl)-[1,2,4]oxadiazol-5-ylmethylen]-cyclohexyl}-methyl)-dimethyl-amin
46. [{4-[3-(4-Chlor-phenyl)-[1,2,4]oxadiazol-5-ylmethylen]-cyclohexyl}-(4-fluor-phenyl)-methyl]-dimethyl-amin (Polareres Diastereomer)
47. [{4-[3-(4-Chlor-phenyl)-[1,2,4]oxadiazol-5-ylmethylen]-cyclohexyl}-(4-fluor-phenyl)-methyl]-dimethyl-amin (Unpolareres Diastereomer)
48. ({4-[3-(2,3-Dichlor-phenyl)-[1,2,4]oxadiazol-5-ylmethylen]-cyclohexyl}-phenyl-methyl)-dimethyl-amin (Polareres Diastereomer)
49. ({4-[3-(2,3-Dichlor-phenyl)-[1,2,4]oxadiazol-5-ylmethylen]-cyclohexyl}-phenyl-methyl)-dimethyl-amin (Unpolareres Diastereomer)
50. [{4-[3-(2,3-Dichlor-phenyl)-[1,2,4]oxadiazol-5-ylmethylen]-cyclohexyl}-(3-fluorphenyl)-methyl]-dimethyl-amin (Polareres Diastereomer)
51. [{4-[3-(2,3-Dichlor-phenyl)-[1,2,4]oxadiazol-5-ylmethylen]-cyclohexyl}-(3-fluorphenyl)-methyl]-dimethyl-amin (Unpolareres Diastereomer)
52. ((4-Chlor-phenyl)-{4-[3-(2,3-dichlor-phenyl)-[1,2,4]oxadiazol-5-ylmethylen]-cyclohexyl}-methyl)-dimethyl-amin (Polareres Diastereomer)
53. ((4-Chlor-phenyl)-{4-[3-(2,3-dichlor-phenyl)-[1,2,4]oxadiazol-5-ylmethylen]-cyclohexyl}-methyl)-dimethyl-amin (Unpolareres Diastereomer)
54. (1-{4-[3-(2,3-Dichlor-phenyl)-[1,2,4]oxadiazol-5-ylmethylen]-cyclohexyl}-3-phenylpropyl)-dimethyl-amin
55. [{4-[3-(2,3-Dichlor-phenyl)-[1,2,4]oxadiazol-5-ylmethylen]-cyclohexyl}-(4-fluorphenyl)-methyl]-dimethyl-amin (Polareres Diastereomer)
56. [{4-[3-(2,3-Dichlor-phenyl)-[1,2,4]oxadiazol-5-ylmethylen]-cyclohexyl}-(4-fluorphenyl)-methyl]-dimethyl-amin (Unpolareres Diastereomer)
57. Dimethyl-(phenyl-{4-[3-(4-trifluormethoxy-phenyl)-[1,2,4]oxadiazol-5-ylmethylen]-cyclohexyl}-methyl)-amin (Polareres Diastereomer)
58. Dimethyl-(phenyl-{4-[3-(4-trifluormethoxy-phenyl)-[1,2,4]oxadiazol-5-ylmethylen]-cyclohexyl}-methyl)-amin (Unpolareres Diastereomer)
59. ((3-Fluor-phenyl)-{4-[3-(4-trifluormethoxy-phenyl)-[1,2,4]oxadiazol-5-ylmethylen]-cyclohexyl}-methyl)-dimethyl-amin (Polareres Diastereomer)
60. ((3-Fluor-phenyl)-{4-[3-(4-trifluormethoxy-phenyl)-[1,2,4]oxadiazol-5-ylmethylen]-cyclohexyl}-methyl)-dimethyl-amin (Unpolareres Diastereomer)
61. ((4-Chlor-phenyl)-{4-[3-(4-trifluormethoxy-phenyl)-[1,2,4]oxadiazol-5-ylmethylen]-cyclohexyl}-methyl)-dimethyl-amin (Polareres Diastereomer)
62. ((4-Chlor-phenyl)-{4-[3-(4-trifluormethoxy-phenyl)-[1,2,4]oxadiazol-5-ylmethylen]-cyclohexyl}-methyl)-dimethyl-amin (Unpolareres Diastereomer)
63. Dimethyl-(3-phenyl-1-{4-[3-(4-trifluormethoxy-phenyl)-[1,2,4]oxadiazol-5-ylmethylen]-cyclohexyl}-propyl)-amin (Polareres Diastereomer)
64. Dimethyl-(3-phenyl-1-{4-[3-(4-trifluormethoxy-phenyl)-[1,2,4]oxadiazol-5-ylmethylen]-cyclohexyl}-propyl)-amin (Unpolareres Diastereomer)
65. ((4-Fluor-phenyl)-{4-[3-(4-trifluormethoxy-phenyl)-[1,2,4]oxadiazol-5-ylmethylen]-cyclohexyl}-methyl)-dimethyl-amin (Polareres Diastereomer)
66. ((4-Fluor-phenyl)-{4-[3-(4-trifluormethoxy-phenyl)-[1,2,4]oxadiazol-5-ylmethylen]-cyclohexyl}-methyl)-dimethyl-amin (Unpolareres Diastereomer)
67. Dimethyl-(thiophen-2-yl-{4-[3-(4-trifluormethoxy-phenyl)-[1,2,4]oxadiazol-5-ylmethylen]-cyclohexyl}-methyl)-amin (Polareres Diastereomer)
68. Dimethyl-(thiophen-2-yl-{4-[3-(4-trifluormethoxy-phenyl)-[1,2,4]oxadiazol-5-ylmethylen]-cyclohexyl}-methyl)-amin (Unpolareres Diastereomer)
69. Dimethyl-{phenyl-[4-(3-phenyl-[1,2,4]oxadiazol-5-ylmethylen)-cyclohexyl]-methyl}-amin (Polareres Diastereomer)
70. Dimethyl-{phenyl-[4-(3-phenyl-[1,2,4]oxadiazol-5-ylmethylen)-cyclohexyl]-methyl}-amin (Unpolareres Diastereomer)
71. {(3-Fluor-phenyl)-[4-(3-phenyl-[1,2,4]oxadiazol-5-ylmethylen)-cyclohexyl]-methyl}-dimethyl-amin (Polareres Diastereomer)
72. {(3-Fluor-phenyl)-[4-(3-phenyl-[1,2,4]oxadiazol-5-ylmethylen)-cyclohexyl]-methyl}-dimethyl-amin (Unpolareres Diastereomer)
73. Dimethyl-{3-phenyl-1-[4-(3-phenyl-[1,2,4]oxadiazol-5-ylmethylen)-cyclohexyl]-propyl}-amin (Polareres Diastereomer)
74. Dimethyl-{3-phenyl-1-[4-(3-phenyl-[1,2,4]oxadiazol-5-ylmethylen)-cyclohexyl]-propyl}-amin (Unpolareres Diastereomer)
75. {(4-Fluor-phenyl)-[4-(3-phenyl-[1,2,4]oxadiazol-5-ylmethylen)-cyclohexyl]-methyl}-dimethyl-amin (Polareres Diastereomer)
76. {(4-Fluor-phenyl)-[4-(3-phenyl-[1,2,4]oxadiazol-5-ylmethylen)-cyclohexyl]-methyl}-dimethyl-amin (Unpolareres Diastereomer)
77. [{4-[3-(2,4-Difluor-phenyl)-[1,2,4]oxadiazol-5-ylmethylen]-cyclohexyl}-(3-fluorphenyl)-methyl]-dimethyl-amin (Polareres Diastereomer)
78. [{4-[3-(2,4-Difluor-phenyl)-[1,2,4]oxadiazol-5-ylmethylen]-cyclohexyl}-(3-fluorphenyl)-methyl]-dimethyl-amin (Unpolareres Diastereomer)
79. ({4-[3-(4-Methoxy-benzyl)-[1,2,4]oxadiazol-5-ylmethylen]-cyclohexyl}-phenyl-methyl)-dimethyl-amin (Polareres Diastereomer)
80. ({4-[3-(4-Methoxy-benzyl)-[1,2,4]oxadiazol-5-ylmethylen]-cyclohexyl}-phenyl-methyl)-dimethyl-amin (Unpolareres Diastereomer)
81. ((3-Fluor-phenyl)-{4-[3-(4-methoxy-benzyl)-[1,2,4]oxadiazol-5-ylmethylen]-cyclohexyl}-methyl)-dimethyl-amin (Polareres Diastereomer)
82. ((3-Fluor-phenyl)-{4-[3-(4-methoxy-benzyl)-[1,2,4]oxadiazol-5-ylmethylen]-cyclohexyl}-methyl)-dimethyl-amin (Unpolareres Diastereomer)
83. ((4-Chlor-phenyl)-{4-[3-(4-methoxy-benzyl)-[1,2,4]oxadiazol-5-ylmethylen]-cyclohexyl}-methyl)-dimethyl-amin (Polareres Diastereomer)
84. ((4-Chlor-phenyl)-{4-[3-(4-methoxy-benzyl)-[1,2,4]oxadiazol-5-ylmethylen]-cyclohexyl}-methyl)-dimethyl-amin (Unpolareres Diastereomer)
85. (1-{4-[3-(4-Methoxy-benzyl)-[1,2,4]oxadiazol-5-ylmethylen]-cyclohexyl}-3-phenylpropyl)-dimethyl-amin (Polareres Diastereomer)
86. (1-{4-[3-(4-Methoxy-benzyl)-[1,2,4]oxadiazol-5-ylmethylen]-cyclohexyl}-3-phenylpropyl)-dimethyl-amin (Unpolareres Diastereomer)
87. ((4-Fluor-phenyl)-{4-[3-(4-methoxy-benzyl)-[1,2,4]oxadiazol-5-ylmethylen]-cyclohexyl}-methyl)-dimethyl-amin (Polareres Diastereomer)
88. ((4-Fluor-phenyl)-{4-[3-(4-methoxy-benzyl)-[1,2,4]oxadiazol-5-ylmethylen]-cyclohexyl}methyl)-dimethyl-amin (Unpolareres Diastereomer)
89. ({4-[3-(4-Chlor-benzyl)-[1,2,4]oxadiazol-5-ylmethylen]-cyclohexyl}-phenyl-methyl)-dimethyl-amin (Polareres Diastereomer)
90. ({4-[3-(4-Chlor-benzyl)-[1,2,4]oxadiazol-5-ylmethylen]-cyclohexyl}-phenyl-methyl)-dimethyl-amin (Unpolareres Diastereomer)
91. [{4-[3-(4-Chlor-benzyl)-[1,2,4]oxadiazol-5-ylmethylen]-cyclohexyl}-(3-fluor-phenyl)-methyl]-dimethyl-amin (Polareres Diastereomer)
92. [{4-[3-(4-Chlor-benzyl)-[1,2,4]oxadiazol-5-ylmethylen]-cyclohexyl}-(3-fluor-phenyl)-methyl]-dimethyl-amin (Unpolareres Diastereomer)
93. [{4-[3-(4-Chlor-benzyl)-[1,2,4]oxadiazol-5-ylmethylen]-cyclohexyl}-(4-chlor-phenyl)-methyl]-dimethyl-amin (Polareres Diastereomer)
94. [{4-[3-(4-Chlor-benzyl)-[1,2,4]oxadiazol-5-ylmethylen]-cyclohexyl}-(4-chlor-phenyl)-methyl]-dimethyl-amin (Unpolareres Diastereomer)
95. (1-{4-[3-(4-Chlor-benzyl)-[1,2,4]oxadiazol-5-ylmethylen]-cyclohexyl}-3-phenylpropyl)-dimethyl-amin (Polareres Diastereomer)
96. (1-{4-[3-(4-Chlor-benzyl)-[1,2,4]oxadiazol-5-ylmethylen]-cyclohexyl}-3-phenylpropyl)-dimethyl-amin (Unpolareres Diastereomer)
97. [{4-[3-(4-Chlor-benzyl)-[1,2,4]oxadiazol-5-ylmethylen]-cyclohexyl}-(4-fluor-phenyl)-methyl]-dimethyl-amin (Polareres Diastereomer)
98. [{4-[3-(4-Chlor-benzyl)-[1,2,4]oxadiazol-5-ylmethylen]-cyclohexyl}-(4-fluor-phenyl)-methyl]-dimethyl-amin (Unpolareres Diastereomer)
99. {(3-Fluor-phenyl)-[4-(3-p-tolyl-[1,2,4]oxadiazol-5-ylmethylen)-cyclohexyl]-methyl}-dimethyl-amin
100. {(4-Chlor-phenyl)-[4-(3-p-tolyl-[1,2,4]oxadiazol-5-ylmethylen)-cyclohexyl]-methyl}-dimethyl-amin
101. {(4-Fluor-phenyl)-[4-(3-p-tolyl-[1,2,4]oxadiazol-5-ylmethylen)-cyclohexyl]-methyl}-dimethyl-amin (Polareres Diastereomer)
102. {(4-Fluor-phenyl)-[4-(3-p-tolyl-[1,2,4]oxadiazol-5-ylmethylen)-cyclohexyl]-methyl}-dimethyl-amin (Unpolareres Diastereomer)
103. ({4-[3-(3,4-Dimethoxy-phenyl)-[1,2,4]oxadiazol-5-ylmethylen]-cyclohexyl}-phenyl-methyl)-dimethyl-amin (Polareres Diastereomer)
104. ({4-[3-(3,4-Dimethoxy-phenyl)-[1,2,4]oxadiazol-5-ylmethylen]-cyclohexyl}-phenyl-methyl)-dimethyl-amin (Unpolareres Diastereomer)
105. [{4-[3-(3,4-Dimethoxy-phenyl)-[1,2,4]oxadiazol-5-ylmethylen]-cyclohexyl}-(3-fluorphenyl)-methyl]-dimethyl-amin (Polareres Diastereomer)
106. [{4-[3-(3,4-Dimethoxy-phenyl)-[1,2,4]oxadiazol-5-ylmethylen]-cyclohexyl}-(3-fluorphenyl)-methyl]-dimethyl-amin (Unpolareres Diastereomer)
107. ((4-Chlor-phenyl)-{4-[3-(3,4-dimethoxy-phenyl)-[1,2,4]oxadiazol-5-ylmethylen]-cyclohexyl}-methyl)-dimethyl-amin (Polareres Diastereomer)
108. ((4-Chlor-phenyl)-{4-[3-(3,4-dimethoxy-phenyl)-[1,2,4]oxadiazol-5-ylmethylen]-cyclohexyl}-methyl)-dimethyl-amin (Unpolareres Diastereomer)
109. (1-{4-[3-(3,4-Dimethoxy-phenyl)-[1,2,4]oxadiazol-5-ylmethylen]-cyclohexyl}-3-phenyl-propyl)-dimethyl-amin (Polareres Diastereomer)
110. (1-{4-[3-(3,4-Dimethoxy-phenyl)-[1,2,4]oxadiazol-5-ylmethylen]-cyclohexyl}-3-phenyl-propyl)-dimethyl-amin (Unpolareres Diastereomer)
111. [{4-[3-(3,4-Dimethoxy-phenyl)-[1,2,4]oxadiazol-5-ylmethylen]-cyclohexyl}-(4-fluorphenyl)-methyl]-dimethyl-amin (Polareres Diastereomer)
112. [{4-[3-(3,4-Dimethoxy-phenyl)-[1,2,4]oxadiazol-5-ylmethylen]-cyclohexyl}-(4-fluorphenyl)-methyl]-dimethyl-amin (Unpolareres Diastereomer)
113. ({4-[3-(3-Chlor-phenyl)-[1,2,4]oxadiazol-5-ylmethylen]-cyclohexyl}-phenyl-methyl)-dimethyl-amin (Polareres Diastereomer)
114. ({4-[3-(3-Chlor-phenyl)-[1,2,4]oxadiazol-5-ylmethylen]-cyclohexyl}-phenyl-methyl)-dimethyl-amin (Unpolareres Diastereomer)
115. [{4-[3-(3-Chlor-phenyl)-[1,2,4]oxadiazol-5-ylmethylen]-cyclohexyl}-(3-fluor-phenyl)-methyl]-dimethyl-amin (Polareres Diastereomer)
116. [{4-[3-(3-Chlor-phenyl)-[1,2,4]oxadiazol-5-ylmethylen]-cyclohexyl}-(3-fluor-phenyl)-methyl]-dimethyl-amin (Unpolareres Diastereomer)
117. ((4-Chlor-phenyl)-{4-[3-(3-chlor-phenyl)-[1,2,4]oxadiazol-5-ylmethylen]-cyclohexyl}-methyl)-dimethyl-amin (Polareres Diastereomer)
118. ((4-Chlor-phenyl)-{4-[3-(3-chlor-phenyl)-[1,2,4]oxadiazol-5-ylmethylen]-cyclohexyl}-methyl)-dimethyl-amin (Unpolareres Diastereomer)
119. (1-{4-[3-(3-Chlor-phenyl)-[1,2,4]oxadiazol-5-ylmethylen]-cyclohexyl}-3-phenylpropyl)-dimethyl-amin (Polareres Diastereomer)
120. (1-{4-[3-(3-Chlor-phenyl)-[1,2,4]oxadiazol-5-ylmethylen]-cyclohexyl}-3-phenylpropyl)-dimethyl-amin (Unpolareres Diastereomer)
121. [{4-[3-(3-Chlor-phenyl)-[1,2,4]oxadiazol-5-ylmethylen]-cyclohexyl}-(4-fluor-phenyl)-methyl]-dimethyl-amin
122. ({4-[3-(4-tert-Butyl-phenyl)-[1,2,4]oxadiazol-5-ylmethylen]-cyclohexyl}-phenyl-methyl)-dimethyl-amin (Polareres Diastereomer)
123. ({4-[3-(4-tert-Butyl-phenyl)-[1,2,4]oxadiazol-5-ylmethylen]-cyclohexyl}-phenyl-methyl)-dimethyl-amin (Unpolareres Diastereomer)
124. [{4-[3-(4-tert-Butyl-phenyl)-[1,2,4]oxadiazol-5-ylmethylen]-cyclohexyl}-(3-fluorphenyl)-methyl]-dimethyl-amin (Polareres Diastereomer)
125. [{4-[3-(4-tert-Butyl-phenyl)-[1,2,4]oxadiazol-5-ylmethylen]-cyclohexyl}-(3-fluorphenyl)-methyl]-dimethyl-amin (Unpolareres Diastereomer)
126. [{4-[3-(4-tert-Butyl-phenyl)-[1,2,4]oxadiazol-5-ylmethylen]-cyclohexyl}-(4-chlorphenyl)-methyl]-dimethyl-amin (Polareres Diastereomer)
127. [{4-[3-(4-tert-Butyl-phenyl)-[1,2,4]oxadiazol-5-ylmethylen]-cyclohexyl}-(4-chlorphenyl)-methyl]-dimethyl-amin (Unpolareres Diastereomer)
128. (1-{4-[3-(4-tert-Butyl-phenyl)-[1,2,4]oxadiazol-5-ylmethylen]-cyclohexyl}-3-phenylpropyl)-dimethyl-amin (Polareres Diastereomer)
129. (1-{4-[3-(4-tert-Butyl-phenyl)-[1,2,4]oxadiazol-5-ylmethylen]-cyclohexyl}-3-phenylpropyl)-dimethyl-amin (Unpolareres Diastereomer)
130. [{4-[3-(4-tert-Butyl-phenyl)-[1,2,4]oxadiazol-5-ylmethylen]-cyclohexyl}-(4-fluorphenyl)-methyl]-dimethyl-amin (Polareres Diastereomer)
131. [{4-[3-(4-tert-Butyl-phenyl)-[1,2,4]oxadiazol-5-ylmethylen]-cyclohexyl}-(4-fluorphenyl)-methyl]-dimethyl-amin (Unpolareres Diastereomer)
132. ({4-[3-(4-tert-Butyl-phenyl)-[1,2,4]oxadiazol-5-ylmethylen]-cyclohexyl}-thiophen-2-yl-methyl)-dimethyl-amin (Polareres Diastereomer)
133. ({4-[3-(4-tert-Butyl-phenyl)-[1,2,4]oxadiazol-5-ylmethylen]-cyclohexyl}-thiophen-2-yl-methyl)-dimethyl-amin (Unpolareres Diastereomer)
134. Dimethyl-{phenyl-[4-(3-m-tolyl-[1,2,4]oxadiazol-5-ylmethylen)-cyclohexyl]-methyl}-amin (Polareres Diastereomer)
135. Dimethyl-{phenyl-[4-(3-m-tolyl-[1,2,4]oxadiazol-5-ylmethylen)-cyclohexyl]-methyl}-amin (Unpolareres Diastereomer)
136. {(3-Fluor-phenyl)-[4-(3-m-tolyl-[1,2,4]oxadiazol-5-ylmethylen)-cyclohexyl]-methyl}-dimethyl-amin (Polareres Diastereomer)
137. {(3-Fluor-phenyl)-[4-(3-m-tolyl-[1,2,4]oxadiazol-5-ylmethylen)-cyclohexyl]-methyl}-dimethyl-amin (Unpolareres Diastereomer)
138. {(4-Chlor-phenyl)-[4-(3-m-tolyl-[1,2,4]oxadiazol-5-ylmethylen)-cyclohexyl]-methyl}-dimethyl-amin (Polareres Diastereomer)
139. {(4-Chlor-phenyl)-[4-(3-m-tolyl-[1,2,4]oxadiazol-5-ylmethylen)-cyclohexyl]-methyl}-dimethyl-amin (Unpolareres Diastereomer)
140. ((4-Fluor-phenyl)-{4-[2-(3-phenyl-[1,2,4]oxadiazol-5-yl)-vinyl]-cyclohexyl}-methyl)-dimethyl-amin
141. [(4-{2-[3-(2,4-Difluor-phenyl)-[1,2,4]oxadiazol-5-yl]-vinyl}-cyclohexyl)-(4-fluorphenyl)-methyl]-dimethyl-amin
142. [(4-Fluor-phenyl)-(4-{2-[3-(4-methoxy-benzyl)-[1,2,4]oxadiazol-5-yl]-vinyl}-cyclohexyl)-methyl]-dimethyl-amin
143. [(4-{2-[3-(4-Chlor-benzyl)-[1,2,4]oxadiazol-5-yl]-vinyl}-cyclohexyl)-(4-fluor-phenyl)-methyl]-dimethyl-amin
144. Dimethyl-(3-phenyl-1-{4-[2-(3-phenyl-[1,2,4]oxadiazol-5-yl)-vinyl]-cyclohexyl}-propyl)-amin
145. [1-(4-{2-[3-(2,4-Difluor-phenyl)-[1,2,4]oxadiazol-5-yl]-vinyl}-cyclohexyl)-3-phenylpropyl]-d imethyl-amin
146. [1-(4-{2-[3-(4-Methoxy-benzyl)-[1,2,4]oxadiazol-5-yl]-vinyl}-cyclohexyl)-3-phenylpropyl]-dimethyl-amin
147. [1-(4-{2-[3-(4-Chlor-benzyl)-[1,2,4]oxadiazol-5-yl]-vinyl}cyclohexyl)-3-phenylpropyl]-dimethyl-amin
148. Dimethyl-(phenyl-{4-[2-(3-phenyl-[1,2,4]oxadiazol-5-yl)-vinyl]-cyclohexyl}-methyl)-amin
149. [(4-{2-[3-(4-Chlor-benzyl)-[1,2,4]oxadiazol-5-yl]-vinyl}-cyclohexyl)-phenyl-methyl]-dimethyl-amin (Polareres Diastereomer)
150. [(4-{2-[3-(4-Chlor-benzyl)-[1,2,4]oxadiazol-5-yl]-vinyl}-cyclohexyl)-phenyl-methyl]-dimethyl-amin (Unpolareres Diastereomer)
151. [(4-Chlor-phenyl)-(4-{2-[3-(2,4-difluor-phenyl)-[1,2,4]oxadiazol-5-yl]-vinyl}-cyclohexyl)-methyl]-dimethyl-amin
152. [(4-Chlor-phenyl)-(4-{2-[3-(4-methoxy-benzyl)-[1,2,4]oxadiazol-5-yl]-vinyl}-cyclohexyl)-methyl]-dimethyl-amin
153. [(4-{2-[3-(4-Chlor-benzyl)-[1,2,4]oxadiazol-5-yl]-vinyl}-cyclohexyl)-(4-chlor-phenyl)-methyl]-dimethyl-amin
154. [(4-{2-[3-(4-Methoxy-benzyl)-[1,2,4]oxadiazol-5-yl]-vinyl}-cyclohexyl)-thiophen-2-yl-methyl]-dimethyl-amin
155. [(4-{2-[3-(4-Chlor-benzyl)-[1,2,4]oxadiazol-5-yl]-vinyl}-cyclohexyl)-thiophen-2-yl-methyl]-dimethyl-amin
156. ((3-Fluor-phenyl)-{4-[2-(3-phenyl-[1,2,4]oxadiazol-5-yl)-vinyl]-cyclohexyl}-methyl)-dimethyl-amin
157. [(3-Fluor-phenyl)-(4-{2-[3-(4-methoxy-benzyl)-[1,2,4]oxadiazol-5-yl]-vinyl}-cyclohexyl)-methyl]-dimethyl-amin
158. ((4-Fluor-phenyl)-{4-[2-(3-p-tolyl-[1,2,4]oxadiazol-5-yl)-vinyl]-cyctohexyl}-methyl)-dimethyl-amin
159. [(4-{2-[3-(3,4-Dimethoxy-phenyl)-[1,2,4]oxadiazol-5-yl]-vinyl}-cyclohexyl)-(4-fluorphenyl)-methyl]-dimethyl-amin
160. Dimethyl-(3-phenyl-1-{4-[2-(3-p-tolyl-[1,2,4]oxadiazol-5-yl)-vinyl]-cyclohexyl}-propyl)-amin
161. [1-(4-{2-[3-(3,4-Dimethoxy-phenyl)-[1,2,4]oxadiazol-5-yl]-vinyl}-cyclohexyl)-3-phenylpropyl]-dimethyl-amin
162. Dimethyl-(phenyl-{4-[2-(3-p-tolyl-[1,2,4]oxadiazol-5-yl)-vinyl]-cyclohexyl}-methyl)-amin
163. [(4-{2-[3-(3,4-Dimethoxy-phenyl)-[1,2,4]oxadiazol-5-yl]-vinyl}-cyclohexyl)-phenyl-methyl]-dimethyl-amin
164. ((4-Chlor-phenyl)-{4-[2-(3-p-tolyl-[1,2,4]oxadiazol-5-yl)-vinyl]-cyclohexyl}-methyl)-dimethyl-amin
165. [(4-Chlor-phenyl)-(4-{2-[3-(3,4-dimethoxy-phenyl)-[1,2,4]oxadiazol-5-yl]-vinyl}-cyclohexyl)-methyl]-dimethyl-amin
166. Dimethyl-(thiophen-2-yl-{4-[2-(3-p-tolyl-[1,2,4]oxadiazol-5-yl)-vinyl]-cyclohexyl}-methyl)-amin
167. [(4-{2-[3-(3,4-Dimethoxy-phenyl)-[1,2,4]oxadiazol-5-yl]-vinyl}-cyclohexyl)-thiophen-2-yl-methyl]-dimethyl-amin
168. ((3-Fluor-phenyl)-{4-[2-(3-p-tolyl-[1,2,4]oxadiazol-5-yl)-vinyl]-cyclohexyl}-methyl)-dimethyl-amin
169. [(4-{2-[3-(3,4-Dimethoxy-phenyl)-[1,2,4]oxadiazol-5-yl]-vinyl}-cyclohexyl)-(3-fluorphenyl)-methyl]-dimethyl-amin
170. [(4-{2-[3-(3-Chlor-phenyl)-[1,2,4]oxadiazol-5-yl]-vinyl}-cyclohexyl)-(4-fluor-phenyl)-methyl]-dimethyl-amin
171. [(4-{2-[3-(4-tert-Butyl-phenyl)-[1,2,4]oxadiazol-5-yl]-vinyl}-cyclohexyl)-(4-fluorphenyl)-methyl]-dimethyl-amin
172. [(4-{2-[3-(4-tert-Butyl-phenyl)-[1,2,4]oxadiazol-5-yl]-vinyl}-cyclohexyl)-(4-fluorphenyl)-methyl]-dimethyl-amin
173. ((4-Fluor-phenyl)-{4-[2-(3-m-tolyl-[1,2,4]oxadiazol-5-yl)-vinyl]-cyclohexyl}-methyl)-dimethyl-amin
174. [1-(4-{2-[3-(3-Chlor-phenyl)-[1,2,4]oxadiazol-5-yl]-vinyl}-cyclohexyl)-3-phenylpropyl]-dimethyl-amin
175. [1-(4-{2-[3-(4-tert-Butyl-phenyl)-[1,2,4]oxadiazol-5-yl]-vinyl}-cyclohexyl)-3-phenylpropyl]-dimethyl-amin (Polareres Isomer)
176. [1-(4-{2-[3-(4-tert-Butyl-phenyl)-[1,2,4]oxadiazol-5-yl]-vinyl}-cyclohexyl)-3-phenylpropyl]-dimethyl-amin (Unpolareres Diastereomer)
177. Dimethyl-(3-phenyl-1-{4-[2-(3-m-tolyl-[1,2,4]oxadiazol-5-yl)-vinyl]-cyclohexyl}-propyl)-amin (Polareres Diastereomer)
178. Dimethyl-(3-phenyl-1-{4-[2-(3-m-tolyl-[1,2,4]oxadiazol-5-yl)-vinyl]-cyclohexyl}-propyl)-amin (Unpolareres Diastereomer)
179. [(4-{2-[3-(3-Chlor-phenyl)-[1,2,4]oxadiazol-5-yl]-vinyl}-cyclohexyl)-phenyl-methyl]-dimethyl-amin
180. [(4-{2-[3-(4-tert-Butyl-phenyl)-[1,2,4]oxadiazol-5-yl]-vinyl}-cyclohexyl)-phenyl-methyl]-dimethyl-amin (Polareres Diastereomer)
181. [(4-{2-[3-(4-tert-Butyl-phenyl)-[1,2,4]oxadiazol-5-yl]-vinyl}-cyclohexyl)-phenyl-methyl]-dimethyl-amin (Unpolareres Diastereomer)
182. Dimethyl-(phenyl-{4-[2-(3-m-tolyl-[1,2,4]oxadiazol-5-yl)-vinyl]-cyclohexyl}-methyl)-amin (Polareres Diastereomer)
183. Dimethyl-(phenyl-{4-[2-(3-m-tolyl-[1,2,4]oxadiazol-5-yl)-vinyl]-cyclohexyl}-methyl)-amin (Unpolareres Diastereomer)
184. [(4-{2-[3-(4-Chlor-phenyl)-[1,2,4]oxadiazol-5-yl]-vinyl}-cyclohexyl)-phenyl-methyl]-dimethyl-amin (Polareres Diastereomer)
185. [(4-{2-[3-(4-Chlor-phenyl)-[1,2,4]oxadiazol-5-yl]-vinyl}-cyclohexyl)-phenyl-methyl]-dimethyl-amin (Unpolareres Diastereomer)
186. [(4-Chlor-phenyl)-(4-{2-[3-(3-chlor-phenyl)-[1,2,4]oxadiazol-5-yl]-vinyl}-cyclohexyl)-methyl]-dimethyl-amin
187. [(4-{2-[3-(4-tert-Butyl-phenyl)-[1,2,4]oxadiazol-5-yl]-vinyl}-cyclohexyl)-(4-chlorphenyl)-methyl]-dimethyl-amin (Polareres Diastereomer)
188. [(4-{2-[3-(4-tert-Butyl-phenyl)-[1,2,4]oxadiazol-5-yl]-vinyl}-cyclohexyl)-(4-chlorphenyl)-methyl]-dimethyl-amin (Unpolareres Diastereomer)
189. ((4-Chlor-phenyl)-{4-[2-(3-m-tolyl-[1,2,4]oxadiazol-5-yl)-vinyl]-cyclohexyl}-methyl)-dimethyl-amin (Polareres Diastereomer)
190. ((4-Chlor-phenyl)-{4-[2-(3-m-tolyl-[1,2,4]oxadiazol-5-yl)-vinyl]-cyclohexyl}-methyl)-dimethyl-amin (Unpolareres Diastereomer)
191. [(4-Chlor-phenyl)-(4-{2-[3-(4-chlor-phenyl)-[1,2,4]oxadiazo1-5-yl]-vinyl}-cyclohexyl)-methyl]-dimethyl-amin
192. [(4-{2-[3-(3-Chlor-phenyl)-[1,2,4]oxadiazol-5-yl]-vinyl}-cyclohexyl)-thiophen-2-yl-methyl]-dimethyl-amin
193. [(4-{2-[3-(4-tert-Butyl-phenyl)-[1,2,4]oxadiazol-5-yl]-vinyl}-cyclohexyl)-thiophen-2-yl-methyl]-dimethyl-amin (Polareres Diastereomer)
194. [(4-{2-[3-(4-tert-Butyl-phenyl)-[1,2,4]oxadiazol-5-yl]-vinyl}-cyclohexyl)-thiophen-2-yl-methyl]-dimethyl-amin (Unpolareres Diastereomer)
195. Dimethyl-(thiophen-2-yl-{4-[2-(3-m-tolyl-[1,2,4]oxadiazol-5-yl)-vinyl]-cyclohexyl}-methyl)-amin
196. [(4-{2-[3-(4-Chlor-phenyl)-[1,2,4]oxadiazol-5-yl]-vinyl}-cyclohexyl)-thiophen-2-yl-methyl]-dimethyl-amin
197. [(4-{2-[3-(3-Chlor-phenyl)-[1,2,4]oxadiazol-5-yl]-vinyl}-cyclohexyl)-(3-fluor-phenyl)-methyl]-dimethyl-amin
198. [(4-{2-[3-(4-tert-Butyl-phenyl)-[1,2,4]oxadiazol-5-yl]-vinyl}-cyclohexyl)-(3-fluorphenyl)-methyl]-dimethyl-amin (Polareres Diastereomer)
199. [(4-{2-[3-(4-tert-Butyl-phenyl)-[1,2,4]oxadiazol-5-yl]-vinyl}-cyclohexyl)-(3-fluorphenyl)-methyl]-dimethyl-amin (Unpolareres Diastereomer)
201. ((3-Fluor-phenyl)-{4-[2-(3-m-tolyl-[1,2,4]oxadiazol-5-yl)-vinyl]-cyclohexyl}-methyl)-dimethyl-amin
202. [(4-{2-[3-(4-Chlor-phenyl)-[1,2,4]oxadiazol-5-yl]-vinyl}-cyclohexyl)-(3-fluor-phenyl)-methyl]-dimethyl-amin
203. [(4-{2-[3-(2,3-Dichlor-phenyl)-[1,2,4]oxadiazol-5-yl]-vinyl}-cyclohexyl)-(4-fluorphenyl)-methyl]-dimethyl-amin
204. [(4-Fluor-phenyl)-(4-{2-[3-(4-trifluormethoxy-phenyl)-[1,2,4]oxadiazol-5-yl]-vinyl}-cyclohexyl)-methyl]-dimethyl-amin
205. [(4-{2-[3-(3,4-Dimethoxy-benzyl)-[1,2,4]oxadiazol-5-yl]-vinyl}-cyclohexyl)-(4-fluorphenyl)-methyl]-dimethyl-amin
206. [(4-{2-[3-(1,5-Dimethyl-1H-pyrrol-2-yl)-[1,2,4]oxadiazol-5-yl]-vinyl}-cyclohexyl)-(4-fluor-phenyl)-methyl]-dimethyl-amin
207. [1-(4-{2-[3-(2,3-Dichlor-phenyl)-[1,2,4]oxadiazol-5-yl]-vinyl}-cyclohexyl)-3-phenylpropyl]-dimethyl-amin
208. Dimethyl-[3-phenyl-1-(4-{2-[3-(4-trifluormethoxy-phenyl)-[1,2,4]oxadiazol-5-yl]-vinyl}-cyclohexyl)-propyl]-amin (Polareres Diastereomer)
209. Dimethyl-[3-phenyl-1-(4-{2-[3-(4-trifluormethoxy-phenyl)-[1,2,4]oxadiazol-5-yl]-vinyl}-cyclohexyl)-propyl]-amin (Unpolareres Diastereomer)
210. [1-(4-{2-[3-(3,4-Dimethoxy-benzyl)-[1,2,4]oxadiazol-5-yl]-vinyl}-cyclohexyl)-3-phenylpropyl]-dimethyl-amin (Polareres Diastereomer)
211. [1-(4-{2-[3-(3,4-Dimethoxy-benzyl)-[1,2,4]oxadiazol-5-yl]-vinyl}-cyclohexyl)-3-phenylpropyl]-dimethyl-amin (Unpolareres Diastereomer)
212. [1-(4-{2-[3-(1,5-Dimethyl-1H-pyrrol-2-yl)-[1,2,4]oxadiazol-5-yl]-vinyl}-cyclohexyl)-3-phenyl-propyl]-dimethyl-amin
213. [(4-{2-[3-(2,3-Dichlor-phenyl)-[1,2,4]oxadiazol-5-yl]-vinyl}-cyclohexyl)-phenyl-methyl]-dimethyl-amin
214. [(4-{2-[3-(3,4-Dimethoxy-benzyl)-[1,2,4]oxadiazol-5-yl]-vinyl}-cyclohexyl)-phenyl-methyl]-dimethyl-amin (Polareres Diastereomer)
215. [(4-{2-[3-(3,4-Dimethoxy-benzyl)-[1,2,4]oxadiazol-5-yl]-vinyl}-cyclohexyl)-phenyl-methyl]-dimethyl-amin (Unpolareres Diastereomer)
217. [(4-{2-[3-(1,5-Dimethyl-1H-pyrrol-2-yl)-[1,2,4]oxadiazol-5-yl]-vinyl}-cyclohexyl)-phenyl-methyl]-dimethyl-amin
218. [(4-Chlor-phenyl)-(4-{2-[3-(2,3-dichlor-phenyl)-[1,2,4]oxadiazol-5-yl]-vinyl}-cyclohexyl)-methyl]-dimethyl-amin
219. [(4-Chlor-phenyl)-(4-{2-[3-(4-trifluormethoxy-phenyl)-[1,2,4]oxadiazol-5-yl]-vinyl}-cyclohexyl)-methyl]-dimethyl-amin
220. [(4-Chlor-phenyl)-(4-{2-[3-(3,4-dimethoxy-benzyl)-[1,2,4]oxadiazol-5-yl]-vinyl}-cyclohexyl)-methyl]-dimethyl-amin
221. [(4-Chlor-phenyl)-(4-{2-[3-(1,5-dimethyl-1H-pyrrol-2-yl)-[1,2,4]oxadiazol-5-yl]-vinyl}-cyclohexyl)-methyl]-dimethyl-amin
222. [(4-{2-[3-(2,3-Dichlor-phenyl)-[1,2,4]oxadiazol-5-yl]-vinyl}-cyclohexyl)-thiophen-2-yl-methyl]-dimethyl-amin
223. Dimethyl-[thiophen-2-yl-(4-{2-[3-(4-trifluormethoxy-phenyl)-[1,2,4]oxadiazol-5-yl]-vinyl}-cyclohexyl)-methyl]-amin
224. [(4-{2-[3-(1,5-Dimethyl-1H-pyrrol-2-yl)-[1,2,4]oxadiazol-5-yl]-vinyl}-cyclohexyl)-thiophen-2-yl-methyl]-dimethyl-amin
225. [(4-{2-[3-(2,3-Dichlor-phenyl)-[1,2,4]oxadiazol-5-yl]-vinyl}-cyclohexyl)-(3-fluorphenyl)-methyl]-dimethyl-amin
226. [(3-Fluor-phenyl)-(4-{2-[3-(4-trifluormethoxy-phenyl)-[1,2,4]oxadiazol-5-yl]-vinyl}-cyclohexyl)-methyl]-dimethyl-amin (Polareres Diastereomer)
227. [(3-Fluor-phenyl)-(4-{2-[3-(4-trifluormethoxy-phenyl)-[1,2,4]oxadiazol-5-yl]-vinyl}-cyclohexyl)-methyl]-dimethyl-amin (Unpolareres Diastereomer)
228. [(4-{2-[3-(3,4-Dimethoxy-benzyl)-[1,2,4]oxadiazol-5-yl]-vinyl}-cyclohexyl)-(3-fluorphenyl)-methyl]-dimethyl-amin (Polareres Diastereomer)
229. [(4-{2-[3-(3,4-Dimethoxy-benzyl)-[1,2,4]oxadiazol-5-yl]-vinyl}-cyclohexyl)-(3-fluorphenyl)-methyl]-dimethyl-amin (Unpolareres Diastereomer)
231. [(4-{2-[3-(1,5-Dimethyl-1H-pyrrol-2-yl)-[1,2,4]oxadiazol-5-yl]-vinyl}-cyclohexyl)-(3-fluor-phenyl)-methyl]-dimethyl-amin
232. {(3-Fluor-phenyl)-[4-(3-phenyl-[1,2,4]oxadiazol-5-ylmethyl)-cyclohexyl]-methyl}-dimethyl-amin
233. {(4-Fluor-phenyl)-[4-(3-phenyl-[1,2,4]oxadiazol-5-ylmethyl)-cyclohexyl]-methyl}-dimethyl-amin
234. Dimethyl-{phenyl-[4-(3-phenyl-[1,2,4]oxadiazol-5-ylmethyl)-cyclohexyl]-methyl}-amin 235. [{4-[3-(2,4-Difluor-phenyl)-[1,2,4]oxadiazol-5-ylmethyl]-cyclohexyl}-(3-fluor-phenyl)-methyl]-dimethyl-amin
236. [{4-[3-(2,4-Difluor-phenyl)-[1,2,4]oxadiazol-5-ylmethyl]-cyclohexyl}-(4-fluor-phenyl)-methyl]-dimethyl-amin
237. ((3-Fluor-phenyl)-{4-[3-(4-methoxy-benzyl)-[1,2,4]oxadiazol-5-ylmethyl]-cyclohexyl}-methyl)-dimethyl-amin
238. ((4-Fluor-phenyl)-{4-[3-(4-methoxy-benzyl)-[1,2,4]oxadiazol-5-ylmethyl]-cyclohexyl}-methyl)-dimethyl-amin
239. ({4-[3-(4-Methoxy-benzyl)-[1,2,4]oxadiazol-5-ylmethyl]-cyclohexyl}-phenyl-methyl)-dimethyl-amin
240. [{4-[3-(4-Chlor-benzyl)-[1,2,4]oxadiazol-5-ylmethyl]-cyclohexyl}-(3-fluor-phenyl)-methyl]-dimethyl-amin
241. [{4-[3-(4-Chlor-benzyl)-[1,2,4]oxadiazol-5-ylmethyl]-cyclohexyl}-(4-fluor-phenyl)-methyl]-dimethyl-amin
242. [{4-[3-(3-Chlor-phenyl)-[1,2,4]oxadiazol-5-ylmethyl]-cyclohexyl}-(4-fluor-phenyl)-methyl]-dimethyl-amin
243. ({4-[3-(3-Chlor-phenyl)-[1,2,4]oxadiazol-5-ylmethyl]-cyclohexyl}-phenyl-methyl)-dimethyl-amin
244. [{4-[3-(2,3-Dichlor-phenyl)-[1,2,4]oxadiazol-5-ylmethyl]-cyclohexyl}-(3-fluor-phenyl)-methyl]-dimethyl-amin
245. [{4-[3-(2,3-Dichlor-phenyl)-[1,2,4]oxadiazol-5-ylmethyl]-cyclohexyl}-(4-fluor-phenyl)-methyl]-dimethyl-amin
246. ({4-[3-(2,3-Dichlor-phenyl)-[1,2,4]oxadiazol-5-ylmethyl]-cyclohexyl}-phenyl-methyl)-dimethyl-amin
247. {(3-Fluor-phenyl)-[4-(3-p-tolyl-[1,2,4]oxadiazol-5-ylmethyl)-cyclohexyl]-methyl}-dimethyl-amin
248. {(4-Fluor-phenyl)-[4-(3-p-tolyl-[1,2,4]oxadiazol-5-ylmethyl)-cyclohexyl]-methyl}-dimethyl-amin
249. Dimethyl-{phenyl-[4-(3-p-tolyl-[1,2,4]oxadiazol-5-ylmethyl)-cyclohexyl]-methyl}-amin
250. [{4-[3-(3,4-Dimethoxy-phenyl)-[1,2,4]oxadiazol-5-ylmethyl]-cyclohexyl}-(3-fluorphenyl)-methyl]-dimethyl-amin
251. [{4-[3-(3,4-Dimethoxy-phenyl)-[1,2,4]oxadiazol-5-ylmethyl]-cyclohexyl}-(4-fluorphenyl)-methyl]-dimethyl-amin
252. ({4-[3-(3,4-Dimethoxy-phenyl)-[1,2,4]oxadiazol-5-ylmethyl]-cyclohexyl}-phenyl-methyl)-dimethyl-amin
253. [{4-[3-(4-tert-Butyl-phenyl)-[1,2,4]oxadiazol-5-ylmethyl]-cyclohexyl}-(3-fluor-phenyl)-methyl]-dimethyl-amin
254. [{4-[3-(4-tert-Butyl-phenyl)-[1,2,4]oxadiazol-5-ylmethyl]-cyclohexyl}-(4-fluor-phenyl)-methyl]-dimethyl-amin
255. ({4-[3-(4-tert-Butyl-phenyl)-[1,2,4]oxadiazol-5-ylmethyl]-cyclohexyl}-phenyl-methyl)-dimethyl-amin
256. {(3-Fluor-phenyl)-[4-(3-m-tolyl-[1,2,4]oxadiazol-5-ylmethyl)-cyclohexyl]-methyl}-dimethyl-amin
257. {(4-Fluor-phenyl)-[4-(3-m-tolyl-[1,2,4]oxadiazol-5-ylmethyl)-cyclohexyl]-methyl}-dimethyl-amin
258. Dimethyl-{phenyl-[4-(3-m-tolyl-[1,2,4]oxadiazol-5-ylmethyl)-cyclohexyl]-methyl}-amin
259. [{4-[3-(4-Chlor-phenyl)-[1,2,4]oxadiazol-5-ylmethyl]-cyclohexyl}-(3-fluor-phenyl)-methyl]-dimethyl-amin
260. ({4-[3-(4-Chlor-phenyl)-[1,2,4]oxadiazol-5-ylmethyl]-cyclohexyl}-phenyl-methyl)-dimethyl-amin
261. ((3-Fluor-phenyl)-{4-[3-(4-trifluormethoxy-phenyl)-[1,2,4]oxadiazol-5-ylmethyl]-cyclohexyl}-methyl)-dimethyl-amin
262. ((4-Fluor-phenyl)-{4-[3-(4-trifluormethoxy-phenyl)-[1,2,4]oxadiazol-5-ylmethyl]-cyclohexyl}-methyl)-dimethyl-amin
263. Dimethyl-(phenyl-{4-[3-(4-trifluormethoxy-phenyl)-[1,2,4]oxadiazol-5-ylmethyl]-cyclohexyl}-methyl)-amin
264. ((4-Fluor-phenyl)-{4-[2-(3-phenyl-[1,2,4]oxadiazol-5-yl)-ethyl]-cyclohexyl}-methyl)-dimethyl-amin
265. Dimethyl-(phenyl-{4-[2-(3-phenyl-[1,2,4]oxadiazol-5-yl)-ethyl]-cyclohexyl}-methyl)-amin
266. Dimethyl-(3-phenyl-1-{4-[2-(3-phenyl-[1,2,4]oxadiazol-5-yl)-ethyl]-cyclohexyl}-propyl)-amin
267. ((3-Fluor-phenyl)-{4-[2-(3-phenyl-[1,2,4]oxadiazol-5-yl)-ethyl]-cyclohexyl}-methyl)-dimethyl-amin
268. [(4-Fluor-phenyl)-(4-{2-[3-(4-methoxy-benzyl)-[1,2,4]oxadiazol-5-yl]-ethyl}-cyclohexyl)-methyl]-dimethyl-amin
269. [(4-{2-[3-(4-Methoxy-benzyl)-[1,2,4]oxadiazol-5-yl]-ethyl}-cyclohexyl)-phenyl-methyl]-dimethyl-amin
270. [1-(4-{2-[3-(4-Methoxy-benzyl)-[1,2,4]oxadiazol-5-yl]-ethyl}-cyclohexyl)-3-phenylpropyl]-dimethyl-amin
271. [(3-Fluor-phenyl)-(4-{2-[3-(4-methoxy-benzyl)-[1,2,4]oxadiazol-5-yl]-ethyl}-cyclohexyl)-methyl]-dimethyl-amin
272. [(4-{2-[3-(4-Chlor-benzyl)-[1,2,4]oxadiazol-5-yl]-ethyl}-cyclohexyl)-(4-fluor-phenyl)-methyl]-dimethyl-amin
273. [(4-{2-[3-(4-Chlor-benzyl)-[1,2,4]oxadiazol-5-yl]-ethyl}-cyclohexyl)-phenyl-methyl]-dimethyl-amin
274. [1-(4-{2-[3-(4-Chlor-benzyl)-[1,2,4]oxadiazol-5-yl]-ethyl}-cyclohexyl)-3-phenylpropyl]-dimethyl-amin
275. [(4-{2-[3-(4-Chlor-benzyl)-[1,2,4]oxadiazol-5-yl]-ethyl}-cyclohexyl)-(3-fluor-phenyl)-methyl]-dimethyl-amin
276. [(4-{2-[3-(3-Chlor-phenyl)-[1,2,4]oxadiazol-5-yl]-ethyl}-cyclohexyl)-(4-fluor-phenyl)-methyl]-dimethyl-amin
277. [(4-{2-[3-(3-Chlor-phenyl)-[1,2,4]oxadiazol-5-yl]-ethyl}-cyclohexyl)-phenyl-methyl]-dimethyl-amin
278. [1-(4-{2-[3-(3-Chlor-phenyl)-[1,2,4]oxadiazol-5-yl]-ethyl}-cyclohexyl)-3-phenylpropyl]-dimethyl-amin
279. [(4-{2-[3-(3-Chlor-phenyl)-[1,2,4]oxadiazol-5-yl]-ethyl}-cyclohexyl)-(3-fluor-phenyl)-methyl]-dimethyl-amin
280. [(4-{2-[3-(2,4-Difluor-phenyl)-[1,2,4]oxadiazol-5-yl]-ethyl}-cyclohexyl)-(4-fluorphenyl)-methyl]-dimethyl-amin
281. [(4-{2-[3-(2,4-Difluor-phenyl)-[1,2,4]oxadiazol-5-yl]-ethyl}-cyclohexyl)-phenyl-methyl]-dimethyl-amin
282. [(4-{2-[3-(2,4-Difluor-phenyl)-[1,2,4]oxadiazol-5-yl]-ethyl}-cyclohexyl)-(3-fluorphenyl)-methyl]-dimethyl-amin
283. [(4-{2-[3-(3,4-Dimethoxy-phenyl)-[1,2,4]oxadiazol-5-yl]-ethyl}-cyclohexyl)-(4-fluorphenyl)-methyl]-dimethyl-amin
284. [(4-{2-[3-(3,4-Dimethoxy-phenyl)-[1,2,4]oxadiazol-5-yl]-ethyl}-cyclohexyl)-phenyl-methyl]-dimethyl-amin
285. [1-(4-{2-[3-(3,4-Dimethoxy-phenyl)-[1,2,4]oxadiazol-5-yl]-ethyl}-cyclohexyl)-3-phenyl-propyl]-dimethyl-amin
286. [(4-{2-[3-(3,4-Dimethoxy-phenyl)-[1,2,4]oxadiazol-5-yl]-ethyl}-cyclohexyl)-(3-fluorphenyl)-methyl]-dimethyl-amin
287. ((4-Fluor-phenyl)-{4-[2-(3-p-tolyl-[1,2,4]oxadiazol-5-yl)-ethyl]-cyclohexyl}-methyl)-dimethyl-amin
288. Dimethyl-(phenyl-{4-[2-(3-p-tolyl-[1,2,4]oxadiazol-5-yl)-ethyl]-cyclohexyl}-methyl)-amin
289. Dimethyl-(3-phenyl-1-{4-[2-(3-p-tolyl-[1,2,4]oxadiazol-5-yl)-ethyl]-cyclohexyl}-propyl)-amin
290. ((3-Fluor-phenyl)-{4-[2-(3-p-tolyl-[1,2,4]oxadiazol-5-yl)-ethyl]-cyclohexyl}-methyl)-dimethyl-amin
291. [(4-{2-[3-(4-tert-Butyl-phenyl)-[1,2,4]oxadiazol-5-yl]-ethyl}-cyclohexyl)-(4-fluorphenyl)-methyl]-dimethyl-amin
292. [(4-{2-[3-(4-tert-Butyl-phenyl)-[1,2,4]oxadiazol-5-yl]-ethyl}-cyclohexyl)-phenyl-methyl]-dimethyl-amin
293. [1-(4-{2-[3-(4-tert-Butyl-phenyl)-[1,2,4]oxadiazol-5-yl]-ethyl}-cyclohexyl)-3-phenylpropyl]-dimethyl-amin
294. [(4-{2-[3-(4-tert-Butyl-phenyl)-[1,2,4]oxadiazol-5-yl]-ethyl}-cyclohexyl)-(3-fluorphenyl)-methyl]-dimethyl-amin
295. ((4-Fluor-phenyl)-{4-[2-(3-m-tolyl-[1,2,4]oxadiazol-5-yl)-ethyl]-cyclohexyl}-methyl)-dimethyl-amin
296. Dimethyl-(phenyl-{4-[2-(3-m-tolyl-[1,2,4]oxadiazol-5-yl)-ethyl]-cyclohexyl}-methyl)-amin
297. Dimethyl-(3-phenyl-1-{4-[2-(3-m-tolyl-[1,2,4]oxadiazol-5-yl)-ethyl]-cyclohexyl}-propyl)-amin
298. ((3-Fluor-phenyl)-{4-[2-(3-m-tolyl-[1,2,4]oxadiazol-5-yl)-ethyl]-cyclohexyl}-methyl)-dimethyl-amin
299. [(4-{2-[3-(4-Chlor-phenyl)-[1,2,4]oxadiazol-5-yl]-ethyl}-cyclohexyl)-(4-fluor-phenyl)-methyl]-dimethyl-amin
300. [(4-{2-[3-(4-Chlor-phenyl)-[1,2,4]oxadiazol-5-yl]-ethyl}-cyclohexyl)-phenyl-methyl]-dimethyl-amin
301. [1-(4-{2-[3-(4-Chlor-phenyl)-[1,2,4]oxadiazol-5-yl]-ethyl}-cyclohexyl)-3-phenylpropyl]-dimethyl-amin
302. [(4-{2-[3-(4-Chlor-phenyl)-[1,2,4]oxadiazol-5-yl]-ethyl}-cyclohexyl)-(3-fluor-phenyl)-methyl]-dimethyl-amin
303. [(4-{2-[3-(2,3-Dichlor-phenyl)-[1,2,4]oxadiazol-5-yl]-ethyl}-cyclohexyl)-(4-fluorphenyl)-methyl]-dimethyl-amin
304. [(4-{2-[3-(2,3-Dichlor-phenyl)-[1,2,4]oxadiazol-5-yl]-ethyl}-cyclohexyl)-phenylmethyl]-dimethyl-amin
305. [1-(4-{2-[3-(2,3-Dichlor-phenyl)-[1,2,4]oxadiazol-5-yl]-ethyl}-cyclohexyl)-3-phenylpropyl]-dimethyl-amin
306. [(4-{2-[3-(2,3-Dichlor-phenyl)-[1,2,4]oxadiazol-5-yl]-ethyl}-cyclohexyl)-(3-fluorphenyl)-methyl]-dimethyl-amin
307. [(4-Fluor-phenyl)-(4-{2-[3-(4-trifluormethoxy-phenyl)-[1,2,4]oxadiazol-5-yl]-ethyl}-cyclohexyl)-methyl]-dimethyl-amin
308. Dimethyl-[phenyl-(4-{2-[3-(4-trifluormethoxy-phenyl)-[1,2,4]oxadiazol-5-yl]-ethyl}-cyclohexyl)-methyl]-amin
309. Dimethyl-[3-phenyl-1-(4-{2-[3-(4-trifluormethoxy-phenyl)-[1,2,4]oxadiazol-5-yl]-ethyl}-cyclohexyl)-propyl]-amin
310. [(3-Fluor-phenyl)-(4-{2-[3-(4-trifluormethoxy-phenyl)-[1,2,4]oxadiazol-5-yl]-ethyl}-cyclohexyl)-methyl]-dimethyl-amin
311 (4-((3-(4-Fluorobenzyl)-1,2,4-oxadiazol-5-yl)methyl)cyclohexyl)(4-fluorophenyl)-N,N-dimethylmethanamin
312 (4-((3-(4-Fluorobenzyl)-1,2,4-oxadiazol-5-yl)methyl)cyclohexyl)-N,N-dimethyl(phenyl)methanamin
313 (4-((3-(3-Chlorobenzyl)-1,2,4-oxadiazol-5-yl)methyl)cyclohexyl)(3-fluorophenyl)-N,N-dimethylmethanamin
314 (4-((3-(3-Chlorobenzyl)-1,2,4-oxadiazol-5-yl)methyl)cyclohexyl)(4-fluorophenyl)-N,N-dimethylmethanamin
315 (4-((3-(3-Chlorobenzyl)-1,2,4-oxadiazol-5-yl)methyl)cyclohexyl)-N,N-dimethyl(phenyl)methanamin
316 (4-((3-(3-Bromophenyl)-1,2,4-oxadiazol-5-yl)methyl)cyclohexyl)(3-fluorophenyl)-N,N-dimethylmethanamin
317 (4-((3-(3-Bromophenyl)-1,2,4-oxadiazol-5-yl)methyl)cyclohexyl)(4-fluorophenyl)-N, N-dimethylmethanamin
318 (4-((3-(3-Bromophenyl)-1,2,4-oxadiazol-5-yl)methyl)cyclohexyl)-N,N-dimethyl(phenyl)methanamin
319 (4-((3-(4-Bromobenzyl)-1,2,4-oxadiazol-5-yl)methyl)cyclohexyl)(3-fluorophenyl)-N,N-dimethylmethanamin
320 (4-((3-(4-Bromobenzyl)-1,2,4-oxadiazol-5-yl)methyl)cyclohexyl)(4-fluorophenyl)-N,N-dimethylmethanamin
321 (4-((3-(4-Bromobenzyl)-1,2,4-oxadiazol-5-yl)methyl)cyclohexyl)-N,N-dimethyl(phenyl)methanamin
322 (3-Fluorophenyl)-N,N-dimethyl(4-((3-(thiophen-2-ylmethyl)-1,2,4-oxadiazol-5-yl)methyl)cyclohexyl)methanamin
323 (4-Fluorophenyl)-N,N-dimethyl(4-((3-(thiophen-2-ylmethyl)-1,2,4-oxadiazol-5-yl)methyl)cyclohexyl)methanamin
324 N,N-Dimethyl(phenyl)(4-((3-(thiophen-2-ylmethyl)-1,2,4-oxadiazol-5-yl)methyl)cyclohexyl)methanamin
325 (4-((3-Benzyl-1,2,4-oxadiazol-5-yl)methyl)cyclohexyl)(3-fluorophenyl)-N,N-dimethylmethanamin
326 (4-((3-Benzyl-1,2,4-oxadiazol-5-yl)methyl)cyclohexyl)(4-fluorophenyl)-N,N-dimethylmethanamin
327 (4-((3-Benzyl-1,2,4-oxadiazol-5-yl)methyl)cyclohexyl)-N,N-dimethyl(phenyl)methanamin
328 (3-Fluorophenyl)-N,N-dimethyl(4-((3-phenethyl-1,2,4-oxadiazol-5-yl)methyl)cyclohexyl)methanamin
329 (4-Fluorophenyl)-N,N-dimethyl(4-((3-phenethyl-1,2,4-oxadiazol-5-yl)methyl)cyclohexyl)methanamin
330 N,N-Dimethyl(4-((3-phenethyl-1,2,4-oxadiazol-5-yl)methyl)cyclohexyl)(phenyl)methanamin
331 (4-((3-((1H-Indol-3-yl)methyl)-1,2,4-oxadiazol-5-yl)methyl)cyclohexyl)(3-fluorophenyl)-N,N-dimethylmethanamin

Ein.weiterer Gegenstand der Erfindung ist ein Verfahren zur Herstellung eines erfindungsgemäßen Oxadiazol-Derivates. Die erfindungsgemäßen Substanzen können hergestellt werden, indem Amidoxime der allgemeinen Formel **D** in einem Reaktionsmedium unter Zugabe einer Base, beispielsweise NaH, mit sowohl gesättigten als auch ungesättigten Estern der allgemeine Formel **A** zu den erfindungsgemäßen Verbindungen umgesetzt werden. 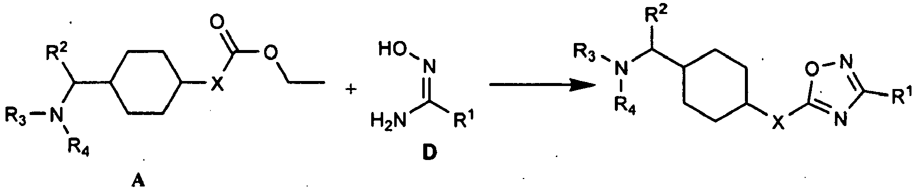 X CH, CH₂, CH=CH, CH₂CH₂, CH₂CH=CH oder CH₂CH₂CH₂
n=0,1,2

Für die Herstellung der Ester der allgemeinen Formel **A** schützt man die Ketofunktion von 4-Oxo-cyclohexancarbonsäureester, 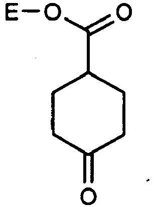 wobei E für einen C₁₋₆-Alkylrest, vorzugsweise Ethyl, steht, nach dem Fachmann bekannten Methoden, 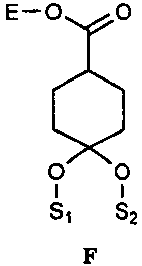 wobei S¹ und S² jeweils für eine Schutzgruppe stehen, vorzugsweise einen Ring bilden und zusammen für -CH₂-CH₂- stehen. Der Ester **F** wird mit einem Reduktionsmittel, beispielsweise Diisobutylaluminiumhydrid zum Aldehyd **G** 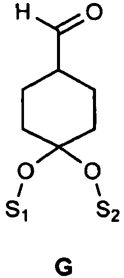 reduziert. Durch Zugabe eines Amins der allgemeinen Formel R³R⁴NH und eines Cyanids, beispielsweise KCN oder NaCN, wird der Aldehyd **G** unter Zugabe einer Säure, beispielsweise Salzsäure, in einem organischen Lösungsmittel, beispielsweise Methanol oder Ethanol, zum Nitril **H** umgesetzt.

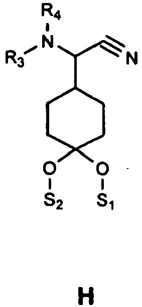

Das Nitril **H** wird mit einem Grignard-Reagenz der allgemeinen Formel R²MgHal, wobei Hal für Br, Cl oder I steht, oder einer metallorganischen Verbindung der allgemeinen Formel R²Li in einem organischen Lösungsmittel, beispielsweise Diethylether, Dioxan oder Tetrahydrofuran, zu einer Verbindung der allgemeinen Formel **J** umgesetzt.

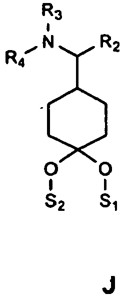

Die Schutzgruppen werden nach den üblichen Methoden abgespalten, wobei man das Keton **K** erhält.

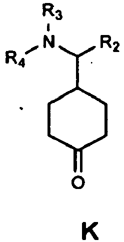

Der Aldehyd **L** 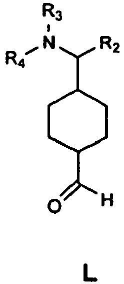 wird durch Umsetzung des Ketons **K** mit
(Methoxymethyl)triphenylphosphoniumchlorid und einer starken Base, beispielsweise Kalium-tert-butylat, bei einer Temperatur von -20°C und +30°C, erhalten. Durch Umsetzung von Aldehyd **L** mit (Methoxymethyl)triphenylphosphoniumchlorid und einer starken Base, beispielsweise Kalium-*tert*-butylat, bei einer Temperatur von - 20°C und +30°C, wird ein Aldehyd der allgemeinen Formel **M** erhalten. Über eine Wiederholung des letzten Reaktionsschrittes können Aldehyde der allgemeinen Formel **N** erhalten werden, bei denen n für 2 steht.

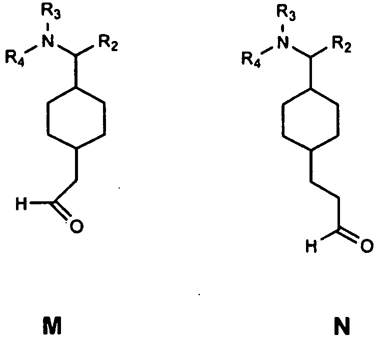

Ein Phosphonoessigsäureester, vorzugsweise Phosphonoessigsäure-trimethylester oder Phosphonoessigsäure-triethylester, wird zunächst mit einer starken Base, vorzugsweise Kalium-*tert*.butylat, Natriumhydrid oder Butyllithium, dann mit einem Keton der allgemeinen Formel **K** oder einem Aldehyd **L**, **M** oder **N** umgesetzt. Dabei entstehen die α,β-ungesättigten Ester der allgemeinen Formel **A**.

Die Doppelbindung kann gegebenenfalls auch zu den gesättigten Esten der allgemeinen Formel **A** reduziert werden. Hierbei wird die Doppelbindung nach literaturbekannten Methoden reduziert, bevorzugt durch heterogene, katalytische Hydrierung an Palladium- oder Platin-Katalysatoren oder durch homogen katalysierte Hydrierung mit Rhodium-Katalysatoren, jeweils bei Temperaturen zwischen RT und 60°C und unter Wasserstoff-Drücken zwischen 1 bar und 6 bar, besonders bevorzugt bei RT unter einem Wasserstoffdruck zwischen 2 und 3 bar an Palladium auf Kohle. Die gegebenenfalls bei den Synthesen anfallenden Diastereomeren können nach dem Fachmann bekannten Methoden zur Trennung von Diastereomeren getrennt werden, z. B. durch Chromatographie, insbesondere an Kieselgel, Normalphase oder Umkehrphase. Besonders geeignet zur Trennung der Diastereomeren ist RP-HPLC (mobile Phase Acetonitril/Wasser oder Methanol/Wasser).

Es hat sich gezeigt, dass die erfindungsgemäßen Substanzen nicht nur an den µ-Opioid-Rezeptor binden, sondern auch die Serotonin- und Noradrenalin-Wiederaufnahme hemmen. Noradrenalin- und Serotonin-Wiederaufnahmehemmer haben eine antidepressive und anxiolytische Wirkung, sind jedoch auch geeignet zur Behandlung von Schmerz (Analgesics - from Chemistry and Pharmacology to Clinical Application, Wiley 2002, S. 265-284)

Die erfindungsgemäßen Substanzen eignen sich als pharmazeutische Wirkstoffe in Arzneimitteln. Ein weiterer Gegenstand der Erfindung sind daher Arzneimittel enthaltend wenigstens ein erfindungsgemäßes substituiertes Oxadiazol-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Oxadiazol-Derivat gegebenenfalls geeignete Zusatz- und/oder Hilfsstoffe, so auch auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Oxadiazol-Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Oxadiazol-Derivate verzögert freisetzen. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,005 bis 20 mg/kg, bevorzugt 0,05 bis 5 mg/kg wenigstens eines erfindungsgemäßen Oxadiazol-Derivats appliziert.

Das Arzneimittel kann ein erfindungsgemäßes Oxadiazol-Derivat als reines Diastereomer und/oder Enantiomer, als Razemat oder als nicht-äquimolare oder äquimolare Mischung der Diastereomere und/oder Enantiomere enthalten.

Ein weiterer Gegenstand der Erfindung ist die Verwendung eines erfindungsgemäßen Oxadiazol-Derivats zur Herstellung eines Arzneimittels zur Behandlung von Schmerz, insbesondere von akutem, neuropathischem oder chronischem Schmerz.

Ein weiterer Gegenstand der Erfindung ist die Verwendung eines erfindungsgemäßen Oxadiazol-Derivats zur Herstellung eines Arzneimittels zur Behandlung von Depressionen und/oder zur Anxiolyse.

Die substituierten Oxadiazol-Derivate der allgemeinen Formel I eignen sich auch zur Behandlung von Harninkontinenz, Diarrhöe, Pruritus, Alkohol- und Drogenmißbrauch, Medikamentenabhängigkeit und Antriebslosigkeit.

Gegenstand der Erfindung ist daher auch die Verwendung eines substituierten Oxadiazol-Derivates der allgemeinen Formel I zur Herstellung eines Arzneimittels zur Behandlung von von Harninkontinenz, Diarrhöe, Pruritus, Alkohol- und Drogenmißbrauch, Medikamentenabhängigkeit und Antriebslosigkeit.

### Beispiele

### Synthese der Amidoxime

Die Amidoxime sind entweder kommerziell erhältlich oder können wie bei L. J. Street et al. J. Med. Chem. 1993, 36, 1529 - 1538 beschrieben, aus den entsprechenden Nitrilen hergestellt werden.

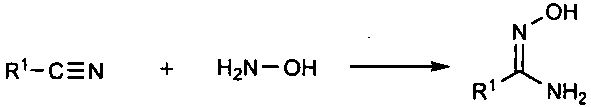

Es wurden die folgenden Amidoxime synthetisiert:
2,4-Difluor-N'-hydroxybenzimidamid **1** (R¹ = 2,4-Difluorphenyl) N'-hydroxybenzimidamid **2** (R¹ = Phenyl)
3,4-Dimethoxy-N'-hydroxybenzimidamid **3** (R¹ = 3,4-Dimethoxyphenyl) 4-Methyl-N'-hydroxybenzimidamid **4** (R' = 4-Methylphenyl)
3-Chlor-N'-hydroxybenzimidamid **5** (R' = 3-Chlorphenyl)
N'-Hydroxy-4-(trifluormethyl)benzimidamid **6** (R' = 4-Trifluormethylphenyl)
2-(4-Chlorphenyl)-N'-hydroxyacetimidamid **7** (R¹ = CH₂-4-Chlorphenyl)
N'-Hydroxy-2-(4-methoxyphenyl)acetimidamid **8** (R' = CH₂-4-Methoxyphenyl) N'-Hydroxyisonicotinimidamid **9** (R¹ = 4-Pyridin)
N'-Hydroxynicotinimidamid **10** (R' = 3-Pyridin)

Synthese der Ketone und Aldehyde der allgemeinen Formeln **K**, **L** und **M**, wobei R³ und R⁴ Methyl bedeuten (allgemeine Formeln **Kₐ**, **Lₐ** und **Mₐ**)

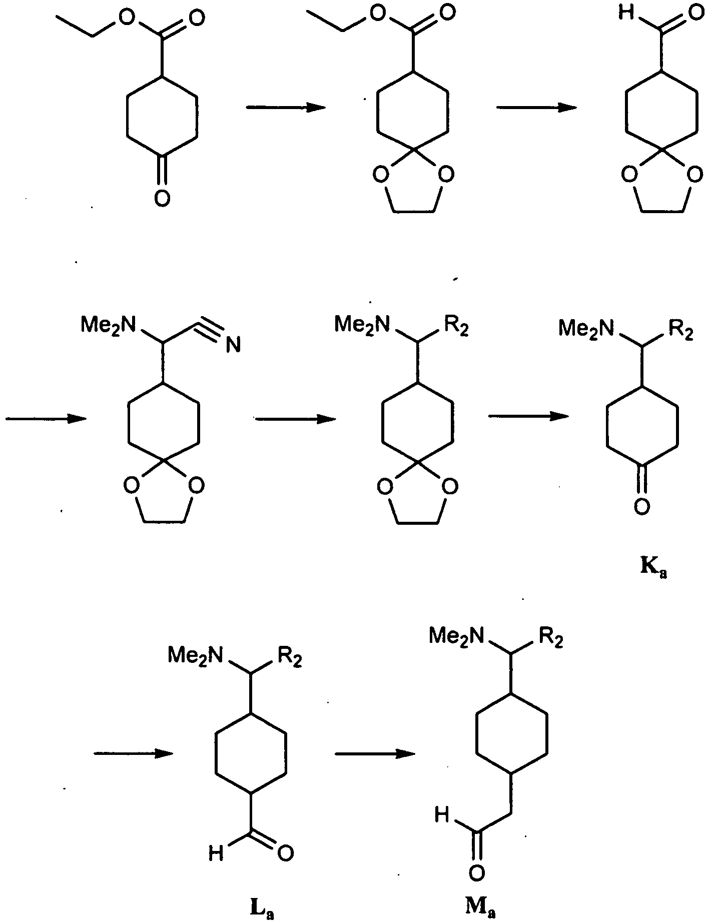

### 1. Stufe

Synthese des 1,4-Dioxa-spiro[4.5]decan-8-carbonsäureethylesters 4-Oxo-cyclohexancarbonsäure-ethylester (52,8 g, 0,31 mol, Merck, Bestell-Nr. 814249), Ethylenglykol (67,4 g, 1,08 mol) und p-Toluolsulfonsäure (0,7 g) in Toluol (160 ml) wurden 20 h bei RT gerührt, die Reaktionslösung in Diethylether (300 ml) gegossen und mit Wasser, Natriumhydrogencarbonat-Lösung und Natriumchlorid-Lösung gewaschen. Die Lösung wurde getrocknet (Na₂SO₄), i. Vak. eingeengt und die verbliebene farblose Flüssigkeit ohne Reinigung weiter verarbeitet.

### 2. Stufe

### Synthese des 1,4-Dioxa-spiro[4.5]decane-8-carbaldehyds

Eine Lösung aus 1,4-Dioxa-spiro[4.5]decan-8-carbonsäureethylester (32,13 g, 150 mmol) in absol.Toluol (160 ml) wurde bei -70 bis -65 °C unter Argon tropfenweise mit Diisobutylaluminiumhydrid (1,5 M Lösung in Toluol, 102 ml, 153 mmol) versetzt und 30 min gerührt. Anschließend wurde der Ansatz bei -70 bis -60 °C durch Zugabe von Methanol (80 ml) gequencht. Die Reaktionslösung wurde auf RT erwärmt, mit gesättigter Natriumchlorid-Lösung (100 ml) versetzt und die Rektionslösung über Kieselgur abgesaugt. Das Kiesegur wurde zweimal mit Essigester gewaschen, die wäßrige Lösung abgetrennt und zweimal mit Essigester extrahiert. Die vereinigten organischen Extrakte wurden mit gesättigter Natriumchlorid-Lösung gewaschen, über Natriumsulfat getrocknet und i. Vak. eingeengt.

### 3. Stufe

### Synthese des Dimethylamino-(1,4-dioxa-spiro[4.5]dec-8-yl)-acetonitrils

Zu einem Gemisch aus 4N Salzsäure (37 ml) und Methanol (22 ml) wurde unter Eiskühlung 40-proz. wässrige Dimethylaminlösung (85 ml, 0,67 mol), 1,4-Dioxa-spiro-[4.5]decan-8-carbaldehyd (240 g, 0,141 mol) und Kaliumcyanid (22,05 g, 0,338 mol) addiert. Die Mischung wurde 4 d bei Raumtemperatur gerührt und anschließend nach Zugabe von Wasser (80 ml) mit Diethylether (4 x 100 ml) extrahiert. Die organische Phase wurde über Natriumsulfat getrocknet, i. Vak. eingeengt und das Produkt als weißer Feststoff erhalten.

### 4. Stufe

### Synthese der [(1,4-Dioxa-spiro[4.5]dec-8-yl)-methyl]-dimethylamine

Eine 1M Lösung des entsprechenden Grignard-Reagenzes in THF oder Diethylether (220 ml, 220 mmol) wurde unter Argon und Eiskühlung tropfenweise mit einer Lösung des Aminonitrils (88 mmol) in absol. THF (160 ml) bzw. absol. Diethylether (160 ml), je nach Lösungsmittel des Grignard-Reagenzes, versetzt und 20 h bei RT gerührt. Zur Aufarbeitung des Reaktionsgemisches wurde unter Eiskühlung gesättigte Ammoniumchlorid-Lösung (100 ml) und Wasser (100 ml) gegeben und mit Diethylether (3 x 100 ml) extrahiert. Die organische Phase wurde mit Wasser und gesättigter Natriumchlorid-Lösung gewaschen, getrocknet (Na₂SO₄) und eingeengt. Das erhaltene Produkt wurde ohne weitere Aufreinigung in die nächste Stufe eingesetzt.

### 5. Stufe

### Synthese der 4-[Dimethylamino-methyl]-cyclohexanone Kₐ

Das Rohprodukt des entsprechenden [(1,4-Dioxa-spiro[4.5]dec-8-yl)-methyl]-dimethylamins (88 mmol) wurde in Wasser (40 ml) gelöst, mit konz. Salzsäure (59 ml) versetzt und 20 h bei RT gerührt. Das Reaktionsgemisch wurde mit Diethylether (2 x 100 ml) extrahiert, die wässrige Phase unter Eiskühlung mit 5N NaOH alkalisch gestellt, mit Dichlormethan (3 x 100 ml) extrahiert, getrocknet und eingeengt. Die Produkte wurden als weiße Feststoffe oder Öle erhalten.

### 6. Stufe

### Synthese der 4-[Dimethylamino-methyl]-cyclohexan-carbaldehyde Lₐ

(Methoxymethyl)triphenylphosphoniumchlorid (25,7 g, 75 mmol) wurde in absol. THF (100 ml) unter Argon suspendiert, bei 0 °C tropfenweise mit Kalium-*tert*-butylat (8,42 g, 75 mmol), gelöst in absol. THF (70 ml) versetzt und anschließend 15 min bei 0 °C nachgerührt.

Bei RT wurde dann das entsprechende 4-[Dimethylamino-methyl]-cyclohexanon **Kₐ** 50 mmol), gelöst in absol. THF (75 ml), zur obigen Lösung zugetropft und über Nacht bei RT gerührt. Unter Eiswasserkühlung wurde tropfenweise mit Wasser (38 ml) und 6N HCl (112 ml) hydrolysiert. Nach 1 h Rühren bei RT wurde mit Ether (10 x 50 ml) extrahiert, die wässrige Phase mit 5N NaOH auf pH 11 gebracht, mit Essigester (3 x 50 ml) ausgeschüttelt, über Na₂SO₄ getrocknet und i. Vak. eingeengt. Das Rohprodukt wurde durch Flashchromatographie mit Essigester/Cyclohexan (1:1) gereinigt. Man erhielt in der Regel zwei Diastereomere in unterschiedlichen Verhältnissen.

### 7. Stufe

### Synthese der {4-[Dimethylamino-methyl]-cyclohexyl}-acetaldehyde Mₐ

(Methoxymethyl)triphenylphosphoniumchlorid (43,53 g, 127 mmol) wurde in absol. THF (200 ml) unter Argon suspendiert, bei 0 °C tropfenweise mit Kalium-*tert*-butylat (14,25 g, 127 mmol), gelöst in absol. THF (130 ml), versetzt und anschließend 15 min bei 0 °C nachgerührt.

Bei RT wurde dann der entsprechende 4-[Dimethylamino-methyl]-cyclohexan-carbaldehyd Lₐ (85 mmol), gelöst in absol. THF (130 ml), zugetropft und über Nacht bei RT gerührt. Unter Eiswasserkühlung wurde tropfenweise mit Wasser (80 ml) und 6N HCl (200 ml) hydrolysiert. Nach 1 h Rühren bei RT wurde zehnmal mit Ether (je 100 ml) extrahiert. Die wässrige Phase wurde mit 5N NaOH auf pH 11 gebracht, dreimal mit Essigester (je 100 ml) ausgeschüttelt, über Na₂S0₄ getrocknet und i. Vak. eingeengt. Das Rohprodukt wurde durch Flashchromatographie mit Essigester/Cyclohexan (1:1 oder 1:2) gereinigt. Man erhielt in der Regel zwei Diastereomere in unterschiedlichen Verhältnissen.

### Synthese der Cyclohexylidenessigester

Die Cyclohexylidenessigester wurden aus den entsprechenden Ketonen mit Phosphono-essigsäure-triethylester nach Horner dargestellt.

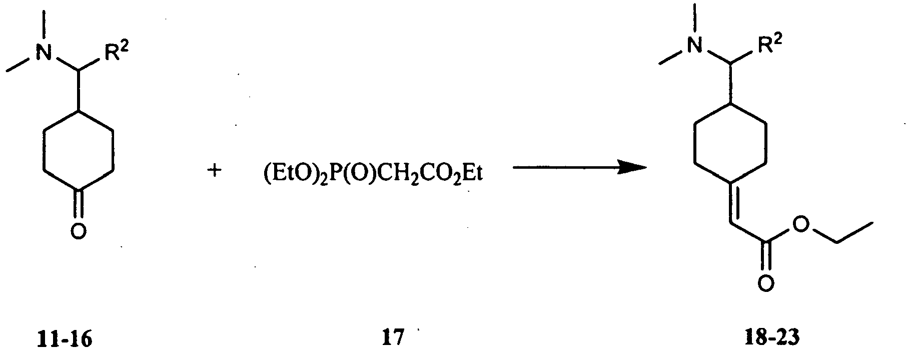

Es wurden die folgenden Ketone verwendet:
4-(Dimethylamino-phenyl-methyl)-cyclohexanon **11** (R² = Phenyl)
4-[Dimethylamino-(3-fluorphenyl)-methyl]-cyclohexanon **12** (R² = 3-Fluorphenyl)
4-[Dimethylamino-(4-fluorphenyl)-methyl]-cyclohexanon **13** (R² = 4-Fluorphenyl)
4-[(4-Chlor-phenyl)-dimethylamino-methyl]-cyclohexanon **14** (R² = 4-Chlorphenyl)
4-(1-Dimethylamino-3-phenyl-propyl)-cyclohexanon **15** (R² = Phenethyl)
4-(Dirnethylamino-thiophen-2-yl-methyl)-cyclohexanon **16** (R² = 2-Thiophen)
[4-(Dimethylamino-phenyl-methyl)-cyclohexyliden]-essigsäure-ethylester **18** (R² = Phenyl)

Zu einer Lösung von Phosphonoessigsäure-triethylester **17** (Acros, Bestell-Nr. 139705000, 30,26 g, 0,135 mol) in absol. DMF (200 ml) wurde unter Argon Kalium-*tert*-butylat (15,15 g, 0,135 mol) gegeben und 10 min gerührt. Anschließend wurde das Keton **11** (20,82 g, 0,09 mol)), gelöst in DMF (200 ml), zugetropft. Nach ca. 20 min fällt ein Feststoff aus. Zur besseren Durchmischung wurde der Ansatz durch Zugabe von DMF (200 ml) verdünnt, 3 h bei RT gerührt und danach auf Eis gegossen. Die Reaktionsmischung wurde mit Diethylether (3 x 100 ml) extrahiert, die organische Phase mit Wasser und gesättigter Natriumchlorid-Lösung gewaschen, getrocknet und im Vakuum eingeengt. Das Rohprodukt wurde durch Flashchromatographie mit Essigester/Cyclohexan (1:2) gereinigt. Man erhält ein Gemisch der E/Z-Isomeren im Verhältnis ca. 1 : 1.
Ausbeute: 21,83 g (80 %), Öl
¹³C-NMR (CDCl₃): 25,93; 26,58; 27,09; 29,21; 29,90; 30,32; 30,73; 30,77; 35,38; 35,66; 38,73; (C₄); 40,06; 40,90; 41,19 (N(CH₃)₂); 48,78; 65,15; 68,22 (CH); 125,36; 127,99; 128,05; 142,69.

### {4-[Dimethylamino-(3-fluorphenyl)-methyl]-cyclohexyliden}-essigsäure-ethylester 19 (R² = 3-Fluorphenyl)

Zu einer Lösung von Phosphonoessigsäure-triethylester **17** (30,26 g, 0,135 mol) in absol. DMF (200 ml) wurde unter Argon Kalium-*tert*-butylat (15,15 g, 0,135 mol) gegeben und 10 min gerührt. Anschließend wurde das Keton **12** (22,43 g, 0,09 mol), gelöst in DMF (200 ml), zugetropft. Nach ca. 20 min fiel ein Feststoff aus. Zur besseren Durchmischung wurde der Ansatz durch Zugabe von DMF (200 ml) verdünnt, 3 h bei RT gerührt und danach auf Eis gegossen. Die Reaktionsmischung wurde mit Diethylether (3 x 100 ml) extrahiert, die organische Phase mit Wasser und gesättigter NaCl-Lösung gewaschen, getrocknet und i. Vak. eingeengt. Das Rohprodukt wurde durch Flash-chromatographie mit Essigester/Cyclohexan (1:2) gereinigt. Man erhält ein Gemisch der E/Z-Isomeren im Verhältnis ca. 1 : 1. Ausbeute: 24,78 g (86 %), Öl
¹³C-NMR (CDCl₃): 14,36; 28,72; 28,92; 29,90; 30,39; 31,76; 30,06; 32,31; 36,96; 37,13; 38,12; 38,17; 41,91; 42,04 (N(CH₃)₂); 59,40; 74,21; 74,25; 113,15; 113,17; 113, 53; 113,56; 113, 74; 113, 77; 115,47; 115, 68; 124, 78; 128, 79; 128, 86; 139,59; 139,66; 139,78; 139,83; 161,09; 162,18; 162,22; 163,52; 166,34; 171, 55.

### {4-[Dimethylamino-(4-fluorphenyl)-methyl]-cyclohexyliden}-essigsäure-ethylester 20 (R² = 4-Fluorphenyl)

Zu einer Lösung von Phosphonoessigsäure-triethylester **17** (26,9 g, 0,12 mol) in absol. DMF (250 ml) wurde unter Argon Kalium-*tert*-butylat (13,46 g, 0,12 mol) gegeben und 10 min gerührt. Anschließend wurde das Keton **13** (19,95 g, 0,08 mol), gelöst in DMF (200 ml), zugetropft. Nach ca. 20 min fiel ein Feststoff aus. Zur besseren Durchmischung wurde der Ansatz durch Zugabe von DMF (200 ml) verdünnt, 3 h bei RT gerührt und danach auf Eis gegossen. Die Reaktionsmischung wurde mit Diethylether (3 x 100 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt. Man erhält ein Gemisch der E/Z-Isomeren im Verhältnis ca. 1 : 1. Ausbeute: 19,7 g (77 %), Öl
¹³C-NMR (CDCl₃): 14,18; 28,56; 28,76; 29,69; 30,17; 31,51; 32,24; 37,03; 38,07; 38,11; 41,80; 41,93; 59,34; 73,80; 73,84; 113,12; 114,24; 114,53; 130,35; 130,45; 132,48; 132,65; 160,11; 162,53; 162,59; 163,35; 166,54.

### {4-[(4-Chlorphenyl)-dimethylamino-methyl]-cyclohexyliden}-essigsäure-ethylester 21 (R² = 4-Chlorphenyl)

Zu einer Lösung von Phosphonoessigsäure-triethylester **17** (23,53 g, 0,105 mol) in absol. DMF (200 ml) wurde unter Argon Kalium-*tert*-butylat (11,78 g, 0,105 mol) gegeben und 10 min gerührt. Anschließend wurde das Keton **14** (18,6 g, 0,07 mol), gelöst in DMF (200 ml) und zugetropft. Nach ca. 20 min fiel ein Feststoff aus. Zur besseren Durchmischung wurde der Ansatz durch Zugabe von DMF (200 ml) verdünnt, 3 h bei RT gerührt und danach auf Eis gegossen. Die Reaktionsmischung wurde mit Diethylether (3 x 100 ml) extrahiert, die organische Phase mit Wasser.und gesättigter NaCl-Lösung gewaschen, getrocknet und i. Vak. eingeengt. Das Rohprodukt wurde durch Flash-chromatographie mit Essigester/Cyclohexan (1:2) gereinigt. Man erhält ein Gemisch der E/Z-Isomeren im Verhältnis ca. 1 : 1. Ausbeute: 18,85 g (80 %), Öl
¹³C-NMR (CDCl₃): 14,36; 25,58; 26,60; 28,37; 28,91; 29,74; 30,06; 30,25; 31,59; 32,32; 33,90; 34,19; 36,94; 37,12; 38,12 (C₄); 41,62; 41,96; 42,09 (N(CH₃)₂); 43,16; 59,40; 60,40; 73,29; 73,53; 73,98; 74,02; 113,15; 124,44; 124,92; 127,56; 127,67; 130,23; 130,42; 130,78; 131,02; 132,41; 134,88; 135,20; 135,31; 135,49; 162,13; 162,18; 166,32; 171,52.

### [4-(1-Dimethylamino-3-phenyl-propyl)-cyclohexyliden)-essigsäure-ethylester 22 (R² = Phenethyl)

Zu einer Lösung von Phosphonoessigsäure-triethylester **17** (29,6 g, 0,132 mol) in absol. DMF (150 ml) wurde unter Argon Kalium-*tert*-butylat (14,8 g, 0,132 mol) gegeben und 10 min gerührt. Anschließend wurde das Keton **15** (22,8 g, 0,088 mol), gelöst in DMF (225 ml), zugetropft. Nach 3 h Nachrühren bei RT wurde die Lösung auf Eis (ca. 1 L) gegossen. Die Reaktionsmischung wurde mit Diethylether (3 x 150 ml) extrahiert, die organische Phase mit Wasser und gesättigter NaCl-Lösung gewaschen, getrocknet (Na₂SO₄) und i. Vak. eingeengt. Das Rohprodukt wurde durch Flashchromatographie mit Essigester/Cyclohexan (1:2) gereinigt. Ausbeute: 18,85 g (80 %), Öl
¹³C-NMR (CDCl₃): 14,28; 29,01; 29,13; 29,18; 31,05; 31,63; 32,15; 35,37; 37,29; 37,36; 37,63; 39,37; 39,60; 41,10; 41,25 (N(CH₃)₂); 59,45; 67,40; 67,46; 112,95; 113,00; 114,10; 125,68; 128,28; 142,71; 142,74; 162,98; 163,06; 166,79.

### [4-(Dimethylamino-thiophen-2-yl-methyl)-cyclohexyliden)-essigsäure-ethylester 23

Zu einer Lösung von Phosphonoessigsäure-triethylester **17** (30,26 g, 0,135 mol) in absol. DMF (200 ml) wurde unter Argon Kalium-*tert*-butylat (15,15 g, 0,135 mol) gegeben und 10 min gerührt. Anschließend wurde das Keton **16** (21,36 g, 0,09 mol), gelöst in DMF (200 ml), zugetropft, 3 h bei RT gerührt und danach auf Eis gegossen. Die Reaktionsmischung wurde mit Diethylether (3 x 100 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 23,9 g (86 %), Öl
¹³C-NMR (CDCl₃): 14,27; 28,57; 28,62; 30,98; 31,34; 31,47; 31,71; 32,09; 36,17; 36,84; 37,62; 40,00; 41,21; 41,33; 59,44; 69,11; 69,20; 113,20; 123,89; 126,09; 126,44; 139,52; 139,71; 162,69; 162,73; 166,69.

### Synthese von Cyclohexylacrylsäureestern

Die Cyclohexylacrylsäureester wurden aus den entsprechenden Aldehyden mit Phosphonoessigsäure-triethylester nach Horner dargestellt.

Es wurden die folgenden Aldehyde verwendet:
4-[Dimethylamino-(phenyl)-methyl]-cyclohexancarbaldehyd **24** (R² = Phenyl)
4-[Dimethylamino-(3-fluor-phenyl)-methyl]-cyclohexancarbaldehyd **25** (R² = 3-Fluorphenyl)
4-[Dimethylamino-(4-fluor-phenyl)-methyl]-cyclohexancarbaldehyd **26** (R² = 4-Fluorphenyl)
4-[(4-Chlor-phenyl)-dimethylamino-methyl]-cyclohexancarbaldehyd **27** (R² = 4-Chlorphenyl)
4-(1-Dimethylamino-3-phenyl-propyl)-cyclohexancarbaldehyd **28** (R² = Phenethyl)
4-(Dimethylamino-thiophen-2-yl-methyl)-cyclohexancarbaldehyd **29** (R² = 2-Thiophen)

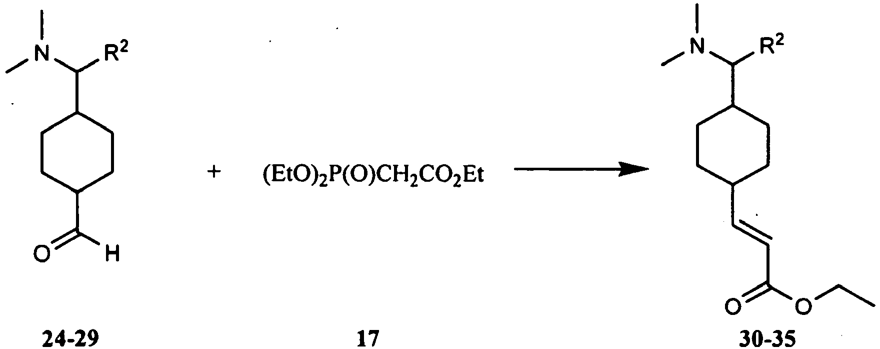

### 3-[4-(Dimethylamino-phenyl-methyl)-cyclohexyl]-acrylsäure-ethylester 30 (R² = Phenyl)

Zu einer Lösung von Phosphonoessigsäure-triethylester **17** (33,62 g, 0,15 mol) in absol. DMF (250 ml) wurde unter Argon Kalium-*tert*-butylat (16,83 g, 0,15 mol) gegeben und 10 min gerührt. Anschließend wurde der Aldehyd **24** (24,27 g, 0,099 mol), gelöst in DMF (250 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt. Man erhält ein Gemisch der E/Z-Isomeren im Verhältnis ca. 6 : 1.
Ausbeute: 27,2 g (87 %), Öl
¹³C-NMR (CDCl₃): 14,22; 25,94; 27,92; 28,23; 28,33; 28,65; 30,18; 30,45; 30,60; 31,45; 31,63; 32,15; 33,03; 37,74; 38,10; 38,55; 40,71; 41,04; 41,30;41,97; 59,67; 60,05; 71,34; 74,89; 75,61; 117,96; 118,97; 120,02; 126,81; 127,55; 137,20; 153,31; 153,90; 155,25; 166,28; 166,99.

### 3-{4-[Dimethylamino-(3-fluorphenyl)-methyl)-cyclohexyl}-acrylsäure-ethylester 31 (R² = 3-Fluorphenyl)

Zu einer Lösung von Phosphonoessigsäure-triethylester **17** (24,66 g, 0,11 mol) in absol. DMF (200 ml) wurde unter Argon Kalium-*tert*-butylat (12,34 g, 0,11 mol) gegeben und 10 min gerührt. Anschließend wurde der Aldehyd **25** (19,3 g, 0,073 mol), gelöst in DMF (200 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 21,9 g (90 %), Öl
¹³C-NMR (CDCl₃): 14,26; 25,82; 28,28; 28,49; 30,09; 30,35; 31,38; 31,56; 32,07; 35,80; 37,54; 38,12; 40,68; 41,05; 41,31; 41,98; 59,75; 60,14; 75,07; 113,57; 113,84; 115,67; 115,94; 118,02; 119,04; 120,12; 125,03; 128,88; 140,13; 153,18; 153,80; 155,20; 160, 92; 164,17; 167,03.

### 3-(4-[Dimethylamino-(4-fluorphenyl)-methyl]-cyclohexyl}acrylsäureethylester 32 (R² = 4-Fluorphenyl)

Zu einer Lösung von Phosphonoessigsäure-triethylester **17** (25,1 g, 0,112 mol) in absol. DMF (150 ml) wurde unter Argon Kalium-*tert-*butylat (12,56 g, 0,112 mol) gegeben und 10 min gerührt. Anschließend wurde der Aldehyd **26** (19,9 g, 0,075 mol), gelöst in DMF (225 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt. Man erhält ein Gemisch der E/Z-Isomeren im Verhältnis ca. 4 : 1.
Ausbeute: 23,7 g (95 %), Öl
¹³C-NMR (CDCl₃): 14,30; 25,78; 26,06; 28,15; 28,32; 28,48; 30,23; 30,48; 31,45; 31,64; 32,32; 37,57; 37,63; 38,28 (C₄); 41,03; 41,80; 41,96 (N(CH₃)₂); 59,62; 60,01; 74,64; 74,80; 114,12; 114,29; 117,86; 118,87; 119,94; 130,28; 132,78; 152,84; 153,46; 154,85; 160,31; 162,73; 165,91; 166,61.

### 3-{4-[(4-Chlorphenyl)-dimethylamino-methyl]-cyclohexyl}-acrylsäureethylester 33 (R² = 4-Chlorphenyl)

Zu einer Lösung von Phosphonoessigsäure-triethylester **17** (16,59 g, 0,074 mol) in absol. DMF (120 ml) wurde unter Argon Kalium-*tert*-butylat (8,3 g, 0,074 mol) gegeben und 10 min gerührt. Anschließend wurde der Aldehyd **27** (13,8 g, 0,049 mol), gelöst in DMF (120 ml), zugetropft. Der Ansatz wurde 3 h bei RT gerührt und danach auf Eis gegossen. Die Reaktionsmischung wurde mit Diethylether (3 x 100 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt. Man erhält ein Gemisch der E/Z-Isomeren im Verhältnis ca. 6 : 1.
Ausbeute: 15,8 g (92 %), Öl
¹³C-NMR (CDCl₃): 14,22; 23,29; 25,71; 25,90; 27,99; 28,20; 28,30; 29,06; 30,07; 31,31; 31,51; 31,99; 32,20; 35,75; 37,53; 38,07; 40,61; 40,97; 41,27; 41,97; 59,69; 60,08; 70,78; 74,77; 117,98; 118,99; 120,07; 127,75; 130,46; 132,48; 134,45; 135,79; 153,10; 153,73; 155,13; 166,96.

### 3-[4-(1-Dimethylamino-3-phenyl-propyl)-cyclohexyl]-acrylsäureethylester 34 (R² = Phenethyl)

Zu einer Lösung von Phosphonoessigsäure-triethylester **17** (26,9 g, 0,120 mol) in absol. DMF (150 ml) wurde unter Argon Kalium-*tert-*butylat (13,46 g, 0,120 mol) gegeben und 10 min gerührt. Anschließend wurde der Aldehyd **28** (21,34 g, 0,080 mol), gelöst in DMF (225 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 19,1 g (71 %), Öl
¹³C-NMR (CDCl₃): 14,14; 28,99; 29,53; 30,56; 31,53; 31,59; 35,26; 39,26; 40,46; 40,72; 41,09; 41,03 (N(CH₃)₂); 59,95; 68,01; 118,84; 125,54; 128,14; 128,17; 142,70; 153,83; 166,86.

### 3-[4-(Dimethylamino-thiophen-2-yl-methyl)-cyclohexyl]-acrylsäureethylester 35 (R² = 2-Thiophenyl)

Zu einer Lösung von Phosphonoessigsäure-triethylester **17** (20,63 g, 0,092 mol) in absol. DMF (200 ml) wurde unter Argon Kalium-fert-butylat (10,3 g, 0,092 mol) gegeben und 10 min gerührt. Anschließend wurde der Aldehyd **29** (15,3, 0,061 mol), gelöst in DMF (200 ml), zugetropft. Der Ansatz wurde 3 h bei RT gerührt und danach auf Eis gegossen. Die Reaktionsmischung wurde mit Diethylether (3 x 200 ml) extrahiert, die organische Phase mit Wasser und gesättigter NaCl-Lösung gewaschen, getrocknet und i. Vak. eingeengt. Das Rohprodukt wurde durch Flashchromatographie mit Essigester/Cyclohexan (1:2) gereinigt.
Ausbeute: 17,4 g (89 %), Öl
¹³C-NMR (CDCl₃): 14,23; 26,01; 26,39; 27,86; 28,04; 29,41; 30,23; 31,34; 31,39; 32,03; 32,08; 37,48; 37,84; 38,05; 40,11; 40,65; 40,74; 40,90; 41,28; 60,09; 70,01; 118,00; 118,99; 119,94; 123,74; 123,80; 126,02; 126,36; 139,88; 140,01; 153,30; 153,84; 155,22; 166,99.

### Synthese der Cyclohexylessigester

Die Cyclohexylessigester wurden durch Hydrierung aus den entsprechenden Cyclohexylidenessigestern in Anwesenheit von Pd/C synthetisiert.

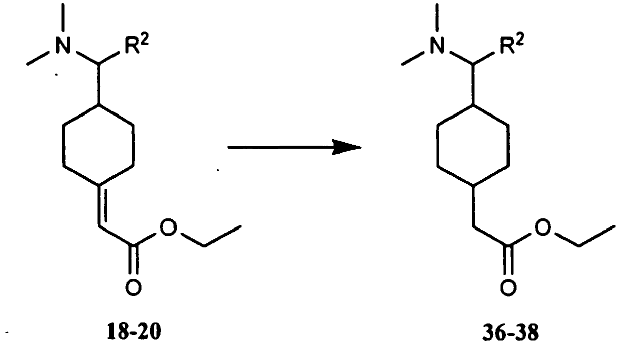

### [4-(Dimethylamino-phenyl-methyl)-cyclohexyl]-essigsäure-ethylester 36 (n = 0; R² = Phenyl)

Der Cyclohexylidenessigester **18** (16,4g, 0,0544 mol) wurde in Methanol (200 ml) gelöst, mit 10%-iger Palladium/Kohle (1,64 g) versetzt und bei 3 bar (RT) 24 h hydriert. Die Pd/C wurde über Kieselgur abgesaugt und das Lösungsmittel i. Vak. entfernt. Der Rückstand wurde in 1 N NaOH (100 ml) und EE (100 ml) gelöst, die organische Phase abgetrennt, mit Wasser gewaschen, getrocknet und eingeengt. Man erhält ein Gemisch der Diastereomeren im Verhältnis ca. 3 : 2.
Ausbeute:15,73 g (95 %), farbloses Öl
¹³C-NMR (CDCl₃): 14,22; 25,41; 25,77; 28,71; 28,88; 30,69; 32,17; 32,84; 35,08; 35,75; 38,26; 38,94; 41,20; 41,98; 42,04 (N(CH₃)₂); 60,01; 71,53; 75,48; 126,73; 126,78; 127,49; 127,57; 129,08; 129,31; 136,23; 137,31; 172,79; 173,30.

### {4-[Dimethylamino-(3-fluorphenyl)-methyl]-cyclohexyl}-essigsäure-ethylester 37 (n = 0; R² = 3-Fluorphenyl)

Der Cyclohexylidenessigester **19** (17,5 g, 0,054 mol) wurde in Methanol (200 ml) gelöst, mit 10%-iger Palladium/Kohle (1,75 g) versetzt und bei 3 bar (RT) 24 h hydriert. Die Pd/C wurde über Kieselgur abgesaugt und das Lösungsmittel i. Vak. entfernt. Der Rückstand wurde in 1 N NaOH (100 ml) und EE (100 ml) gelöst, die organische Phase abgetrennt, mit Wasser gewaschen, getrocknet und eingeengt. Man erhält ein Gemisch der Diastereomeren im Verhältnis ca. 2 : 1.
Ausbeute:15,5 g (90 %), farbloses Öl
¹³C-NMR (CDCl₃): 14,37; 25,39; 25,82; 28,85; 30,74; 32,23; 32,72; 32,91; 35,17; 38,45; 39,00; 41,27; 41,68; 42,04 (N(CH₃)₂); 60,04; 71,24; 75,11; 113,37; 113,42; 113,58; 113,63; 115,55; 115,76; 124,89; 128,65; 128,74; 128,82; 139,12; 139,18; 140,18; 140,24; 161,09; 163,51; 172,64; 172,93.

### {4-[Dimethylamino-(4-fluorphenyl)-methyl]-cyclohexyl}-essigsäure-ethylester 38 (n = 0; R² = 4-Fluorphenyl)

Der Cyclohexylidenessigester **20** (14,0 g, 0,044 mol) wurde in Methanol (200 ml) gelöst, mit 10%-iger Palladium/Kohle (1,4 g) versetzt und bei 3 bar (RT) 24 h hydriert. Die Pd/C wurde über Kieselgur abgesaugt und das Lösungsmittel i. Vak. entfernt. Der Rückstand wurde in 1 N NaOH (100 ml) und EE (100 ml) gelöst, die organische Phase abgetrennt, mit Wasser gewaschen, getrocknet und eingeengt. Man erhält ein Gemisch der Diastereomeren im Verhältnis ca. 3 : 2.
Ausbeute:136 g (96 %), farbloses Öl
¹³C-NMR (CDCl₃): 14,19; 25,17; 25,72; 28,64; 28,76; 30,65; 32,06; 32,58; 32,77; 35,02; 35,99; 38,39; 38,83; 41,14; 41,93; 59,98; 70,82; 74,70; 114,15; 114,24; 114,43; 130,44; 130,54; 132,00; 133,05; 133,09; 160,10; 163,64; 172,90; 173,19.

### Synthese der Cyclohexylpropionsäureester

Die beschriebenen Cyclohexylpropionsäureester wurden durch Hydrierung aus den entsprechenden Cyclohexylacrylsäureester in Anwesenheit von Pd/C synthetisiert.

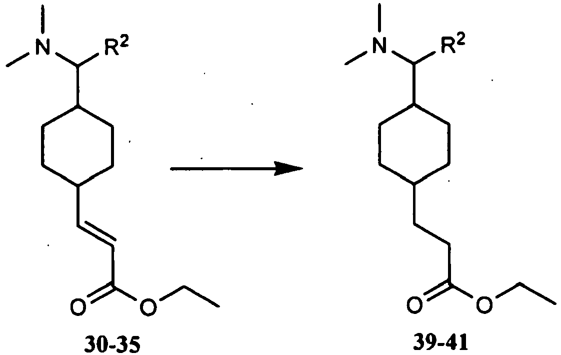

### 3-[4-(Dimethylamino-phenyl-methyl)-cyclohexyl]-propionsäureethylester 39 (n = 1; R² = Phenyl)

Der Cyclohexylacrylsäureester **30** (20,9 g, 0,066 mol) wurde in Methanol (150 ml) gelöst, mit 10%-iger Palladium/Kohle (2,0 g) versetzt und bei 3 bar (RT) 24 h hydriert. Die Pd/C wurde über Kieselgur abgesaugt und das Lösungsmittel im Vakuum entfernt. Der Rückstand wurde in 1N NaOH (100 ml) und EE (100 ml) gelöst, die organische Phase abgetrennt, mit Wasser gewaschen, getrocknet und eingeengt. Man erhält ein Gemisch der Diastereomeren im Verhältnis ca. 6 : 1.
Ausbeute:18,6 g (89 %), farbloses Öl
¹³C-NMR (CDCl₃): 14,15; 25,49; 25,79; 28,54; 29,00; 30,84; 31,62; 31,91; 32,15; 32,35; 32,59; 32,76; 34,62; 35,80; 37,18; 37,37; 38,57; 41,14; 41,96; 60,05; 71,33; 75,55; 126,65; 127,43; 127,50; 127,95; 129,06; 129,27; 136,25; 137,40; 173,97.

### 3-{4-[Dimethylamino-(3-fluorphenyl)-methyl]-cyclohexyl}-propionsäureethyl-ester 40 (n = 1; R² = 3-Fluorphenyl)

Der Cyclohexylacrylsäureester **31** (14,98 g, 0,045 mol) wurde in Methanol (100 ml) gelöst, mit 10%-iger Palladium/Köhle (1,5 g) versetzt und bei 3 bar (RT) 24 h hydriert. Die Pd/C wurde über Kieselgur abgesaugt und das Lösungsmittel im Vakuum entfernt. Der Rückstand wurde in 1 N NaOH (100 ml) und EE (100 ml) gelöst, die organische Phase abgetrennt, mit Wasser gewaschen, getrocknet und eingeengt.
Ausbeute: 14,3 g (95 %), farbloses Öl
¹³C-NMR (CDCl₃): 14,18; 25,36; 25,72; 28,55; 28,86; 30,77; 31,94; 32,14; 32,38; 32,54; 32,73; 34,58; 35,94; 37,38; 38,64; 41,16; 41,98; 60,12; 71,08; 75,19; 113,41; 113,68; 115,64; 115,91; 125,03; 128,75; 128,86; 140,40; 160,86; 164,11; 174,02.

### 3-{4-[Dimethylamino-(4-fluorphenyl)-methyl]-cyclohexyl}-propionsäureethyl-ester 41 (n = 1; R² = 4-Fluorphenyl)

Der Cyclohexylacrylsäureester **32** (12,3g, 0,050 mol) wurde in Methanol (100 ml) gelöst, mit 10%-iger Palladium/Kohle (1,63 g) versetzt und bei 3 bar (RT) 24 h hydriert. Die Pd/C wurde über Kieselgur abgesaugt und das Lösungsmittel im Vakuum entfernt. Der Rückstand wurde in 1 N NaOH (100 ml) und EE (100 ml) gelöst, die organische Phase abgetrennt, mit Wasser gewaschen, getrocknet und eingeengt. Man erhält ein Gemisch der Diastereomeren im Verhältnis ca. 4 : 1.
Ausbeute:16,7 g (100 %), farbloses Öl
¹³C-NMR (CDCl₃): 14,32; 25,43; 25,93; 28,67; 28,72; 28,93; 31,00; 32,05; 32,50; 32,70; 32,88; 34,69; 36,26; 38,90 (C₄); 41,24; 42,08 (N(CH₃)₂); 60,11; 70,79 74,87; 114,08; 114,16; 114,27; 130,35; 130,43; 132,03; 133,17; 160,32; 162,74; 173,71.

### 3-[4-(1-Dimethylamino-3-phenyl-propyl)-cyclohexyl]-propionsäureethylester 42 (n = 1; R² = Phenethyl)

Der Cyclohexylacrylsäureester **34** (14,04 g, 0,041 mol) wurde in Methanol (100 ml) gelöst, mit 10%-iger Palladium/Kohle (1,4 g) versetzt und bei 3 bar (RT) 48 h hydriert. Die Pd/C wurde über Kieselgur abgesaugt und das Lösungsmittel im Vakuum entfernt. Der Rückstand wurde in 1 N NaOH (100 ml) und EE (100 ml) gelöst, die organische Phase abgetrennt, mit Wasser gewaschen, getrocknet und eingeengt.
Ausbeute: 11,7 g (82 %), farbloses Öl
¹³C-NMR (CDCl₃): 14,18; 25,68; 26,37; 28,36; 29,11; 30,01; 31,23; 31,65; 32,18; 32,50; 32,85; 32,90; 34,12; 35,37; 37,25; 38,73; 39,78; 40,84; 41,17; 60,07; 65,41; 68,25; 125,56; 128,24; 142,93; 174,01.

### Synthese der erfindungsgemäßen Verwindungen

### {{4-{[3-(4-Chlorbenzyl)-1,2,4-oxadiazol-5-yl]methyl}cyclohexyl}}-N,N-dimethyl(phenyl)methanamin 43 (n = 0; R² = Phenyl; R¹ = CH₂-(4-Chlorphenyl))

Das Amidoxim **7** (0,18g, 0,99 mmol) wurde in THF (3 ml) gelöst und mit NaH (0,019 g, 0,79 mmol) versetzt. Die Reaktionsmischung wurde für 20 min bei 60°C gerührt. Nach dem Abkühlen auf Raumtemperatur wurde **36** (0,20g, 0,66 mmol) gelöst in THF (1 ml) zugetropft und für eine Stunde zum Rückfluss erhitzt. Nach Abkühlen auf RT wurde mit Wasser (1ml) und 1 ml NaOH (1ml) gequencht und über Filtererde abfiltriert. Anschließend wurden die Phasen getrennt und die wässrige zweimal mit Ether extrahiert. Die vereinigten org. Phasen wurden über MgSO₄ getrocknet und eingeengt. Das Rohprodukt wurde durch Flashchromatographie mit Essigester gereinigt.

### {{4-{[3-(4-Methoxybenzyl)-1,2,4-oxadiazol-5-yl]methyl}cyclohexyl}}-N,N-dimethyl(phenyl)methanamin 44 (n = 0; R² = Phenyl; R¹ = CH₂-(4-Methoxyphenyl))

Das **8** (0,18g, 0,99 mmol) wurde in THF (3 ml) gelöst und mit NaH (0,019 g, 0,79 mmol) versetzt. Die Reaktionsmischung wurde für 20 min bei 60°C gerührt. Nach dem Abkühlen auf Raumtemperatur wurde **36** (0,20g, 0,66 mmol) gelöst in THF (1 ml) zugetropft und für eine Stunde zum Rückfluss erhitzt. Nach Abkühlen auf RT wurde mit Wasser (1 ml) und 1 ml NaOH (1ml) gequencht und über Filtererde abfiltriert. Anschließend wurden die Phasen getrennt und die wässrige zweimal mit Ether extrahiert. Die vereinigten org. Phasen wurden über MgSO₄ getrocknet und eingeengt. Das Rohprodukt wurde durch Flashchromatographie mit Essigester gereinigt.

### Automatisierte Synthese

In ein trockenes Gewindeglas mit Septumkappe wurde bei RT Amidoxim-Lösung (250µmol, 1 ml, 0,25 M in THF) vorgelegt und mit NaH (200 µmmol, 8 mg, 60%ig in Mineralöl) versetzt. Die Reaktionslösung wurde für 20 min bei 60°C erhitzt und anschließend mit Ester-Lösung (100 µmol, 1 ml, 0,1 M in THF) versetzt. Die Reaktionslösung wurde 5 h in einem Synthesis 1 Solid 24 von Heidolph unter Rückfluss geschüttelt. Nach erfolgter Reaktion wurde bei RT mit 1 ml H₂O und 1ml 5N NaOH gequencht und weitere 15min bei RT geschüttelt.
Die Aufarbeitung der Synthesen erfolgt auf dem Myriad-Allex-System von Mettler-Toledo. Dabei wurde die organische Phase abgenommen und gesammelt. Die wäßrige Phase wurde zweimal mit je 2ml CH₂Cl₂ versetzt, die Phasen getrennt. Anschließend wurden die vereinigten, organischen Phasen in der GeneVac eingeengt. Man erhält zum Teil mehrere Isomere, insbesondere E/Z-Isomere, deren Konfiguration nicht bestimmt wurde. Aus diesem Grund werden Isomere im Folgenden als polareres oder unpolareres Diastereomer bezeichnet. Die Polarität wurde über RP-HPLC bestimmt.

Es wurden die folgenden Beispiele hergestellt:

| **Nr.** | **Name** | **Gefundene Masse(ESI)** |
|---|---|---|
| 45. | ((4-Chlor-phenyl)-{4-[3-(4-chlor-phenyl)-[1,2,4]oxadiazol-5-ylmethylen]-cyclohexyl}-methyl)-dimethyl-amin | 442,1 [M⁺+1]; 444,1; 446,1 |
| 46. | [{4-[3-(4-Chlor-phenyl)-[1,2,4]oxadiazol-5-ylmethylen]-cyclohexyl}-(4-fluor-phenyl)-methyl]-dimethyl-amin (Polareres Diastereomer) | 426,0 [M⁺+1]; 428,0 |
| 47. | [{4-[3-(4-Chlor-phenyl)-[1,2,4]oxadiazol-5-ylmethylen]-cyclohexyl}-(4-fluor-phenyl)-methyl]-dimethyl-amin (Unpolareres Diastereomer) | 426,0 [M⁺+1]; 428,0 |
| 48. | ({4-[3-(2,3-Dichlor-phenyl)-[1,2,4]oxadiazol-5-ylmethylen]-cyclohexyl}-phenyl-methyl)-dimethyl-amin (Polareres Diastereomer) | 442,0 [M⁺+1]; 444,0 |
| 49. | ({4-[3-(2,3-Dichlor-phenyl)-[1,2,4]oxadiazol-5-ylmethylen]-cyclohexyl}-phenyl-methyl)-dimethyl-amin (Unpolareres Diastereomer) | 442,0 [M⁺+1]; 444,0 |
| 50. | [{4-[3-(2,3-Dichlor-phenyl)-[1,2,4]oxadiazol-5-ylmethylen]-cyclohexyl}-(3-fluor-phenyl)-methyl]-dimethyl-amin (Polareres Diastereomer) | 460,1 [M⁺+1]; 462,0; 464,0 |
| 51. | [{4-[3-(2,3-Dichlor-phenyl)-[1,2,4]oxadiazol-5-ylmethylen]-cyclohexyl}-(3-fluor-phenyl)-methyl]-dimethyl-amin (Unpolareres Diastereomer) | 460,1 [M⁺+1]; 462,0; 464,0 |
| 52. | ((4-Chlor-phenyl)-{4-[3-(2,3-dichlor-phenyl)-[1,2,4]oxadiazol-5-ylmethylen]-cyclohexyl}-methyl)-dimethyl-amin (Polareres Diastereomer) | 476,0 [M⁺+1]; 478,0; 480,0 |
| 53. | ((4-Chlor-phenyl)-{4-[3-{2,3-dichlor-phenyl)-[1,2,4]oxadiazol-5-ylmethylen]-cyclohexyl}-methyl)-dimethyl-amin (Unpolareres Diastereomer) | 476,0 [M⁺+1]; 478,0; 480,0 |
| 54. | (1-{4-[3-(2,3-Dichlor-phenyl)-[1,2,4]oxadiazol-5-ylmethylen]-cyclohexyl}-3-phenyl-propyl)-dimethyl-amin | 470,0 [M⁺+1], 472,0 |
| 55. | [{4-[3-(2,3-Dichlor-phenyl)-[1,2,4]oxadiazol-5-ylmethylen]-cyclohexyl}-(4-fluor-phenyl)-methyl]-dimethyl-amin (Polareres Diastereomer) | 460,0 [M⁺+1]; 462,0 |
| 56. | [{4-[3-(2,3-Dichlor-phenyl)-[1,2,4]oxadiazol-5-ylmethylen]-cyclohexyl}-(4-fluor-phenyl)-methyl]-dimethyl-amin (Unpolareres Diastereomer) | 460,0 [M⁺+1]; 462,0 |
| 57. | Dimethyl-(phenyl-{4-[3-(4-trifluormethoxy-phenyl)-[1,2,4]oxadiazol-5-ylmethylen]-cyclohexyl}-methyl)-amin (Polareres Diastereomer) | 458,2 [M⁺+1] |
| 58. | Dimethyl-(phenyl-{4-[3-(4-trifluormethoxy-phenyl)-[1,2,4]oxadiazol-5-ylmethylen]-cyclohexyl}-methyl)-amin (Unpolareres Diastereomer) | 458,2 [M⁺+1] |
| 59. | ((3-Fluor-phenyl)-{4-[3-(4-trifluormethoxy-phenyl)-[1,2,4]oxadiazol-5-ylmethylen]-cyclohexyl}-methyl)-dimethyl-amin (Polareres Diastereomer) | 476,2 [M⁺+1] |
| 60. | ((3-Fluor-phenyl)-{4-[3-(4-trifluormethoxy-phenyl)-[1,2,4]oxadiazol-5-ylmethylen]-cyclohexyl}-methyl)-dimethyl-amin (Unpolareres Diastereomer) | 476,2 [M⁺+1] |
| 61. | ((4-Chlor-phenyl)-{4-[3-(4-trifluormethoxy-phenyt)-[1,2,4]oxadiazol-5-ylmethylen]-cyclohexyl}-methyl)-dimethyl-amin (Polareres Diastereomer) | 492,0 [M⁺+1]; 494.0 |
| 62. | ((4-Chlor-phenyl)-{4-[3-(4-trifluormethoxy-phenyl)-[1,2,4]oxadiazol-5-ylmethylen]-cyclohexyl)-methyl)-dimethyl-amin (Unpolareres Diastereomer) | 492,0 [M⁺+1]; 494.0 |
| 63. | Dimethyl-(3-phenyl-1-{4-[3-(4-trifluormethoxy-phenyl)-[1,2,4]oxadiazol-5-ylmethylen]-cyclohexyl}-propyl)-amin (Polareres Diastereomer) | 486,2 [M⁺+1] |
| 64. | Dimethyl-(3-phenyl-1-{4-[3-(4-trifluormethoxy-phenyl)-[1,2,4]oxadiazol-5-ylmethylen]-cyclohexyl}-propyl)-amin (Unpolareres Diastereomer) | 486,2 [M⁺+1] |
| 65. | ((4-Fluor-phenyl)-{4-[3-(4-trifluormethoxy-phenyl)-[1,2,4]oxadiazol-5-ylmethylen]-cyclohexyl}-methyl)-dimethyl-amin (Polareres Diastereomer) | 476,2 [M⁺+1] |
| 66. | ((4-Fluor-phenyl)-{4-[3-(4-trifluormethoxy-phenyl)-[1,2,4]oxadiazol-5-ylmethylen]-cyclohexyl)-methyl)-dimethyl-amin (Unpolareres Diastereomer) | 476,2 [M⁺+1] |
| 67. | Dimethyl-(thiophen-2-yl-{4-[3-(4-trifluormethoxy-phenyl)-[1,2,4]oxadiazol-5-ylmethylen]-cyclohexyl}-methyl)-amin (Polareres Diastereomer) | 464,2 [M⁺+1] |
| 68. | Dimethyl-(thiophen-2-yl-{4-[3-(4-trifluormethoxy-phenyl)-[1,2,4]oxadiazol-5-ylmethylen]-cyclohexyl}-methyl)-amin (Unpolareres Diastereomer) | 464,2 [M⁺+1] |
| 69. | Dimethyl-{phenyl-[4-(3-phenyl-[1,2,4]oxadiazol-5-ylmethylen)-cyclohexyl]-methyl}-amin (Polareres Diastereomer) | 374,2 [M⁺+1] |
| 70. | Dimethyl-{phenyl-[4-(3-phenyl-[1,2,4]oxadiazol-5-ylmethylen)-cyclohexyl]-methyl}-amin (Unpolareres Diastereomer) | 374,2 [M⁺+1] |
| 71. | {(3-Fluor-phenyl)-(4-(3-phenyl-[1,2,4]oxadiazol-5-ylmethylen)-cyclohexyl]-methyl}-dimethyl-amin (Polareres Diastereomer) | 392,2 [M⁺+1] |
| 72. | {(3-Fluor-phenyl)-[4-(3-phenyl-[1,2,4]oxadiazol-5-ylmethylen)-cyclohexyl]-methyl}-dimethyl-amin (Unpolareres Diastereomer) | 392,2 [M⁺+1] |
| 73. | Dimethyl-{3-phenyl-1-[4-(3-phenyl-[1,2,4]oxadiazol-5-ylmethylen)-cyclohexyl]-propyl)-amin (Polareres Diastereomer) | 402,2 [M⁺+1] |
| 74. | Dimethyl-{3-phenyl-1-[4-(3-phenyl-[1,2,4]oxadiazol-5-ylmethylen)-cyclohexyl]-propyl}-amin (Unpolareres Diastereomer) | 402,2 [M⁺+1] |
| 75. | {(4-Fluor-phenyl)-[4-(3-phenyl-[1,2,4]oxadiazol-5-ylmethylen)-cyclohexyl]-methyl}-dimethyl-amin (Polareres Diastereomer) | 392,2 [M⁺+1] |
| 76. | {(4-Fluor-phenyl)-[4-(3-phenyl-[1,2,4]oxadiazol-5-ylmethylen)-cyclohexyl]-methyl}-dimethyl-amin (Unpolareres Diastereomer) | 392,2 [M⁺+1] |
| 77. | [{4-[3-(2,4-Difluor-phenyl)-[1,2,4]oxadiazol-5-ylmethylen]-cyclohexyl}-(3-fluor-phenyl)-methyl]-dimethyl-amin (Polareres Diastereomer) | 428,2 [M⁺+1] |
| 78. | [{4-[3-(2,4-Difluor-phenyl)-[1,2,4]oxadiazol-5-ylmethylen]-cyclohexyl}-(3-fluor-phenyl)-methyl]-dimethyl-amin (Unpolareres Diastereomer) | 428,2 [M⁺+1] |
| 79. | ({4-[3-(4-Methoxy-benzyl)-[1,2,4]oxadiazol-5-ylmethylen]-cyclohexyl}-phenyl-methyl)-dimethyl-amin (Polareres Diastereomer) | 418,2 [M⁺+1] |
| 80. | ({4-[3-(4-Methoxy-benzyl)-[1,2,4]oxadiazol-5-ylmethylen]-cyclohexyl}-phenyl-methyl)-dimethyl-amin (Unpolareres Diastereomer) | 418,2 [M⁺+1] |
| 81. | ((3-Fluor-phenyl)-{4-[3-(4-methoxy-benzyl)-[1,2,4]oxadiazol-5-ylmethylen]-cyclohexyl}-methyl)-dimethyl-amin (Polareres Diastereomer) | 436,2 [M⁺+1] |
| 82. | ((3-Fluor-phenyl)-{4-(3-(4-methoxy-benzyl)-[1,2,4]oxadiazol-5-ylmethylen]-cyclohexyl}-methyl)-dimethyl-amin (Unpolareres Diastereomer) | 436,2 [M⁺+1] |
| 83. | ((4-Chlor-phenyl)-{4-[3-(4-methoxy-benzyl)-[1,2,4]oxadiazol-5-ylmethylen]-cyclohexyl}-methyl)-dimethyl-amin (Polareres Diastereomer) | 452,0 [M⁺+1]; 454,0 |
| 84. | ((4-Chlor-phenyl)-{4-[3-(4-methoxy-benzyl)-[1,2,4]oxadiazol-5-ylmethylen]-cyclohexyl}-methyl)-dimethyl-amin (Unpolareres Diastereomer) | 452,0 [M⁺+1]: 454,0 |
| 85. | (1-{4-[3-(4-Methoxy-benzyl)-[1,2,4]oxadiazol-5-ylmethylen]-cyclohexyl}-3-phenyl-propyl)-dimethyl-amin (Polareres Diastereomer) | 446,3 [M⁺+1] |
| 86. | (1-{4-[3-(4-Methoxy-benzyl)-[1,2,4]oxadiazol-5-ylmethylen]-cyclohexyl}-3-phenyl-propyl)-dimethyl-amin (Unpolareres Diastereomer) | 446,3 [M⁺+1] |
| 87. | ((4-Fluor-phenyl)-{4-[3-(4-methoxy-benzyl)-[1,2,4]oxadiazol-5-ylmethylen]-cyclohexyl}-methyl)-dimethyl-amin (Polareres Diastereomer) | 436,2 [M⁺+1] |
| 88. | ((4-Fluor-phenyl)-{4-[3-(4-methoxy-benzyl)-[1,2,4]oxadiazol-5-ylmethylen]-cyclohexyl}-methyl)-dimethyl-amin (Unpolareres Diastereomer) | 436,2 [M⁺+1] |
| 89. | ({4-[3-(4-Chlor-benzyl)-[1,2,4]oxadiazol-5-ylmethylen]-cyclohexyl}-phenyl-methyl)-dimethyl-amin (Polareres Diastereomer) | 422,1 [M⁺+1]; 424,0 |
| 90. | ({4-[3-(4-Chlor-benzyl)-[1,2,4]oxadiazol-5-ylmethylen]-cyclohexyl)-phenyl-methyl)-dimethyl-amin (Unpolareres Diastereomer) | 422,1 [M⁺+1]; 424,0 |
| 91. | [{4-[3-(4-Chlor-benzyl)-[1,2,4]oxadiazol-5-ylmethylen]-cyclohexyl}-(3-fluor-phenyl)-methyl]-dimethyl-amin (Polareres Diastereomer) | 440,0 [M⁺+1]; 442,0 |
| 92. | [{4-[3-(4-Chlor-benzyl)-[1,2,4]oxadiazol-5-ylmethylen]-cyclohexyl}-(3-fluor-phenyl)-methyl]-dimethyl-amin (Unpolareres Diastereomer) | 440,0 [M⁺+1]; 442,0 |
| 93. | [{4-[3-(4-Chlor-benzyl)-[1,2,4]oxadiazol-5-ylmethylen]-cyclohexyl}-(4-chlor-phenyl)-methyl]-dimethyl-amin (Polareres Diastereomer) | 456,0 [M⁺+1]; 458,0 |
| 94. | [{4-[3-(4-Chlor-benzyl)-[1,2,4]oxadiazol-5-ylmethylen]-cyclohexyl}-(4-chlor-phenyl)-methyl]-dimethyl-amin (Unpolareres Diastereomer) | 456,0 [M⁺+1]; 458,0 |
| 95. | (1-{4-[3-(4-Chlor-benzyl)-[1,2,4]oxadiazol-5-ylmethylen]-cyclohexyl}-3-phenyl-propyl)-dimethyl-amin (Polareres Diastereomer) | 450,0 [M⁺+1]; 452,0 |
| 96. | (1-{4-[3-(4-Chlor-benzyl)-[1,2,4]oxadiazol-5-ylmethylen]-cyclohexyl}-3-phenyl-propyl)-dimethyl-amin (Unpolareres Diastereomer) | 450,0 [M⁺+1]; 452,0 |
| 97. | [{4-[3-(4-Chlor-benzyl)-[1,2,4]oxadiazol-5-ylmethylen]-cyclohexyl}-(4-fluor-phenyl)-methyl]-dimethyl-amin (Polareres Diastereomer) | 440,0 [M⁺+1]; 442.0 |
| 98. | [{4-[3-(4-Chlor-benzyl)-[1,2,4]oxadiazol-5-ylmethylen]-cyclohexyl}-(4-fluor-phenyl)-methyl]-dimethyl-amin (Unpolareres Diastereomer) | 440,0 [M⁺+1]; 442.0 |
| 99. | {(3-Fluor-phenyl)-[4-(3-p-tolyl-[1,2,4]oxadiazol-5-ylmethylen)-cyclohexyl]-methyl}-dimethyl-amin | 406,2 [M⁺+1] |
| 100. | {(4-Chlor-phenyl)-[4-(3-p-tolyl-[1,2,4]oxadiazol-5-ylmethylen)-cyclohexyl]-methyl}-dimethyl-amin | 422,0 [M⁺+1]; 424,0 |
| 101. | {(4-Fluor-phenyl)-[4-(3-p-tolyl-[1,2,4]oxadiazol-5-ylmethylen)-cyclohexyl]-methyl}-dimethyl-amin (Polareres Diastereomer) | 406,2 [M⁺+1] |
| 102. | {(4-Fluor-phenyl)-[4-(3-p-tolyl-[1,2,4]oxadiazol-5-ylmethylen)--cyclohexyl]-methyl}-dimethyl-amin (Unpolareres Diastereomer) | 406,2 [M⁺+1] |
| 103. | ({4-[3-(3,4-Dimethoxy-phenyl)-[1,2,4]oxadiazol-5-ylmethylen]-cyclohexyl}-phenyl-methyl)-dimethyl-amin (Polareres Diastereomer) | 434,2 [M⁺+1] |
| 104. | ({4-[3-(3,4-Dimethoxy-phenyl)-[1,2,4]oxadiazol-5-ylmethylen]-cyclohexyl}-phenyl-methyl)-dimethyl-amin (Unpolareres Diastereomer) | 434,2 [M⁺+1] |
| 105. | [{4-[3-(3,4-Dimethoxy-phenyl)-[1,2,4]oxadiazol-5-ylmethylen]-cyclohexyl}-(3-fluor-phenyl)-methyl]-dimethyl-amin (Polareres Diastereomer) | 452,2 [M⁺+1] |
| 106. | [{4-[3-(3,4-Dimethoxy-phenyl)-[1,2,4]oxadiazol-5-ylmethylen]-cyclohexyl}-(3-fluor-phenyl)-methyl]-dimethyl-amin (Unpolareres Diastereomer) | 452,2 [M⁺+1] |
| 107. | ((4-Chlor-phenyl)-{4-[3-(3,4-dimethoxy-phenyl)-[1,2,4]oxadiazol-5-ylmethylen]-cyclohexyl}-methyl)-dimethyl-amin (Polareres Diastereomer) | 468,0 [M⁺+1]; 470,0 |
| 108. | ((4-Chlor-phenyl)-{4-[3-(3,4-dimethoxy-phenyl)-[1,2,4]oxadiazol-5-ylmethylen]-cyclohexyl)-methyl)-dimethyl-amin (Unpolareres Diastereomer) | 468,0 [M⁺+1]; 470,0 |
| 109. | (1-{4-(3-(3,4-Dimethoxy-phenyl)-[1,2,4]oxadiazol-5-ylmethylen]-cyclohexyl}-3-phenyl-propyl)-dimethyl-amin (Polareres Diastereomer) | 462,3 |
| 110. | (1-{4-[3-(3,4-Dimethoxy-phenyl)-[1,2,4]oxadiazol-5-ylmethylen]-cyclohexyl}-3-phenyl-propyl)-dimethyl-amin (Unpolareres Diastereomer) | 462,3 [M⁺+1] |
| 111. | [{4-[3-(3,4-Dimethoxy-phenyl)-[1,2,4]oxadiazol-5-ylmethylen]-cyclohexyl}-(4-fluor-phenyl)-methyl]-dimethyl-amin (Polareres Diastereomer) | 452,2 [M⁺+1] |
| 112. | [{4-[3-(3,4-Dimethoxy-phenyl)-[1,2,4]oxadiazol-5-ylmethylen]-cyclohexyl}-(4-fluor-phenyl)-methyl]-dimethyl-amin (Unpolareres Diastereomer) | 452, 2 [M⁺+1] |
| 113. | ({4-[3-(3-Chlor-phenyl)-[1,2,4]oxadiazol-5-ylmethylen]-cyclohexyl}-phenyl-methyl)-dimethyl-amin (Polareres Diastereomer) | 408,0 [M⁺+1]; 410,0 |
| 114. | ({4-[3-(3-Chlor-phenyl)-[1,2,4]oxadiazol-5-ylmethylen]-cyclohexyl}-phenyl-methyl)-dimethyl-amin (Unpolareres Diastereomer) | 408,0 [M⁺+1]; 410,0 |
| 115. | [{4-[3-(3-Chlor-phenyl)-[1,2,4]oxadiazol-5-ylmethylen]-cyclohexyl}-(3-fluor-phenyl)-methyl]-dimethyl-amin (Polareres Diastereomer) | 426,0 [M⁺+1]; 428.0 |
| 116. | [{4-[3-(3-Chlor-phenyl)-[1,2,4]oxadiazol-5-ylmethylen]-cyclohexyl}-(3-fluor-phenyl)-methyl]-dimethyl-amin (Unpolareres Diastereomer) | 426,0 [M⁺+1]; 428.0 |
| 117. | ((4-Chlor-phenyl)-{4-[3-(3-chlor-phenyl)-[1,2,4]oxadiazol-5-ylmethylen]-cyclohexyl)-methyl)-dimethyl-amin (Polareres Diastereomer) | 442,0 [M⁺+1]; 444,0 |
| 118. | ((4-Chlor-phenyl)-{4-[3-(3-chlor-phenyl)-[1,2,4]oxadiazol-5-ylmethylen]-cyclohexyl}-methyl)-dimethyl-amin (Unpolareres Diastereomer) | 442,0 [M⁺+1]; 444,0 |
| 119. | (1-{4-[3-(3-Chlor-phenyl)-[1,2,4]oxadiazol-5-ylmethylen]-cyclohexyl}-3-phenyl-propyl)-dimethyl-amin (Polareres Diastereomer) | 436,0 [M⁺+1]; 438,0 |
| 120. | (1-{4-[3-(3-Chlor-phenyl)-[1,2,4]oxadiazol-5-ylmethylen]-cyclohexyl}-3-phenyl-propyl)-dimethyl-amin (Unpolareres Diastereomer) | 436,0 [M⁺+1]; 438,0 |
| 121. | [{4-[3-(3-Chlor-phenyl)-[1,2,4]oxadiazol-5-ylmethylen]-cyclohexyl}-(4-fluor-phenyl)-methyl]-dimethyl-amin | 426,0 [M⁺+1]; 428,0 |
| 122. | ({4-[3-(4-tert-Butyl-phenyl)-[1,2,4]oxadiazol-5-ylmethylen]-cyclohexyl}-phenyl-methyl)-dimethyl-amin (Polareres Diastereomer) | 430,3 [M⁺+1] |
| 123. | ({4-[3-(4-tert-Butyl-phenyl)-[1,2,4]oxadiazol-5-ylmethylen]-cyclohexyl}-phenyl-methyl)-dimethyl-amin (Unpolareres Diastereomer) | 430,3 [M⁺+1] |
| 124. | [{4-[3-(4-tert-Butyl-phenyl)-[1,2,4]oxadiazol-5-ylmethylen]-cyclohexyl}-(3-fluor-phenyl)-methyl]-dimethyl-amin (Polareres Diastereomer) | 448,3 [M⁺+1] |
| 125. | [{4-[3-(4-tert-Butyl-phenyl)-[1,2,4]oxadiazol-5-ylmethylen)-cyclohexyl}-(3-fluor-phenyl)-methyl]-dimethyl-amin (Unpolareres Diastereomer) | 448,3 [M⁺+1] |
| 126. | [{4-[3-(4-tert-Butyl-phenyl)-[1,2,4]oxadiazol-5-ylmethylen]-cyclohexyl}-(4-chlor-phenyl)-methyl]-dimethyl-amin (Polareres Diastereomer) | 464,1 [M⁺+1]; 466,1 |
| 127. | [{4-[3-(4-tert-Butyl-phenyl)-[1,2,4]oxadiazol-5-ylmethylen]-cyclohexyl}-(4-chlor-phenyl)-methyl]-dimethyl-amin (Unpolareres Diastereomer) | 464,1 [M⁺+1]; 466,1 |
| 128. | (1-{4-[3-(4-tert-Butyl-phenyl)-[1,2,4]oxadiazol-5-ylmethylen]-cyclohexyl)-3-phenyl-propyl)-dimethyl-amin (Polareres Diastereomer) | 458,3 [M⁺+1] |
| 129. | (1-{4-[3-(4-tert-Butyl-phenyl)-[1,2,4]oxadiazol-5-ylmethylen]-cyclohexyl}-3-phenyl-propyl)-dimethyl-amin (Unpolareres Diastereomer) | 458,3 [M⁺+1] |
| 130. | [{4-[3-(4-tert-Butyl-phenyl)-[1,2,4]oxadiazol-5-ylmethylen]-cyclohexyl}-(4-fluor-phenyl)-methyl]-dimethyl-amin (Polareres Diastereomer) | 448,3 [M⁺+1] |
| 131. | [{4-[3-(4-tert-Butyl-phenyl)-[1,2,4]oxadiazol-5-ylmethylen]-cyclohexyl}-(4-fluor-phenyl)-methyl]-dimethyl-amin (Unpolareres Diastereomer) | 448,3 [M⁺+1] |
| 132. | ({4-[3-(4-tert-Butyl-phenyl)-[1,2,4]oxadiazol-5-ylmethylen]-cyclohexyl}-thiophen-2-yl-methyl)-dimethyl-amin (Polareres Diastereomer) | 436,2 [M⁺+1] |
| 133. | ({4-[3-(4-tert-Butyl-phenyl)-[1,2,4]oxadiazol-5-ylmethylen]-cyclohexyl}-thiophen-2-yl-methyl)-dimethyl-amin (Unpolareres Diastereomer) | 436,2 [M⁺+1] |
| 134. | Dimethyl-{phenyl-[4-(3-m-tolyl-[1,2,4]oxadiazol-5-ylmethylen)-cyclohexyl]-methyl}-amin (Polareres Diastereomer) | 388,2 [M⁺+1] |
| 135. | Dimethyl-{phenyl-[4-(3-m-tolyl-[1,2,4]oxadiazol-5-ylmethylen)-cyclohexyl)-methyl}-amin (Unpolareres Diastereomer) | 388,2 [M⁺+1] |
| 136. | {(3-Fluor-phenyl)-[4-(3-m-tolyl-[1,2,4]oxadiazol-5-ylmethylen)-cyclohexyl]-methyl}-dimethyl-amin (Polareres Diastereomer) | 406,2 [M⁺+1] |
| 137. | {(3-Fluor-phenyl)-[4-(3-m-tolyl-[1,2,4]oxadiazol-5-ylmethylen)-cyclohexyl]-methyl}-dimethyl-amin (Unpolareres Diastereomer) | 406,2 [M⁺+1] |
| 138. | {(4-Chlor-phenyl)-[4-(3-m-tolyl-[1,2,4]oxadiazol-5-ylmethylen)-cyclohexyl]-methyl}-dimethyl-amin (Polareres Diastereomer) | 422,1 [M⁺+1]; 424.0 |
| 139. | {(4-Chlor-phenyl)-[4-(3-m-tolyl-[1,2,4]oxadiazol-5-ylmethylen)-cyclohexyl]-methyl}-dimethyl-amin (Unpolareres Diastereomer) | 422,1 [M⁺+1]; 424.0 |
| 140. | ((4-Fluor-phenyl)-{4-[2-(3-pheny)-[1,2,4]oxadiazol-5-yl)-vinyl]-cyclohexyl}-methyl)-dimethyl-amin | 406,2 [M⁺+1] |
| 141. | [(4-{2-[3-(2,4-Difluor-phenyl)-[1,2,4]oxadiazol-5-yl]-vinyl}-cyclohexyl)-(4-fluor-phenyl)-methyl]-dimethyl-amin | 442,2 [M⁺+1] |
| 142. | [(4-Fluor-phenyl)-(4-{2-[3-(4-methoxy-benzyl)-[1,2,4]oxadiazol-5-yl]-vinyl}-cyclohexyl)-methyl]-dimethyl-amin | 450,2 [M⁺+1] |
| 143. | [(4-{2-[3-(4-Chlor-benzyl)-[1,2,4]oxadiazol-5-yl]-vinyl}-cyclohexyl)-(4-fluor-phenyl)-methyl]-dimethyl-amin | 454,1 [M⁺+1]; 456,0 |
| 144. | Dimethyl-(3-phenyl-1-{4-[2-(3-phenyl-[1,2,4]oxadiazol-5-yl)-vinyl]-cyclohexyl}-propyl)-amin | 416,3 [M⁺+1] |
| 145. | [1-(4-{2-[3-(2,4-Difluor-phenyl)-[1,2,4]oxadiazol-5-yl}-vinyl}-cyclohexyl)-3-phenyl-propyl]-dimethyl-amin | 452,2 [M⁺+1] |
| 146. | [1-(4-{2-[3-(4-Methoxy-benzyl)-[1,2,4]oxadiazol-5-yl]-vinyl}-cyclohexyl)-3-phenyl-propyl]-dimethyl-amin | 460,3 [M⁺+1] |
| 147. | [1-(4-{2-[3-(4-Chlor-benzyl)-[1,2,4]oxadiazol-5-yl]-vinyl}-cyclohexyl)-3-phenyl-propyl]-dimethyl-amin | 464,1 [M⁺+1]; 466,0 |
| 148. | Dimethyl-(phenyl-{4-[2-(3-phenyl-[1,2,4]oxadiazol-5-yl)-vinyl]-cyclohexyl}-methyl)-amin | 388,2 [M⁺+1] |
| 149. | [(4-{2-[3-(4-Chlor-benzyl)-[1,2,4]oxadiazol-5-yl]-vinyl}-cyclohexyl)-phenyl-methyl]-dimethyl-amin (Polareres Diastereomer) | 436,1 [M⁺+1]; 438,0 |
| 150. | [(4-{2-[3-(4-Chlor-benzyl)-[1,2,4]oxadiazol-5-yl]-vinyl}-cyclohexyl)-phenyl-methyl)-dimethyl-amin (Unpolareres Diastereomer) | 436,1 [M⁺+1]; 438,0 |
| 151. | [(4-Chlor-phenyl)-(4-{2-[3-(2,4-difluor-phenyl)-[1,2,4]oxadiazol-5-yl]-vinyl}-cyclohexyl)-methyl]-dimethyl-amin | 458,1 [M⁺+1]; 460,0 |
| 152. | [(4-Chlor-phenyl)-(4-{2-[3-(4-methoxy-benzyl)-[1,2,4]oxadiazol-5-y)]-vinyl}-cyclohexyl)-methyl]-dimethyl-amin | 466,1 [M⁺+1]; 468,0 |
| 153. | [(4-{2-[3-(4-Chlor-benzyl)-[1,2,4]oxadiazol-5-yl]-vinyl}-cyclohexyl)-(4-chlor-phenyl)-methyl]-dimethyl-amin | 470,0 [M⁺+1]; 472,0 |
| 154. | [(4-{2-[3-(4-Methoxy-benzyl)-[1,2,4]oxadiazol-5-yl]-vinyl}-cyclohexyl)-thiophen-2-yl-methyl]-dimethyl-amin | 438,2 [M⁺+1] |
| 155. | [(4-{2-[3-(4-Chlor-benzyl)-[1,2,4]oxadiazol-5-yl]-vinyl}-cyclohexyl)-thiophen-2-yl-methyl]-dimethyl-amin | 442,1 [M⁺+1]; 444,0 |
| 156. | ((3-Fluor-phenyl)-{4-[2-(3-phenyl-[1,2,4]oxadiazol-5-yl)-vinyl]-cyclohexyl)-methyl)-dimethyl-amin | 406,2 [M⁺+1] |
| 157. | [(3-Fluor-phenyl)-(4-{2-[3-(4-methoxy-benzyl)-[1,2,4]oxadiazol-5-yl]-vinyl}-cyclohexyl)-methyl]-dimethyl-amin | 450,2 [M⁺+1] |
| 158. | ((4-Fluor-phenyl)-{4-[2-(3-p-tolyl-[1,2,4]oxadiazol-5-yl)-vinyl]-cyclohexyl}-methyl)-dimethyl-amin | 420,2 [M⁺+1] |
| 159. | [(4-{2-[3-(3,4-Dimethoxy-phenyl)-[1,2,4]oxadiazol-5-yl]-vinyl}-cyclohexyl)-(4-fluor-phenyl)-methyl]-dimethyl-amin | 466,2 [M⁺+1] |
| 160. | Dimethyl-(3-phenyl-1-{4-[2-(3-p-tolyl-[1,2,4]oxadiazol-5-yl)-vinyl]-cyclohexyl}-propyl)-amin | 430,3 [M⁺+1] |
| 161. | [1-(4-{2-[3-(3,4-Dimethoxy-phenyl)-[1,2,4]oxadiazol-5-yl]-vinyl}-cyclohexyl)-3-phenyl-propyl]-dimethyl-amin | 476,3 [M⁺+1] |
| 162. | Dimethyl-(phenyl-{4-[2-(3-p-tolyl-[1,2,4]oxadiazol-5-yl)-vinyl]-cyclohexyl}methyl)-amin | 402,2 [M⁺+1] |
| 163. | [(4-{2-[3-(3,4-Dimethoxy-phenyl)-[1,2,4]oxadiazol-5-yl]-vinyl}-cyclohexyl)-phenyl-methyl]-dimethyl-amin | 448,3 [M⁺+1] |
| 164. | ((4-Chlor-phenyl)-{4-[2-(3-p-tolyl-[1,2,4]oxadiazol-5-yl)-vinyl]-cyclohexyl}-methyl)-dimethyl-amin | 436,1 [M⁺+1]; 438,0 |
| 165. | [(4-Chlor-phenyl)-(4-{2-[3-(3,4-dimethoxy-phenyl)-[1,2,4]oxadiazol-5-yl]-vinyl}-cyclohexyl)-methyl]-dimethyl-amin | 482,0 [M⁺+1]; 484,1 |
| 166. | Dimethyl-(thiophen-2-yl-{4-[2-(3-p-tolyl-[1,2,4]oxadiazol-5-yl)-vinyl]-cyclohexyl}-methyl)-amin | 408,2 [M⁺+1] |
| 167. | [(4-{2-[3-(3,4-Dimethoxy-phenyl)-[1,2,4]oxadiazol-5-yl]-vinyl}cyclohexyl)-thiophen-2-yl-methyl]-dimethyl-amin | 454,2 [M⁺+1] |
| 168. | ((3-Fluor-phenyl)-{4-[2-(3-p-tolyl-[1,2,4]oxadiazol-5-yl)vinyl]-cyclohexyl}-methyl)-dimethyl-amin | 420,2 [M⁺+1] |
| 169. | [(4-{2-[3-(3,4-Dimethoxy-phenyl)-[1,2,4]oxadiazol-5-yl]-vinyl}-cyclohexyl)-(3-fluor-phenyl)-methyl]-dimethyl-amin | 466,2 [M⁺+1] |
| 170. | [(4-{2-[3-(3-Chlor-phenyl)-[1,2,4]oxadiazol-5-yl]-vinyl}-cyclohexyl)-(4-fluor-phenyl)-methyl]-dimethyl-amin | 440,0 [M⁺+1]; 442,0 |
| 171. | [(4-{2-[3-(4-tert-Butyl-phenyl)-[1,2,4]oxadiazol-5-yl]-vinyl}-cyclohexyl)-(4-fluor-phenyl)-methyl]-dimethyl-amin | 462,3 [M⁺+1] |
| 172. | [(4-{2-[3-(4-tert-Butyl-phenyl)-[1,2,4]oxadiazol-5-yl]-vinyl}-cyclohexyl)-(4-fluor-phenyl)-methyl]-dimethyl-amin | 462,3 [M⁺+1] |
| 173. | ((4-Fluor-phenyl)-{4-[2-(3-m-tolyl-[1,2,4]oxadiazol-5-yl)-vinyl]-cyclohexyl)-methyl)-dimethyl-amin | 420,2 [M⁺+1] |
| 174. | [1-(4-{2-[3-(3-Chlor-phenyl)-[1,2,4]oxadiazo)-5-yl]-vinyl}-cyclohexyl)-3-phenyl-propyl]-dimethyl-amin | 450,1 [M⁺+1]; 452,0 |
| 175. | [1-(4-{2-[3-(4-tert-Butyl-phenyl)-[1,2,4]oxadiazol-5-yl]-vinyl}-cyclohexyl)-3-phenyl-propyl]-dimethyl-amin (Polareres Diastereomer) | 472,3 [M⁺+1] |
| 176. | [1-(4-{2-[3-(4-tert-Butyl-phenyl)-[1,2,4]oxadiazol-5-yl]-vinyl}-cyclohexyl)-3-phenyl-propyl]-dimethyl-amin (Unpolareres Diastereomer) | 472,3 [M⁺+1] |
| 177. | Dimethyl-(3-phenyl-1-{4-[2-(3-m-tolyl-[1,2,4]oxadiazol-5-yl)-vinyl]-cyclohexyl}-propyl)-amin (Polareres Diastereomer) | 430,3 [M⁺+1] |
| 178. | Dimethyl-(3-phenyl-1-{4-[2-(3-m-tolyl-[1,2,4]oxadiazol-5-yl)-vinyl]-cyclohexyl}-propyl)-amin (Unpolareres Diastereomer) | 430,3 [M⁺+1] |
| 179. | [(4-{2-[3-(3-Chlor-phenyl)-[1,2,4]oxadiazol-5-yl]-vinyl}-cyclohexyl)-phenyl-methyl]-dimethyl-amin | 422,1 [M⁺+1]; 424,0 |
| 180. | [(4-{2-[3-(4-tert-Butyl-phenyl)-[1,2,4]oxadiazol-5-yl]-vinyl}-cyclohexyl)-phenyl-methyl]-dimethyl-amin (Polareres Diastereomer) | 444,3 [M⁺+1] |
| 181. | [(4-{2-[3-(4-tert-Butyl-phenyl)-[1,2,4]oxadiazol-5-yl]-vinyl}-cyclohexyl)-phenyl-methyl]-dimethyl-amin (Unpolareres Diastereomer) | 444,3 [M⁺+1] |
| 182. | Dimethyl-(phenyl-{4-[2-(3-m-tolyl-[1,2,4]oxadiazol-5-yl)-vinyl]-cyclohexyl}-methyl)-amin (Polareres Diastereomer) | 402,2 [M⁺+1] |
| 183. | Dimethyl-(phenyl-{4-[2-(3-m-tolyl-[1,2,4]oxadiazol-5-yl)-vinyl]-cyclohexyl}-methyl)-amin (Unpolareres Diastereomer) | 402,2 [M⁺+1] |
| 184. | [(4-{2-[3-(4-Chlor-phenyl)-[1,2,4]oxadiazol-5-yl]-vinyl}-cyclohexyl)-phenyl-methyl]-dimethyl-amin (Polareres Diastereomer) | 422,1 [M⁺+1]; 424,0 |
| 185. | [(4-{2-[3-(4-Chlor-phenyl)-[1,2,4]oxadiazol-5-yl]-vinyl}-cyclohexyl)-phenyl-methyl]-dimethyl-amin (Unpolareres Diastereomer) | 422,1 [M⁺+1]; 424,0 |
| 186. | [(4-Chlor-phenyl)-(4-{2-[3-(3-chlor-phenyl)-[1,2,4]oxadiazol-5-yl]-vinyl}-cyclohexyl)-methyl]-dimethyl-amin | 456,1 [M⁺+1]; 458,0 |
| 187. | [(4-{2-[3-(4-tert-Butyl-phenyl)-[1,2,4]oxadiazol-5-yl]-vinyl}-cyclohexyl)-(4-chlor-phenyl)-methyl]-dimethyl-amin (Polareres Diastereomer) | 478,2 [M⁺+1]; 480,0 |
| 188. | [(4-{2-[3-(4-tert-Butyl-phenyl)-[1,2,4]oxadiazol-5-yl]-vinyl}-cyclohexyl)-(4-chlor-phenyl)-methyl]-dimethyl-amin (Unpolareres Diastereomer) | 478,2 [M⁺+1]; 480,0 |
| 189. | ((4-Chlor-phenyl)-{4-[2-(3-m-tolyl-[1,2,4]oxadiazol-5-yl)-vinyl]-cyclohexyl}-methyl)-dimethyl-amin (Polareres Diastereomer) | 436,0 [M⁺+1]; 438,0 |
| 190. | ((4-Chlor-phenyl)-{4-[2-(3-m-tolyl-[1,2,4]oxadiazol-5-yl)-vinyl]-cyclohexyl}-methyl)-dimethyl-amin (Unpolareres Diastereomer) | 436,0 [M⁺+1]; 438,0 |
| 191. | [(4-Chlor-phenyl)-(4-{2-[3-(4-chlor-phenyl)-[1,2,4]oxadiazol-5-yl]-vinyl}-cyclohexyl)-methyl]-dimethyl-amin | 456,0 [M⁺+1]; 458,0 |
| 192. | [(4-{2-[3-(3-Chlor-phenyl)-[1,2,4]oxadiazol-5-yl]-vinyl}-cyclohexyl)-thiophen-2-yl-methyl]-dimethyl-amin | 428,0 [M⁺+1]; 430,0 |
| 193. | [(4-{2-[3-(4-tert-Butyl-phenyl)-[1,2,4]oxadiazol-5-yl]-vinyl}-cyclohexyl)-thiophen-2-yl-methyl]-dimethyl-amin (Polareres Diastereomer) | 450,3 [M⁺+1] |
| 194. | [(4-{2-[3-(4-tert-Butyl-phenyl)-[1,2,4]oxadiazol-5-yl]-vinyl}-cyclohexyl)-thiophen-2-yl-methyl]-dimethyl-amin (Unpolareres Diastereomer) | 450,3 [M⁺+1] |
| 195. | Dimethyl-(thiophen-2-yl-{4-[2-(3-m-tolyl-[1,2,4]oxadiazol-5-yl)-vinyl]-cyclohexyl}-methyl)-amin | 408,2 [M⁺+1] |
| 196. | [(4-{2-[3-(4-Chlor-phenyl)-[1,2,4]oxadiazol-5-yl]-vinyl}-cyclohexyl)-thiophen-2-yl-methyl]-dimethyl-amin | 428,0 [M⁺+1]; 430,0 |
| 197. | [(4-{2-[3-(3-Chlor-phenyl)-[1,2,4]oxadiazol-5-yl]-vinyl}-cyclohexyl)-(3-fluor-phenyl)-methyl]-dimethyl-amin | 440,0 [M⁺+1]; 442,0 |
| 198. | [(4-{2-[3-(4-tert-Butyl-phenyl)-[1,2,4]oxadiazol-5-yl]-vinyl}-cyclohexyl)-(3-fluor-phenyl)-methyl]-dimethyl-amin (Polareres Diastereomer) | 462,3 [M⁺+1] |
| 199. | [(4-{2-[3-(4-tert-Butyl-phenyl)-[1,2,4]oxadiazol-5-yl]-vinyl}-cyclohexyl)-(3-fluor-phenyl)-methyl]-dimethyl-amin (Unpolareres Diastereomer) | 462,3 [M⁺+1] |
| 201. | ((3-Fluor-phenyl)-{4-[2-(3-m-tolyl-[1,2,4]oxadiazol-5-yl)-vinyl]-cyclohexyl}-methyl)-dimethyl-amin | 420,2 [M⁺+1] |
| 202. | [(4-{2-[3-(4-Chlor-phenyl)-[1,2,4]oxadiazol-5-yl]-vinyl}-cyclohexyl)-(3-fluor-phenyl)-methyl]-dimethyl-amin | 440,0; 442,0 [M⁺+1] |
| 203. | [(4-{2-[3-(2,3-Dichlor-phenyl)-[1,2,4]oxadiazol-5-yl]-vinyl}-cyclohexyl)-(4-fluor-phenyl)-methyl]-dimethyl-amin | 474,1; 476,1 [M⁺+1] |
| 204. | [(4-Fluor-phenyl)-(4-{2-[3-(4-trifluormethoxy-phenyl)-[1,2,4]oxadiazol-5-yl]-vinyl}-cyclohexyl)-methyl]-dimethyl-amin | 490,2 [M⁺+1] |
| 205. | [(4-{2-[3-(3,4-Dimethoxy-benzyl)-[1,2,4]oxadiazol-5-yl]-vinyl}-cyclohexyl)-(4-fluor-phenyl)-methyl]-dimethyl-amin | 480,3 [M⁺+1] |
| 206. | [(4-{2-[3-(1,5-Dimethyl-1H-pyrrol-2-yl)-[1,2,4]oxadiazol-5-yl]-vinyl}-cyclohexyl)-(4-fluor-phenyl)-methyl]-dimethyl-amin | 423,2 [M⁺+1] |
| 207. | [1-(4-{2-[3-(2,3-Dichlor-phenyl)-[1,2,4]oxadiazol-5-yl]-vinyl}-cyclohexyl)-3-phenyl-propyl]-dimethyl-amin | 484,1 [M⁺+1]; 486,0 |
| 208. | Dimethyl-[3-phenyl-1-(4-{2-[3-(4-trifluormethoxy-phenyl)-[1,2,4]oxadiazol-5-yl]-vinyl}-cyclohexyl)-propyl]-amin (Polareres Diastereomer) | 500,2 [M⁺+1] |
| 209. | Dimethyl-[3-phenyl-1-(4-{2-[3-(4-trifluormethoxy-phenyl)-[1,2,4]oxadiazol-5-yl]-vinyl}-cyclohexyl)-propyl]-amin (Unpolareres Diastereomer) | 500,2 [M⁺+1] |
| 210. | [1-(4-{2-[3-(3,4-Dimethoxy-benzyl)-[1,2,4]oxadiazol-5-yl]-vinyl}-cyclohexyl)-3-phenyl-propyl]-dimethyl-amin (Polareres Diastereomer) | 490,3 [M⁺+1] |
| 211. | [1-(4-{2-[3-(3,4-Dimethoxy-benzyl)-[1,2,4]oxadiazol-5-yl)-vinyl}-cyclohexyl)-3-phenyl-propyl]-dimethyl-amin (Unpolareres Diastereomer) | 490,3 [M⁺+1] |
| 212. | [1-(4-{2-[3-(1,5-Dimethyl-1H-pyrrol-2-yl)-[1,2,4]oxadiazol-5-yl]-vinyl}-cyclohexyl)-3-phenyl-propyl]-dimethyl-amin | 433,3 [M⁺+1] |
| 213. | [(4-{2-[3-(2,3-Dichlor-phenyl)-[1,2,4]oxadiazol-5-yl]-vinyl}-cyclohexyl)-phenyl-methyl]-dimethyl-amin | 456,0 [M⁺+1]; 458,0 |
| 214. | [(4-{2-[3-(3,4-Dimethoxy-benzyl)-[1,2,4]oxadiazol-5-yl]-vinyl}-cyclohexyl)-phenyl-methyl]-dimethyl-amin (Polareres Diastereomer) | 462,3 [M⁺+1] |
| 215. | [(4-{2-[3-(3,4-Dimethoxy-benzyl)-[1,2,4]oxadiazol-5-yl]-vinyl}-cyclohexyl)-phenyl-methyl]-dimethyl-amin (Unpolareres Diastereomer) | 462,3 [M⁺+1] |
| 217. | [(4-{2-[3-(1,5-Dimethyl-1H-pyrrol-2-yl)-[1,2,4]oxadiazol-5-yl]-vinyl}-cyclohexyl)-phenyl-methyl]-dimethyl-amin | 405,3 [M⁺+1] |
| 218. | [(4-Chlor-phenyl)-(4-{2-[3-(2,3-dichlor-phenyl)-[1,2,4]oxadiazol-5-yl]-vinyl}-cyclohexyl)-methyl]-dimethyl-amin | 490,1 [M⁺+1]; 492,0; 494,0 |
| 219. | [(4-Chlor-phenyl)-(4-{2-[3-(4-trifluormethoxy-phenyl)-[1,2,4]oxadiazol-5-yl]-vinyl}-cyclohexyl)-methyl]-dimethyl-amin | 506,0 [M⁺+1]; 508,0 |
| 220. | [(4-Chlor-phenyl)-(4-{2-[3-(3,4-dimethoxy-benzyl)-[1,2,4]oxadiazol-5-yl]-vinyl}-cyclohexyl)-methyl]-dimethyl-amin | 496,1 [M⁺+1]; 498,0 |
| 221. | [(4-Chlor-phenyl)-(4-{2-[3-(1,5-dimethyl-1H-pyrrol-2-yl)-[1,2,4]oxadiazol-5-yl]-vinyl}-cyclohexyl)-methyl]-dimethyl-amin | 439,1 [M⁺+1]; 441,0 |
| 222. | [(4-{2-[3-(2,3-Dichlor-phenyl)-[1,2,4]oxadiazol-5-yl]-vinyl}-cyclohexyl)-thiophen-2-yl-methyl]-dimethyl-amin | 462,0 [M⁺+1]; 464,0; 466,0 |
| 223. | Dimethyl-[thiophen-2-yl-(4-{2-[3-(4-trifluormethoxy-phenyl)-[1,2,4]oxadiazol-5-yl]-vinyl}-cyclohexyl)-methyl]-amin | 478,2 [M⁺+1] |
| 224. | [(4-{2-[3-(1,5-Dimethyl-1H-pyrrol-2-yl)-[1,2,4]oxadiazol-5-yl]-vinyl}-cyclohexyl)-thiophen-2-yl-methyl]-dimethyl-amin | 411,2 [M⁺+1] |
| 225. | [(4-{2-[3-(2,3-Dichlor-phenyl)-[1,2,4]oxadiazol-5-yl]-vinyl}-cyclohexyl)-(3-fluor-phenyl)-methyl]-dimethyl-amin | 474,1 [M⁺+1]; 476,0; 478,0 |
| 226. | [(3-Fluor-phenyl)-(4-{2-[3-(4-trifluormethoxy-phenyl)-[1,2,4]oxadiazol-5-yl]-vinyl}-cyclohexyl)-methyl]-dimethyl-amin (Polareres Diastereomer) | 490,2 [M⁺+1] |
| 227. | [(3-Fluor-phenyl)-(4-{2-[3-(4-trifluormethoxy-phenyl)-[1,2,4]oxadiazol-5-yl]-vinyl}-cyclohexyl)-methyl]-dimethyl-amin (Unpolareres Diastereomer) | 490,2 [M⁺+1] |
| 228. | [(4-{2-[3-(3,4-Dimethoxy-benzyl)-[1,2,4joxadiazol-5-yl]-vinyl}-cyclohexyl)-(3-fluor-phenyl)-methyl]-dimethyl-amin (Polareres Diastereomer) | 480,3 [M⁺+1] |
| 229. | [(4-{2-[3-(3,4-Dimethoxy-benzyl)-[1,2,4]oxadiazol-5-yl]-vinyl}-cyclohexyl)-(3-fluor-phenyl)-methyl]-dimethyl-amin (Unpolareres Diastereomer) | 480,3 [M⁺+1] |
| 231. | [(4-{2-[3-(1,5-Dimethyl-1H-pyrrol-2-yl)-[1,2,4)oxadiazol-5-yl]-vinyl}-cyclohexyl)-(3-fluor-phenyl)-methyl]-dimethyl-amin | 423,2 [M⁺+1] |
| 232. | {(3-Fluor-phenyl)-[4-(3-phenyl-[1,2,4]oxadiazol-5-ylmethyl)-cyclohexyl]-methyl}-dimethyl-amin | 394,2 [M⁺+1] |
| 233. | {(4-Fluor-phenyl)-[4-(3-phenyl-[1,2,4]oxadiazol-5-ylmethyl)-cyclohexyl]-methyl}-dimethyl-amin | 394,2 [M⁺+1] |
| 234. | Dimethyl-{phenyl-[4-(3-phenyl-[1,2,4]oxadiazol-5-ylmethyl)-cyclohexyl)-methyl}-amin | 376,2 [M⁺+1] |
| 235. | [{4-[3-(2,4-Difluor-phenyl)-[1,2,4]oxadiazol-5-ylmethyl]-cyclohexyl}-(3-fluor-phenyl)-methyl]-dimethyl-amin | 430,2 [M⁺+1] |
| 236. | [{4-[3-(2,4-Difluor-phenyl)-[1,2,4]oxadiazol-5-ylmethyl]-cyclohexyl}-(4-fluor-phenyl)-methyl]-dimethyl-amin | 430,2 [M⁺+1] |
| 237. | ((3-Fluor-phenyl)-{4-[3-(4-methoxy-benzyl)-[1,2,4]oxadiazol-5-ylmethyl]-cyclohexyl}-methyl)-dimethyl-amin | 438,2 [M⁺+1] |
| 238. | ((4-Fluor-phenyl)-{4-[3-(4-methoxy-benzyl)-[1,2,4]oxadiazol-5-ylmethyl]-cyclohexyl}-methyl)-dimethyl-amin | 438,2 [M⁺+1] |
| 239. | ({4-[3-(4-Methoxy-benzyl)-[1,2,4]oxadiazol-5-ylmethyl]-cyclohexyl}-phenyl-methyl)-dimethyl-amin | 420,3 [M⁺+1] |
| 240. | [{4-[3-(4-Chlor-benzyl)-[1,2,4]oxadiazol-5-ylmethyl]-cyclohexyl}-(3-fluor-phenyl)-methyl]-dimethyl-amin | 442,1 [M⁺+1]; 444,0 |
| 241. | [{4-[3-(4-Chlor-benzyl)-[1,2,4]oxadiazol-5-ylmethyl]-cyclohexyl}-(4-fluor-phenyl)-methyl]-dimethyl-amin | 442,1 [M⁺+1]; 444,0 |
| 242. | [{4-[3-(3-Chlor-phenyl)-[1,2,4]oxadiazol-5-ylmethylj-cyclohexyl}-(4-fluor-phenyl)-methyl]-dimethyl-amin | 428,0 [M⁺+1]; 430,0 |
| 243. | ({4-[3-(3-Chlor-phenyl)-[1,2,4]oxadiazol-5-ylmethyl]-cyclohexyl}-phenyl-methyl)-dimethyl-amin | 410,1 [M⁺+1]; 412,0 |
| 244. | [{4-[3-(2,3-Dichlor-phenyl)-[1,2,4]oxadiazol-5-ylmethyl]-cyclohexyl}-(3-fluor-phenyl)-methyl]-dimethyl-amin | 462,1 [M⁺+1]; 464,0 |
| 245. | [{4-[3-(2,3-Dichlor-phenyl)-[1,2,4]oxadiazol-5-ylmethyl]-cyclohexyl}-(4-fluor-phenyl)-methyl]-dimethyl-amin | 462,1 [M⁺+1]; 464,0; 466,0 |
| 246. | ({4-[3-(2,3-Dichlor-phenyl)-[1,2.4]oxadiazol-5-ylmethyl]-cyclohexyl}-phenyl-methyl)-dimethyl-amin | 444,0 [M⁺+1]; 446,0; 448,0 |
| 247. | {(3-Fluor-phenyl)-(4-(3-p-tolyl-[1,2,4]oxadiazol-5-ylmethyl)-cyclohexyl]-methyl}-dimethyl-amin | 408,2 [M⁺+1] |
| 248. | {(4-Fluor-phenyl)-[4-(3-p-tolyl-[1,2,4]oxadiazol-5-ylmethyl)-cyclohexyl]-methyl}dimethyl-amin | 408,2 [M⁺+1] |
| 249. | Dimethyl-{phenyl-[4-(3-p-tolyl-[1,2,4]oxadiazol-5-ylmethyl)-cyclohexyl]-methyl}-amin | 390,2 [M⁺+1] |
| 250. | [{4-[3-(3,4-Dimethoxy-phenyl)-[1,2,4]oxadiazol-5-ylmethyl]-cyclohexyl}-(3-fluor-phenyl)-methyl]-dimethyl-amin | 454,2 [M⁺+1] |
| 251. | [{4-[3-(3,4-Dimethoxy-phenyl)-[1,2,4]oxadiazol-5-ylmethyl]-cyclohexyl)-(4-fluor-phenyl)-methyl]-dimethyl-amin | 454,2 [M⁺+1] |
| 252. | ({4-[3-(3,4-Dimethoxy-phenyl)-[1,2,4]oxadiazol-5-ylmethyl]-cyclohexyl}-phenyl-methyl)-dimethyl-amin | 436,3 [M⁺+1] |
| 253. | [{4-[3-(4-tert-Butyl-phenyl)-[1,2,4]oxadiazol-5-ylmethyl]-cyclohexyl}-(3-fluor-phenyl)-methyl]-dimethyl-amin | 450,3 [M⁺+1] |
| 254. | [{4-[3-(4-tert-Butyl-phenyl)-[1,2,4]oxadiazol-5-ylmethyl]-cyclohexyl}-(4-fluor-phenyl)-methyl]-dimethyl-amin | 450,3 [M⁺+1] |
| 255. | ({4-[3-(4-tert-Buty)-phenyl)-[1,2,4]oxadiazol-5-ylmethyl]-cyclohexyl}-phenyl-methyl)-dimethyl-amin | 432,3 [M⁺+1] |
| 256. | {(3-Fluor-phenyl)-[4-(3-m-tolyl-[1,2,4]oxadiazol-5-ylmethyl)-cyclohexyl]-methyl}-dimethyl-amin | 408,2 [M⁺+1] |
| 257. | {(4-Fluor-phenyl)-[4-(3-m-tolyl-[1,2,4]oxadiazol-5-ylmethyl)-cyclohexyl]-methyl}-dimethyl-amin | 408,2 [M⁺+1] |
| 258. | Dimethyl-{phenyl-[4-(3-m-tolyl-[1,2,4]oxadiazol-5-ylmethyl)-cyclohexyl]-methyl}-amin | 390,2 [M⁺+1] |
| 259. | [{4-[3-(4-Chlor-phenyl)-[1,2,4]oxadiazol-5-ylmethyl]-cyclohexyl}-(3-fluor-phenyl)-methyl]-dimethyl-amin | 428,1 [M⁺+1]; 430,0 |
| 260. | ({4-[3-(4-Chlor-phenyl)-[1,2,4]oxadiazol-5-ylmethyl]-cyclohexyl}-phenyl-methyl)-dimethyl-amin | 410,1 [M⁺+1]; 412,0 |
| 261. | ((3-Fluor-phenyl)-{4-[3-(4-trifluormethoxy-phenyl)-[1,2,4]oxadiazol-5-ylmethyl]-cyclohexyl}-methyl)-dimethyl-amin | 478,2 [M⁺+1] |
| 262. | ((4-Fluor-phenyl)-{4-[3-(4-trifluormethoxy-phenyl)-[1,2,4]oxadiazol-5-ylmethyl]-cyclohexyl}-methyl)-dimethyl-amin | 478,2 [M⁺+1] |
| 263. | Dimethyl-(phenyl-{4-[3-(4-trifluormethoxy-phenyl)-[1,2,4]oxadiazol-5-ylmethyl]-cyclohexyl}-methyl)-amin | 460,2 [M⁺+1] |
| 264. | ((4-Fluor-phenyl)-{4-[2-(3-phenyl-[1,2,4]oxadiazol-5-yl)-ethyl]-cyclohexyl}-methyl)-dimethyl-amin | 408,2 [M⁺+1] |
| 265. | Dimethyl-(phenyl-{4-[2-(3-phenyl-[1,2,4]oxadiazol-5-yl)-ethyl]-cyclohexyl}-methyl)-amin | 390,2 [M⁺+1] |
| 266. | Dimethyl-(3-phenyl-1-{4-[2-(3-phenyl-[1,2,4]oxadiazol-5-yl)-ethyl]-cyclohexyl}-propyl)-amin | 418,3 [M⁺+1] |
| 267. | ((3-Fluor-phenyl)-{4-[2-(3-phenyl-[1,2,4]oxadiazol-5-yl)-ethyl]-cyclohexyl}-methyl)-dimethyl-amin | 408,2 [M⁺+1] |
| 268. | [(4-Fluor-phenyl)-(4-{2-[3-(4-methoxy-benzyl)-[1,2,4]oxadiazol-5-yl]-ethyl}-cyclohexyl)-methyl]-dimethyl-amin | 452,3 (M⁺+1)] |
| 269. | [(4-{2-[3-(4-Methoxy-benzyl)-[1,2,4]oxadiazol-5-yl]-ethyl}-cyclohexyl)-phenyl-methyl]-dimethyl-amin | 434,3 [M⁺+1] |
| 270. | [1-(4-{2-[3-(4-Methoxy-benzyl)-[1,2,4]oxadiazol-5-yl]-ethyl}-cyclohexyl)-3-phenyl-propyl]-dimethyl-amin | 462,3 [M⁺+1] |
| 271. | [(3-Fluor-phenyl)-(4-{2-[3-(4-methoxy-benzyl)-[1,2,4]oxadiazol-5-yl]-ethyl}-cyclohexyl)-methyl]-dimethyl-amin | 452,3 [M⁺+1] |
| 272. | [(4-{2-[3-(4-Chlor-benzyl)-[1,2,4]oxadiazol-5-yl]-ethyl}-cyclohexyl)-(4-fluor-phenyl)-methyl]-dimethyl-amin | 456,1 [M⁺+1]; 458,0 |
| 273. | [(4-{2-[3-(4-Chlor-benzyl)-[1,2,4]oxadiazol-5-yl]-ethyl}-cyclohexyl)-phenyl-methyl]-dimethyl-amin | 438,1 [M⁺+1]; 440,1 |
| 274. | [1-(4-{2-[3-(4-Chlor-benzyl)-[1,2,4]oxadiazol-5-yl]-ethyl}-cyclohexyl)-3-phenyl-propyl]-dimethyl-amin | 466,1 [M⁺+1]; 468,1 |
| 275. | [(4-{2-[3-(4-Chlor-benzyl)-[1,2,4]oxadiazol-5-yl]-ethyl}-cyclohexyl)-(3-fluor-phenyl)-methyl]-dimethyl-amin | 456,1 [M⁺+1]; 458,1 |
| 276. | [(4-{2-[3-(3-Chlor-phenyl)-[1,2,4]oxadiazol-5-yl]-ethyl}-cyclohexyl)-(4-fluor-phenyl)-methyl]-dimethyl-amin | 442,1 [M⁺+1]; 444,1 |
| 277. | [(4-{2-[3-(3-Chlor-phenyl)-[1,2,4]oxadiazol-5-yl]-ethyl}-cyclohexyl)-phenyl-methyl]-dimethyl-amin | 424,1 [M⁺+1]; 426,1 |
| 278. | [1-(4-{2-[3-(3-Chlor-phenyl)-[1,2,4]oxadiazol-5-yl]-ethyl}-cyclohexyl)-3-phenyl-propyl]-dimethyl-amin | 452,1 [M⁺+1]; 454,1 |
| 279. | [(4-{2-[3-(3-Chlor-phenyl)-[1,2,4]oxadiazol-5-yl]-ethyl}-cyclohexyl)-(3-fluor-phenyl)-methyl]-dimethyl-amin | 442,1 [M⁺+1]; 444,0 |
| 280. | [(4-{2-[3-(2.4-Difluor-phenyl)-[1,2,4]oxadiazol-5-yl]-ethyl}-cyclohexyl)-(4-fluor-phenyl)-methyl]-dimethyl-amin | 444,2 [M⁺+1] |
| 281. | [(4-{2-[3-(2,4-Difluor-phenyl)-[1,2,4]oxadiazo)-5-yl]-ethyl}-cyclohexyl)-phenyl-methyl]-dimethyl-amin | 426,2 [M⁺+1] |
| 282. | [(4-{2-[3-(2,4-Difluor-phenyl)-[1,2,4]oxadiazol-5-yl]-ethyl}-cyclohexyl)-(3-fluor-phenyl)-methyl]-dimethyl-amin | 444,2 [M⁺+1] |
| 283. | [(4-{2-[3-(3,4-Dimethoxy-phenyl)-[1,2,4]oxadiazol-5-yl]-ethyl}-cyclohexyl)-(4-fluor-phenyl)-methyl]-dimethyl-amin | 468,3 [M⁺+1] |
| 284. | [(4-{2-[3-(3,4-Dimethoxy-phenyl)-[1,2,4]oxadiazol-5-yl]-ethyl}-cyclohexyl)-phenyl-methyl]-dimethyl-amin | 450,3 [M⁺+1] |
| 285. | [1-(4-{2-[3-(3,4-Dimethoxy-phenyl)-[1,2,4]oxadiazol-5-yl]-ethyl}-cyclohexyl)-3-phenyl-propyl]-dimethyl-amin | 478,3 [M⁺+1] |
| 286. | [(4-{2-[3-(3,4-Dimethoxy-phenyl)-[1,2,4]oxadiazol-5-yl]-ethyl}-cyclohexyl)-(3-fluor-phenyl)-methyl]-dimethyl-amin | 468,3 [M⁺+1] |
| 287. | ((4-Fluor-phenyl)-{4-[2-(3-p-tolyl-[1,2,4]oxadiazol-5-yl)-ethyl]-cyclohexyl}-methyl)-dimethyl-amin | 422,3 [M⁺+1] |
| 288. | Dimethyl-(phenyl-{4-[2-(3-p-tolyl-[1,2,4]oxadiazol-5-yl)-ethyl]-cyclohexyl}-methyl)-amin | 404,3 [M⁺+1] |
| 289. | Dimethyl-(3-phenyl-1-{4-[2-(3-p-tolyl-[1,2,4]oxadiazol-5-yl)-ethyl]-cyclohexyl}-propyl)-amin | 432,3 [M⁺+1] |
| 290. | ((3-Fluor-phenyl)-{4-[2-(3-p-tolyl-[1,2,4]oxadiazol-5-yl)-ethyl]-cyclohexyl}-methyl)-dimethyl-amin | 422,3 [M⁺+1] |
| 291. | [(4-{2-[3-(4-tert-Butyl-phenyl)-[1,2,4]oxadiazol-5-yl]-ethyl}-cyclohexyl)-(4-fluor-phenyl)-methyl]-dimethyl-amin | 464,3 [M⁺+1] |
| 292. | [(4-{2-[3-(4-tert-Butyl-phenyl)-[1,2,4]oxadiazol-5-yl]-ethyl}-cyclohexyl)-phenyl-methyl]-dimethyl-amin | 446,3 [M⁺+1] |
| 293. | [1-(4-{2-[3-(4-tert-Butyl-phenyl)-[1,2,4]oxadiazol-5-yl]-ethyl}-cyclohexyl)-3-phenyl-propyl]-dimethyl-amin | 474,3 [M⁺+1] |
| 294. | [(4-{2-[3-(4-tert-Butyl-phenyl)-[1,2,4]oxadiazol-5-yl]-ethyl}-cyclohexyl)-(3-fluor-phenyl)-methyl]-dimethyl-amin | 464,3 [M⁺+1] |
| 295. | ((4-Fluor-phenyl)-{4-[2-(3-m-tolyl-[1,2,4]oxadiazol-5-yl)-ethyl]-cyclohexyl}-methyl)-dimethyl-amin | 422,3 [M⁺+1] |
| 296. | Dimethyl-(phenyl-{4-[2-(3-m-tolyl-[1,2,4]oxadiazol-5-yl)-ethyl]-cyclohexyl}-methyl)-amin | 404,3 [M⁺+1] |
| 297. | Dimethyl-(3-phenyl-1-{4-[2-(3-m-tolyl-[1,2,4]oxadiazol-5-yl)-ethyl]-cyclohexyl}-propyl)-amin | 432,3 [M⁺+1] |
| 298. | ((3-Fluor-phenyl)-{4-[2-(3-m-tolyl-[1,2,4]oxadiazol-5-yl)-ethyl]-cyclohexyl}-methyl)-dimethyl-amin | 422,3 [M⁺+1] |
| 299. | [(4-{2-[3-(4-Chlor-phenyl)-[1,2,4]oxadiazol-5-yl]-ethyl}-cyclohexyl)-(4-fluor-phenyl)-methyl]-dimethyl-amin | 442,1 [M⁺+1]; 444,0 |
| 300. | [(4-{2-[3-(4-Chlor-phenyl)-[1,2,4]oxadiazol-5-yl]-ethyl}-cyclohexyl)-phenyl-methyl]-dimethyl-amin | 424,1 [M⁺+1]; 426,0 |
| 301. | [1-(4-{2-[3-(4-Chlor-phenyl)-[1,2,4]oxadiazol-5-yl]-ethyl}-cyclohexyl)-3-phenyl-propyl]-dimethyl-amin | 452,1 [M⁺+1]; 454,1 |
| 302. | [(4-{2-[3-(4-Chlor-phenyl)-[1,2,4]oxadiazol-5-yl]-ethyl}-cyclohexyl)-(3-fluor-phenyl)-methyl]-dimethyl-amin | 442,0 [M⁺+1]; 444,0 |
| 303. | [(4-{2-[3-(2,3-Dichlor-phenyl)-[1,2,4]oxadiazol-5-yl]-ethyl}-cyclohexyl)-(4-fluor-phenyl)-methyl]-dimethyl-amin | 476,0 [M⁺+1]; 478,0; 480,0 |
| 304. | [(4-{2-[3-(2,3-Dichlor-phenyl)-[1,2,4]oxadiazol-5-yl]-ethyl}-cyclohexyl)-phenyl-methyl]-dimethyl-amin | 458,0 [M⁺+1]; 460,0; 462,0 |
| 305. | [1-(4-{2-[3-(2,3-Dichlor-phenyl)-[1,2,4]oxadiazol-5-yl]-ethyl}-cyclohexyl)-3-phenyl-propyl]-dimethyl-amin | 486,0 [M⁺+1]; 488,0 |
| 306. | [(4-{2-[3-(2,3-Dichlor-phenyl)-[1,2,4]oxadiazol-5-yl]-ethyl}-cyclohexyl)-(3-fluor-phenyl)-methyl]-dimethyl-amin | 476,0 [M⁺+1]; 478,0 |
| 307. | [(4-Fluor-phenyl)-(4-{2-[3-(4-trifluormethoxy-phenyl)-[1,2,4]oxadiazol-5-yl]-ethyl}-cyclohexyl)-methyl]-dimethyl-amin | 492,2 [M⁺+1] |
| 308. | Dimethyl-[phenyl-(4-{2-[3-(4-trifluormethoxy-phenyl)-[1,2,4]oxadiazol-5-yl]-ethyl}-cyclohexyl)-methyl]-amin | 474,2 [M⁺+1] |
| 309. | Dimethyl-[3-phenyl-1-(4-{2-[3-(4-trifluormethoxy-phenyl)-[1,2,4]oxadiazol-5-yl]-ethyl}-cyclohexyl)-propyl]-amin | 502,3 [M⁺+1] |
| 310. | [(3-Fluor-phenyl)-(4-{2-[3-(4-trifluormethoxy-phenyl)-[1,2,4]oxadiazol-5-yl]-ethyl}-cyclohexyl)-methyl]-dimethyl-amin | 492,2 [M⁺+1] |
| 311 | (4-((3-(4-Fluorobenzyl)-1,2,4-oxadiazol-5-yl)methyl)cyclohexyl)(4-fluorophenyl)-N,N-dimethylmethanamin | 426,2 [M⁺+1] |
| 312 | (4-((3-(4-Fluorobenzyl)-1,2,4-oxadiazol-5-yl)methyl)cyclohexyl)-N,N-dimethyl(phenyl)methanamin | 408,2 [M⁺+1] |
| 313 | (4-((3-(3-Chlorobenzyl)-1,2,4-oxadiazol-5-yl)methyl)cyclohexyl)(3-fluorophenyl)-N,N-dimethylmethanamin | 442,1 [M⁺+1]; 444,2 |
| 314 | (4-((3-(3-Chlorobenzyl)-1,2,4-oxadiazol-5-yl)methyl)cyclohexyl)(4-fluorophenyl)-N,N-dimethylmethanamin | 442,1 [M⁺+1]; 444,2 |
| 315 | (4-((3-(3-Chlorobenzyl)-1,2,4-oxadiazol-5-yl)methyl)cyclohexyl)-N,N-dimethyl(phenyl)methanamin | 424,1 [M⁺+1]; 426,2 |
| 316 | (4-((3-(3-Bromophenyl)-1,2,4-oxadiazol-5-yl)methyl)cyclohexyl)(3-fluorophenyl)-N,N-dimethylmethanamin | 472,1 [M⁺+1]; 474,1 |
| 317 | (4-((3-(3-Bromophenyl)-1,2,4-oxadiazol-5-yl)methyl)cyclohexyl)(4-fluorophenyl)-N,N-dimethylmethanamin | 472,1 [M⁺+1]; 474,1 |
| 318 | (4-((3-(3-Bromophenyl)-1,2,4-oxadiazol-5-yl)methyl)cyclohexyl)-N,N-dimethyl(phenyl)methanamin | 454,1 [M⁺+1]; 456,1 |
| 319 | (4-((3-(4-Bromobenzyl)-1,2,4-oxadiazol-5-yl)methyl)cyclohexyl)(3-fluorophenyl)-N,N-dimethylmethanamin | 486,1 [M⁺+1]; 488,1 |
| 320 | (4-((3-(4-Bromobenzyl)-1,2,4-oxadiazol-5-yl)methyl)cyclohexyl)(4-fluorophenyl)-N,N-dimethylmethanamin | 486,1 [M⁺+1]; 488,1 |
| 321 | (4-((3-(4-Bromobenzyl)-1,2,4-oxadiazol-5-yl)methyl)cyclohexyl)-N,N-dimethyl(phenyl)methanamin | 468,1 [M⁺+1]; 470,1 |
| 322 | (3-Fluorophenyl)-N,N-dimethyl(4-((3-(thiophen-2-ylmethyl)-1,2,4-oxadiazol-5-yl)methyl)cyclohexyl)methanamin | 414,2 [M⁺+1] |
| 323 | (4-Fluorophenyl)-N,N-dimethyl(4-((3-(thiophen-2-ylmethyl)-1,2,4-oxadiazol-5-yl)methyl)cyclohexyl)methanamin | 414,2 [M⁺+1] |
| 324 | N,N-Dimethyl(phenyl)(4-((3-(thiophen-2-ylmethyl)-1,2,4-oxadiazol-5-yl)methyl)cyclohexyl)methanamin | 396,2 [M⁺+1] |
| 325 | (4-((3-Benzyl-1,2,4-oxadiazol-5-yl)methyl)cyclohexyl)(3-fluorophenyl)-N,N-dimethylmethanamin | 408,2 [M⁺+1] |
| 326 | (4-((3-Benzyl-1,2,4-oxadiazol-5-yl)methyl)cyclohexyl)(4-fluorophenyl)-N,N-dimethylmethanamin | 408,2 [M⁺+1] |
| 327 | (4-((3-Benzyl-1,2,4-oxadiazol-5-yl)methyl)cyclohexyl)-N,N-dimethyl(phenyl)methanamin | 390,2 [M⁺+1] |
| 328 | (3-Fluorophenyl)-N, N-dimethyl(4-((3-phenethyl-1,2,4-oxadiazol-5-yl)methyl)cyclohexyl)methanamin | 422,3 [M⁺+1] |
| 329 | (4-Fluorophenyl)-N,N-dimethyl(4-((3-phenethyl-1,2,4-oxadiazol-5-yl)methyl)cyclohexyl)methanamin | 422,3 [M⁺+1] |
| 330 | N,N-Dimethyl(4-((3-phenethyl-1,2,4-oxadiazol-5-yl)methyl)cyclohexyl)(phenyl)methanamin | 404,3 [M⁺+1] |
| 331 | (4-((3-((1H-Indol-3-yl)methyl)-1,2,4-oxadiazol-5-yl)methyl)cyclohexyl)(3-fluorophenyl)-N,N-dimethylmethanamin | 447,2 [M⁺+1] |

### Trennung der Diastereomeren

Bei den Synthesen entstanden Diastereomeren, bei denen es sich insbesondere um E/Z-Isomeren handelte. In den Fällen, in denen Diastereomere getrennt wurden, wurde die Trennung nach der folgenden Methode durchgeführt:

An einer HPLC-Säule VP 100/21 Nucleodur C 18 (5µm), 100 mm, 21 mm Innendurchmesser von Macherey-Nagel wurde mit Hilfe einer Waters 600 HPLC-Pumpe/mobile Phase Wasser/Methanol bei einem Starteluenten von 20-50% Wasser bei 25°C und einem Fluss von 20 ml/min das Rohprodukt aufgetragen. Innerhalb von 8-12 min wurde der Methanol-Anteil des Eluenten kontinuierlich auf 100% erhöht. Detektiert wurde mit einem Waters 2487 UV Detektor bei 220 und 254 nm sowie ES-MS. Die getrennten Fraktionen wurden gesammelt, eingeengt und mit Hilfe von ES Massenspektroskopie analysiert. In der vorliegenden Erfindung wurden die Beispielverbindungen, die in der ersten Fraktion eluiert wurden, als "polareres Diastereomer" und in der zweiten Fraktion als "unpolareres Diastereomer" bezeichnet.

**Die beispielhaft genannten Verbindungen haben das folgende Substitutionsmuster (R³, R⁴ = CH₃):**

| Nr. | X | R² | R¹ |
|---|---|---|---|
| 45 | CH | 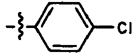 | 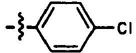 |
| 46 | CH | 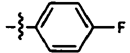 | 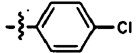 |
| 47 | CH | 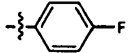 | 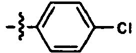 |
| 48 | CH | 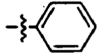 | 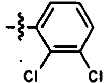 |
| 49 | CH | 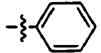 | 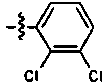 |
| 50 | CH | 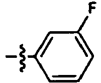 | 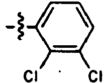 |
| 51 | CH | 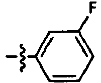 | 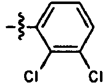 |
| 52 | CH | 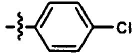 | 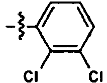 |
| 53 | CH | 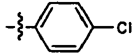 | 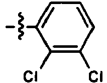 |
| 54 | CH | 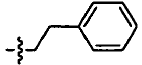 | 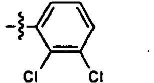 |
| 55 | CH | 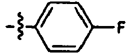 | 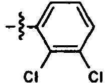 |
| 56 | CH | 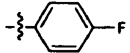 | 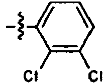 |
| 57 | CH | 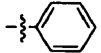 | 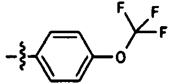 |
| 58 | CH | 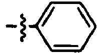 | 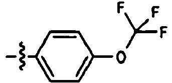 |
| 59 | CH | 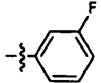 | 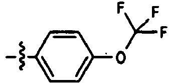 |
| 60 | CH | 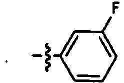 | 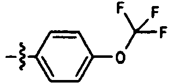 |
| 61 | CH | 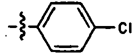 | 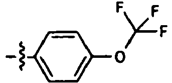 |
| 62 | CH | 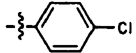 | 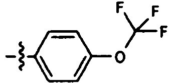 |
| 63 | CH | 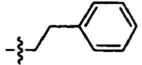 | 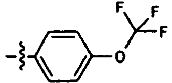 |
| 64 | CH | 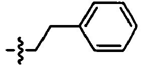 | 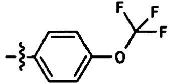 |
| 65 | CH | 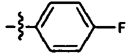 | 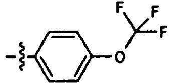 |
| 66 | CH | 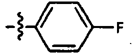 | 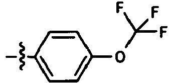 |
| 67 | CH | 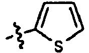 | 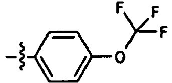 |
| 68 | CH | 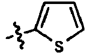 | 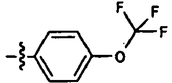 |
| 69 | CH | 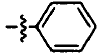 | 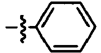 |
| 70 | CH | 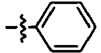 | 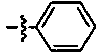 |
| 71 | CH | 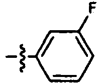 | 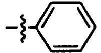 |
| 72 | CH | 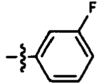 | 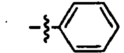 |
| 73 | CH | 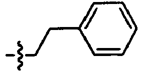 | 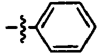 |
| 74 | CH | 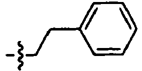 | 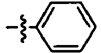 |
| 75 | CH | 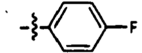 | 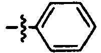 |
| 76 | CH | 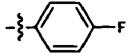 | 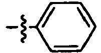 |
| 77 | CH | 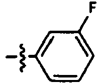 | 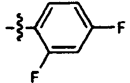 |
| 78 | CH | 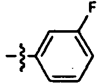 | 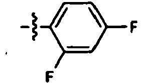 |
| 79 | CH | 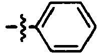 | 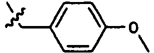 |
| 80 | CH | 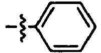 | 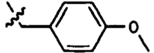 |
| 81 | CH | 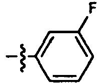 | 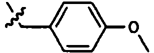 |
| 82 | CH | 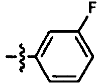 | 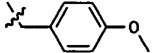 |
| 83 | CH | 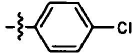 | 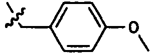 |
| 84 | CH | 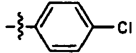 | 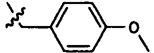 |
| 85 | CH | 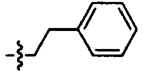 | 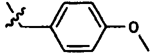 |
| 86 | CH | 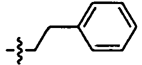 | 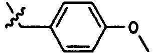 |
| 87 | CH | 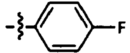 | 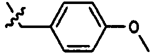 |
| 88 | CH | 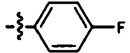 | 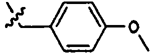 |
| 89 | CH | 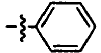 | 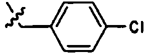 |
| 90 | CH | 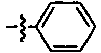 | 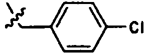 |
| 91 | CH | 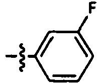 | 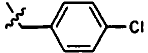 |
| 92 | CH | 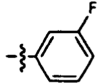 | 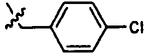 |
| 93 | CH | 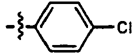 | 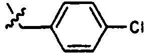 |
| 94 | CH | 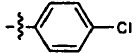 | 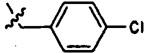 |
| 95 | CH | 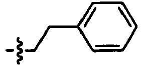 | 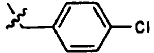 |
| 96 | CH | 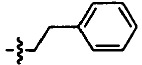 | 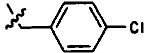 |
| 97 | CH | 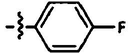 | 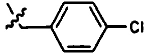 |
| 98 | CH | 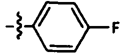 | 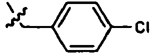 |
| 99 | CH | 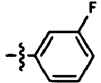 | 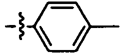 |
| 100 | CH | 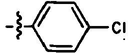 | 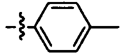 |
| 101 | CH | 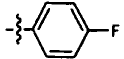 | 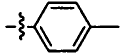 |
| 102 | CH | 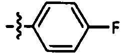 | 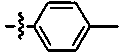 |
| 103 | CH | 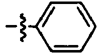 | 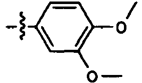 |
| 104 | CH | 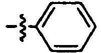 | 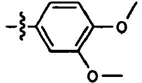 |
| 105 | CH | 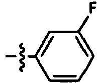 | 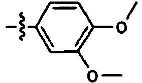 |
| 106 | CH | 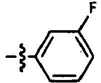 | 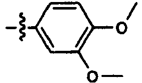 |
| 107 | CH | 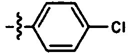 | 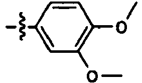 |
| 108 | CH | 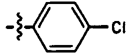 | 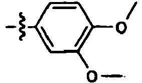 |
| 109 | CH | 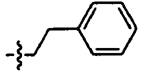 | 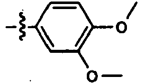 |
| 110 | CH | 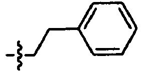 | 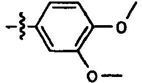 |
| 111 | CH | 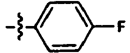 | 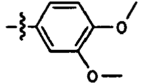 |
| 112 | CH | 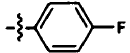 | 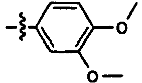 |
| 113 | CH | 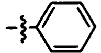 | 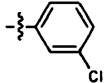 |
| 114 | CH | 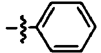 | 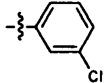 |
| 115 | CH | 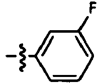 | 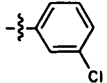 |
| 116 | CH | 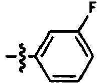 | 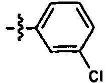 |
| 117 | CH | 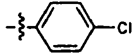 | 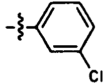 |
| 118 | CH | 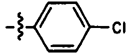 | 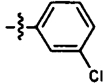 |
| 119 | CH | 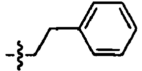 | 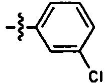 |
| 120 | CH | 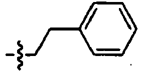 | 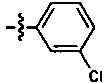 |
| 121 | CH | 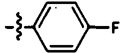 | 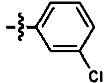 |
| 122 | CH | 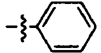 | 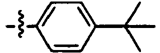 |
| 123 | CH | 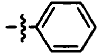 | 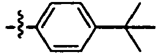 |
| 124 | CH | 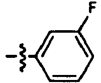 | 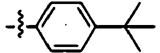 |
| 125 | CH | 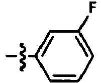 | 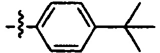 |
| 126 | CH | 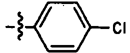 | 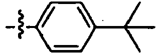 |
| 127 | CH | 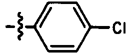 | 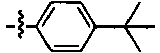 |
| 128 | CH | 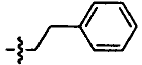 | 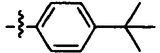 |
| 129 | CH | 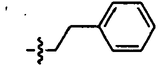 | 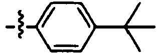 |
| 130 | CH | 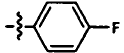 | 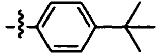 |
| 131 | CH | 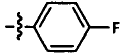 | 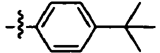 |
| 132 | CH | 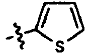 | 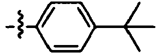 |
| 133 | CH | 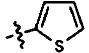 | 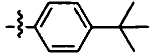 |
| 134 | CH | 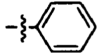 | 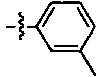 |
| 135 | CH | 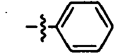 | 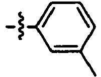 |
| 136 | CH | 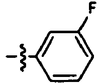 | 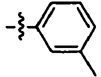 |
| 137 | CH | 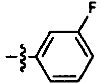 | 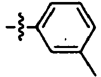 |
| 138 | CH | 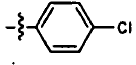 | 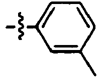 |
| 139 | CH | 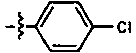 | 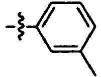 |
| 140 | CH=CH | 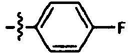 | 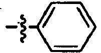 |
| 141 | CH=CH | 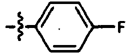 | 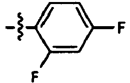 |
| 142 | CH=CH | 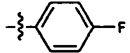 | 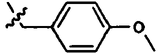 |
| 143 | CH=CH | 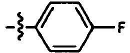 | 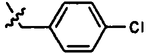 |
| 144 | CH=CH | 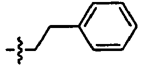 | 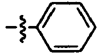 |
| 145 | CH=CH | 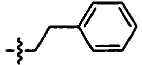 | 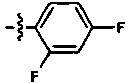 |
| 146 | CH=CH | 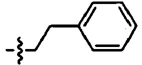 | 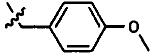 |
| 147 | CH=CH | 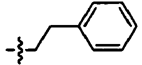 | 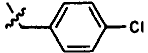 |
| 148 | CH=CH | 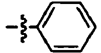 | 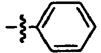 |
| 149 | CH=CH | 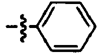 | 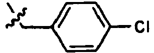 |
| 150 | CH=CH | 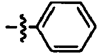 | 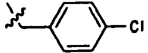 |
| 151 | CH=CH | 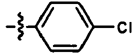 | 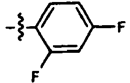 |
| 152 | CH=CH | 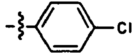 | 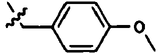 |
| 153 | CH=CH | 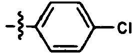 | 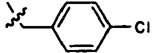 |
| 154 | CH=CH | 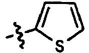 | 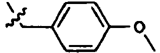 |
| 155 | CH=CH | 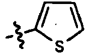 | 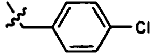 |
| 156 | CH=CH | 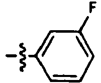 | 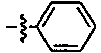 |
| 157 | CH=CH | 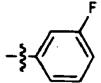 | 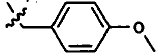 |
| 158 | CH=CH | 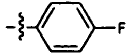 | 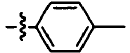 |
| 159 | CH=CH | 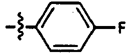 | 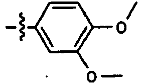 |
| 160 | CH=CH | 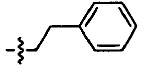 | 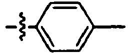 |
| 161 | CH=CH | 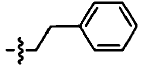 | 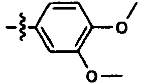 |
| 162 | CH=CH | 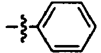 | 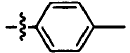 |
| 163 | CH=CH | 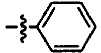 | 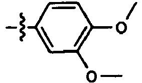 |
| 164 | CH=CH | 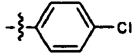 | 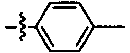 |
| 165 | CH=CH | 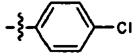 | 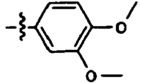 |
| 166 | CH=CH | 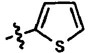 | 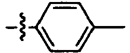 |
| 167 | CH=CH | 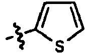 | 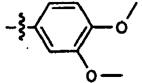 |
| 168 | CH=CH | 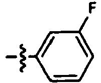 | 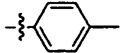 |
| 169 | CH=CH | 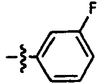 | 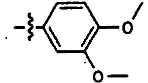 |
| 170 | CH=CH | 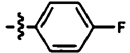 | 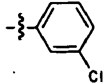 |
| 171 | CH=CH | 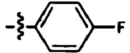 | 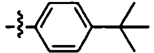 |
| 172 | CH=CH | 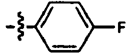 | 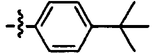 |
| 173 | CH=CH | 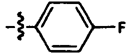 | 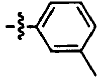 |
| 174 | CH=CH | 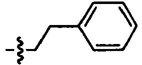 | 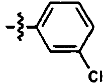 |
| 175 | CH=CH | 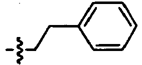 | 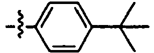 |
| 176 | CH=CH | 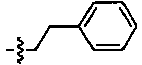 | 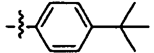 |
| 177 | CH=CH | 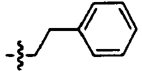 | 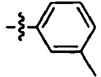 |
| 178 | CH=CH | 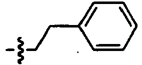 | 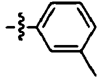 |
| 179 | CH=CH | 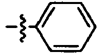 | 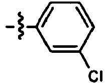 |
| 180 | CH=CH | 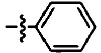 | 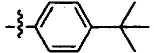 |
| 181 | CH=CH | 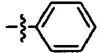 | 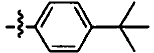 |
| 182 | CH=CH | 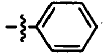 | 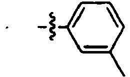 |
| 183 | CH=CH | 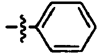 | 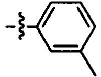 |
| 184 | CH=CH | 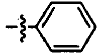 | 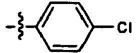 |
| 185 | CH=CH | 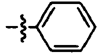 | 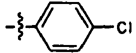 |
| 186 | CH=CH | 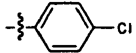 | 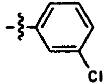 |
| 187 | CH=CH | 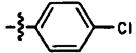 | 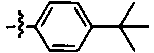 |
| 188 | CH=CH | 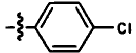 | 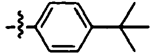 |
| 189 | CH=CH | 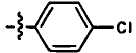 | 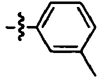 |
| 190 | CH=CH | 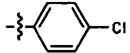 | 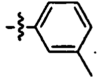 |
| 191 | CH=CH | 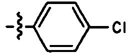 | 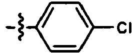 |
| 192 | CH=CH | 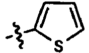 | 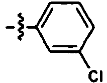 |
| 193 | CH=CH | 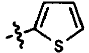 | 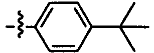 |
| 194 | CH=CH | 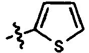 | 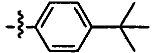 |
| 195 | CH=CH | 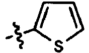 | 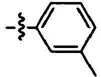 |
| 196 | CH=CH | 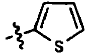 | 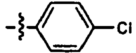 |
| 197 | CH=CH | 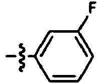 | 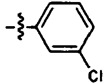 |
| 198 | CH=CH | 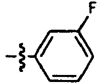 | 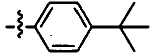 |
| 199 | CH=CH | 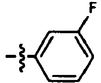 | 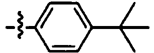 |
| 201 | CH=CH | 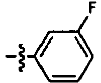 | 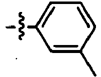 |
| 202 | CH=CH | 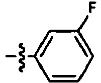 | 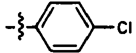 |
| 203 | CH=CH | 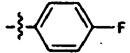 | 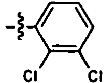 |
| 204 | CH=CH | 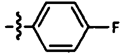 | 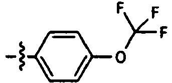 |
| 205 | CH=CH | 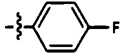 | 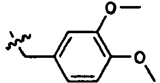 |
| 206 | CH=CH | 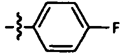 | 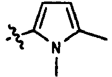 |
| 207 | CH=CH | 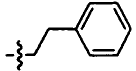 | 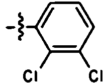 |
| 208 | CH=CH | 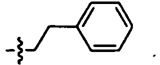 | 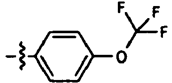 |
| 209 | CH=CH | 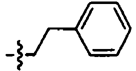 | 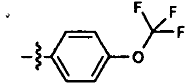 |
| 210 | CH=CH | 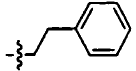 | 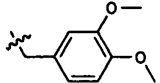 |
| 211 | CH=CH | 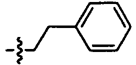 | 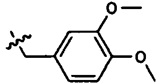 |
| 212 | CH=CH | 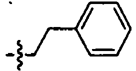 | 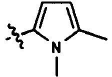 |
| 213 | CH=CH | 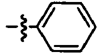 | 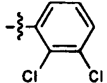 |
| 214 | CH=CH | 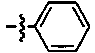 | 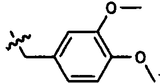 |
| 215 | CH=CH | 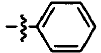 | 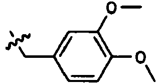 |
| 217 | CH=CH | 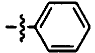 | 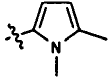 |
| 218 | CH=CH | 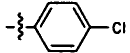 | 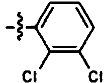 |
| 219 | CH=CH | 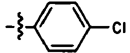 | 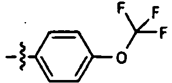 |
| 220 | CH=CH | 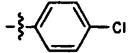 | 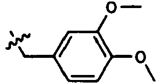 |
| 221 | CH=CH | 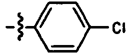 | 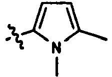 |
| 222 | CH=CH | 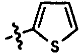 | 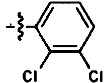 |
| 223 | CH=CH | 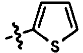 | 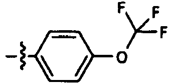 |
| 224 | CH=CH | 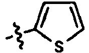 | 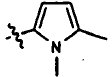 |
| 225 | CH=CH | 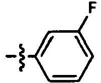 | 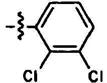 |
| 226 | CH=CH | 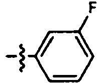 | 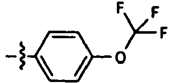 |
| 227 | CH=CH | 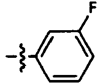 | 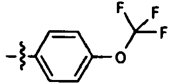 |
| 228 | CH=CH | 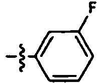 | 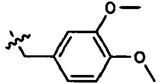 |
| 229 | CH=CH | 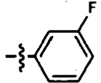 | 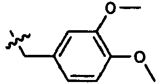 |
| 231 | CH=CH | 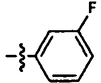 | 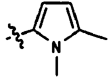 |
| 232 | CH₂ | 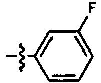 | 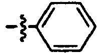 |
| 233 | CH₂ | 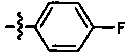 | 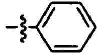 |
| 234 | CH₂ | 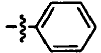 | 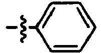 |
| 235 | CH₂ | 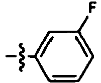 | 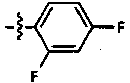 |
| 236 | CH₂ | 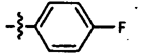 | 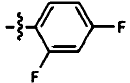 |
| 237 | CH₂ | 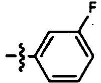 | 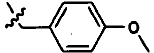 |
| 238 | CH₂ | 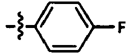 | 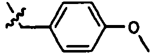 |
| 239 | CH₂ | 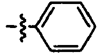 | 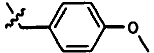 |
| 240 | CH₂ | 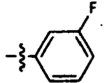 | 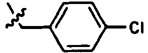 |
| 241 | CH₂ | 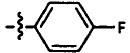 | 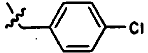 |
| 242 | CH₂ | 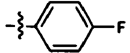 | 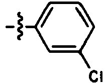 |
| 243 | CH₂ | 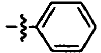 | 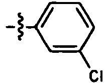 |
| 244 | CH₂ | 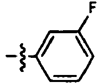 | 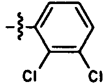 |
| 245 | CH₂ | 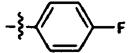 | 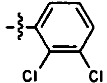 |
| 246 | CH₂ | 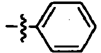 | 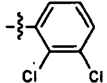 |
| 247 | CH₂ | 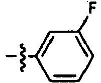 | 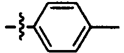 |
| 248 | CH₂ | 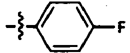 | 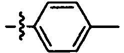 |
| 249 | CH₂ | 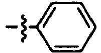 | 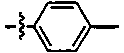 |
| 250 | CH₂ | 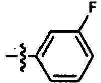 | 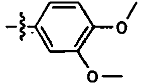 |
| 251 | CH₂ | 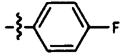 | 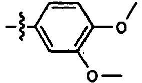 |
| 252 | CH₂ | 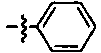 | 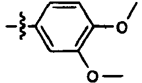 |
| 253 | CH₂ | 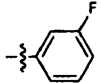 | 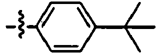 |
| 254 | CH₂ | 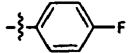 | 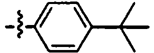 |
| 255 | CH₂ | 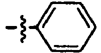 | 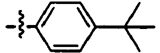 |
| 256 | CH₂ | 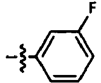 | 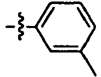 |
| 257 | CH₂ | 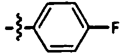 | 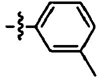 |
| 258 | CH₂ | 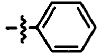 | 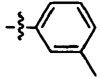 |
| 259 | CH₂ | 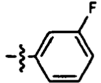 | 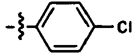 |
| 260 | CH₂ | 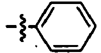 | 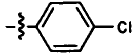 |
| 261 | CH₂ | 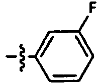 | 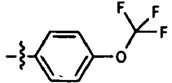 |
| 262 | CH₂ | 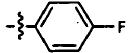 | 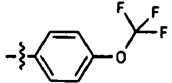 |
| 263 | CH₂ | 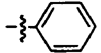 | 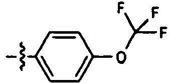 |
| 264 | CH₂CH₂ | 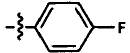 | 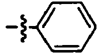 |
| 265 | CH₂CH₂ | 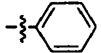 | 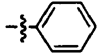 |
| 266 | CH₂CH₂ | 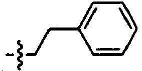 | 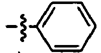 |
| 267 | CH₂CH₂ | 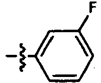 | 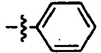 |
| 268 | CH₂CH₂ | 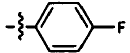 | 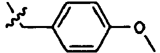 |
| 269 | CH₂CH₂ | 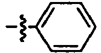 | 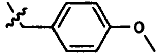 |
| 270 | CH₂CH₂ | 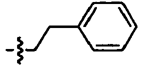 | 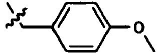 |
| 271 | CH₂CH₂ | 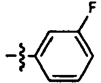 | 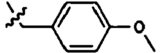 |
| 272 | CH₂CH₂ | 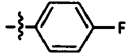 | 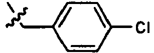 |
| 273 | CH₂CH₂ | 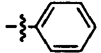 | 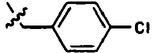 |
| 274 | CH₂CH₂ | 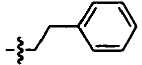 | 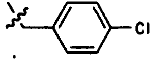 |
| 275 | CH₂CH₂ | 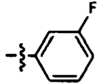 | 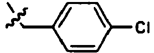 |
| 276 | CH₂CH₂ | 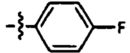 | 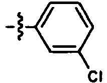 |
| 277 | CH₂CH₂ | 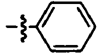 | 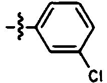 |
| 278 | CH₂CH₂ | 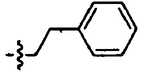 | 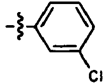 |
| 279 | CH₂CH₂ | 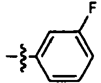 | 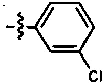 |
| 280 | CH₂CH₂ | 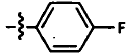 | 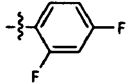 |
| 281 | CH₂CH₂ | 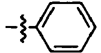 | 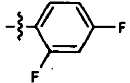 |
| 282 | CH₂CH₂ | 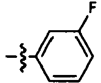 | 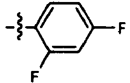 |
| 283 | CH₂CH₂ | 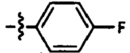 | 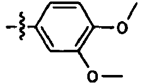 |
| 284 | CH₂CH₂ | 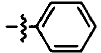 | 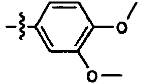 |
| 285 | CH₂CH₂ | 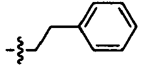 | 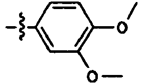 |
| 286 | CH₂CH₂ | 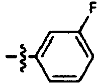 | 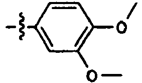 |
| 287 | CH₂CH₂ | 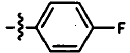 | 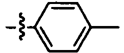 |
| 288 | CH₂CH₂ | 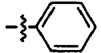 | 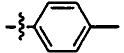 |
| 289 | CH₂CH₂ | 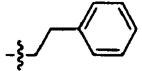 | 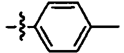 |
| 290 | CH₂CH₂ | 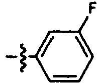 | 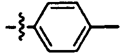 |
| 291 | CH₂CH₂ | 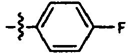 | 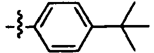 |
| 292 | CH₂CH₂ | 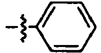 | 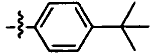 |
| 293 | CH₂CH₂ | 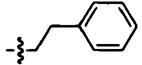 | 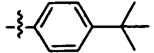 |
| 294 | CH₂CH₂ | 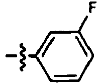 | 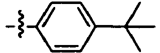 |
| 295 | CH₂CH₂ | 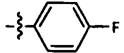 | 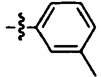 |
| 296 | CH₂CH₂ | 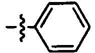 | 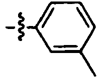 |
| 297 | CH₂CH₂ | 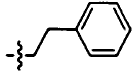 | 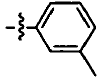 |
| 298 | CH₂CH₂ | 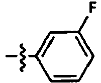 | 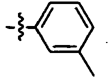 |
| 299 | CH₂CH₂ | 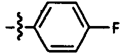 | 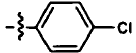 |
| 300 | CH₂CH₂ | 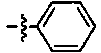 | 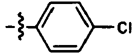 |
| 301 | CH₂CH₂ | 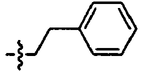 | 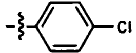 |
| 302 | CH₂CH₂ | 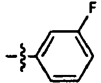 | 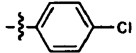 |
| 303 | CH₂CH₂ | 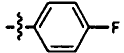 | 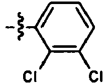 |
| 304 | CH₂CH₂ | 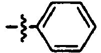 | 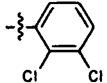 |
| 305 | CH₂CH₂ | 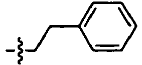 | 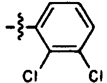 |
| 306 | CH₂CH₂ | 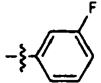 | 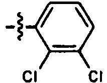 |
| 307 | CH₂CH₂ | 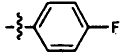 | 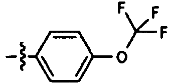 |
| 308 | CH₂CH₂ | 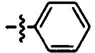 | 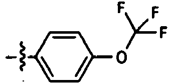 |
| 309 | CH₂CH₂ | 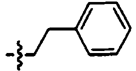 | 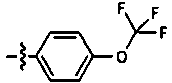 |
| 310 | CH₂CH₂ | 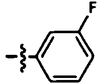 | 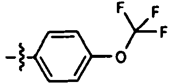 |
| 311 | CH₂ | 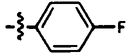 | 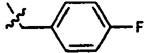 |
| 312 | CH₂ | 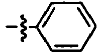 | 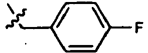 |
| 313 | CH₂ | 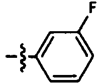 | 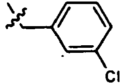 |
| 314 | CH₂ | 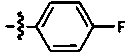 | 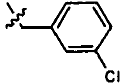 |
| 315 | CH₂ | 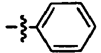 | 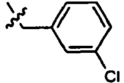 |
| 316 | CH₂ | 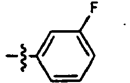 | 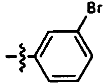 |
| 317 | CH₂ | 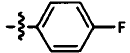 | 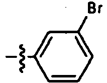 |
| 318 | CH₂ | 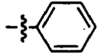 | 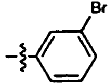 |
| 319 | CH₂ | 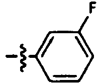 | 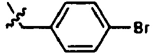 |
| 320 | CH₂ | 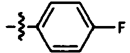 | 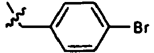 |
| 321 | CH₂ | 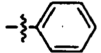 | 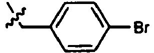 |
| 322 | CH₂ | 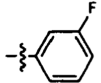 | 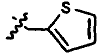 |
| 323 | CH₂ | 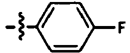 | 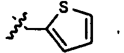 |
| 324 | CH₂ | 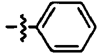 | 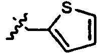 |
| 325 | CH₂ | 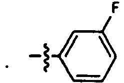 | 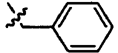 |
| 326 | CH₂ | 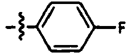 | 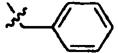 |
| 327 | CH₂ | 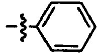 | 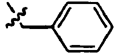 |
| 328 | CH₂ | 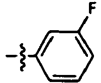 | 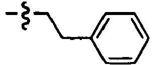 |
| 329 | CH₂ | 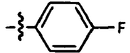 | 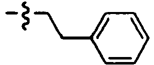 |
| 330 | CH₂ | 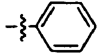 | 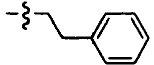 |
| 331 | CH₂ | 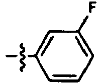 | 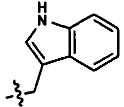 |

### Untersuchungen zur Wirksamkeit der erfindungsgemäßen Verbindungen

### Methode zur Bestimmung der Affinität zum humanen µ-Opiatrezeptor

Die Rezeptoraffinität zum humanen µ-Opiatrezeptor wird in einem homogenen Ansatz in Mikrotiterplatten bestimmt. Hierzu werden Verdünnungsreihen der zu prüfenden Substanzen mit einer Rezeptormembranpräparation (15 - 40 µg Protein /250 µl Inkubationsansatz) von CHO-K1-Zellen, welche den humanen µ-Opiatrezeptor exprimieren (RB-HOM-Rezeptormembran-Präparation von Fa PerkinElmer Life Sciences, Zaventem, Belgien) in Gegenwart von 1 nmol/l des radioaktiven Liganden [³H]-Naloxon (NET719, Fa. PerkinElmer Life Sciences, Zaventem, Belgien) sowie von 1 mg WGA-SPA-Beads (Wheat germ agglutinin SPA Beads der FA. Amersham/Pharmacia, Freiburg, Deutschland) in einem Gesamtvolumen von 250 µl für 90 Minuten bei Raumtemperatur inkubiert. Als Inkubationspuffer wird 50 mmol/l Tris-HCl supplementiert mit 0,06 % bovinem Serumalbumin verwendet. Zur Bestimmung der unspezifischen Bindung wird zusätzlich 100 µmol/l Naloxon zugegeben. Nach Beendigung der neunzigminütigen Inkubationszeit werden die Mikrotiterplatten für 20 Minuten bei 1000 g abzentrifugiert und die Radioaktivität in einem β-Counter (Microbeta-Trilux, Fa. PerkinElmer Wallac, Freiburg, Deutschland) vermessen. Es wird die prozentuale Verdrängung des radioaktiven Liganden aus seiner Bindung zum humanen µ-Opiatrezeptor bei einer Konzentration der Prüfsubstanzen von 1 µmol/l bestimmt und als Prozent Hemmung der spezifischen Bindung angegeben. Ausgehend von der prozentualen Verdrängung durch unterschiedliche Konzentrationen der Prüfsubstanzen werden IC₅₀ Hemmkonzentrationen berechnet, die eine 50-prozentige Verdrängung des radioaktiven Liganden bewirken. Durch Umrechnung mittels der Cheng-Prusoff-Beziehung werden Kᵢ-Werte für die Prüfsubstanzen erhalten.

### Noradrenalin (NA)- und Serotonin(5HT)-Wiederaufnahme-Inhibierung

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

**Tabelle 1: µ-Affinität der Oxadiazole**

| Nr. | µ-Opioid-Rezeptor, % Hemmung [1 µM] | µ-Opioid-Rezeptor, Kᵢ [µM) |
|---|---|---|
| 44 | 75 | 0,072 |
| 43 | 83 | 0,044 |
| 55 | 53 | |
| 69 | 40 | |
| 79 | 43 | |
| 135 | 44 | |
| 149 | 42 | |
| 150 | 56 | |
| 155 | 52 | |
| 183 | 40 | |
| 195 | 48 | |
| 224 | 54 | |
| 232 | 41 | |
| 234 | 64 | |
| 239 | 66 | |
| 243 | 55 | 0,32 |
| 246 | 56 | |
| 258 | 53 | |
| 269 | 43 | |
| 273 | 68 | |
| 281 | 69 | |
| 296 | 45 | |
| 304 | 51 | |

**Tabelle 2: Monoamin-Wiederaufnahme Inhibierung ausgewählter Oxadiazole**

| Nr. | Serotonin-Wiederaufnahme, % Hemmung [10 µM] | Noradrenalin-Wiederaufnahme, % Hemmung [10 µM] |
|---|---|---|
| 43 | 95 | 95 |
| 44 | 99 | 100 |

## Patentansprüche

1. Substituierte Oxadiazol-Derivate der allgemeinen Formel I, 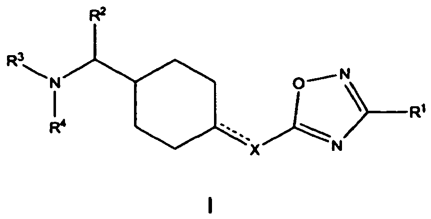 , worin
X CH, CH₂, CH=CH, CH₂CH₂, CH₂CH=CH oder CH₂CH₂CH₂ bedeutet
R¹ Aryl, Heteroaryl, jeweils unsubstituiert oder einfach oder mehrfach substituiert; über eine C₁₋₃-Alkylgruppe verknüpften Aryl oder Heteroarylrest, jeweils unsubstituiert oder einfach oder mehrfach substituiert; bedeutet;
R² Aryl oder Heteroaryl, jeweils unsubstituiert oder einfach oder mehrfach substituiert; einen über eine C₁₋₃-Alkylkette gebundenen Arylrest, jeweils unsubstituiert oder einfach oder mehrfach substituiert, bedeutet;
R³ und R⁴ unabhängig voneinander H; C₁₋₆-Alkyl, jeweils gesättigt oder ungesättigt, verzweigt oder unverzweigt, bedeutet, wobei R³ und R⁴ nicht gleichzeitig H bedeuten,
oder die Reste R³ und R⁴ zusammen CH₂CH₂OCH₂CH₂, oder (CH₂)₃₋₆ bedeuten,
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, wobei
im Zusammenhang mit "Alkyl" unter dem Begriff "substituiert" die Substitution eines Wasserstoffrestes durch F, Cl, Br, I, -CN, NH₂, NH-C₁₋₆-Alkyl, NH-C₁₋₆-Alkyl-OH, C₁₋₆-Alkyl, N(C₁₋₆-Alkyl)₂, N(C₁₋₆-A)kyl-OH)₂. NO₂. SH, S-C₁₋₆-Alkyl, S-Benzyl, O-C₁₋₆-Alkyl, OH, O-C₁₋₆-Alkyl-OH, =O, O-Benzyl, C(=O)C₁₋₆-Alkyl, CO₂H, CO₂-C₁₋₆-Alkyl oder Benzyl verstanden wird, und wobei
in Bezug auf "Aryl" und "Heteroaryl" unter "ein oder mehrfach substituiert" die ein oder mehrfache Substitution eines oder mehrerer Wasserstoffatome des Ringsystems durch F, Cl, Br, I, CN, NH₂, NH-C₁₋₆-Alkyl, NHC₁₋₆-Alkyl-OH, N(C₁₋₆-Alkyl)₂, N(C₁₋₆-Alkyl-OH)₂, NO₂, SH, S-C₁₋₆-Alkyl, OH, O-C₁₋₆-Alkyl, O-C₁₋₆-Alkyl-OH, C(=O)C₁₋₆-Alkyl, CO₂H, CO₂-C₁₋₆-Alkyl, CF₃, oder C₁₋₆-Alkyl verstanden wird.

2. Substituierte Oxadiazol-Derivate gemäß Anspruch 1, worin R¹ Phenyl, Naphthyl, Thienyl, Pyridyl oder Pyrrolyl, Benzyl, Methylindolyl, Methylthienyl oder Phenethyl, unsubstituiert oder einfach oder mehrfach substituiert mit F, Cl, Br, CN, OCF₃, NH₂, NO₂, SH, S-C₁₋₆-Alkyl, OH, O-C₁₋₆-Alkyl, CO₂H, CO₂-C₁₋₆-Alkyl, CF₃ oder C₁₋₆-Alkyl bedeutet.

3. Substituierte Oxadiazol-Derivate gemäß Anspruch 2, worin R¹ Phenyl, Thienyl, Benzyl, Methylindolyl, Methylthienyl oder Pyrrolyl, unsubstituiert oder einfach oder mehrfach substituiert mit Cl, Br, OCH₃, CH₃, F, OCF₃, CF₃ oder tert.-Butyl bedeutet.

4. Substituierte Oxadiazol-Derivate gemäß Anspruch 1 , worin R² Phenyl oder Thienyl, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl; einen über eine C₁₋₃-Alkylkette gebundenen Phenylrest, jeweils unsubstituiert oder einfach oder mehrfach substituiert mit F, Cl, CN, NO₂, SH, S-C₁₋₆-Alkyl, OH, O-C₁₋₆-Alkyl, CO₂H, CO₂-C₁₋₆-Alkyl, CF₃, C₁₋₆-Alkyl, bedeutet;

5. Substituierte Oxadiazol-Derivate gemäß Anspruch 4, worin R² Phenyl, unsubstituiert oder einfach substituiert mit Cl oder F, Phenethyl oder Thienyl bedeutet.

6. Substituierte Oxadiazol-Derivate gemäß Anspruch 1, worin R³ und R⁴ unabhängig voneinander H oder C₁₋₆-Alkyl bedeuten, wobei R³ und R⁴ nicht gleichzeitig H bedeuten, oder die Reste R³ und R⁴ zusammen CH₂CH₂OCH₂CH₂, oder (CH₂)₄₋₅ bedeuten.

7. Substituierte Oxadiazol-Derivate gemäß Anspruch 6, worin R³ und R⁴ CH₃ bedeuten.

8. Substituierte Oxadiazol-Derivate gemäß Anspruch 1 aus der Gruppe
43. (4-((3-(4-chlorbenzyl)-1,2,4-oxadiazol-5-yl)methyl)cyclohexyl)-N,N-dimethyl(phenyl)methanamin
44. (4-((3-(4-methoxybenzyl)-1,2,4-oxadiazol-5-yl)methyl)cyclohexyl)-N,N-dimethyl(phenyl)methanamin
45. ((4-Chlor-phenyl)-{4-[3-(4-chlor-phenyl)-[1,2,4]oxadiazol-5-ylmethylen]-cyclohexyl}-methyl)-dimethyl-amin
46. [{4-[3-(4-Chlor-phenyl)-[1,2,4]oxadiazol-5-ylmethylen]-cyclohexyl}-(4-fluor-phenyl)-methyl]-dimethyl-amin (Polareres Diastereomer)
47. [{4-[3-(4-Chlor-phenyl)-[1,2,4]oxadiazol-5-ylmethylen]-cyclohexyl}-(4-fluor-phenyl)-methyl]-dimethyl-amin (Unpolareres Diastereomer)
48. ({4-[3-(2,3-Dichlor-phenyl)-[1,2,4]oxadiazol-5-ylmethylen]-cyclohexyl}-phenylmethyl}-dimethyl-amin (Polareres Diastereomer)
49. ({4-[3-(2,3-Dichlor-phenyl)-[1,2,4]oxadiazol-5-ylmethylen]-cyclohexyl}-phenylmethyl)-dimethyl-amin (Unpolareres Diastereomer)
50. [{4-[3-(2.3-Dichlor-phenyl)-[1,2,4]oxadiazol-5-ylmethylen]-cyclohexyl}-(3-fluorphenyl)-methyl]-dimethyl-amin (Polareres Diastereomer)
51. [{4-[3-(2,3-Dichlor-phenyl)-[1,2,4]oxadiazol-5-ylmethylen]-cyclohexyl}-(3-fluorphenyl)-methyl]-dimethyl-amin (Unpolareres Diastereomer)
52. ((4-Chlor-phenyl)-{4-[3-(2,3-dichlor-phenyl)-[1,2,4]oxadiazol-5-ylmethylen]-cyclohexyl}-methyl)-dimethyl-amin (Polareres Diastereomer)
53. ((4-Chlor-phenyl)-{4-[3-(2,3-dichlor-phenyl)-[1,2,4]oxadiazol-5-ylmethylen]-cyclohexyl}-methyl)-dimethyl-amin (Unpolareres Diastereomer)
54. (1-{4-[3-(2,3-Dichlor-phenyl)-[1,2,4]oxadiazol-5-ylmethylen]-cyclohexyl}-3-phenylpropyl)-dimethyl-amin
55. [{4-[3-(2,3-Dichlor-phenyl)-[1,2,4]oxadiazol-5-ylmethylen]-cyclohexyl}-(4-fluorphenyl)-methyl]-dimethyl-amin (Polareres Diastereomer)
56. [{4-[3-(2,3-Dichlor-phenyl)-[1,2,4]oxadiazol-5-ylmethylen]-cyclohexyl}-(4-fluorphenyl)-methyl]-dimethyl-amin (Unpolareres Diastereomer)
57. Dimethyl-(phenyl-{4-[3-(4-trifluormethoxy-phenyl)-[1,2,4]oxadiazol-5-ylmethylen]-cyclohexyl}-methyl)-amin (Polareres Diastereomer)
58. Dimethyl-(phenyl-{4-[3-(4-trifluormethoxy-phenyl)-[1,2,4]oxadiazol-5-ylmethylen]-cyclohexyl}-methyl)-amin (Unpolareres Diastereomer)
59. ((3-Fluor-phenyl)-{4-[3-(4-trifluormethoxy-phenyl)-[1,2,4]oxadiazol-5-ylmethylen]-cyclohexyl}-methyl)-dimethyl-amin (Polareres Diastereomer)
60. ((3-Fluor-phenyl)-{4-[3-(4-trifluormethoxy-phenyl)-[1,2,4]oxadiazol-5-ylmethylen]-cyclohexyl}-methyl)-dimethyl-amin (Unpolareres Diastereomer)
61. ((4-Chlor-phenyl)-{4-[3-(4-trifluormethoxy-phenyl)-[1,2,4]oxadiazol-5-ylmethylen]-cyclohexyl}-methyl)-dimethyl-amin (Polareres Diastereomer)
62. ((4-Chlor-phenyl)-{4-[3-(4-trifluormethoxy-phenyl)-[1,2,4]oxadiazol-5-ylmethylen]-cyclohexyl}-methyl)-dimethyl-amin (Unpolareres Diastereomer)
63. Dimethyl-(3-phenyl-1-{4-[3-(4-trifluormethoxy-phenyl)-[1,2,4]oxadiazol-5-ylmethyien]-cyclohexyl}-propyl)-amin (Polareres Diastereomer)
64. Dimethyl-(3-phenyl-1-{4-[3-(4-trifluormethoxy-phenyl)-[1,2,4]oxadiazol-5-ylmethylen]-cyclohexyl}-propyl)-amin (Unpolareres Diastereomer)
65. ((4-Fluor-phenyl)-{4-[3-(4-trifluormethoxy-phenyl)-[1,2,4]oxadiazol-5-ylmethylen]-cyclohexyl}-methyl)-dimethyl-amin (Polareres Diastereomer)
66. ((4-Fluor-phenyl)-{4-[3-(4-trifluormethoxy-phenyl)-[1,2,4]oxadiazol-5-ylmethylen]-cyclohexyl}-methyl)-dimethyl-amin (Unpolareres Diastereomer)
67. Dimethyl-(thiophen-2-yl-{4-[3-(4-trifluormethoxy-phenyl)-[1,2,4]oxadiazol-5-ylmethylen]-cyclohexyl}-methyl)-amin (Polareres Diastereomer)
68. Dimethyl-(thiophen-2-yl-{4-[3-(4-trifluormethoxy-phenyl)-[1,2,4]oxadiazol-5-ylmethylen]-cyclohexyl}-methyl)-amin (Unpolareres Diastereomer)
69. Dimethyl-{phenyl-[4-(3-phenyl-[1,2,4]oxadiazol-5-ylmethylen)-cyclohexyl]-methyl}-amin (Polareres Diastereomer)
70. Dimethyl-{phenyl-[4-(3-phenyl-[1,2,4]oxadiazol-5-ylmethylen)-cyclohexyl]-methyl}-amin (Unpolareres Diastereomer)
71. {(3-Fluor-phenyl)-[4-(3-phenyl-[1,2,4]oxadiazol-5-ylmethylen)-cyclohexyl]-methyl}-dimethyl-amin (Polareres Diastereomer)
72. {(3-Fluor-phenyl)-[4-(3-phenyl-[1,2,4]oxadiazol-5-ylmethylen)-cyclohexyl]-methyl}-dimethyl-amin (Unpolareres Diastereomer)
73. Dimethyl-{3-phenyl-1-[4-(3-phenyl-[1,2,4]oxadiazol-5-ylmethylen)-cyclohexyl]-propyl}-amin (Polareres Diastereomer)
74. Dimethyl-{3-phenyl-1-[4-(3-phenyl-[1,2,4]oxadiazol-5-ylmethylen)-cyclohexyl]-propyl} amin (Unpolareres Diastereomer)
75. {(4-Fluor-phenyl)-[4-(3-phenyl-[1,2,4]oxadiazol-5-ylmethylen)-cyclohexyl]-methyl}-dimethyl-amin (Polareres Diastereomer)
76. {(4-Fluor-phenyl)-[4-(3-phenyl-[1,2,4]oxadiazol-5-ylmethylen)-cyclohexyl]-methyl}-dimethyl-amin (Unpolareres Diastereomer) .
77. [{4-[3-(2,4-Difluor-phenyl)-[1,2,4]oxadiazol-5-ylmethylen]-cyclohexyl}-(3-fluorphenyl)-methyl]-dimethyl-amin (Polareres Diastereomer)
78. [{4-[3-(2,4-Difluor-phenyl)-[1,2,4]oxadiazol-5-ylmethylen]-cyclohexyl}-(3-fluorphenyl)-methyl]-dimethyl-amin (Unpolareres Diastereomer)
79. ({4-[3-(4-Methoxy-benzyl)-[1,2,4]oxadiazol-5-ylmethylen]-cyclohexyl}-phenylmethyl)-dimethyl-amin (Polareres Diastereomer)
80. ({4-[3-(4-Methoxy-benzyl)-[1,2,4]oxadiazol-5-ylmethylen]-cyclohexyl}-phenylmethyl)-dimethyl-amin (Unpolareres Diastereomer)
81. ((3-Fluor-phenyl)-{4-[3-(4-methoxy-benzyl)-[1,2,4]oxadiazol-5-ylmethylen]-cyclohexyl}-methyl)-dimethyl-amin (Polareres Diastereomer)
82. ((3-Fluor-phenyl)-{4-[3-(4-methoxy-benzyl)-[1,2,4]oxadiazol-5-ylmethylen]-cyclohexyl}-methyl)-dimethyl-amin (Unpolareres Diastereomer)
83. ((4-Chlor-phenyl)-{4-[3-(4-methoxy-benzyl)-[1,2,4]oxadiazol-5-ylmethylen]-cyclohexyl}-methyl)-dimethyl-amin (Polareres Diastereomer)
84. ((4-Chlor-phenyl)-{4-[3-(4-methoxy-benzyl)-[1,2,4]oxadiazol-5-ylmethylen]-cyclohexyl}-methyl}-dimethyl-amin (Unpolareres Diastereomer)
85. (1-{4-[3-(4-Methoxy-benzyl)-[1,2,4]oxadiazol-5-ylmethylen]-cyclohexyl}-3-phenylpropyl)-dimethyl-amin (Polareres Diastereomer)
86. (1-{4-[3-(4-Methoxy-benzyl)-[1,2,4]oxadiazol-5-ylmethylen]-cyclohexyl}-3-phenylpropyl)-dimethyl-amin (Unpolareres Diastereomer)
87. ((4-Fluor-phenyl)-{4-[3-(4-methoxy-benzyl)-[1,2,4]oxadiazol-5-ylmethylen]-cyclohexyl}-methyl)-dimethyl-amin (Polareres Diastereomer)
88. ((4-Fluor-phenyl)-{4-[3-(4-methoxy-benzyl)-[1,2,4]oxadiazol-5-ylmethylen]-cyclohexyl}-methyl)-dimethyl-amin (Unpolareres Diastereomer)
89. ({4-[3-(4-Chlor-benzyl)-[1,2,4]oxadiazol-5-ylmethylen]-cyclohexyl}-phenyl-methyl)-dimethyl-amin (Polareres Diastereomer)
90. ({4-[3-(4-Chlor-benzyl)-[1,2,4]oxadiazol-5-ylmethylen]-cyclohexyl}-phenyl-methyl)-dimethyl-amin (Unpolareres Diastereomer)
91. [{4-[3-(4-Chlor-benzyl)-[1,2,4]oxadiazol-5-ylmethylen]-cyclohexyl}-(3-fluor-phenyl)-methyl]-dimethyl-amin (Polareres Diastereomer)
92. [{4-[3-(4-Chlor-benzyl)-[1,2,4]oxadiazol-5-ylmethylen]-cyclohexyl}-(4-chlor-phenyl)-methyl]-dimethyl-amin (Unpolareres Diastereomer)
93. [{4-[3-(4-Chlor-benzyl)-[1,2,4]oxadiazol-5-ylmethylen]-cyclohexyl}-(4-chlor-phenyl)-methyl]-dimethyl-amin (Polareres Diastereomer)
94. [{4-[3-(4-Chlor-benzyl)-[1,2,4]oxadiazol-5-ylmethylen]-cyclohexyl}-(4-chlor-phenyl)-methyl]-dimethyl-amin (Unpolareres Diastereomer)
95. (1-{4-[3-(4-Chlor-benzyl)-[1,2,4]oxadiazol-5-ylmethylen]-cyclohexyl}-3-phenyl-propyl)-dimethyl-amin (Polareres Diastereomer)
96. (1-{4-[3-(4-Chlor-benzyl)-[1,2,4]oxadiazol-5-ylmethylen]-cyclohexyl}-3-phenyl-propyl)-dimethyl-amin (Unpolareres Diastereomer)
97. [{4-[3-(4-Chlor-benzyl)-[1,2,4]oxadiazol-5-ylmethylen]-cyclohexyl}-(4-fluor-phenyl)-methyl]-dimethyl-amin (Polareres Diastereomer)
98. [{4-[3-(4-Chlor-benzyl)-[1,2,4]oxadiazol-5-ylmethylen]-cyclohexyl}-(4-fluor-phenyl)-methyl]-dimethyl-amin (Unpolareres Diastereomer)
99. {(3-Fluor-phenyl)-[4-(3-p-tolyl-[1,2,4]oxadiazol-5-ylmethylen)-cyclohexyl]-methyl}-dimethyl-amin
100. {(4-Chlor-phenyl)-[4-(3-p-tolyl-[1,2,4]oxadiazol-5-ylmethylen)-cyclohexyl]-methyl}-dimethyl-amin
101. {(4-Fluor-phenyl)-[4-(3-p-tolyl-[1,2,4]oxadiazol-5-ylmethylen)-cyclohexyl]-methyl}-dimethyl-amin (Polareres Diastereomer)
102. {(4-Fluor-phenyl)-[4-(3-p-tolyl-[1,2,4]oxadiazol-5-ylmethylen)-cyclohexyl]-methyl}-dimethyl-amin (Unpolareres Diastereomer)
103. ({4-[3-(3,4-Dimethoxy-phenyl)-[1,2,4]oxadiazol-5-ylmethylen]-cyclohexyl}-phenylmethyl)-dimethyl-amin (Polareres Diastereomer)
104. ({4-[3-(3,4-Dimethoxy-phenyl)-[1,2,4]oxadiazol-5-ylmethylen]-cyclohexyl}-phenylmethyl)-dimethyl-amin (Unpolareres Diastereomer)
105. [{4-[3-(3,4-Dimethoxy-phenyl)-[1,2,4]oxadiazol-5-ylmethylen]-cyclohexyl}-(3-fluorphenyl)-methyl]-dimethyl-amin (Polareres Diastereomer)
106. [{4-[3-(3,4-Dimethoxy-phenyl)-[1,2,4]oxadiazol-5-ylmethylen]-cyclohexyl}-(3-fluorphenyl)-methyl]-dimethyl-amin (Unpolareres Diastereomer)
107. ((4-Ch)or-phenyl)-{4-[3-(3,4-dimethoxy-phenyl)-[1,2,4]oxadiazol-5-ylmethylen]-cyclohexyl}-methyl)-dimethyl-amin (Polareres Diastereomer)
108. ((4-Chlor-phenyl)-{4-[3-(3,4-dimethoxy-phenyl)-[1,2,4]oxadiazol-5-ylmethylen]-cyclohexyl}-methyl)-dimethyl-amin (Unpolareres Diastereomer)
109. (1-{4-[3-(3,4-Dimethoxy-phenyl)-[1,2,4]oxadiazol-5-ylmethylen]-cyclohexyl}-3- phenylpropyl)-dimethyl-amin (Polareres Diastereomer)
110. (1-{4-[3-(3,4-Dimethoxy-phenyl)-[1,2,4]oxadiazol-5-ylmethylen]-cyclohexyl}-3- phenylpropyl)-dimethyl-amin (Unpolareres Diastereomer)
111. [{4-[3-(3,4-Dimethoxy-phenyl)-[1,2,4]oxadiazol-5-ylmethylen]-cyclohexyl}-(4-fluorphenyl)-methyl]-dimethyl-amin (Polareres Diastereomer)
112. [{4-[3-(3,4-Dimethoxy-phenyl)-[1,2,4]oxadiazol-5-ylmethylen]-cyclohexyl}-(4-fluorphenyl)-methyl]-dimethyl-amin (Unpolareres Diastereomer)
113. ({4-[3-(3-Chlor-phenyl)-[1,2,4]oxadiazol-5-ylmethylen]-cyclohexyl}-phenyl-methyl)-dimethyl-amin (Polareres Diastereomer)
114. ({4-[3-(3-Chlor-phenyl)-[1,2,4]oxadiazol-5-ylmethylen]-cyclohexyl}-phenyl-methyl)-dimethyl-amin (Unpolareres Diastereomer)
115. [{4-[3-(3-Chlor-phenyl)-[1,2,4]oxadiazol-5-ylmethylen]-cyclohexyl}-(3-fluor-phenyl)-methyl]-dimethyl-amin (Polareres Diastereomer)
116. [{4-[3-(3-Chlor-phenyl)-[1,2,4]oxadiazol-5-ylmethylen]-cyclohexyl}-(3-fluor-phenyl)-methyl]-dimethyl-amin (Unpolareres Diastereomer)
117. ((4-Chlor-phenyl)-{4-[3-(3-chlor-phenyl)-[1,2,4]oxadiazol-5-ylmethylen]-cyclohexyl}-methyl)-dimethyl-amin (Polareres Diastereomer)
118. ((4-Chlor-phenyl)-{4-[3-(3-chlor-phenyl)-[1,2,4]oxadiazol-5-ylmethylen]-cyclohexyl}-methyl)-dimethyl-amin (Unpolareres Diastereomer)
119. (1-{4-[3-(3-Chlor-phenyl)-[1,2,4]oxadiazol-5-ylmethylen]-cyclohexyl}-3-phenylpropyl)-dimethyl-amin (Polareres Diastereomer)
120. (1-{4-[3-(3-Chlor-phenyl)-[1,2,4]oxadiazol-5-ylmethylen]-cyclohexyl}-3-phenylpropyl)-dimethyl-amin (Unpolareres Diastereomer)
121. [{4-[3-(3-Chlor-phenyl)-[1,2,4]oxadiazol-5-ylmethylen]-cyclohexyl}-(4-fluor-phenyl)-methyl]-dimethyl-amin
122. ({4-[3-(4-tert-Butyl-phenyl)-[1,2,4]oxadiazol-5-ylmethylen]-cyclohexyl}-phenylmethyl)-dimethyl-amin (Polareres Diastereomer)
123. ({4-[3-(4-tert-Butyl-phenyl)-[1,2,4]oxadiazol-5-ylmethylen]-cyclohexyl}-phenylmethyl)-dimethyl-amin (Unpolareres Diastereomer)
124. [{4-[3-(4-tert-Butyl-phenyl)-[1,2,4]oxadiazol-5-ylmethylen]-cyclohexyl}-(3-fluorphenyl)-methyl]-dimethyl-amin (Polareres Diastereomer)
125. [{4-[3-(4-tert-Butyl-phenyl)-[1,2,4]oxadiazol-5-ylmethylen]-cyclohexyl}-(3-fluorphenyl)-methyl]-dimethyl-amin (Unpolareres Diastereomer)
126. [{4-[3-(4-tert-Butyl-phenyl)-[1,2,4]oxadiazol-5-ylmethylen]-cyclohexyl}-(4-chlorphenyl)-methyl]-dimethyl-amin (Polareres Diastereomer)
127. [{4-[3-(4-tert-Butyl-phenyl)-[1,2,4]oxadiazol-5-ylmethylen]-cyclohexyl}-(4-chlorphenyl)-methyl]-dimethyl-amin (Unpolareres Diastereomer)
128. (1-{4-[3-(4-tert-Butyl-phenyl)-[1,2,4]oxadiazol-5-ylmethylen]-cyclohexyl}-3-phenylpropyl)-dimethyl-amin (Polareres Diastereomer)
129. (1-{4-[3-(4-tert-Butyl-phenyl)-[1,2,4]oxadiazol-5-ylmethylen]-cyclohexyl}-3-phenylpropyl)-dimethyl-amin (Unpolareres Diastereomer)
130. [{4-[3-(4-tert-Butyl-phenyl)-[1,2,4]oxadiazol-5-ylmethylen]-cyclohexyl}-(4-fluorphenyl)-methyl]-dimethyl-amin (Polareres Diastereomer)
131. [{4-[3-(4-tert-Butyl-phenyl)-[1,2,4]oxadiazol-5-ylmethylen]-cyclohexyl)-(4-fluorphenyl)-methyl]-dimethyl-amin (Unpolareres Diastereomer)
132. ({4-[3-(4-tert-Butyl-phenyl)-[1,2,4]oxadiazol-5-ylmethylen]-cyclohexyl}-thiophen-2-ylmethyl)-dimethyl-amin (Polareres Diastereomer)
133. ({4-[3-(4-tert-Butyl-phenyl)-[1,2,4]oxadiazol-5-ylmethylen]-cyclohexyl}-thiophen-2-ylmethyl)-dimethyl-amin (Unpolareres Diastereomer)
134. Dimethyl-{phenyl-[4-(3-m-tolyl-[1,2,4]oxadiazol-5-ylmethylen)-cyclohexyl]-methyl}-amin (Polareres Diastereomer)
135. Dimethyl-{phenyl-[4-(3-m-tolyl-[1,2,4]oxadiazol-5-ylmethylen)-cyclohexyl]-methyl}-amin (Unpolareres Diastereomer)
136. {(3-Fluor-phenyl)-[4-(3-m-tolyl-[1,2,4]oxadiazol-5-ylmethylen)-cyclohexyl]-methyl}-dimethyl-amin (Polareres Diastereomer)
137. {(3-Fluor-phenyl)-[4-(3-m-tolyl-[1,2,4]oxadiazol-5-ylmethylen)-cyclohexyl]-methyl}-dimethyl-amin (Unpolareres Diastereomer)
138. {(4-Chlor-phenyl)-[4-(3-m-tolyl-[1,2,4]oxadiazol-5-ylmethylen)-cyclohexyl]-methyl}-dimethyl-amin (Polareres Diastereomer)
139. {(4-Chlor-phenyl)-[4-(3-m-tolyl-[1,2,4]oxadiazol-5-ylmethylen)-cyclohexyl]-methyl}-dimethyl-amin (Unpolareres Diastereomer)
140. ((4-Fluor-phenyl)-{4-[2-(3-phenyl-[1,2,4]oxadiazol-5-yl)-vinyl]-cyclohexyl}-methyl)-dimethyl-amin
141. [(4-{2-[3-(2,4-Difluor-phenyl)-[1,2,4]oxadiazol-5-yl]-vinyl}-cyclohexyl)-(4-fluorphenyl)-methyl]-dimethyl-amin
142. [(4-Fluor-phenyl)-(4-{2-[3-(4-methoxy-benzyl)-[1,2,4]oxadiazol-5-yl]-vinyl}-cyclohexyl)-methyl]-dimethyl-amin
143. [(4-{2-[3-(4-Chlor-benzyl)-[1,2,4]oxadiazol-5-yl]-vinyl}-cyclohexyl)-(4-fluor-phenyl)-methyl]-dimethyl-amin
144. Dimethyl-(3-phenyl-1-{4-[2-(3-phenyl-[1,2,4]oxadiazol-5-yl)-vinyl]-cyclohexyl}-propyl)-amin
145. [1-(4-{2-[3-(2,4-Difluor-phenyl)-[1 I2,4]oxadiazol-5-yl]-vinyl}-cyclohexyl)-3-phenylpropyl]-dimethyl-amin
146. [1-(4-{2-[3-(4-Methoxy-benzyl)-[1,2,4]oxadiazol-5-yl]-vinyl}-cyclohexyl)-3-phenylpropyl]-dimethyl-amin
147. [1-(4-{2-[3-(4-Chlor-benzyl)-[1,2,4]oxadiazol-5-yl]-vinyl}-cyclohexyl)-3-phenylpropyl]-dimethyl-amin
148. Dimethyl-(phenyl-{4-[2-(3-phenyl-[1,2,4]oxadiazol-5-yl)-vinyl]-cyclohexyl}-methyl)-amin
149. [(4-{2-[3-(4-Chlor-benzyl)-[1,2,4]oxadiazol-5-yl]-vinyl}-cyclohexyl)-phenyl-methyl]-dimethyl-amin (Polareres Diastereomer)
150. [(4-{2-[3-(4-Chlor-benzyl)-[1,2,4]oxadiazol-5-yl]-vinyl}-cyclohexyl)-phenyl-methyl]-dimethyl-amin (Unpolareres Diastereomer)
151. [(4-Chlor-phenyl)-(4-{2-[3-(2,4-difluor-phenyl)-[1,2,4]oxadiazol-5-yl]-vinyl}-cyclohexyl)-methyl]-dimethyl-amin
152. [(4-Chlor-phenyl)-(4-{2-[3-(4-methoxy-benzyl)-[1,2,4]oxadiazol-5-yl]-vinyl}-cyclohexyl)-methyl]-dimethyl-amin
153. [(4-{2-[3-(4-Chlor-benzyl)-[1,2,4]oxadiazol-5-yl]-vinyl}-cyclohexyl)-(4-chlor-phenyl)-methyl]-dimethyl-amin
154. [(4-{2-[3-(4-Methoxy-benzyl)-[1,2,4]oxadiazol-5-yl]-vinyl}-cyclohexyl)-thiophen-2-ylmethyl]-dimethyl-amin
155. [(4-{2-[3-(4-Chlor-benzyl)-[1,2,4]oxadiazol-5-yl]-vinyl}-cyclohexyl)-thiophen-2-ylmethyl]-dimethyl-amin
156. ((3-Fluor-phenyl)-{4-[2-(3-phenyl-[1,2,4]oxadiazol-5-yl)-vinyl]-cyclohexyl}-methyl)-dimethyl-amin
157. [(3-Fluor-phenyl)-(4-{2-[3-(4-methoxy-benzyl)-[1,2,4]oxadiazol-5-yl]-vinyl}-cyclohexyl)-methyl]-dimethyl-amin
158. ((4-Fluor-phenyl)-{4-[2-(3-p-tolyl-[1,2,4]oxadiazol-5-yl)-vinyl]-cyclohexyl}-methyl)-dimethyl-amin
159. [(4-{2-[3-(3,4-Dimethoxy-phenyl)-[1,2,4]oxadiazol-5-yl]-vinyl}-cyclohexyl)-(4-fluorphenyl)-methyl]-dimethyl-amin
160. Dimethyl-(3-phenyl-1 -{4-[2-(3-p-tolyl-[1,2,4]oxadiazol-5-yl)-vinyl]-cyclohexyl}-propyl)-amin
161. [1-(4-{2-[3-(3,4-Dimethoxy-phenyl)-[1,2,4]oxadiazol-5-yl]-vinyl}-cyclohexyl)-3-phenylpropyl]-dimethyl-amin
162. Dimethyl-(phenyl-{4-[2-(3-p-tolyl-[1,2,4]oxadiazol-5-yl)-vinyl]-cyclohexyl}-methyl)-amin
163. [(4-{2-[3-(3,4-Dimethoxy-Phenyl)-[1,2,4]oxadiazol-5-yl)-vinyl]-cyclohexyl)-phenyl-methyl]-dimethyl-amin
164. ((4-Chlor-phenyl)-{4-[2-(3-p-tolyl-[1,2,4]oxadiazol-5-yl)-vinyl]-cyclohexyl}-methyl)-dimethyl-amin
165. [(4-Chlor-phenyl)-(4-{2-[3-(3,4-dimethoxy-phenyl)-[1,2,4]oxadiazol-5-yl]-vinyl}-cyclohexyl)-methyl]-dimethyl-amin
166. Dimethyl-(thiophen-2-yl-{4-[2-(3-p-tolyl-[1,2,4]oxadiazol-5-yl)-vinyl]-cyclohexyl}-methyl)-amin
167. [(4-{2-[3-(3,4-Dimethoxy-phenyl)-[1,2,4]oxadiazol-5-yl]-vinyl}-cyclohexyl)-thiophen2-yl-methyl]-dimethyl-amin
168. ((3-Fluor-phenyl)-{4-[2-(3-p-tolyl-[1,2,4]oxadiazol-5-yl)-vinyl]-cyclohexyl}-methyl)-dimethyl-amin
169. [(4-{2-[3-(3,4-Dimethoxy-phenyl)-[1,2,4]oxadiazol-5-yl]-vinyl}-cyclohexyl)-(3-fluorphenyl)-methyl]-dimethyl-amin
170. [(4-(2-[3-(3-Chlor-phenyl)-[1,2,4]oxadiazol-5-yl]-vinyl}-cyclohexyl)-(4-fluor-phenyl)-methyl]-dimethyl-amin
171. [(4-{2-[3-(4-tert-Butyl-phenyl)-[1,2,4]oxadiazol-5-yl]-vinyl}-cyclohexyl)-(4-fluorphenyl)-methyl]-dimethyl-amin
172. [(4-{2-[3-(4-tert-Butyl-phenyl)-[1,2,4]oxadiazol-5-yl]-vinyl}-cyclohexyl)-(4-fluorphenyl)-methyl]-dimethyl-amin
173. ((4-Fluor-phenyl)-{4-[2-(3-m-tolyl-[1,2,4]oxadiazol-5-yl)-vinyl]-cyclohexyl}-methyl)-dimethyl-amin
174. [1-(4-{2-[3-(3-Chlor-phenyl)-[1,2,4]oxadiazol-5-yl]-vinyl}-cyclohexyl)-3-phenylpropyl]-dimethyl-amin
175. [1-(4-{2-[3-(4-tert-Butyl-phenyl)-[1,2,4]oxadiazol-5-yl]-vinyl}-cyclohexyl)-3-phenylpropyl]-dimethyl-amin (Polareres Diastereomer)
176. [1-(4-{2-[3-(4-tert-Butyl-phenyl)-[1,2,4]oxadiazol-5-yl]-vinyl}-cyclohexyl)-3-phenylpropyl]-dimethyl-amin (Unpolareres Diastereomer)
177. Dimethyl-(3-phenyl-1-{4-[2-(3-m-tolyl-[1,2,4]oxadiazol-5-yl)-vinyl]-cyclohexyl}-propyl)-amin (Polareres Diastereomer)
178. Dimethyl-(3-phenyl-1-{4-[2-(3-m-tolyl-[1,2,4]oxadiazol-5-yl)-vinyl]-cyclohexyl}-propyl)-amin (Unpolareres Diastereomer)
179. [(4-{2-[3-(3-Chlor-phenyl)-[1,2,4]oxadiazol-5-yl]-vinyl}-cyclohexyl)-phenyl-methyl]-dimethyl-amin
180. [(4-{2-[3-(4-tert-Butyl-phenyl)-[1,2,4]oxadiazol-5-yl]-vinyl}-cyclohexyl)-phenylmethyl]-dimethyl-amin (Polareres Diastereomer)
181. [(4-{2-[3-(4-tert-Butyl-phenyl)-[1,2,4]oxadiazol-5-yl]-vinyl}-cyclohexyl)-phenylmethyl]-dimethyl-amin (Unpolareres Diastereomer)
182. Dimethyl-(phenyl-{4-[2-(3-m-tolyl-[1,2,4]oxadiazol-5-yl)-vinyl]-cyclohexyl}-methyl)-amin (Polareres Diastereomer)
183. Dimethyl-(phenyl-{4-[2-(3-m-tolyl-[1,2,4]oxadiazol-5-yl)-vinyl]-cyclohexyl}-methyl)-amin (Unpolareres Diastereomer)
184. [(4-{2-[3-(4-Chlor-phenyl)-[1,2,4]oxadiazol-5-yl]-vinyl}-cyclohexyl)-phenyl-methyl]-dimethyl-amin (Polareres Diastereomer)
185. [(4-{2-[3-(4-Chlor-phenyl)-[1,2,4]oxadiazol-5-yl]-vinyl}-cyclohexyl)-phenyl-methyl]-dimethyl-amin (Unpolareres Diastereomer)
186. [(4-Chlor-phenyl)-(4-{2-[3-(3-chlor-phenyl)-[1,2,4]oxadiazol-5-yl]-vinyl}-cyclohexyl)-methyl]-dimethyl-amin
187. [(4-{2-[3-(4-tert-Butyl-phenyl)-[1,2,4]oxadiazol-5-yl]-vinyl}-cyclohexyl)-(4-chlorphenyl)-methyl]-dimethyl-amin (Polareres Diastereomer)
188. [(4-{2-[3-(4-tert-Butyl-phenyl)-[1,2,4]oxadiazol-5-yl]-vinyl}-cyclohexyl)-(4-chlorphenyl)-methyl]-dimethyl-amin (Unpolareres Diastereomer)
189. ((4-Ch)or-phenyl)-{4-[2-(3-m-tolyl-[1,2,4]oxadiazol-5-yl)-vinyl]-cyclohexyl}-methyl)-dimethyl-amin (Polareres Diastereomer)
190. ((4-Chlor-phenyl)-{4-[2-(3-m-tolyl-[1,2,4]oxadiazol-5-yl)-vinyl]-cyclohexyl}-methyl)-dimethyl-amin (Unpolareres Diastereomer)
191. [(4-Chlor-phenyl)-(4-{2-[3-(4-chlor-phenyl)-[1,2,4]oxadiazo1-5-yl]-vinyl}-cyclohexyl)-methyl]-dimethyl-amin
192. [(4-{2-[3-(3-Chlor-phenyl)-[1,2,4]oxadiazol-5-yl]-vinyl}-cyclohexyl)-thiophen-2-ylmethyl]-dimethyl-amin
193. [(4-{2-[3-(4-tert-Butyl-phenyl)-[1,2,4]oxadiazol-5-yl]-vinyl}-cyclohexyl)-thiophen-2-ylmethyl]-dimethyl-amin (Polareres Diastereomer)
194. [(4-{2-[3-(4-tert-Butyl-phenyl)-[1,2,4]oxadiazol-5-yl]-vinyl}-cyclohexyl)-thiophen-2-yl-methyl]-dimethyl-amin (Unpolareres Diastereomer)
195. Dimethyl-(thiophen-2-yl-{4-[2-(3-m-tolyl-[1,2,4]oxadiazol-5-yl)-vinyl]-cyclohexyl}-methyl)-amin
196. [(4-{2-[3-(4-Chlor-phenyl)-[1,2,4]oxadiazol-5-yl]-vinyl}-cyclohexyl)-thiophen-2-ylmethyl]-dimethyl-amin
197. [(4-{2-[3-(4-Chlor-phenyl)-[1,2,4]oxadiazol-5-yl]-vinyl}-cyclohexyl)-(3-fluor-phenyl)-methyl]-dimethyl-amin
198. [(4-{2-[3-(4-tert-Butyl-phenyl)-[1,2,4]oxadiazol-5-yl]-vinyl}-cyclohexyl)-(3-fluorphenyl)-methyl]-dimethyl-amin (Polareres Diastereomer)
199. [(4-{2-[3-(4-tert-Butyl-phenyl)-[1,2,4]oxadiazol-5-yl]-vinyl}-cyclohexyl)-(3-fluorphenyl)-methyl]-dimethyl-amin (Unpolareres Diastereomer)
201. ((3-Fluor-phenyl)-{4-[2-(3-m-tolyl-[1,2,4]oxadiazol-5-yl)-vinyl]-cyclohexyl}-methyl)-dimethyl-amin
202. [(4-{2-[3-(4-Chlor-phenyl)-[1,2,4]oxadiazol-5-yl]-vinyl}-cyclohexyl)-(3-fluor-phenyl)-methyl]-dimethyl-amin
203. [(4-{2-[3-(2,3-Dichlor-phenyl)-[1,2,4]oxadiazol-5-yl]-vinyl}-cyclohexyl)-(4-fluorphenyl)-methyl]-dimethyl-amin
204. [(4-Fluor-phenyl)-(4-{2-[3-(4-trifluormethoxy-phenyl)-[1,2,4]oxadiazol-5-yl]-vinyl}-cyclohexyl)-methyl]-dimethyl-amin
205. [(4-{2-[3-(3,4-Dimethoxy-benzyl)-[1,2,4]oxadiazol-5-yl]-vinyl}-cyclohexyl)-(4-fluorphenyl)-methyl]-dimethyl-amin
206. [(4-{2-[3-(1,5-Dimethyl-1H-pyrrol-2-yl)-[1,2,4]oxadiazol-5-yl]-vinyl}-cyclohexyl)-(4-fluorphenyl)-methyl]-dimethyl-amin
207. [1-(4-{2-[3-(2,3-Dichlor-phenyl)-[1,2,4]oxadiazol-5-yl]-vinyl}-cyclohexyl)-3-phenylpropyl]-dimethyl-amin
208. Dimethyl-[3-phenyl-1-(4-{2-[3-(4-trifluormethoxy-phenyl)-[1,2,4]oxadiazol-5-yl]-vinyl}-cyclohexyl)-propyl]-amin (Polareres Diastereomer)
209. Dimethyl-[3-phenyl-1-(4-{2-[3-(4-trifluormethoxy-phenyl)-[1,2,4]oxadiazol-5-yl]-vinyl}-cyclohexyl)-propyl]-amin (Unpolareres Diastereomer)
210. [1-(4-{2-[3-(3,4-Dimethoxy-benzyl)-[1,2,4]oxadiazol-5-yl]-vinyl}-cyclohexyl)-3-phenylpropyl]-dimethyl-amin (Polareres Diastereomer)
211. [1-(4-{2-[3-(3,4-Dimethoxy-benzyl)-[1,2,4]oxadiazol-5-yl]-vinyl}-cyclohexyl)-3-phenylpropyl]-dimethyl-amin (Unpolareres Diastereomer)
212. [1-(4-{2-[3-(1,5-Dimethyl-1H-pyrrol-2-yl)-[1,2,4]oxadiazol-5-yl]-vinyl}-cyclohexyl)-3-phenyl-propyl]-dimethyl-amin
213. [(4-{2-[3-(2,3-Dichlor-phenyl)-[1,2,4]oxadiazol-5-yl]-vinyl}-cyclohexyl)-phenylmethyl]-dimethyl-amin
214. [(4-{2-[3-(3,4-Dimethoxy-benzyl)-[1,2,4]oxadiazol-5-yl]-vinyl}-cyclohexyl)-phenylmethyl]-dimethyl-amin (Polareres Diastereomer)
215. [(4-{2-[3-(3,4-Dimethoxy-benzyl)-[1,2,4]oxadiazol-5-yl]-vinyl}-cyclohexyl)-phenylmethyl]-dimethyl-amin (Unpolareres Diastereomer)
217. [(4-{2-[3-(1,5-Dimethyl-1H-pyrrol-2-yl)-[1,2,4]oxadiazol-5-yl]-vinyl}-cyclohexyl)-phenyl-methyl]-dimethyl-amin
218. [(4-Chlor-phenyl)-(4-{2-[3-(2,3-dichlor-phenyl)-[1,2,4]oxadiazol-5-yl]-vinyl}-cyclohexyl)-methyl]-dimethyl-amin
219. [(4-Chlor-phenyl)-{4-{2-[3-(4-trifluormethoxy-phenyl)-[1,2,4Joxadiazol-5-yl]-vinyl}-cyclohexyl)-methyl]-dimethyl-amin
220. [(4-Chlor-phenyl)-(4-{2-[3-(3,4-dimethoxy-benzyl)-[1,2,4]oxadiazol-5-yl]-vinyl}-cyclohexyl)-methyl]-dimethyl-amin
221. [(4-Chlor-phenyl)-(4-{2-[3-(1,5-dimethyl-1H-pyrrol-2-yl)-[1,2,4]oxadiazol-5-yl]-vinyl}-cyclohexyl)-methyl]-dimethyl-amin
222. [(4-{2-[3-(2,3-Dichlor-phenyl)-[1,2,4]oxadiazol-5-yl]-vinyl}-cyclohexyl)-thiophen-2-ylmethyl]-dimethyl-amin
223. Dimethyl-[thiophen-2-yl-(4-{2-[3-(4-trifluormethoxy-phenyl)-[1,2,4]oxadiazol-5-yl]-vinyl}-cyclohexyl)-methyl]-amin
224. [(4-{2-[3-(1 ,5-Dimethyl-1H-pyrrol-2-yl)-[1,2,4]oxadiazol-5-yl]-vinyl}-cyclohexyl)-thiophen-2-yl-methyl]-dimethyl-amin
225. [(4-{2-[3-(2,3-Dichlor-phenyl)-[1,2,4]oxadiazol-5-yl]-vinyl}-cyclohexyl)-(3-fluorphenyl)-methyl]-dimethyl-amin
226. [(3-Fluor-phenyl)-(4-{2-[3-(4-trifluormethoxy-phenyl)-[1,2,4]oxadiazol-5-yl]-vinyl}-cyclohexyl)-methyl]-dimethyl-amin (Polareres Diastereomer)
227. [(3-Fluor-phenyl)-(4-{2-[3-(4-trifluormethoxy-phenyl)-[1,2,4]oxadiazol-5-yl]-vinyl}-cyclohexyl)-methyl]-dimethyl-amin (Unpolareres Diastereomer)
228. [(4-{2-[3-(3,4-Dimethoxy-benzyl)-[1,2,4]oxadiazol-5-yl]-vinyl}-cyclohexyl)-(3-fluorphenyl)-methyl]-dimethyl-amin (Polareres Diastereomer)
229. [(4-{2-[3-(3,4-Dimethoxy-benzyl)-[1,2,4]oxadiazol-5-yl]-vinyl}-cyclohexyl)-(3-fluorphenyl)-methyl]-dimethyl-amin (Unpolareres Diastereomer)
231. [(4-{2-[3-(1,5-Dimethy)-1H-pyrrol-2-yl)-[12,4]oxadiazol-5-yl]-vinyl}-cyclohexyl)-(3-fluorphenyl)-methyl]-dimethyl-amin
232. {(3-Fluor-phenyl)-[4-(3-phenyl-[1,2,4]oxadiazol-5-ylmethyl)-cyclohexyl]-methyl}-dimethyl-amin
233. {(4-Fluor-phenyl)-[4-(3-phenyl-[1,2,4]oxadiazol-5-ylmethyl)-cyclohexyl]-methyl}-dimethyl-amin
234. Dimethyl-{phenyl-[4-(3-phenyl-[1,2,4]oxadiazol-5-ylmethyl)-cyclohexyl]-methyl}-amin
235. [{4-[3-(2,4-Difluor-phenyl)-[1,2,4]oxadiazol-5-ylmethyl]-cyclohexyl}-(3-fluor-phenyl)-methyl]-dimethyl-amin
236. [{4-[3-(2,4-Difluor-phenyl)-[1,2,4]oxadiazol-5-ylmethyl]-cyclohexyl}-(4-fluor-phenyl)-methyl]-dimethyl-amin
237. ((3-Fluor-phenyl)-{4-[3-(4-methoxy-benzyl)-[1,2,4]oxadiazol-5-ylmethyl]-cyclohexyl}-methyl)-dimethyl-amin
238. ((4-Fluor-phenyl)-{4-[3-(4-methoxy-benzyl)-[1,2,4]oxadiazol-5-ylmethyl]-cyclohexyl}-methyl)-dimethyl-amin
239. ({4-[3-(4-Methoxy-benzyl)-[1,2,4]oxadiazol-5-ylmethyl]-cyclohexyl}-phenyl-methyl)-dimethyl-amin
240. [{4-[3-(4-Chlor-benzyl)-[1,2,4]oxadiazol-5-ylmethyl]-cyclohexyl}-(3-fluor-phenyl)-methyl]-dimethyl-amin
241. [{4-[3-(4-Chlor-benzyl)-[1,2,4]oxadiazol-5-ylmethyl]-cyclohexyl}-(4-fluor-phenyl)-methyl]-dimethyl-amin
242. [{4-[3-(3-Chlor-phenyl)-[1,2,4]oxadiazol-5-ylmethyl]-cyclohexyl}-(4-fluor-phenyl)-methyl]-dimethyl-amin
243. ({4-[3-(3-Chlor-phenyl)-[1,2,4]oxadiazol-5-ylmethyl]-cyclohexyl}-phenyl-methyl)-dimethyl-amin
244. [{4-[3-(2,3-Dichlor-phenyl)-[1,2,4]oxadiazol-5-ylmethyl]-cyclohexyl}-(3-fluor-phenyl)-methyl]-dimethyl-amin
245. [{4-[3-(2,3-Dichlor-phenyl)-[1,2,4]oxadiazol-5-ylmethyl]-cyclohexyl}-(4-fluor-phenyl)-methyl]-dimethyl-amin
246. ({4-[3-(2,3-Dichlor-phenyl)-[1 I2I4]oxadiazol-5-ylmethyl]-cyclohexyl}-phenyl-methyl)-dimethyl-amin
247. {(3-Fluor-phenyl)-[4-(3-p-tolyl-[1,2,4]oxadiazol-5-ylmethyl)-cydohexyl]-methyl}-dimethylamin
248. {(4-Fluor-phenyl)-[4-(3-p-tolyl-[1,2,4]oxadiazol-5-ylmethyl)-cyclohexyl]-methyl}-dimethylamin
249. Dimethyl-{phenyl-[4-(3-p-tolyl-[1,2,4]oxadiazol-5-ylmethyl)-cyclohexyl]-methyl}-amin
250. [{4-[3-(3,4-Dimethoxy-phenyl)-[1,2,4]oxadiazol-5-ylmethyl]-cyclohexyl}-(3-fluorphenyl)-methyl]-dimethyl-amin
251. [{4-[3-(3,4-Dimethoxy-phenyl)-[1,2,4]oxadiazol-5-ylmethyl]-cyclohexyl}-(4-fluorphenyl)-methyl]-dimethyl-amin
252. ({4-[3-(3,4-Dimethoxy-phenyl)-[1,2,4]oxadiazol-5-ylmethyl]-cyclohexyl}-phenyl-methyl)-dimethyl-amin
253. [{4-[3-(4-tert-Butyl-phenyl)-[1,2,4]oxadiazol-5-ylmethyl]-cyclohexyl}-(3-fluor-phenyl)-methyl]-dimethyl-amin
254. [{4-[3-(4-tert-Butyl-phenyl)-[1,2,4]oxadiazol-5-ylmethyl]-cyclohexyl}-(4-fluor-phenyl)-methyl]-dimethyl-amin
255. ({4-[3-(4-tert-Butyl-phenyl)-[1,2,4]oxadiazol-5-ylmethyl]-cyclohexyl}-phenyl-methyl)-dimethyl-amin
256. {(3-Fluor-phenyl)-[4-(3-m-tolyl-[1,2,4]oxadiazol-5-ylmethyl)-cyclohexyl]-methyl}-dimethyl-amin
257. {(4-Fluor-phenyl)-[4-(3-m-tolyl-[1,2,4]oxadiazol-5-ylmethyl)-cyclohexyl]-methyl}-dimethyl-amin
258. Dimethyl-(phenyl-[4-(3-m-tolyl-[1 I2,4]oxadiazol-5-ylmethyl)-cyclohexyl]-methyl}-amin
259. [{4-[3-(4-Chlor-phenyl)-[1,2,4]oxadiazol-5-ylmethyl]-cyclohexyl}-(3-fluor-phenyl)-methyl]-dimethyl-amin
260. ({4-[3-(4-Chlor-phenyl)-[1,2,4]oxadiazol-5-ylmethyl]-cyclohexyl}-phenyl-methyl)-dimethyl-amin
261. ((3-Fluor-phenyl)-{4-[3-(4-trifluormethoxy-phenyl)-[1,2,4]oxadiazol-5-ylmethyl]-cyclohexyl}-methyl)-dimethyl-amin
262. ((4-Fluor-phenyl)-{4-[3-(4-trifluormethoxy-phenyl)-[1,2,4]oxadiazol-5-ylmethyl]-cyclohexyl}-methyl)-dimethyl-amin
263. Dimethyl-(phenyl-{4-[3-(4-trifluormethoxy-phenyl)-[1,2,4]oxadiazol-5-ylmethyl]-cyclohexyl}-methyl)-amin
264. ((4-Fluor-phenyl)-{4-[2-(3-phenyl-[1,2,4]oxadiazol-5-yl)-ethyl]-cyclohexyl}-methyl)-dimethyl-amin
265. Dimethyl-(phenyl-{4-[2-(3-phenyl-[1,2,4]oxadiazol-5-yl)-ethyl]-cyclohexyl}-methyl)-amin
266. Dimethyl-(3-phenyl-1-{4-[2-(3-phenyl-[1,2,4]oxadiazol-5-yl)-ethyl]-cyclohexyl}-propyl)-amin
267. ((3-Fluor-phenyl)-{4-[2-(3-phenyl-[1,2,4]oxadiazol-5-yl)-ethyl]-cyclohexyl}-methyl)-dimethyl-amin
268. [(4-Fluor-phenyl)-(4-{2-[3-(4-methoxy-benzyl)-[1,2,4]oxadiazol-5-yl]-ethyl}-cyclohexyl)-methyl]-dimethyl-amin
269. [(4-{2-[3-(4-Methoxy-benzyl)-[1,2,4]oxadiazol-5-yl]-ethyl}-cyclohexyl)-phenylmethyl]-dimethyl-amin
270. [1-(4-{2-[3-(4-Methoxy-benzyl)-[1,2,4]oxadiazol-5-yl]-ethyl}-cyclohexyl)-3-phenylpropyl]-dimethyl-amin
271. [(3-Fluor-phenyl)-(4-{2-[3-(4-methoxy-benzyl)-[1,2,4]oxadiazol-5-yl]-ethyl}-cyclohexyl)-methyl]-dimethyl-amin
272. [(4-{2-[3-(4-Chlor-benzyl)-[1,2,4]oxadiazol-5-yl]-ethyl}-cyclohexyl)-(4-fluor-phenyl)-methyl]-dimethyl-amin
273. [(4-{2-[3-(4-Chlor-benzyl)-[1,2,4]oxadiazol-5-yl]-ethyl}-cyclohexyl)-phenyl-methyl]-dimethyl-amin
274. [1-(4-{2-[3-(4-Chlor-benzyl)-[1,2,4]oxadiazol-5-yl]-ethyl}-cyclohexyl)-3-phenylpropyl]-dimethyl-amin
275. [(4-{2-[3-(4-Chlor-benzyl)-[1,2,4]oxadiazol-5-yl]-ethyl}-cyclohexyl)-(3-fluor-phenyl)-methyl]-dimethyl-amin
276. [(4-{2-[3-(3-Chlor-phenyl)-[1,2,4]oxadiazol-5-yl]-ethyl}-cyclohexyl)-(4-fluor-phenyl)-methyl]-dimethyl-amin
277. [(4-{2-[3-(3-Chlor-phenyl)-[1,2,4]oxadiazol-5-yl]-ethyl}-cyclohexyl)-phenyl-methyl]-dimethyl-amin
278. [1-(4-{2-[3-(3-Chlor-phenyl)-[1,2,4]oxadiazol-5-yl]-ethyl}-cyclohexyl)-3-phenylpropyl]-dimethyl-amin
279. [(4-{2-[3-(3-Chlor-phenyl)-[1,2,4]oxadiazol-5-yl]-ethyl}-cyclohexyl)-(3-fluor-phenyl)-methyl]-dimethyl-amin
280. [(4-{2-[3-(2,4-Difluor-phenyl)-[1,2,4]oxadiazol-5-yl]-ethyl}-cyclohexyl)-(4-fluorphenyl)-methyl]-dimethyl-amin
281. [(4-{2-[3-(2,4-Difluor-phenyl)-[1,2,4]oxadiazol-5-yl]-ethyl}-cyclohexyl)-phenylmethyl]-dimethyl-amin
282. [(4-{2-[3-(2,4-Difluor-phenyl)-[1,2,4]oxadiazol-5-yl]-ethyl}-cyclohexyl)-(3-fluorphenyl)-methyl]-dimethyl-amin
283. [(4-{2-[3-(3,4-Dimethoxy-phenyl)-[1,2,4]oxadiazol-5-yl]-ethyl}-cyclohexyl)-(4-fluorphenyl)-methyl]-dimethyl-amin
284. [(4-{2-[3-(3,4-Dimethoxy-phenyl)-[1,2,4]oxadiazol-5-yl]-ethyl}-cyclohexyl)-phenyl-methyl]-dimethyl-amin
285. [1-(4-{2-[3-(3,4-Dimethoxy-phenyl)-[1,2,4]oxadiazol-5-yl]-ethyl}-cyclohexyl)-3-phenylpropyl]-dimethyl-amin
286. [(4-{2-[3-(3,4-Dimethoxy-phenyl)-[1,2,4]oxadiazol-5-yl]-ethyl}-cyclohexyl)-(3-fluorphenyl)-methyl]-dimethyl-amin
287. ((4-Fluor-phenyl)-{4-[2-(3-p-tolyl-[1,2,4]oxadiazol-5-yl)-ethyl]-cyclohexyl}-methyl)-dimethyl-amin
288. Dimethyl-(phenyl-{4-[2-(3-p-tolyl-[1,2,4]oxadiazol-5-yl)-ethyl]-cyclohexyl}-methyl)-amin
289. Dimethyl-(3-phenyl-1-{4-[2-(3-p-tolyl-[1,2,4]oxadiazol-5-yl)-ethyl]-cyclohexyl}-propyl)-amin
290. ((3-Fluor-phenyl)-{4-[2-(3-p-tolyl-[1,2,4]oxadiazol-5-yl)-ethyl]-cyclohexyl}-methyl)-dimethyl-amin
291. [(4-{2-[3-(4-tert-Butyl-phenyl)-[1,2,4]oxadiazol-5-yl]-ethyl}-cyclohexyl)-(4-fluor-phenyl)-methyl]-dimethyl-amin
292. [(4-{2-[3-(4-tert-Butyl-phenyl)-[1,2,4]oxadiazol-5-yl]-ethyl}-cyclohexyl)-phenylmethyl]-dimethyl-amin
293. [1-(4-{2-[3-(4-tert-Butyl-phenyl)-[1,2,4]oxadiazol-5-yl]-ethyl}-cyclohexyl)-3-phenylpropyl]-dimethyl-amin
294. [(4-{2-[3-(4-tert-Butyl-phenyl)-[1,2,4]oxadiazol-5-yl]-ethyl}-cyclohexyl)-(3-fluorphenyl)-methyl]-dimethyl-amin
295. ((4-Fluor-phenyl)-{4-[2-(3-m-tolyl-[1,2,4]oxadiazol-5-yl)-ethyl]-cyclohexyl}-methyl)-dimethyl-amin
296. Dimethyl-(phenyl-{4-[2-(3-m-tolyl-[1,2,4]oxadiazol-5-yl)-ethyl]-cyclohexyl}-methyl)-amin
297. Dimethyl-(3-phenyl-1-{4-[2-(3-m-tolyl-[1,2,4]oxadiazol-5-yl)-ethyl]-cyclohexyl}-propyl)-amin
298. ((3-Fluor-phenyl)-{4-[2-(3-m-tolyl-[1,2,4]oxadiazol-5-yl)-ethyl]-cyclohexyl}-methyl)-dimethyl-amin
299. [(4-{2-[3-(4-Chlor-phenyl)-[1,2,4]oxadiazol-5-yl]-ethyl}-cyclohexyl)-(4-fluor-phenyl)-methyl]-dimethyl-amin
300. [(4-{2-[3-(4-Chlor-phenyl)-[1,2,4]oxadiazol-5-yl]-ethyl}-cyclohexyl)-phenyl-methyl]-dimethyl-amin
301. [1-(4-{2-[3-(4-Chlor-phenyl)-[1,2,4]oxadiazol-5-yl]-ethyl}-cyclohexyl)-3-phenylpropyl]-dimethyl-amin
302. [(4-{2-[3-(4-Chlor-phenyl)-[1,2,4]oxadiazol-5-yl]-ethyl}-cyclohexyl)-(3-fluor-phenyl)-methyl]-dimethyl-amin
303. [(4-{2-[3-(2,3-Dichlor-phenyl)-[1,2,4]oxadiazol-5-yl]-ethyl}-cyclohexyl)-(4-fluorphenyl)-methyl]-dimethyl-amin
304. [(4-{2-[3-(2,3-Dichlor-phenyl)-[1,2,4]oxadiazol-5-yl]-ethyl}-cyclohexyl)-phenylmethyl]-dimethyl-amin
305. [1-(4-{2-[3-(2,3-Dichlor-phenyl)-[1,2,4]oxadiazol-5-yl]-ethyl}-cyclohexyl)-3-phenylpropyl]-dimethyl-amin
306. [(4-{2-[3-(2,3-Dichlor-phenyl)-[1,2,4]oxadiazol-5-yl]-ethyl}-cyclohexyl)-(3-fluorphenyl)-methyl]-dimethyl-amin
307. [(4-Fluor-phenyl)-(4-{2-[3-(4-trifluormethoxy-phenyl)-[1,2,4]oxadiazol-5-yl]-ethyl}-cyclohexyl)-methyl]-dimethyl-amin
308. Dimethyl-[phenyl-(4-{2-[3-(4-trifluormethoxy-phenyl)-[1,2,4]oxadiazol-5-yl]-ethyl}-cyclohexyl)-methyl]-amin
309. Dimethyl-[3-phenyl-1-(4-{2-[3-(4-trifluormethoxy-phenyl)-[1,2,4]oxadiazol-5-yl]-ethyl}-cyclohexyl)-propyl]-amin
310. [(3-Fluor-phenyl)-(4-{2-[3-(4-trifluormethoxy-phenyl)-[1,2,4]oxadiazol-5-yl]-ethyl}-cyclohexyl)-methyl]-dimethyl-amin
311. (4-((3-(4-Fluorobenzyl)-1,2,4-oxadiazol-5-yl)methyl)cyclohexyl)(4-fluorophenyl)-N,N-dimethylmethanamin
312. (4-((3-(4-Fluorobenzyl)-1,2,4-oxadiazol-5-yl)methyl)cyclohexyl)-N,N-dimethyl(phenyl)methanamin
313. (4-((3-(3-Chlorobenzyl)-1,2,4-oxadiazol-5-yl)methyl)cyclohexyl)(3-fluorophenyl)-N,N-dimethylmethanamin
314. (4-((3-(3-Chlorobenzyl)-1,2,4-oxadiazol-5-yl)methyl)cyclohexyl)(4-fluorophenyl)-N,N-dimethylmethanamin
315. (4-((3-(3-Chlorobenzyl)-1,2,4-oxadiazol-5-yl)methyl)cyclohexyl)-N,N-dimethyl(phenyl)methanamin
316. (4-((3-(3-Bromophenyl)-1,2,4-oxadiazol-5-yl)methyl)cyclohexyl)(3-fluorophenyl)-N,N-dimethylmethanamin
317. (4-((3-(3-Bromophenyl)-1,2,4-oxadiazol-5-yl)methyl)cyclohexyl)(4-fluorophenyl)-N,N-dimethylmethanamin
318. (4-((3-(3-Bromophenyl)-1,2,4-oxadiazol-5-yl)methyl)cyclohexyl)-N,N-dimethyl(phenyl)methanamin
319. (4-((3-(4-Bromobenzyl)-1,2,4-oxadiazol-5-yl)methyl)cyclohexyl)(3-fluorophenyl)-N,N-dimethylmethanamin
320. (4-((3-(4-Bromobenzyl)-1,2,4-oxadiazol-5-yl)methyl)cyclohexyl)(4-fluorophenyl)-N,N-dimethylmethanamin
321. (4-((3-(4-Bromobenzyl)-1,2,4-oxadiazol-5-yl)methyl)cyclohexyl)-N,N-dimethyl(phenyl)methanamin
322. (3-Fluorophenyl)-N,N-dimethyl(4-((3-(thiophen-2-ylmethyl)-1,2,4-oxadiazol-5-yl)methyl)cyclohexyl)methanamin
323. (4-Fluorophenyl)-N,N-dimethyl(4-((3-(thiophen-2-ylmethyl)-1,2,4-oxadiazol-5-yl)methyl)cyclohexyl)methanamin
324. N,N-Dimethyl(phenyl)(4-((3-(thiophen-2-ylmethyl)-1,2,4-oxadiazol-5-yl)methyl)cyclohexyl)methanamin
325. (4-((3-Benzyl-1,2,4-oxadiazol-5-yl)methyl)cyclohexyl)(3-fluorophenyl)-N,N-dimethylmethanamin
326. (4-((3-Benzyl-1,2,4-oxadiazol-5-yl)methyl)cyclohexyl)(4-fluorophenyl)-N,N-dimethylmethanamin
327. (4-((3-Benzyl-1,2,4-oxadiazol-5-yl)methyl)cyclohexyl)-N,N-dimethyl(phenyl)methanamin
328. (3-Fluorophenyl)-N,N-dimethyl(4-((3-phenethyl-1,2,4-oxadiazol-5-yl)methyl)cyclohexyl)methanamin
329. (4-Fluorophenyl)-N,N-dimethyl(4-((3-phenethyl-1,2,4-oxadiazol-5-yl)methyl)cyclohexyl)methanamin
330. N,N-Dimethyl(4-((3-phenethyl-1,2,4-oxadiazol-5-yl)methyl)cyclohexyl)(phenyl)methanamin
331. (4-((3-((1H-Indol-3-yl)methyl)-1,2,4-oxadiazol-5-yl)methyl)cyclohexyl)(3- fluorophenyl)-N,N-dimethylmethanamin

9. Verfahren zur Herstellung eines Oxadiazol-Derivates gemäß Anspruch 1, wobei Amidoxime der allgemeinen Formel D in einem Reaktionsmedium unter Zugabe einer Base, beispielsweise NaH, mit sowohl gesättigten als auch ungesättigten Estern der allgemeinen Formel A zu den erfindungsgemäßen Verbindungen umgesetzt werden.

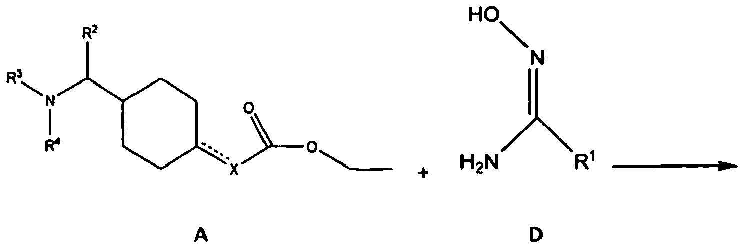

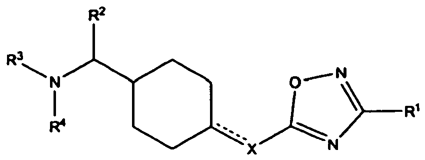

10. Arzneimittel enthaltend wenigstens ein substituiertes Oxadiazol-Derivat gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, sowie gegebenenfalls enthaltend geeignete Zusatzund/oder Hilfsstoffe und/oder gegebenenfalls weiterer Wirkstoffe.

11. Verwendung eines substituierten Oxadiazol-Derivats gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Schmerz, insbesondere von akutem, neuropathischem oder chronischem Schmerz.

12. Verwendung eines substituierten Oxadiazol-Derivats gemäß Anspruch 1, gegebenenfalls in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren, zur Herstellung eines Arzneimittels zur Behandlung von Depressionen, Harninkontinenz, Diarrhöe, Pruritus, Alkoholund Drogenmissbrauch, Medikamentenabhängigkeit, Antriebslosigkeit und/oder zur Anxiolyse.

## Claims

1. Substituted oxadiazole derivatives of the general formula I, in which 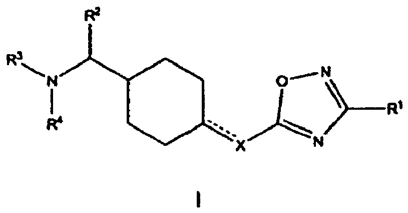 X means CH, CH₂, CH=CH, CH₂CH₂, CH₂CH=CH or CH₂CH₂CH₂
R¹ means aryl, heteroaryl, in each case unsubstituted or mono- or polysubstituted; a C₁₋₃ alkyl group-linked aryl or heteroaryl residue, in each case unsubstituted or mono- or polysubstituted;
R² means aryl or heteroaryl, in each case unsubstituted or mono- or polysubstituted; a C₁₋₃ alkyl chain-attached aryl residue, in each case unsubstituted or mono- or polysubstituted;
R³ and R⁴ mutually independently mean H; C₁₋₆ alkyl, in each case saturated or unsaturated, branched or unbranched, wherein R³ and R⁴ do not simultaneously mean H,
or the residues R³ and R⁴ together mean CH₂CH₂OCH₂CH₂, or (CH₂)₃₋₆,
in the form of the racemate; the enantiomers, diastereomers, mixtures of the enantiomers or diastereomers or of an individual enantiomer or diastereomer; the bases and/or salts of physiologically acceptable acids, wherein
in connection with "alkyl", the term "substituted" is taken to mean the substitution of a hydrogen residue by F, Cl, Br, I, -CN, NH₂, NH-C₁₋₆-alkyl, NH-C₁₋₆-alkyl-OH, -C₁₋₆ alkyl, N(C₁₋₆-alkyl)₂, N(C₁₋₆-alkyl-OH)₂, -NO₂, SH, S-C₁₋₆-alkyl, S-benzyl, -O-C₁₋₆-alkyl, OH, O-C₁₋₆-alkyl-OH, =O, O-benzyl, C(=O)-C₁₋₆-alkyl, CO₂H, CO₂-C₁₋₆-alkyl, or benzyl, and wherein
with regard to "aryl" and "heteroaryl", "mono- or polysubstituted" is taken to mean mono- or polysubstitution of one or more hydrogen atoms of the ring system by F, Cl, Br, I, CN, NH₂, NH-C₁₋₆-alkyl, NH-C₁₋₆-alkyl-OH, N(C₁₋₆-alkyl)₂, N(C₁₋₆-alkyl-OH)₂, NO₂, SH, -S-C₁₋₆-alkyl, OH, O-C₁₋₆-alkyl, O-C₁₋₆-alkyl-OH, C(=O)-C₁₋₆-alkyl, CO₂H, CO₂-C₁₋₆-alkyl, CF₃, C₁₋₆ alkyl.

2. Substituted oxadiazole derivatives according to claim 1, in which R¹ means phenyl, naphthyl, thienyl, pyridyl or pyrrolyl, benzyl, methylindolyl, methylthienyl or phenethyl, unsubstituted or mono- or polysubstituted with F, Cl, Br, CN, OCF₃, NH₂, NO₂, SH, S-C₁₋₆-alkyl, OH, O-C₁₋₆-alkyl, CO₂H, CO₂-C₁₋₆-alkyl, CF₃ or C₁₋₆ alkyl.

3. Substituted oxadiazole derivatives according to claim 2, in which R¹ means phenyl, thienyl, benzyl, methylindolyl, methylthienyl or pyrrolyl, unsubstituted or mono- or polysubstituted with Cl, Br, OCH₃, CH₃, F, OCF₃, CF₃ or *tert*.-butyl.

4. Substituted oxadiazole derivatives according to claim 1, in which R² means phenyl or thienyl, in each case unsubstituted or mono- or polysubstituted with F, Cl, CN, NO₂, SH, S-C₁₋₆-alkyl, OH, O-C₁₋₆-alkyl, CO₂H, CO₂-C₁₋₆-alkyl, CF₃, C₁₋₆ alkyl; a C₁₋₃ alkyl chain-attached phenyl residue, in each case unsubstituted or mono- or polysubstituted with F, Cl, CN, NO₂, SH, S-C₁₋₆-alkyl, OH, O-C₁₋₆-alkyl, CO₂H, CO₂-C₁₋₆-alkyl, CF₃, C₁₋₆ alkyl.

5. Substituted oxadiazole derivatives according to claim 4, in which R² means phenyl, unsubstituted or monosubstituted with Cl or F, phenethyl or thienyl.

6. Substituted oxadiazole derivatives according to claim 1, in which R³ and R⁴ mutually independently mean H or C₁₋₆ alkyl, wherein R³ and R⁴ do not simultaneously mean H,
or the residues R³ and R⁴ together mean CH₂CH₂OCH₂CH₂, or (CH₂)₄₋₅.

7. Substituted oxadiazole derivatives according to claim 6, in which R³ and R⁴ mean CH₃.

8. Substituted oxadiazole derivatives according to claim 1 from the group 43. (4-((3-(4-chlorobenzyl)-1,2,4-oxadiazol-5-yl)methyl)cyclohexyl)-N,N-dimethyl(phenyl)methanamine
44. (4-((3-(4-methoxybenzyl)-1,2,4-oxadiazol-5-yl)methyl)cyclohexyl)-N,N-dimethyl(phenyl)methanamine
45. ((4-chlorophenyl)-{4-[3-(4-chlorophenyl)-[1,2,4]oxadiazol-5-ylmethylene]-cyclohexyl}-methyl)-dimethylamine
46. [{4-[3-(4-chlorophenyl)-[1,2,4]oxadiazol-5-ylmethylene]-cyclohexyl}-(4-fluorophenyl)-methyl]-dimethylamine (more highly polar diastereomer)
47. [{4-[3-(4-chlorophenyl)-[1,2,4]oxadiazol-5-ylmethylene]-cyclohexyl}-(4-fluorophenyl)-methyl]-dimethylamine (more highly nonpolar diastereomer)
48. ({4-[3-(2,3-dichlorophenyl)-[1,2,4]oxadiazol-5-ylmethylene]-cyclohexyl}-phenylmethyl)-dimethylamine (more highly polar diastereomer)
49. ({4-[3-(2,3-dichlorophenyl)-[1,2,4]oxadiazol-5-ylmethylene]-cyclohexyl}-phenylmethyl)-dimethylamine (more highly nonpolar diastereomer)
50. [{4-[3-(2,3-dichlorophenyl)-[1,2,4]oxadiazol-5-ylmethylene]-cyclohexyl}-(3-fluorophenyl)-methyl]-dimethylamine (more highly polar diastereomer)
51. [{4-[3-(2,3-dichlorophenyl)-[1,2,4]oxadiazol-5-ylmethylene]-cyclohexyl}-(3-fluorophenyl)-methyl]-dimethylamine (more highly nonpolar diastereomer)
52. ((4-chlorophenyl)-{4-[3-(2,3-dichlorophenyl)-[1,2,4]oxadiazol-5-ylmethylene]-cyclohexyl}-methyl)-dimethylamine (more highly polar diastereomer)
53. ((4-chlorophenyl)-{4-[3-(2,3-dichlorophenyl)-[1,2,4]oxadiazol-5-ylmethylene]-cyclohexyl}-methyl)-dimethylamine (more highly nonpolar diastereomer)
54. (1-{4-[3-(2,3-dichlorophenyl)-[1,2,4]oxadiazol-5-ylmethylene]-cyclohexyl}-3-phenylpropyl)-dimethylamine
55. [{4-[3-(2,3-dichlorophenyl)-[1,2,4]oxadiazol-5-ylmethylene]-cyclohexyl}-(4-fluorophenyl)-methyl]-dimethylamine (more highly polar diastereomer)
56. [{4-[3-(2,3-dichlorophenyl)-[1,2,4]oxadiazol-5-ylmethylene]-cyclohexyl}-(4-fluorophenyl)-methyl]-dimethylamine (more highly nonpolar diastereomer)
57. dimethyl-(phenyl-{4-[3-(4-trifluoromethoxyphenyl)-[1,2,4]oxadiazol-5-ylmethylene]-cyclohexyl}-methyl)-amine (more highly polar diastereomer)
58. dimethyl-(phenyl-{4-[3-(4-trifluoromethoxyphenyl)-[1,2,4]oxadiazol-5-ylmethylene]-cyclohexyl}-methyl)-amine (more highly nonpolar diastereomer)
59. ((3-fluorophenyl)-{4-[3-(4-trifluoromethoxyphenyl)-[1,2,4]oxadiazol-5-ylmethylene]-cyclohexyl}-methyl)-dimethylamine (more highly polar diastereomer)
60. ((3-fluorophenyl)-{4-[3-(4-trifluoromethoxyphenyl)-[1,2,4]oxadiazol-5-ylmethylene]-cyclohexyl}-methyl)-dimethylamine (more highly nonpolar diastereomer)
61. ((4-chlorophenyl)-{4-[3-(4-trifluoromethoxyphenyl)-[1,2,4]oxadiazol-5-ylmethylene]-cyclohexyl}-methyl)-dimethylamine (more highly polar diastereomer)
62. ((4-chlorophenyl)-{4-[3-(4-trifluoromethoxyphenyl)-[1,2,4]oxadiazol-5-ylmethylene]-cyclohexyl}-methyl)-dimethylamine (more highly nonpolar diastereomer)
63. dimethyl-(3-phenyl-1-{4-[3-(4-trifluoromethoxyphenyl)-[1,2,4]oxadiazol-5-ylmethylene]-cyclohexyl}-propyl)-amine (more highly polar diastereomer)
64. dimethyl-(3-phenyl-1-{4-[3-(4-trifluoromethoxyphenyl)-[1,2,4]oxadiazol-5-ylmethylene]-cyclohexyl}-propyl)-amine (more highly nonpolar diastereomer)
65. ((4-fluorophenyl)-{4-[3-(4-trifluoromethoxyphenyl)-[1,2,4]oxadiazol-5-ylmethylene]-cyclohexyl}-methyl)-dimethylamine (more highly polar diastereomer)
66. ((4-fluorophenyl)-{4-[3-(4-trifluoromethoxyphenyl)-[1,2,4]oxadiazol-5-ylmethylene]-cyclohexyl}-methyl)-dimethylamine (more highly nonpolar diastereomer)
67. dimethyl-(thiophen-2-yl-{4-[3-(4-trifluoromethoxyphenyl)-[1,2,4]oxadiazol-5-ylmethylene]-cyclohexyl}-methyl)-amine (more highly polar diastereomer)
68. dimethyl-(thiophen-2-yl-{4-[3-(4-trifluoromethoxyphenyl)-[1,2,4]oxadiazol-5-ylmethylene]-cyclohexyl}-methyl)-amine (more highly nonpolar diastereomer)
69. dimethyl-{phenyl-[4-(3-phenyl-[1,2,4]oxadiazol-5-ylmethylene)-cyclohexyl]-methyl}-amine (more highly polar diastereomer)
70. dimethyl-{phenyl-[4-(3-phenyl-[1,2,4]oxadiazol-5-ylmethylene)-cyclohexyl]-methyl}-amine (more highly nonpolar diastereomer)
71. {(3-fluorophenyl)-[4-(3-phenyl-[1,2,4]oxadiazol-5-ylmethylene)-cyclohexyl]-methyl}-dimethylamine (more highly polar diastereomer)
72. {(3-fluorophenyl)-[4-(3-phenyl-[1,2,4]oxadiazol-5-ylmethylene)-cyclohexyl]-methyl}-dimethylamine (more highly nonpolar diastereomer)
73. dimethyl-{3-phenyl-1-[4-(3-phenyl-[1,2,4]oxadiazol-5-ylmethylene)-cyclohexyl]-propyl}-amine (more highly polar diastereomer)
74. dimethyl-{3-phenyl-1-[4-(3-phenyl-[1,2,4]oxadiazol-5-ylmethylene)-cyclohexyl]-propyl}-amine (more highly nonpolar diastereomer)
75. {(4-fluorophenyl)-[4-(3-phenyl-[1,2,4]oxadiazol-5-ylmethylene)-cyclohexyl]-methyl}-dimethylamine (more highly polar diastereomer)
76. {(4-fluorophenyl)-[4-(3-phenyl-[1,2,4]oxadiazol-5-ylmethylene)-cyclohexyl]-methyl}-dimethylamine (more highly nonpolar diastereomer)
77. [{4-[3-(2,4-difluorophenyl)-[1,2,4]oxadiazol-5-ylmethylene]-cyclohexyl}-(3-fluorophenyl)-methyl]-dimethylamine (more highly polar diastereomer)
78. [{4-[3-(2,4-difluorophenyl)-[1,2,4]oxadiazol-5-ylmethylene]-cyclohexyl}-(3-fluorophenyl)-methyl]-dimethylamine (more highly nonpolar diastereomer)
79. ({4-[3-(4-methoxybenzyl)-[1,2,4]oxadiazol-5-ylmethylene]-cyclohexyl}-phenylmethyl)-dimethylamine (more highly polar diastereomer)
80. ({4-[3-(4-methoxybenzyl)-[1,2,4]oxadiazol-5-ylmethylene]-cyclohexyl}-phenylmethyl)-dimethylamine (more highly nonpolar diastereomer)
81. ((3-fluorophenyl)-{4-[3-(4-methoxybenzyl)-[1,2,4]oxadiazol-5-ylmethylene]-cyclohexyl}-methyl)-dimethylamine (more highly polar diastereomer)
82. ((3-fluorophenyl)-{4-[3-(4-methoxybenzyl)-[1,2,4]oxadiazol-5-ylmethylene]-cyclohexyl}-methyl)-dimethylamine (more highly nonpolar diastereomer)
83. ((4-chlorophenyl)-{4-[3-(4-methoxybenzyl)-[1,2,4]oxadiazol-5-ylmethylene]-cyclohexyl}-methyl)-dimethylamine (more highly polar diastereomer)
84. ((4-chlorophenyl)-{4-[3-(4-methoxybenzyl)-[1,2,4]oxadiazol-5-ylmethylene]-cyclohexyl}-methyl)-dimethylamine (more highly nonpolar diastereomer)
85. (1-{4-[3-(4-methoxybenzyl)-[1,2,4]oxadiazol-5-ylmethylene]-cyclohexyl}-3-phenylpropyl)-dimethylamine (more highly polar diastereomer)
86. (1-{4-[3-(4-methoxybenzyl)-[1,2,4]oxadiazol-5-ylmethylene]-cyclohexyl}-3-phenylpropyl)-dimethylamine (more highly nonpolar diastereomer)
87. ((4-fluorophenyl)-{4-[3-(4-methoxybenzyl)-[1,2,4]oxadiazol-5-ylmethylene]-cyclohexyl}-methyl)-dimethylamine (more highly polar diastereomer)
88. ((4-fluorophenyl)-{4-[3-(4-methoxybenzyl)-[1,2,4]oxadiazol-5-ylmethylene]-cyclohexyl}-methyl)-dimethylamine (more highly nonpolar diastereomer)
89. ({4-[3-(4-chlorobenzyl)-[1,2,4]oxadiazol-5-ylmethylene]-cyclohexyl}-phenylmethyl)-dimethylamine (more highly polar diastereomer)
90. ({4-[3-(4-chlorobenzyl)-[1,2,4]oxadiazol-5-ylmethylene]-cyclohexyl}-phenylmethyl)-dimethylamine (more highly nonpolar diastereomer)
91. [{4-[3-(4-chlorobenzyl)-[1,2,4]oxadiazol-5-ylmethylene]-cyclohexyl}-(3-fluorophenyl)-methyl]-dimethylamine (more highly polar diastereomer)
92. [{4-[3-(4-chlorobenzyl)-[1,2,4]oxadiazol-5-ylmethylene]-cyclohexyl}-(3-fluorophenyl)-methyl]-dimethylamine (more highly nonpolar diastereomer)
93. [{4-[3-(4-chlorobenzyl)-[1,2,4]oxadiazol-5-ylmethylene]-cyclohexyl}-(4-chlorophenyl)-methyl]-dimethylamine (more highly polar diastereomer)
94. [{4-[3-(4-chlorobenzyl)-[1,2,4]oxadiazol-5-ylmethylene]-cyclohexyl}-(4-chlorophenyl)-methyl]-dimethylamine (more highly nonpolar diastereomer)
95. (1-{4-[3-(4-chlorobenzyl)-[1,2,4]oxadiazol-5-ylmethylene]-cyclohexyl}-3-phenylpropyl)-dimethylamine (more highly polar diastereomer)
96. (1-{4-[3-(4-chlorobenzyl)-[1,2,4]oxadiazol-5-ylmethylene]-cyclohexyl}-3-phenylpropyl)-dimethylamine (more highly nonpolar diastereomer)
97. [{4-[3-(4-chlorobenzyl)-[1,2,4]oxadiazol-5-ylmethylene]-cyclohexyl}-(4-fluorophenyl)-methyl]-dimethylamine (more highly polar diastereomer)
98. [{4-[3-(4-chlorobenzyl)-[1,2,4]oxadiazol-5-ylmethylene]-cyclohexyl}-(4-fluorophenyl)-methyl]-dimethylamine (more highly nonpolar diastereomer)
99. {(3-fluorophenyl)-[4-(3-p-tolyl-[1,2,4]oxadiazol-5-ylmethylene)-cyclohexyl]-methyl}-dimethylamine
100. {(4-chlorophenyl)-[4-(3-p-tolyl-[1,2,4]oxadiazol-5-ylmethylene)-cyclohexyl]-methyl}-dimethylamine
101. {(4-fluorophenyl)-[4-(3-p-tolyl-[1,2,4]oxadiazol-5-ylmethylene)-cyclohexyl]-methyl}-dimethylamine (more highly polar diastereomer)
102. {(4-fluorophenyl)-[4-(3-p-tolyl-[1,2,4]oxadiazol-5-ylmethylene)-cyclohexyl]-methyl}-dimethylamine (more highly nonpolar diastereomer)
103. ({4-[3-(3,4-dimethoxyphenyl)-[1,2,4]oxadiazol-5-ylmethylene]-cyclohexyl}-phenylmethyl)-dimethylamine (more highly polar diastereomer)
104. ({4-[3-(3,4-dimethoxyphenyl)-[1,2,4]oxadiazol-5-ylmethylene]-cyclohexyl}-phenylmethyl)-dimethylamine (more highly nonpolar diastereomer)
105. [{4-[3-(3,4-dimethoxyphenyl)-[1,2,4]oxadiazol-5-ylmethylene]-cyclohexyl}-(3-fluorophenyl)-methyl]-dimethylamine (more highly polar diastereomer)
106. [{4-[3-(3,4-dimethoxyphenyl)-[1,2,4]oxadiazol-5-ylmethylene]-cyclohexyl}-(3-fluorophenyl)-methyl]-dimethylamine (more highly nonpolar diastereomer)
107. ((4-chlorophenyl)-{4-[3-(3,4-dimethoxyphenyl)-[1,2,4]oxadiazol-5-ylmethylene]-cyclohexyl}-methyl)-dimethylamine (more highly polar diastereomer)
108. ((4-chlorophenyl)-{4-[3-(3,4-dimethoxyphenyl)-[1,2,4]oxadiazol-5-ylmethylene]-cyclohexyl}-methyl)-dimethylamine (more highly nonpolar diastereomer)
109. (1-{4-[3-(3,4-dimethoxyphenyl)-[1,2,4]oxadiazol-5-ylmethylene]-cyclohexyl}-3-phenylpropyl)-dimethylamine (more highly polar diastereomer)
110. (1-{4-[3-(3,4-dimethoxyphenyl)-[1,2,4]oxadiazol-5-ylmethylene]-cyclohexyl}-3-phenylpropyl)-dimethylamine (more highly nonpolar diastereomer)
111. [{4-[3-(3,4-dimethoxyphenyl)-[1,2,4]oxadiazol-5-ylmethylene]-cyclohexyl}-(4-fluorophenyl)-methyl]-dimethylamine (more highly polar diastereomer)
112. ({4-[3-(3,4-dimethoxyphenyl)-[1,2,4]oxadiazol-5-ylmethylene]-cyclohexyl}-(4-fluorophenyl)-methyl]-dimethylamine (more highly nonpolar diastereomer)
113. ({4-[3-(3-chlorophenyl)-[1,2,4]oxadiazol-5-ylmethylene]-cyclohexyl}-phenylmethyl)-dimethylamine (more highly polar diastereomer)
114. ({4-[3-(3-chlorophenyl)-[1,2,4]oxadiazol-5-ylmethylene]-cyclohexyl}-phenylmethyl)-dimethylamine (more highly nonpolar diastereomer)
115. [{4-[3-(3-chlorophenyl)-[1,2,4]oxadiazol-5-ylmethylene]-cyclohexyl}-(3-fluorophenyl)-methyl]-dimethylamine (more highly polar diastereomer)
116. [{4-[3-(3-chlorophenyl)-[1,2,4]oxadiazol-5-ylmethylene]-cyclohexyl}-(3-fluorophenyl)-methyl]-dimethylamine (more highly nonpolar diastereomer)
117. ((4-chlorophenyl)-{4-[3-(3-chlorophenyl)-[1,2,4]oxadiazol-5-ylmethylene]-cyclohexyl}-methyl)-dimethylamine (more highly polar diastereomer)
118. ((4-chlorophenyl)-{4-[3-(3-chlorophenyl)-[1,2,4]oxadiazol-5-ylmethylene]-cyclohexyl}-methyl)-dimethylamine (more highly nonpolar diastereomer)
119. (1-{4-[3-(3-chlorophenyl)-[1,2,4]oxadiazol-5-ylmethylene]-cyclohexyl}-3-phenylpropyl)-dimethylamine (more highly polar diastereomer)
120. (1-{4-[3-(3-chlorophenyl)-[1,2,4]oxadiazol-5-ylmethylene]-cyclohexyl}-3-phenylpropyl)-dimethylamine (more highly nonpolar diastereomer)
121. [{4-[3-(3-chlorophenyl)-[1,2,4]oxadiazol-5-ylmethylene]-cyclohexyl}-(4-fluorophenyl)-methyl]-dimethylamine
122. ({4-[3-(4-*tert*.-butylphenyl)-[1,2,4]oxadiazol-5-ylmethylene]-cyclohexyl}-phenylmethyl)-dimethylamine (more highly polar diastereomer)
123. ({4-[3-(4-*tert*.-butylphenyl)-[1,2,4]oxadiazol-5-ylmethylene]-cyclohexyl}-phenylmethyl)-dimethylamine (more highly nonpolar diastereomer)
124. [{4-[3-(4-*tert*.-butylphenyl)-[1,2,4]oxadiazol-5-ylmethylene]-cyclohexyl}-(3-fluorophenyl)-methyl]-dimethylamine (more highly polar diastereomer)
125. [{4-[3-(4-*tert*.-butylphenyl)-[1,2,4]oxadiazol-5-ylmethylene]-cyclohexyl}-(3-fluorophenyl)-methyl]-dimethylamine (more highly nonpolar diastereomer)
126. [{4-[3-(4-*tert*.-butylphenyl)-[1,2,4]oxadiazol-5-ylmethylene]-cyclohexyl}-(4-chlorophenyl)-methyl]-dimethylamine (more highly polar diastereomer)
127. [{4-[3-(4-*tert*.-butylphenyl)-[1,2,4]oxadiazol-5-ylmethylene]-cyclohexyl}-(4-chlorophenyl)-methyl]-dimethylamine (more highly nonpolar diastereomer)
128. (1-{4-[3-(4-*tert*.-butylphenyl)-[1,2,4]oxadiazol-5-ylmethylene]-cyclohexyl}-3-phenylpropyl)-dimethylamine (more highly polar diastereomer)
129. (1-{4-[3-(4-*tert*.-butylphenyl)-[1,2,4]oxadiazol-5-ylmethylene]-cyclohexyl}-3-phenylpropyl)-dimethylamine (more highly nonpolar diastereomer)
130. [{4-[3-(4-*tert*.-butylphenyl)-[1,2,4]oxadiazol-5-ylmethylene]-cyclohexyl}-(4-fluorophenyl)-methyl]-dimethylamine (more highly polar diastereomer)
131. [{4-[3-(4-*tert*.-butylphenyl)-[1,2,4]oxadiazol-5-ylmethylene]-cyclohexyl}-(4-fluorophenyl)-methyl]-dimethylamine (more highly nonpolar diastereomer)
132. ({4-[3-(4-*tert*.-butylphenyl)-[1,2,4]oxadiazol-5-ylmethylene]-cyclohexyl}-thiophen-2-yl-methyl)-dimethylamine (more highly polar diastereomer)
133. ({4-[3-(4-*tert*.-butylphenyl)-[1,2,4]oxadiazol-5-ylmethylene]-cyclohexyl}-thiophen-2-yl-methyl)-dimethylamine (more highly nonpolar diastereomer)
134. dimethyl-{phenyl-[4-(3-m-tolyl-[1,2,4]oxadiazol-5-ylmethylene)-cyclohexyl]-methyl}-amine (more highly polar diastereomer)
135. dimethyl-{phenyl-[4-(3-m-tolyl-[1,2,4]oxadiazol-5-ylmethylene)-cyclohexyl]-methyl}-amine (more highly nonpolar diastereomer)
136. {(3-fluorophenyl)-[4-(3-m-tolyl-[1,2,4]oxadiazol-5-ylmethylene)-cyclohexyl]-methyl}-dimethylamine (more highly polar diastereomer)
137. {(3-fluorophenyl)-[4-(3-m-tolyl-[1,2,4]oxadiazol-5-ylmethylene)-cyclohexyl]-methyl}-dimethylamine (more highly nonpolar diastereomer)
138. {(4-chlorophenyl)-[4-(3-m-tolyl-[1,2,4]oxadiazol-5-ylmethylene)-cyclohexyl]-methyl}-dimethylamine (more highly polar diastereomer)
139. {(4-chlorophenyl)-[4-(3-m-tolyl-[1,2,4]oxadiazol-5-ylmethylene)-cyclohexyl]-methyl}-dimethylamine (more highly nonpolar diastereomer)
140. ((4-fluorophenyl)-{4-[2-(3-phenyl-[1,2,4]oxadiazol-5-yl)-vinyl]-cyclohexyl}-methyl)-dimethylamine
141. [(4-{2-[3-(2,4-difluorophenyl)-[1,2,4]oxadiazol-5-yl]-vinyl}-cyclohexyl)-(4-fluorophenyl)-methyl]-dimethylamine
142. [(4-fluorophenyl)-(4-{2-[3-(4-methoxybenzyl)-[1,2,4]oxadiazol-5-yl]-vinyl}-cyclohexyl)-methyl]-dimethylamine
143. [(4-{2-[3-(4-chlorobenzyl)-[1,2,4]oxadiazol-5-yl]-vinyl}-cyclohexyl)-(4-fluorophenyl)-methyl]-dimethylamine
144. dimethyl-(3-phenyl-1-{4-[2-(3-phenyl-[1,2,4]oxadiazol-5-yl)-vinyl]-cyclohexyl}-propyl)-amine
145. [1-(4-{2-[3-(2,4-difluorophenyl)-[1,2,4]oxadiazol-5-yl]-vinyl}-cyclohexyl)-3-phenylpropyl]-dimethylamine
146. [1-(4-{2-[3-(4-methoxybenzyl)-[1,2,4]oxadiazol-5-yl]-vinyl}-cyclohexyl)-3-phenylpropyl]-dimethylamine
147. [1-(4-{2-[3-(4-chlorobenzyl)-[1,2,4]oxadiazol-5-yl]-vinyl}-cyclohexyl)-3-phenylpropyl]-dimethylamine
148. dimethyl-(phenyl-{4-[2-(3-phenyl-[1,2,4]oxadiazol-5-yl)-vinyl]-cyclohexyl}-methyl)-amine
149. [(4-{2-[3-(4-chlorobenzyl)-[1,2,4]oxadiazol-5-yl]-vinyl}-cyclohexyl)-phenylmethyl]-dimethylamine (more highly polar diastereomer)
150. [(4-{2-[3-(4-chlorobenzyl)-[1,2,4]oxadiazol-5-yl]-vinyl}-cyclohexyl)-phenylmethyl]-dimethylamine (more highly nonpolar diastereomer)
151. [(4-chlorophenyl)-(4-{2-[3-(2,4-difluorophenyl)-[1,2,4]oxadiazol-5-yl]-vinyl}-cyclohexyl)-methyl]-dimethylamine
152. [(4-chlorophenyl)-(4-{2-[3-(4-methoxybenzyl)-[1,2,4]oxadiazol-5-yl]-vinyl}-cyclohexyl)-methyl]-dimethylamine
153. [(4-{2-[3-(4-chlorobenzyl)-[1,2,4]oxadiazol-5-yl]-vinyl}-cyclohexyl)-(4-chlorophenyl)-methyl]-dimethylamine
154. [(4-{2-[3-(4-methoxybenzyl)-[1,2,4]oxadiazol-5-yl]-vinyl}-cyclohexyl)-thiophen-2-yl-methyl]-dimethylamine
155. [(4-{2-[3-(4-chlorobenzyl)-[1,2,4]oxadiazol-5-yl]-vinyl}-cyclohexyl)-thiophen-2-yl-methyl]-dimethylamine
156. ((3-fluorophenyl)-{4-[2-(3-phenyl-[1,2,4]oxadiazol-5-yl)-vinyl]-cyclohexyl}-methyl)-dimethylamine
157. [(3-fluorophenyl)-(4-{2-[3-(4-methoxybenzyl)-[1,2,4]oxadiazol-5-yl]-vinyl}-cyclohexyl)-methyl]-dimethylamine
158. ((4-fluorophenyl)-{4-[2-(3-p-tolyl-[1,2,4]oxadiazol-5-yl)-vinyl]-cyclohexyl}-methyl)-dimethylamine
159. [(4-{2-[3-(3,4-dimethoxyphenyl)-[1,2,4]oxadiazol-5-yl]-vinyl}-cyclohexyl)-(4-fluorophenyl)-methyl]-dimethylamine
160. dimethyl-(3-phenyl-1-{4-[2-(3-p-tolyl-[1,2,4]oxadiazol-5-yl)-vinyl]-cyclohexyl}-propyl)-amine
161. [1-(4-{2-[3-(3,4-dimethoxyphenyl)-[1,2,4]oxadiazol-5-yl]-vinyl}-cyclohexyl)-3-phenylpropyl]-dimethylamine
162. dimethyl-(phenyl-{4-[2-(3-p-tolyl-[1,2,4]oxadiazol-5-yl)-vinyl]-cyclohexyl}-methyl)-amine
163. [(4-{2-[3-(3,4-dimethoxyphenyl)-[1,2,4]oxadiazol-5-yl]-vinyl}-cyclohexyl)-phenylmethyl]-dimethylamine
164. ((4-chlorophenyl)-{4-[2-(3-p-tolyl-[1,2,4]oxadiazol-5-yl)-vinyl]-cyclohexyl}-methyl)-dimethylamine
165. [(4-chlorophenyl)-(4-{2-[3-(3,4-dimethoxyphenyl)-[1,2,4]oxadiazol-5-yl]-vinyl}-cyclohexyl)-methyl]-dimethylamine
166. dimethyl-(thiophen-2-yl-{4-[2-(3-p-tolyl-[1,2,4]oxadiazol-5-yl)-vinyl]-cyclohexyl}-methyl)-amine
167. [(4-{2-[3-(3,4-dimethoxyphenyl)-[1,2,4]oxadiazol-5-yl]-vinyl}-cyclohexyl)-thiophen-2-yl-methyl]-dimethylamine
168. ((3-fluorophenyl)-{4-[2-(3-p-tolyl-[1,2,4]oxadiazol-5-yl)-vinyl]-cyclohexyl}-methyl)-dimethylamine
169. [(4-{2-[3-(3,4-dimethoxyphenyl)-[1,2,4]oxadiazol-5-yl]-vinyl}-cyclohexyl)-(3-fluorophenyl)-methyl]-dimethylamine
170. [(4-{2-[3-(3-chlorophenyl)-[1,2,4]oxadiazol-5-yl]-vinyl}-cyclohexyl)-(4-fluorophenyl)-methyl]-dimethylamine
171. [(4-{2-[3-(4-*tert*.-butylphenyl)-[1,2,4]oxadiazol-5-yl]-vinyl}-cyclohexyl)-(4-fluorophenyl)-methyl]-dimethylamine
172. [(4-{2-[3-(4-*tert*.-butylphenyl)-[1,2,4]oxadiazol-5-yl]-vinyl}-cyclohexyl)-(4-fluorophenyl)-methyl]-dimethylamine
173. ((4-fluorophenyl)-{4-[2-(3-m-tolyl-[1,2,4]oxadiazol-5-yl)-vinyl]-cyclohexyl}-methyl)-dimethylamine
174. [1-(4-{2-[3-(3-chlorophenyl)-[1,2,4]oxadiazol-5-yl]-vinyl}-cyclohexyl)-3-phenylpropyl]-dimethylamine
175. [1-(4-{2-[3-(4-*tert*.-butylphenyl)-[1,2,4]oxadiazol-5-yl]-vinyl}-cyclohexyl)-3-phenylpropyl]-dimethylamine (more highly polar diastereomer)
176. [1-(4-{2-[3-(4-*tert*.-butylphenyl)-[1,2,4]oxadiazol-5-yl]-vinyl}-cyclohexyl)-3-phenylpropyl]-dimethylamine (more highly nonpolar diastereomer)
177. dimethyl-(3-phenyl-1-{4-[2-(3-m-tolyl-[1,2,4]oxadiazol-5-yl)-vinyl]-cyclohexyl}-propyl)-amine (more highly polar diastereomer)
178. dimethyl-(3-phenyl-1-{4-[2-(3-m-tolyl-[1,2,4]oxadiazol-5-yl)-vinyl]-cyclohexyl}-propyl)-amine (more highly nonpolar diastereomer)
179. [(4-{2-[3-(3-chlorophenyl)-[1,2,4]oxadiazol-5-yl]-vinyl}-cyclohexyl)-phenylmethyl]-dimethylamine
180. [(4-{2-[3-(4-*tert*.-butylphenyl)-[1,2,4]oxadiazol-5-yl]-vinyl}-cyclohexyl)-phenylmethyl]-dimethylamine (more highly polar diastereomer)
181. [(4-{2-[3-(4-*tert*.-butylphenyl)-[1,2,4]oxadiazol-5-yl]-vinyl}-cyclohexyl)-phenylmethyl]-dimethylamine (more highly nonpolar diastereomer)
182. dimethyl-(phenyl-{4-[2-(3-m-tolyl-[1,2,4]oxadiazol-5-yl)-vinyl]-cyclohexyl}-methyl)-amine (more highly polar diastereomer)
183. dimethyl-(phenyl-{4-[2-(3-m-tolyl-[1,2,4]oxadiazol-5-yl)-vinyl]-cyclohexyl}-methyl)-amine (more highly nonpolar diastereomer)
184. [(4-{2-[3-(4-chlorophenyl)-[1,2,4]oxadiazol-5-yl]-vinyl}-cyclohexyl)-phenylmethyl]-dimethylamine (more highly polar diastereomer)
185. [(4-{2-[3-(4-chlorophenyl)-[1,2,4]oxadiazol-5-yl]-vinyl}-cyclohexyl)-phenylmethyl]-dimethylamine (more highly nonpolar diastereomer)
186. [(4-chlorophenyl)-(4-{2-[3-(3-chlorophenyl)-[1,2,4]oxadiazol-5-yl]-vinyl}-cyclohexyl)-methyl]-dimethylamine
187. [(4-{2-[3-(4-*tert*.-butylphenyl)-[1,2,4]oxadiazol-5-yl]-vinyl}-cyclohexyl)-(4-chlorophenyl)-methyl]-dimethylamine (more highly polar diastereomer)
188. [(4-{2-[3-(4-*tert*.-butylphenyl)-[1,2,4]oxadiazol-5-yl]-vinyl}-cyclohexyl)-(4-chlorophenyl)-methyl]-dimethylamine (more highly nonpolar diastereomer)
189. ((4-chlorophenyl)-{4-[2-(3-m-tolyl-[1,2,4]oxadiazol-5-yl)-vinyl]-cyclohexyl}-methyl)-dimethylamine (more highly polar diastereomer)
190. ((4-chlorophenyl)-{4-[2-(3-m-tolyl-[1,2,4]oxadiazol-5-yl)-vinyl]-cyclohexyl}-methyl)-dimethylamine (more highly nonpolar diastereomer)
191. [(4-chlorophenyl)-(4-{2-[3-(4-chlorophenyl)-[1,2,4]oxadiazol-5-yl]-vinyl}-cyclohexyl)-methyl]-dimethylamine
192. [(4-{2-[3-(3-chlorophenyl)-[1,2,4]oxadiazol-5-yl]-vinyl}-cyclohexyl)-thiophen-2-yl-methyl]-dimethylamine
193. [(4-{2-[3-(4-*tert*.-butylphenyl)-[1,2,4]oxadiazol-5-yl]-vinyl}-cyclohexyl)-thiophen-2-yl-methyl]-dimethylamine (more highly polar diastereomer)
194. [(4-{2-[3-(4-*tert*.-butylphenyl)-[1,2,4]oxadiazol-5-yl]-vinyl}-cyclohexyl)-thiophen-2-yl-methyl]-dimethylamine (more highly nonpolar diastereomer)
195. dimethyl-(thiophen-2-yl-{4-[2-(3-m-tolyl-[1,2,4]oxadiazol-5-yl)-vinyl]-cyclohexyl}-methyl)-amine
196. [(4-{2-[3-(4-chlorophenyl)-[1,2,4]oxadiazol-5-yl]-vinyl}-cyclohexyl)-thiophen-2-yl-methyl]-dimethylamine
197. [(4-{2-[3-(3-chlorophenyl)-[1,2,4]oxadiazol-5-yl]-vinyl}-cyclohexyl)-(3-fluorophenyl)-methyl]-dimethylamine
198. [(4-{2-[3-(4-*tert*.-butylphenyl)-[1,2,4]oxadiazol-5-yl]-vinyl}-cyclohexyl)-(3-fluorophenyl)-methyl]-dimethylamine (more highly polar diastereomer)
199. [(4-{2-[3-(4-*tert*.-butylphenyl)-[1,2,4]oxadiazol-5-yl]-vinyl}-cyclohexyl)-(3-fluorophenyl)-methyl]-dimethylamine (more highly nonpolar diastereomer) 201. ((3-fluorophenyl)-{4-[2-(3-m-tolyl-[1,2,4]oxadiazol-5-yl)-vinyl]-cyclohexyl}-methyl)-dimethylamine
202. [(4-{2-[3-(4-chlorophenyl)-[1,2,4]oxadiazol-5-yl]-vinyl}-cyclohexyl)-(3-fluorophenyl)-methyl]-dimethylamine
203. [(4-{2-[3-(2,3-dichlorophenyl)-[1,2,4]oxadiazol-5-yl]-vinyl}-cyclohexyl)-(4-fluorophenyl)-methyl]-dimethylamine
204. [(4-fluorophenyl)-(4-{2-[3-(4-trifluoromethoxyphenyl)-[1,2,4]oxadiazol-5-yl]-vinyl}-cyclohexyl)-methyl]-dimethylamine
205. [(4-{2-[3-(3,4-dimethoxybenzyl)-[1,2,4]oxadiazol-5-yl]-vinyl}-cyclohexyl)-(4-fluorophenyl)-methyl]-dimethylamine
206. [(4-{2-[3-(1,5-dimethyl-1H-pyrrol-2-yl)-[1,2,4]oxadiazol-5-yl]-vinyl}-cyclohexyl)-(4-fluorophenyl)-methyl]-dimethylamine
207. [1-(4-{2-[3-(2,3-dichlorophenyl)-[1,2,4]oxadiazol-5-yl]-vinyl}-cyclohexyl)-3-phenylpropyl]-dimethylamine
208. dimethyl-[3-phenyl-1-(4-{2-[3-(4-trifluoromethoxyphenyl)-[1,2,4]oxadiazol-5-yl]-vinyl}-cyclohexyl)-propyl]-amine (more highly polar diastereomer)
209. dimethyl-[3-phenyl-1-(4-{2-[3-(4-trifluoromethoxyphenyl)-[1,2,4]oxadiazol-5-yl]-vinyl}-cyclohexyl)-propyl]-amine (more highly nonpolar diastereomer)
210. [1-(4-{2-[3-(3,4-dimethoxybenzyl)-[1,2,4]oxadiazol-5-yl]-vinyl}-cyclohexyl)-3-phenylpropyl]-dimethylamine (more highly polar diastereomer)
211. [1-(4-{2-[3-(3,4-dimethoxybenzyl)-[1,2,4]oxadiazol-5-yl]-vinyl}-cyclohexyl)-3-phenylpropyl]-dimethylamine (more highly nonpolar diastereomer)
212. [1-(4-{2-[3-(1,5-dimethyl-1H-pyrrol-2-yl)-[1,2,4]oxadiazol-5-yl]-vinyl}-cyclohexyl)-3-phenylpropyl]-dimethylamine
213. [(4-{2-[3-(2,3-dichlorophenyl)-[1,2,4]oxadiazol-5-yl]-vinyl}-cyclohexyl)-phenylmethyl]-dimethylamine
214. [(4-{2-[3-(3,4-dimethoxybenzyl)-[1,2,4]oxadiazol-5-yl]-vinyl}-cyclohexyl)-phenylmethyl]-dimethylamine (more highly polar diastereomer)
215. [(4-{2-[3-(3,4-dimethoxybenzyl)-[1,2,4]oxadiazol-5-yl]-vinyl}-cyclohexyl)-phenylmethyl]-dimethylamine (more highly nonpolar diastereomer) 217. [(4-{2-[3-(1,5-dimethyl-1H-pyrrol-2-yl)-[1,2,4]oxadiazol-5-yl]-vinyl}-cyclohexyl)-phenylmethyl]-dimethylamine
218. [(4-chlorophenyl)-(4-{2-[3-(2,3-dichlorophenyl)-[1,2,4]oxadiazol-5-yl]-vinyl}-cyclohexyl)-methyl]-dimethylamine
219. [(4-chlorophenyl)-(4-{2-[3-(4-trifluoromethoxyphenyl)-[1,2,4]oxadiazol-5-yl]-vinyl}-cyclohexyl)-methyl]-dimethylamine
220. [(4-chlorophenyl)-(4-{2-[3-(3,4-dimethoxybenzyl)-[1,2,4]oxadiazol-5-yl]-vinyl}-cyclohexyl)-methyl]-dimethylamine
221. [(4-chlorophenyl)-(4-{2-[3-(1,5-dimethyl-1H-pyrrol-2-yl)-[1,2,4]oxadiazol-5-yl]-vinyl}-cyclohexyl)-methyl]-dimethylamine
222. [(4-{2-[3-(2,3-dichlorophenyl)-[1,2,4]oxadiazol-5-yl]-vinyl}-cyclohexyl)-thiophen-2-yl-methyl]-dimethylamine
223. dimethyl-[thiophen-2-yl-(4-{2-[3-(4-trifluoromethoxyphenyl)-[1,2,4]oxadiazol-5-yl]-vinyl}-cyclohexyl)-methyl]-amine
224. [(4-{2-[3-(1,5-dimethyl-1H-pyrrol-2-yl)-[1,2,4]oxadiazol-5-yl]-vinyl}-cyclohexyl-thiophen-2-yl-methyl]-dimethylamine
225. [(4-{2-[3-(2,3-dichlorophenyl)-[1,2,4]oxadiazol-5-yl]-vinyl}-cyclohexyl)-(3-fluorophenyl)-methyl]-dimethylamine
226. [(3-fluorophenyl)-(4-{2-[3-(4-trifluoromethoxyphenyl)-[1,2,4]oxadiazol-5-yl]-vinyl}-cyclohexyl)-methyl]-dimethylamine (more highly polar diastereomer)
227. [(3-fluorophenyl)-(4-{2-[3-(4-trifluoromethoxyphenyl)-[1,2,4]oxadiazol-5-yl]-vinyl}-cyclohexyl)-methyl]-dimethylamine (more highly nonpolar diastereomer)
228. [(4-{2-[3-(3,4-dimethoxybenzyl)-[1,2,4]oxadiazol-5-yl]-vinyl}-cyclohexyl)-(3-fluorophenyl)-methyl]-dimethylamine (more highly polar diastereomer)
229. [(4-{2-[3-(3,4-dimethoxybenzyl)-[1,2,4]oxadiazol-5-yl]-vinyl}-cyclohexyl)-(3-fluorophenyl)-methyl]-dimethylamine (more highly nonpolar diastereomer) 231. [(4-{2-[3-(1,5-dimethyl-1H-pyrrol-2-yl)-[1,2,4]oxadiazol-5-yl]-vinyl}-cyclohexyl)-(3-fluorophenyl)-methyl]-dimethylamine
232. {(3-fluorophenyl)-[4-(3-phenyl-[1,2,4]oxadiazol-5-ylmethyl)-cyclohexyl]-methyl}-dimethylamine
233. {(4-fluorophenyl)-[4-(3-phenyl-[1,2,4]oxadiazol-5-ylmethyl)-cyclohexyl]-methyl}-dimethylamine
234. dimethyl-{phenyl-[4-(3-phenyl-[1,2,4]oxadiazol-5-ylmethyl)-cyclohexyl]-methyl}-amine
235. [{4-[3-(2,4-difluorophenyl)-[1,2,4]oxadiazol-5-ylmethyl]-cyclohexyl}-(3-fluorophenyl)-methyl]-dimethylamine
236. [{4-[3-(2,4-difluorophenyl)-[1,2,4]oxadiazol-5-ylmethyl]-cyclohexyl}-(4-fluorophenyl)-methyl]-dimethylamine
237. ((3-fluorophenyl)-{4-[3-(4-methoxybenzyl)-[1,2,4]oxadiazol-5-ylmethyl]-cyclohexyl}-methyl)-dimethylamine
238. ((4-fluorophenyl)-{4-[3-(4-methoxybenzyl)-[1,2,4]oxadiazol-5-ylmethyl]-cyclohexyl}-methyl)-dimethylamine
239. ({4-[3-(4-methoxybenzyl)-[1,2,4]oxadiazol-5-ylmethyl]-cyclohexyl}-phenylmethyl)-dimethylamine
240. [{4-[3-(4-chlorobenzyl)-[1,2,4]oxadiazol-5-ylmethyl]-cyclohexyl}-(3-fluorophenyl)-methyl]-dimethylamine
241. [{4-[3-(4-chlorobenzyl)-[1,2,4]oxadiazol-5-ylmethyl]-cyclohexyl}-(4-fluorophenyl)-methyl]-dimethylamine
242. [{4-[3-(3-chlorophenyl)-[1,2,4]oxadiazol-5-ylmethyl]-cyclohexyl}-(4-fluorophenyl)-methyl]-dimethylamine
243. ({4-[3-(3-chlorophenyl)-[1,2,4]oxadiazol-5-ylmethyl]-cyclohexyl}-phenylmethyl)-dimethylamine
244. [{4-[3-(2,3-dichlorophenyl)-[1,2,4]oxadiazol-5-ylmethyl]-cyclohexyl}-(3-fluorophenyl)-methyl]-dimethylamine
245. [{4-[3-(2,3-dichlorophenyl)-[1,2,4]oxadiazol-5-ylmethyl]-cyclohexyl}-(4-fluorophenyl)-methyl]-dimethylamine
246. ({4-[3-(2,3-dichlorophenyl)-[1,2,4]oxadiazol-5-ylmethyl]-cyclohexyl}-phenylmethyl)-dimethylamine
247. {(3-fluorophenyl)-[4-(3-p-tolyl-[1,2,4]oxadiazol-5-ylmethyl)-cyclohexyl]-methyl}-dimethylamine
248. {(4-fluorophenyl)-[4-(3-p-tolyl-[1,2,4]oxadiazol-5-ylmethyl)-cyclohexyl]-methyl}-dimethylamine
249. dimethyl-{phenyl-[4-(3-p-tolyl-[1,2,4]oxadiazol-5-ylmethyl)-cyclohexyl]-methyl}-amine
250. [{4-[3-(3,4-dimethoxyphenyl)-[1,2,4]oxadiazol-5-ylmethyl]-cyclohexyl}-(3-fluorophenyl)-methyl]-dimethylamine
251. [{4-[3-(3,4-dimethoxyphenyl)-[1,2,4]oxadiazol-5-ylmethyl]-cyclohexyl}-(4-fluorophenyl)-methyl]-dimethylamine
252. ({4-[3-(3,4-dimethoxyphenyl)-[1,2,4]oxadiazol-5-ylmethyl]-cyclohexyl}-phenylmethyl)-dimethylamine
253. [{4-[3-(4-tert.-butylphenyl)-[1,2,4]oxadiazol-5-ylmethyl]-cyclohexyl}-(3-fluorophenyl)-methyl]-dimethylamine
254. [{4-[3-(4-tert.-butylphenyl)-[1,2,4]oxadiazol-5-ylmethyl]-cyclohexyl}-(4-fluorophenyl)-methyl]-dimethylamine
255. ({4-[3-(4-tert.-butylphenyl)-[1,2,4]oxadiazol-5-ylmethyl]-cyclohexyl}-phenylmethyl)-dimethylamine
256. {(3-fluorophenyl)-[4-(3-m-tolyl-[1,2,4]oxadiazol-5-ylmethyl)-cyclohexyl]-methyl}-dimethylamine
257. {(4-fluorophenyl)-[4-(3-m-tolyl-[1,2,4]oxadiazol-5-ylmethyl)-cyclohexyl]-methyl}-dimethylamine
258. dimethyl-{phenyl-[4-(3-m-tolyl-[1,2,4]oxadiazol-5-ylmethyl)-cyclohexyl]-methyl}-amine
259. [{4-[3-(4-chlorophenyl)-[1,2,4]oxadiazol-5-ylmethyl]-cyclohexyl}-(3-fluorophenyl)-methyl]-dimethylamine
260. ({4-[3-(4-chlorophenyl)-[1,2,4]oxadiazol-5-ylmethyl]-cyclohexyl}-phenylmethyl)-dimethylamine
261. ((3-fluorophenyl)-{4-[3-(4-trifluoromethoxyphenyl)-[1,2,4]oxadiazol-5-ylmethyl]-cyclohexyl}-methyl)-dimethylamine
262. ((4-fluorophenyl)-{4-[3-(4-trifluoromethoxyphenyl)-[1,2,4]oxadiazol-5-ylmethyl]-cyclohexyl}-methyl)-dimethylamine
263. dimethyl-(phenyl-{4-[3-(4-trifluoromethoxyphenyl)-[1,2,4]oxadiazol-5-ylmethyl]-cyclohexyl}-methyl)-amine
264. ((4-fluorophenyl)-{4-[2-(3-phenyl-[1,2,4]oxadiazol-5-yl)-ethyl]-cyclohexyl}-methyl)-dimethylamine
265. dimethyl-(phenyl-{4-[2-(3-phenyl-[1,2,4]oxadiazol-5-yl)-ethyl]-cyclohexyl}-methyl)-amine
266. dimethyl-(3-phenyl-1-{4-[2-(3-phenyl-[1,2,4]oxadiazol-5-yl)-ethyl]-cyclohexyl}-propyl)-amine
267. ((3-fluorophenyl)-{4-[2-(3-phenyl-[1,2,4]oxadiazol-5-yl)-ethyl]-cyclohexyl}-methyl)-dimethylamine
268. [(4-fluorophenyl)-(4-{2-[3-(4-methoxybenzyl)-[1,2,4]oxadiazol-5-yl]-ethyl}-cyclohexyl)-methyl]-dimethylamine
269. [(4-{2-[3-(4-methoxybenzyl)-[1,2,4]oxadiazol-5-yl]-ethyl}-cyclohexyl)-phenylmethyl]-dimethylamine
270. [1-(4-{2-[3-(4-methoxybenzyl)-[1,2,4]oxadiazol-5-yl]-ethyl}-cyclohexyl)-3-phenylpropyl]-dimethylamine
271. [(3-fluorophenyl)-(4-{2-[3-(4-methoxybenzyl)-[1,2,4]oxadiazol-5-yl]-ethyl}-cyclohexyl)-methyl]-dimethylamine
272. [(4-{2-[3-(4-chlorobenzyl)-[1,2,4]oxadiazol-5-yl]-ethyl}-cyclohexyl)-(4-fluorophenyl)-methyl]-dimethylamine
273. [(4-{2-[3-(4-chlorobenzyl)-[1,2,4]oxadiazol-5-yl]-ethyl}-cyclohexyl)-phenylmethyl]-dimethylamine
274. [1-(4-{2-[3-(4-chlorobenzyl)-[1,2,4]oxadiazol-5-yl]-ethyl}-cyclohexyl)-3-phenylpropyl]-dimethylamine
275. [(4-{2-[3-(4-chlorobenzyl)-[1,2,4]oxadiazol-5-yl]-ethyl}-cyclohexyl)-(3-fluorophenyl)-methyl]-dimethylamine
276. [(4-{2-[3-(3-chlorophenyl)-[1,2,4]oxadiazol-5-yl]-ethyl}-cyclohexyl)-(4-fluorophenyl)-methyl]-dimethylamine
277. [(4-{2-[3-(3-chlorophenyl)-[1,2,4]oxadiazol-5-yl]-ethyl}-cyclohexyl)-phenylmethyl]-dimethylamine
278. [1-(4-{2-[3-(3-chlorophenyl)-[1,2,4]oxadiazol-5-yl]-ethyl}-cyclohexyl)-3-phenylpropyl]-dimethylamine
279. [(4-{2-[3-(3-chlorophenyl)-[1,2,4]oxadiazol-5-yl]-ethyl}-cyclohexyl)-(3-fluorophenyl)-methyl]-dimethylamine
280. [(4-{2-[3-(2,4-difluorophenyl)-[1,2,4]oxadiazol-5-yl]-ethyl}-cyclohexyl)-(4-fluorophenyl)-methyl]-dimethylamine
281. [(4-{2-[3-(2,4-difluorophenyl)-[1,2,4]oxadiazol-5-yl]-ethyl}-cyclohexyl)-phenylmethyl]-dimethylamine
282. [(4-{2-[3-(2,4-difluorophenyl)-[1,2,4]oxadiazol-5-yl]-ethyl}-cyclohexyl)-(3-fluorophenyl)-methyl]-dimethylamine
283. [(4-{2-[3-(3,4-dimethoxyphenyl)-[1,2,4]oxadiazol-5-yl]-ethyl}-cyclohexyl)-(4-fluorophenyl)-methyl]-dimethylamine
284. [(4-{2-[3-(3,4-dimethoxyphenyl)-[1,2,4]oxadiazol-5-yl]-ethyl}-cyclohexyl)-phenylmethyl]-dimethylamine
285. [1-(4-{2-[3-(3,4-dimethoxyphenyl)-[1,2,4]oxadiazol-5-yl]-ethyl}-cyclohexyl)-3-phenylpropyl]-dimethylamine
286. [(4-{2-[3-(3,4-dimethoxyphenyl)-[1,2,4]oxadiazol-5-yl]-ethyl}-cyclohexyl)-(3-fluorophenyl)-methyl]-dimethylamine
287. ((4-fluorophenyl)-{4-[2-(3-p-tolyl-[1,2,4]oxadiazol-5-yl)-ethyl]-cyclohexyl}-methyl)-dimethylamine
288. dimethyl-(phenyl-{4-[2-(3-p-tolyl-[1,2,4]oxadiazol-5-yl)-ethyl]-cyclohexyl}-methyl)-amine
289. dimethyl-(3-phenyl-1-{4-[2-(3-p-tolyl-[1,2,4]oxadiazol-5-yl)-ethyl]-cyclohexyl}-propyl)-amine
290. ((3-fluorophenyl)-{4-[2-(3-p-tolyl-[1,2,4]oxadiazol-5-yl)-ethyl]-cyclohexyl}-methyl)-dimethylamine
291. [(4-{2-[3-(4-*tert*.-butylphenyl)-[1,2,4]oxadiazol-5-yl]-ethyl}-cyclohexyl)-(4-fluorophenyl)-methyl]-dimethylamine
292. [(4-{2-[3-(4-*tert*.-butylphenyl)-[1,2,4]oxadiazol-5-yl]-ethyl}-cyclohexyl)-phenylmethyl]-dimethylamine
293. [1-(4-{2-[3-(4-*tert*.-butylphenyl)-[1,2,4]oxadiazol-5-yl]-ethyl}-cyclohexyl)-3-phenylpropyl]-dimethylamine
294. [(4-{2-[3-(4-*tert*.-butylphenyl)-[1,2,4]oxadiazol-5-yl]-ethyl}-cyclohexyl)-(3-fluorophenyl)-methyl]-dimethylamine
295. ((4-fluorophenyl)-{4-[2-(3-m-tolyl-[1,2,4]oxadiazol-5-yl)-ethyl]-cyclohexyl}-methyl)-dimethylamine
296. dimethyl-(phenyl-{4-[2-(3-m-tolyl-[1,2,4]oxadiazol-5-yl)-ethyl]-cyclohexyl}-methyl)-amine
297. dimethyl-(3-phenyl-1-{4-[2-(3-m-tolyl-[1,2,4]oxadiazol-5-yl)-ethyl]-cyclohexyl}-propyl)-amine
298. ((3-fluorophenyl)-{4-[2-(3-m-tolyl-[1,2,4]oxadiazol-5-yl)-ethyl]-cyclohexyl}-methyl)-dimethylamine
299. [(4-{2-[3-(4-chlorophenyl)-[1,2,4]oxadiazol-5-yl]-ethyl}-cyclohexyl)-(4-fluorophenyl)-methyl]-dimethylamine
300. [(4-{2-[3-(4-chlorophenyl)-[1,2,4]oxadiazol-5-yl]-ethyl}-cyclohexyl)-phenylmethyl]-dimethylamine
301. [1-(4-{2-[3-(4-chlorophenyl)-[1,2,4]oxadiazol-5-yl]-ethyl}-cyclohexyl)-3-phenylpropyl]-dimethylamine
302. [(4-{2-[3-(4-chlorophenyl)-[1,2,4]oxadiazol-5-yl]-ethyl}-cyclohexyl)-(3-fluorophenyl)-methyl]-dimethylamine
303. [(4-{2-[3-(2,3-dichlorophenyl)-[1,2,4]oxadiazol-5-yl]-ethyl}-cyclohexyl)-(4-fluorophenyl)-methyl]-dimethylamine
304. [(4-{2-[3-(2,3-dichlorophenyl)-[1,2,4]oxadiazol-5-yl]-ethyl}-cyclohexyl)-phenylmethyl]-dimethylamine
305. [1-(4-{2-[3-(2,3-dichlorophenyl)-[1,2,4]oxadiazol-5-yl]-ethyl}-cyclohexyl)-3-phenylpropyl]-dimethylamine
306. [(4-{2-[3-(2,3-dichlorophenyl)-[1,2,4]oxadiazol-5-yl]-ethyl}-cyclohexyl)-(3-fluorophenyl)-methyl]-dimethylamine
307. [(4-fluorophenyl)-(4-{2-[3-(4-trifluoromethoxyphenyl)-[1,2,4]oxadiazol-5-yl]-ethyl}-cyclohexyl)-methyl]-dimethylamine
308. dimethyl-[phenyl-(4-{2-[3-(4-trifluoromethoxyphenyl)-[1,2,4]oxadiazol-5-yl]-ethyl}-cyclohexyl)-methyl]-amine
309. dimethyl-[3-phenyl-1-(4-{2-[3-(4-trifluoromethoxyphenyl)-[1,2,4]oxadiazol-5-yl]-ethyl}-cyclohexyl)-propyl]-amine
310. [(3-fluorophenyl)-(4-{2-[3-(4-trifluoromethoxyphenyl)-[1,2,4]oxadiazol-5-yl]-ethyl}-cyclohexyl)-methyl]-dimethylamine
311. (4-((3-(4-fluorobenzyl)-1,2,4-oxadiazol-5-yl)methyl)cyclohexyl)(4-fluorophenyl)-N,N-dimethylmethanamine
312. (4-((3-(4-fluorobenzyl)-1,2,4-oxadiazol-5-yl)methyl)cyclohexyl)-N,N-dimethyl(phenyl)methanamine
313. (4-((3-(3-chlorobenzyl)-1,2,4-oxadiazol-5-yl)methyl)cyclohexyl)(3-fluorophenyl)-N,N-dimethylmethanamine
314. (4-((3-(3-chlorobenzyl)-1,2,4-oxadiazol-5-yl)methyl)cyclohexyl)(4-fluorophenyl)-N,N-dimethylmethanamine
315. (4-((3-(3-chlorobenzyl)-1,2,4-oxadiazol-5-yl)methyl)cyclohexyl)-N,N-dimethyl(phenyl)methanamine
316. (4-((3-(3-bromophenyl)-1,2,4-oxadiazol-5-yl)methyl)cyclohexyl)(3-fluorophenyl)-N,N-dimethylmethanamine
317. (4-((3-(3-bromophenyl)-1,2,4-oxadiazol-5-yl)methyl)cyclohexyl)(4-fluorophenyl)-N,N-dimethylmethanamine
318. (4-((3-(3-bromophenyl)-1,2,4-oxadiazol-5-yl)methyl)cyclohexyl)-N,N-dimethyl(phenyl)methanamine
319. (4-((3-(4-bromobenzyl)-1,2,4-oxadiazol-5-yl)methyl)cyclohexyl)(3-fluorophenyl)-N,N-dimethylmethanamine
320. (4-((3-(4-bromobenzyl)-1,2,4-oxadiazol-5-yl)methyl)cyclohexyl)(4-fluorophenyl)-N,N-dimethylmethanamine
321. (4-((3-(4-bromobenzyl)-1,2,4-oxadiazol-5-yl)methyl)cyclohexyl)-N,N-dimethyl(phenyl)methanamine
322. (3-fluorophenyl)-N,N-dimethyl(4-((3-(thiophen-2-ylmethyl)-1,2,4-oxadiazol-5-yl)methyl)cyclohexyl)methanamine
323. (4-fluorophenyl)-N,N-dimethyl(4-((3-(thiophen-2-ylmethyl)-1,2,4-oxadiazol-5-yl)methyl)cyclohexyl)methanamine
324. N,N-dimethyl(phenyl)(4-((3-(thiophen-2-ylmethyl)-1,2,4-oxadiazol-5-yl)methyl)cyclohexyl)methanamine
325. (4-((3-benzyl-1,2,4-oxadiazol-5-yl)methyl)cyclohexyl)(3-fluorophenyl)-N,N-dimethylmethanamine
326. (4-((3-benzyl-1,2,4-oxadiazol-5-yl)methyl)cyclohexyl)(4-fluorophenyl)-N,N-dimethylmethanamine
327. (4-((3-benzyl-1,2,4-oxadiazol-5-yl)methyl)cyclohexyl)-N,N-dimethyl(phenyl)methanamine
328. (3-fluorophenyl)-N,N-dimethyl(4-((3-phenethyl-1,2,4-oxadiazol-5-yl)methyl)cyclohexyl)methanamine
329. (4-fluorophenyl)-N,N-dimethyl(4-((3-phenethyl-1,2,4-oxadiazol-5-yl)methyl)cyclohexyl)methanamine
330. N,N-dimethyl(4-((3-phenethyl-1,2,4-oxadiazol-5-yl)methyl)cyclohexyl)(phenyl)methanamine
331. (4-((3-((1H-indol-3-yl)methyl)-1,2,4-oxadiazol-5-yl)methyl)cyclohexyl)(3-fluorophenyl)-N,N-dimethylmethanamine

9. A method for producing an oxadiazole derivative according to claim 1, wherein amide oximes of the general formula D are reacted in a reaction medium with addition of a base, for example NaH, with both saturated and unsaturated esters of the general formula A to yield the compounds according to the invention.

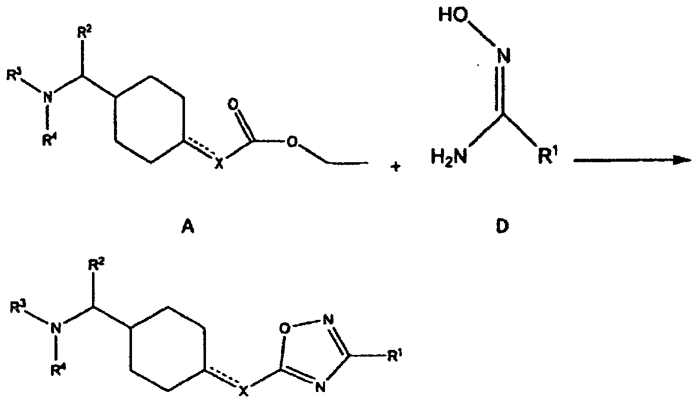

10. A medicament containing at least one substituted oxadiazole derivative according to claim 1, optionally in the form of the racemate; the enantiomers, diastereomers, mixtures of the enantiomers or diastereomers or of an individual enantiomer or diastereomer; the bases and/or salts of physiologically acceptable acids, and optionally containing suitable additives and/or auxiliary substances and/or optionally further active ingredients.

11. Use of a substituted oxadiazole derivative according to claim 1, optionally in the form of the racemate; the enantiomers, diastereomers, mixtures of the enantiomers or diastereomers or of an individual enantiomer or diastereomer; the bases and/or salts of physiologically acceptable acids, for producing a medicament for treating pain, in particular acute, neuropathic or chronic pain.

12. Use of a substituted oxadiazole derivative according to claim 1, optionally in the form of the racemate; the enantiomers, diastereomers, mixtures of the enantiomers or diastereomers or of an individual enantiomer or diastereomer; the bases and/or salts of physiologically acceptable acids, for producing a medicament for treating depression, urinary incontinence, diarrhoea, pruritus, alcohol and drug abuse, dependency on medicines, lack of drive and/or for anxiolysis.

## Revendications

1. Dérivés substitués d'oxadiazole de formule
générale I, 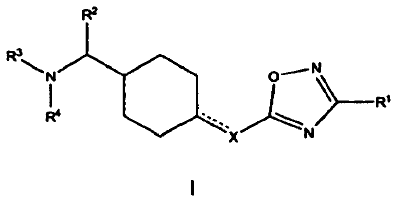 où
X signifie CH, CH₂, CH=CH, CH₂CH₂, CH₂CH=CH ou CH₂CH₂CH₂
R¹ signifie aryle, hétéroaryle, à chaque fois non substitué ou monosubstitué ou polysubstitué ; un radical aryle ou hétéroaryle, à chaque fois non substitué ou monosubstitué ou polysubstitué, lié via un groupe C₁₋₃-alkyle ;
R² signifie aryle ou hétéroaryle, à chaque fois non substitué ou monosubstitué ou polysubstitué ; un radical aryle, à chaque fois non substitué ou monosubstitué ou polysubstitué, lié via une chaîne C₁₋₃-alkyle ;
R³ et R⁴ signifient, indépendamment l'un de l'autre H ; C₁₋₆-alkyle, à chaque fois saturé ou insaturé, ramifié ou non ramifié, R³ et R⁴ ne signifiant pas simultanément H,
ou les radicaux R³ et R⁴ signifient ensemble CH₂CH₂OCH₂CH₂ ou (CH₂)₃₋₆,
sous forme du racémate ; des énantiomères, diastéréo-isomères, mélanges des énantiomères ou diastéréo-isomères ou d'un seul énantiomère ou diastéréo-isomère ; des bases et/ou des sels d'acides physiologiquement acceptables,
le concept "substitué" associé à "alkyle" désignant la substitution d'un radical hydrogène par F, Cl, Br, I, -CN, NH₂, NH-C₁₋₆-alkyle, NH-C₁₋₆-alkyl-OH, C₁₋₆-alkyle, N(C₁₋₆-alkyle)₂, N(C₁₋₆-alkyl-OH)₂, NO₂, SH, S-C₁₋₆-alkyle, S-benzyle, O-C₁₋₆-alkyle, OH, O-C₁₋₆-alkyl-OH, =O, O-benzyle, C(=O)C₁₋₆-alkyle, CO₂H, CO₂-C₁₋₆-alkyle ou benzyle, et
le concept "monosubstitué ou polysubstitué" en association avec "aryle" et "hétéroaryle" désignant la monosubstitution ou la polysubstitution d'un ou de plusieurs atomes d'hydrogène du système cyclique par F, Cl, Br, I, CN, NH₂, NH-C₁₋₆-alkyle, NHC₁₋₆-alkyl-OH, N(C₁₋₆-alkyle)₂, N(C₁₋₆-alkyl-OH)₂, NO₂, SH, S-C₁₋₆-alkyle, OH, O-C₁₋₆-alkyle, O-C₁₋₆-alkyl-OH, C(=O)C₁₋₆-alkyle, CO₂H, CO₂-C₁₋₆-alkyle, CF₃ ou C₁₋₆-alkyle.

2. Dérivés substitués d'oxadiazole selon la revendication 1, dans lesquels R¹ signifie phényle, naphtyle, thiényle, pyridyle ou pyrrolyle, benzyle, méthylindolyle, méthylthiényle ou phénéthyle, non substitué, ou monosubstitué ou polysubstitué par F, Cl, Br, CN, OCF₃, NH₂, NO₂, SH, S-C₁₋₆-alkyle, OH, O-C₁₋₆-alkyle, CO₂H, CO₂-C₁₋₆-alkyle, CF₃ ou C₁₋₆-alkyle.

3. Dérivés substitués d'oxadiazole selon la revendication 2, dans lesquels R¹ signifie phényle, thiényle, benzyle, méthylindolyle, méthylthiényle ou pyrrolyle, non substitué ou monosubstitué ou polysubstitué par Cl, Br, OCH₃, CH₃, F, OCF₃, CF₃ ou tert-butyle.

4. Dérivés substitués d'oxadiazole selon la revendication 1, dans lesquels R² signifie phényle ou thiényle, à chaque fois non substitué ou monosubstitué ou polysubstitué par F, Cl, CN, NO₂, SH, S-C₁₋₆-alkyle, OH, O-C₁₋₆-alkyle, CO₂H, CO₂-C₁₋₆-alkyle, CF₃, C₁₋₆-alkyle ; un radical phényle lié via une chaîne C₁₋₃-alkyle, à chaque fois non substitué ou monosubstitué ou polysubstitué par F, Cl, CN, NO₂, SH, S-C₁₋₆-alkyle, OH, O-C₁₋₆-alkyle, CO₂H, CO₂-C₁₋₆-alkyle, CF₃, C₁₋₆-alkyle.

5. Dérivés substitués d'oxadiazole selon la revendication 4, dans lesquels R² signifie phényle, non substitué ou monosubstitué par Cl ou F, phénéthyle ou thiényle.

6. Dérivés substitués d'oxadiazole selon la revendication 1, dans lesquels R³ et R⁴ signifient, indépendamment l'un de l'autre H ou C₁₋₆-alkyle, R³ et R⁴ ne signifiant pas simultanément H, ou les radicaux R³ et R⁴ signifient ensemble CH₂CH₂OCH₂CH₂ ou (CH₂)₄₋₅.

7. Dérivés substitués d'oxadiazole selon la revendication 6, dans lesquels R³ et R⁴ signifient CH₃.

8. Dérivés substitués d'oxadiazole selon la revendication 1, du groupe formé par
43. la (4-((3-(4-chlorobenzyl)-1,2,4-oxadiazol-5-yl)méthyl)cyclohexyl)-N,N-diméthyl(phényl)méthanamine
44. la (4-((3-(4-méthoxybenzyl)-1,2,4-oxadiazol-5-yl)méthyl)cyclohexyl)-N,N-diméthyl(phényl)méthanamine
45. la ((4-chlorophényl)-{4-[3-(4-chlorophényl)-[1,2,4]oxadiazol-5-ylméthylène]-cyclohexyl}-méthyl)-diméthylamine
46. la [{4-[3-(4-chlorophényl)-[1,2,4]oxadiazol-5-ylméthylène]-cyclohexyl}-(4-fluorophényl)-méthyl]-diméthylamine (diastéréo-isomère polaire)
47. la [{4-[3-(4-chlorophényl)-[1,2,4]oxadiazol-5-ylméthylène]-cyclohexyl}-(4-fluorophényl)-méthyl]-diméthylamine (diastéréo-isomère non polaire)
48. la ({4-[3-(2,3-dichlorophényl)-[1,2,4]oxadiazol-5-ylméthylène]-cyclohexyl}-phénylméthyl)-diméthylamine (diastéréo-isomère polaire)
49. la ({4-[3-(2,3-dichlorophényl)-[1,2,4]oxadiazol-5-ylméthylène]-cyclohexyl}-phénylméthyl)-diméthylamine (diastéréo-isomère non polaire)
50. la [{4-[3-(2,3-dichlorophényl)-[1,2,4]oxadiazol-5-ylméthylène]-cyclohexyl}-(3-fluorophényl)-méthyl]-diméthylamine (diastéréo-isomère polaire)
51. la [{4-[3-(2,3-dichlorophényl)-[1,2,4]oxadiazol-5-ylméthylène]-cyclohexyl}-(3-fluorophényl)-méthyl]-diméthylamine (diastéréo-isomère non polaire)
52. la ((4-chlorophényl)-{4-[3-(2,3-dichlorophényl)-[1,2,4]oxadiazol-5-ylméthylène]-cyclohexyl}-méthyl)-diméthylamine (diastéréo-isomère polaire)
53. la ((4-chlorophényl)-{4-[3-(2,3-dichlorophényl)-[1,2,4]oxadiazol-5-ylméthylène]-cyclohexyl}-méthyl)-diméthylamine (diastéréo-isomère non polaire)
54. la (1-{4-[3-(2,3-dichlorophényl)-[1,2,4]oxadiazol-5-ylméthylène]-cyclohexyl}-3-phénylpropyl)-diméthylamine
55. la [{4-[3-(2,3-dichlorophényl)-[1,2,4]oxadiazol-5-ylméthylène]-cyclohexyl}-(4-fluorophényl)-méthyl]-diméthylamine (diastéréo-isomère polaire)
56. la [{4-[3-(2,3-dichlorophényl)-[1,2,4]oxadiazol-5-ylméthylène]-cyclohexyl}-(4-fluorophényl)-méthyl]-diméthylamine (diastéréo-isomère non polaire)
57. la diméthyl-(phényl-{4-[3-(4-trifluorométhoxyphényl)-[1,2,4]oxadiazol-5-ylméthylène]-cyclohexyl}-méthyl)-amine (diastéréo-isomère polaire)
58. la diméthyl-(phényl-{4-[3-(4-trifluorométhoxyphényl)-[1,2,4]oxadiazol-5-ylméthylène]-cyclohexyl}-méthyl)-amine (diastéréo-isomère non polaire)
59. la ((3-fluorophényl)-{4-[3-(4-trifluorométhoxyphényl)-[1,2,4]oxadiazol-5-ylméthylène]-cyclohexyl}-méthyl)-diméthylamine (diastéréo-isomère polaire)
60. la ((3-fluorophényl)-{4-[3-(4-trifluorométhoxyphényl)-[1,2,4]oxadiazol-5-ylméthylène]-cyclohexyl}-méthyl)-diméthylamine (diastéréo-isomère non polaire)
61. la ((4-chlorophényl)-{4-[3-(4-trifluorométhoxyphényl)-[1,2,4]oxadiazol-5-ylméthylène]-cyclohexyl}-méthyl)-diméthylamine (diastéréo-isomère polaire)
62. la ((4-chlorophényl)-{4-[3-(4-trifluorométhoxyphényl)-[1,2,4]oxadiazol-5-ylméthylène]-cyclohexyl}-méthyl)-diméthylamine (diastéréo-isomère non polaire)
63. la diméthyl-(3-phényl-1-{4-[3-(4-trifluorométhoxyphényl)-[1,2,4]oxadiazol-5-ylméthylène]-cyclohexyl}-propyl)-amine (diastéréo-isomère polaire)
64. la diméthyl-(3-phényl-1-{4-[3-(4-trifluorométhoxyphényl)-[1,2,4]oxadiazol-5-ylméthylène]-cyclohexyl}-propyl)-amine (diastéréo-isomère non polaire)
65. la ((4-fluorophényl)-{4-[3-(4-trifluorométhoxyphényl)-[1,2,4]oxadiazol-5-ylméthylène]-cyclohexyl}-méthyl)-diméthylamine (diastéréo-isomère polaire)
66. la ((4-fluorophényl)-{4-[3-(4-trifluorométhoxyphényl)-[1,2,4]oxadiazol-5-ylméthylène]-cyclohexyl}-méthyl)-diméthylamine (diastéréo-isomère non polaire)
67. la diméthyl-(thiophén-2-yl-{4-[3-(4-trifluorométhoxyphényl)-[1,2,4]oxadiazol-5-ylméthylène]-cyclohexyl}-méthyl)-amine (diastéréo-isomère polaire)
68. la diméthyl-(thiophén-2-yl-{4-[3-(4-trifluorométhoxyphényl)-[1,2,4]oxadiazol-5-ylméthylène]-cyclohexyl}-méthyl)-amine (diastéréo-isomère non polaire)
69. la diméthyl-{phényl-[4-(3-phényl-[1,2,4]oxadiazol-5-ylméthylène)-cyclohexyl]-méthyl}-amine (diastéréo-isomère polaire)
70. la diméthyl-{phényl-[4-(3-phényl-[1,2,4]oxadiazol-5-ylméthylène)-cyclohexyl]-méthyl}-amine (diastéréo-isomère non polaire)
71. la {(3-fluorophényl)-[4-(3-phényl-[1,2,4]oxadiazol-5-ylméthylène)-cyclohexyl]-méthyl}-diméthylamine (diastéréo-isomère polaire)
72. la {(3-fluorophényl)-[4-(3-phényl-[1,2,4]oxadiazol-5-ylméthylène)-cyclohexyl]-méthyl}-diméthylamine (diastéréo-isomère non polaire)
73. la diméthyl-{3-phényl-1[4-[-(3-phényl-[1,2,4]oxadiazol-5-ylméthylène)-cyclohexyl]-propyl}-amine (diastéréo-isomère polaire)
74. la diméthyl-{3-phényl-1-[4-(3-phényl-[1,2,4]oxadiazol-5-ylméthylène)-cyclohexyl]-propyl}amine (diastéréo-isomère non polaire)
75. la {(4-fluorophényl)-[4-(3-phényl-[1,2,4]oxadiazol-5-ylméthylène)-cyclohexyl]-méthyl}-diméthylamine (diastéréo-isomère polaire)
76. la {(4-fluorophényl)-[4-(3-phényl-[1,2,4]oxadiazol-5-ylméthylène)-cyclohexyl]-méthyl}-diméthylamine (diastéréo-isomère non polaire)
77. la [{4-[3-(2,4-difluorophényl)-[1,2,4]oxadiazol-5-ylméthylène]-cyclohexyl}-(3-fluorophényl)-méthyl]-diméthylamine (diastéréo-isomère polaire)
78. la [{4-[3-(2,4-difluorophényl)-[1,2,4]oxadiazol-5-ylméthylène]-cyclohexyl}-(3-fluorophényl)-méthyl]-diméthylamine (diastéréo-isomère non polaire)
79. la ({4-[3-(4-méthoxybenzyl)-[1,2,4]oxadiazol-5-ylméthylène]-cyclohexyl}-phénylméthyl)-diméthylamine (diastéréo-isomère polaire)
80. la ({4-[3-(4-méthoxybenzyl)-[1,2,4]oxadiazol-5-ylméthylène]-cyclohexyl}-phénylméthyl)-diméthylamine (diastéréo-isomère non polaire)
81. la ((3-fluorophényl)-{4-[3-(4-méthoxybenzyl)-[1,2,4]oxadiazol-5-ylméthylène]-cyclohexyl}-méthyl)-diméthylamine (diastéréo-isomère polaire)
82. la ((3-fluorophényl)-{4-[3-(4-méthoxybenzyl)-[1,2,4]oxadiazol-5-ylméthylène]-cyclohexyl}-méthyl)-diméthylamine (diastéréo-isomère non polaire)
83. la ((4-chlorophényl)-{4-[3-(4-méthoxybenzyl)-[1,2,4]oxadiazol-5-ylméthylène]-cyclohexyl}-méthyl)-diméthylamine (diastéréo-isomère polaire)
84. la ((4-chlorophényl)-{4-[3-(4-méthoxybenzyl)-[1,2,4]oxadiazol-5-ylméthylène]-cyclohexyl}-méthyl)-diméthylamine (diastéréo-isomère non polaire)
85. la (1-{4-[3-(4-méthoxybenzyl)-[1,2,4]oxadiazol-5-ylméthylène]-cyclohexyl}-3-phénylpropyl)-diméthylamine (diastéréo-isomère polaire)
86. la (1-{4-[3-(4-méthoxybenzyl)-[1,2,4]oxadiazol-5-ylméthylène]-cyclohexyl}-3-phénylpropyl)-diméthylamine (diastéréo-isomère non polaire)
87. la ((4-fluorophényl)-{4-[3-(4-méthoxybenzyl)-[1,2,4]oxadiazol-5-ylméthylène]-cyclohexyl}-méthyl)-diméthylamine (diastéréo-isomère polaire)
88. la ((4-fluorophényl)-{4-[3-(4-méthoxybenzyl)-[1,2,4]oxadiazol-5-ylméthylène]-cyclohexyl}-méthyl)-diméthylamine (diastéréo-isomère non polaire)
89. la ({4-[3-(4-chlorobenzyl)-[1,2,4]oxadiazol-5-ylméthylène]-cyclohexyl}-phénylméthyl)-diméthylamine (diastéréo-isomère polaire)
90. la ({4-[3-(4-chlorobenzyl)-[1,2,4]oxadiazol-5-ylméthylène]-cyclohexyl}-phénylméthyl)-diméthylamine (diastéréo-isomère non polaire)
91. la [{4-[3-(4-chlorobenzyl)-[1,2,4]oxadiazol-5-ylméthylène]-cyclohexyl}-(3-fluorophényl)-méthyl]-diméthylamine (diastéréo-isomère polaire)
92. la [{4-[3-(4-chlorobenzyl)-[1,2,4]oxadiazol-5-ylméthylène]-cyclohexyl}-(4-chlorophényl)-méthyl]-diméthylamine (diastéréo-isomère non polaire)
93. la [{4-[3-(4-chlorobenzyl)-[1,2,4]oxadiazol-5-ylméthylène]-cyclohexyl}-(4-chlorophényl)-méthyl]-diméthylamine (diastéréo-isomère polaire)
94. la [{4-[3-(4-chlorobenzyl)-[1,2,4]oxadiazol-5-ylméthylène]-cyclohexyl}-(4-chlorophényl)-méthyl]-diméthylamine (diastéréo-isomère non polaire)
95. la (1-{4-[3-(4-chlorobenzyl)-[1,2,4]oxadiazol-5-ylméthylène]-cyclohexyl}-3-phénylpropyl)-diméthylamine (diastéréo-isomère polaire)
96. la (1-{4-[3-(4-chlorobenzyl)-[1,2,4]oxadiazol-5-ylméthylène]-cyclohexyl}-3-phénylpropyl)-diméthylamine (diastéréo-isomère non polaire)
97. la [{4-[3-(4-chlorobenzyl)-[1,2,4]oxadiazol-5-ylméthylène]-cyclohexyl}-(4-fluorophényl)-méthyl]-diméthylamine (diastéréo-isomère polaire)
98. la [{4-[3-(4-chlorobenzyl)-[1,2,4]oxadiazol-5-ylméthylène]-cyclohexyl}-(4-fluorophényl)-méthyl]-diméthylamine (diastéréo-isomère non polaire)
99. la {(3-fluorophényl)-[4-(3-p-toluyl-[1,2,4]oxadiazol-5-ylméthylène)-cyclohexyl]-méthyl}-diméthylamine
100. la {(4-chlorophényl)-[4-(3-p-toluyl-[1,2,4]oxadiazol-5-ylméthylène)-cyclohexyl]-méthyl}-diméthylamine
101. la {(4-fluorophényl)-[4-(3-p-toluyl-[1,2,4]oxadiazol-5-ylméthylène)-cyclohexyl]-méthyl}-diméthylamine (diastéréo-isomère polaire)
102. la {(4-fluorophényl)-[4-(3-p-toluyl-[1,2,4]oxadiazol-5-ylméthylène)-cyclohexyl]-méthyl}-diméthylamine (diastéréo-isomère non polaire)
103. la ({4-[3-(3,4-diméthoxyphényl)-[1,2,4]oxadiazol-5-ylméthylène]-cyclohexyl}-phénylméthyl)-diméthylamine (diastéréo-isomère polaire)
104. la [{4-[3-(3,4-diméthoxyphényl)-[1,2,4]oxadiazol-5-ylméthylène]-cyclohexyl}-phénylméthyl)-diméthylamine (diastéréo-isomère non polaire)
105. la [{4-[3-(3,4-diméthoxyphényl)-[1,2,4]oxadiazol-5-ylméthylène]-cyclohexyl}-(3-fluorophényl)-méthyl]-diméthylamine (diastéréo-isomère polaire)
106. la [{4-[3-(3,4-diméthoxyphényl)-[1,2,4]oxadiazol-5-ylméthylène]-cyclohexyl}-(3-fluorophényl)-méthyl]-diméthylamine (diastéréo-isomère non polaire)
107. la ((4-chlorophényl)-{4-[3-(3,4-diméthoxyphényl)-[1,2,4]oxadiazol-5-ylméthylène]-cyclohexyl}-méthyl)-diméthylamine (diastéréo-isomère polaire)
108. la ((4-chlorophényl)-{4-[3-(3,4-diméthoxyphényl)-[1,2,4]oxadiazol-5-ylméthylène]-cyclohexyl}-méthyl)-diméthylamine (diastéréo-isomère non polaire)
109. la (1-{4-[3-(3,4-diméthoxyphényl)-[1,2,4]oxadiazol-5-ylméthylène]-cyclohexyl}-3-phénylpropyl)-diméthylamine (diastéréo-isomère polaire)
110. la (1-{4-[3-(3,4-diméthoxyphényl)-[1,2,4]oxadiazol-5-ylméthylène]-cyclohexyl}-3-phénylpropyl)-diméthylamine (diastéréo-isomère non polaire)
111. la [{4-[3-(3,4-diméthoxyphényl)-[1,2,4]oxadiazol-5-ylméthylène]-cyclohexyl}-(4-fluorophényl)-méthyl]-diméthylamine (diastéréo-isomère polaire)
112. la [{4-[3-(3,4-diméthoxyphényl)-[1,2,4]oxadiazol-5-ylméthylène]-cyclohexyl}-(4-fluorophényl)-méthyl]-diméthylamine (diastéréo-isomère non polaire)
113. la ({4-[3-(3-chlorophényl)-[1,2,4]oxadiazol-5-ylméthylène]-cyclohexyl}-phénylméthyl)-diméthylamine (diastéréo-isomère polaire)
114. la ({4-[3-(3-chlorophényl)-[1,2,4]oxadiazol-5-ylméthylène]-cyclohexyl}-phénylméthyl)-diméthylamine (diastéréo-isomère non polaire)
115. la [{4-[3-(3-chlorophényl)-[1,2,4]oxadiazol-5-ylméthylène]-cyclohexyl}-(3-fluorophényl)-méthyl]-diméthylamine (diastéréo-isomère polaire)
116. la [{4-[3-(3-chlorophényl)-[1,2,4]oxadiazol-5-ylméthylène]-cyclohexyl}-(3-fluorophényl)-méthyl]-diméthylamine (diastéréo-isomère non polaire)
117. la ((4-chlorophényl)-{4-[3-(3-chlorophényl)-[1,2,4]oxadiazol-5-ylméthylène]-cyclohexyl}-méthyl)-diméthylamine (diastéréo-isomère polaire)
118. la ((4-chlorophényl)-{4-[3-(3-chlorophényl)-[1,2,4]oxadiazol-5-ylméthylène]-cyclohexyl}-méthyl)-diméthylamine (diastéréo-isomère non polaire)
119. la (1-{4-[3-(3-chlorophényl)-[1,2,4]oxadiazol-5-ylméthylène]-cyclohexyl}-3-phénylpropyl)-diméthylamine (diastéréo-isomère polaire)
120. la (1-{4-[3-(3-chlorophényl)-[1,2,4]oxadiazol-5-ylméthylène]-cyclohexyl}-3-phénylpropyl)-diméthylamine (diastéréo-isomère non polaire)
121. la [{4-[3-(3-chlorophényl)-[1,2,4]oxadiazol-5-ylméthylène]-cyclohexyl}-(4-fluorophényl)-méthyl]-diméthylamine
122. la ({4-[3-(4-tert-butylphényl)-[1,2,4]oxadiazol-5-ylméthylène]-cyclohexyl}-phénylméthyl)-diméthylamine (diastéréo-isomère polaire)
123. la ({4-[3-(4-tert-butylphényl)-[1,2,4]oxadiazol-5-ylméthylène]-cyclohexyl}-phénylméthyl)-diméthylamine (diastéréo-isomère non polaire)
124. la [{4-[3-(4-tert-butylphényl)-[1,2,4]oxadiazol-5-ylméthylène]-cyclohexyl}-(3-fluorophényl)-méthyl]-diméthylamine (diastéréo-isomère polaire)
125. la [{4-[3-(4-tert-butylphényl)-[1,2,4]oxadiazol-5-ylméthylène]-cyclohexyl}-(3-fluorophényl)-méthyl]-diméthylamine (diastéréo-isomère non polaire)
126. la [{4-[3-(4-tert-butylphényl)-[1,2,4]oxadiazol-5-ylméthylène]-cyclohexyl}-(4-chlorophényl)-méthyl]-diméthylamine (diastéréo-isomère polaire)
127. la [{4-[3-(4-tert-butylphényl)-[1,2,4]oxadiazol-5-ylméthylène]-cyclohexyl}-(4-chlorophényl)-méthyl]-diméthylamine (diastéréo-isomère non polaire)
128. la (1-{4-[3-(4-tert-butylphényl)-[1,2,4]oxadiazol-5-ylméthylène]-cyclohexyl}-3-phénylpropyl)-diméthylamine (diastéréo-isomère polaire)
129. la (1-{4-[3-(4-tert-butylphényl)-[1,2,4]oxadiazol-5-ylméthylène]-cyclohexyl}-3-phénylpropyl)-diméthylamine (diastéréo-isomère non polaire)
130. la [{4-[3-(4-tert-butylphényl)-[1,2,4]oxadiazol-5-ylméthylène]-cyclohexyl}-(4-fluorophényl)-méthyl]-diméthylamine (diastéréo-isomère polaire)
131. la [{4-[3-(4-tert-butylphényl)-[1,2,4]oxadiazol-5-ylméthylène]-cyclohexyl}-(4-fluorophényl)-méthyl]-diméthylamine (diastéréo-isomère non polaire)
132. la ({4-[3-(4-tert-butylphényl)-[1,2,4]oxadiazol-5-ylméthylène]-cyclohexyl}-thiophén-2-ylméthyl)-diméthylamine (diastéréo-isomère polaire)
133. la ({4-[3-(4-tert-butylphényl)-[1,2,4]oxadiazol-5-ylméthylène]-cyclohexyl}-thiophén-2-ylméthyl)-diméthylamine (diastéréo-isomère non polaire)
134. la diméthyl-{phényl-[4-(3-m-toluyl-[1,2,4]oxadiazol-5-ylméthylène)-cyclohexyl]-méthyl}-amine (diastéréo-isomère polaire)
135. la diméthyl-{phényl-[4-(3-m-toluyl-[1,2,4]oxadiazol-5-ylméthylène)-cyclohexyl]-méthyl}-amine (diastéréo-isomère non polaire)
136. la {(3-fluorophényl)-[4-(3-m-toluyl[1,2,4]oxadiazol-5-ylméthylène)-cyclohexyl]-méthyl}-diméthylamine (diastéréo-isomère polaire)
137. la {(3-fluorophényl)-[4-(3-m-toluyl-[1,2,4]oxadiazol-5-ylméthylène)-cyclohexyl]-méthyl}-diméthylamine (diastéréo-isomère non polaire)
138. la {(4-chlorophényl)-[4-(3-m-toluyl-[1,2,4]oxadiazol-5-ylméthylène)-cyclohexyl]-méthyl}-diméthylamine (diastéréo-isomère polaire)
139. la {(4-chlorophényl)-[4-(3-m-toluyl-[1,2,4]oxadiazol-5-ylméthylène)-cyclohexyl]-méthyl}-diméthylamine (diastéréo-isomère non polaire)
140. la ((4-fluorophényl)-{4-[2-(3-phényl-[1,2,4]oxadiazol-5-yl)-vinyl]-cyclohexyl}-méthyl)-diméthylamine
141. la [(4-{2-[3-(2,4-difluorophényl)-[1,2,4]oxadiazol-5-yl]-vinyl}-cyclohexyl)-(4-fluorophényl)-méthyl]-diméthylamine
142. la [(4-fluorophényl)-(4-{2-[3-(4-méthoxybenzyl)-[1,2,4]oxadiazol-5-yl]-vinyl}-cyclohexyl)-méthyl]-diméthylamine
143. la [(4-{2-[3-(4-chlorobenzyl)-[1,2,4]oxadiazol-5-yl]-vinyl}-cyclohexyl)-(4-fluorophényl)-méthyl]-diméthylamine
144. la diméthyl-(3-phényl-1-{4-[2-(3-phényl-[1,2,4]oxadiazol-5-yl)-vinyl]-cyclohexyl}-propyl)-amine
145. la [1-(4-{2-[3-(2,4-difluorophényl)-[1,2,4]oxadiazol-5-yl]-vinyl}-cyclohexyl)-3-phénylpropyl]-diméthylamine
146. la [1-(4-{2-[3-(4-méthoxybenzyl)-[1,2,4]oxadiazol-5-yl]-vinyl}-cyclohexyl)-3-phénylpropyl]-diméthylamine
147. la [1-(4-{2-[3-(4-chlorobenzyl)-[1,2,4]oxadiazol-5-yl]-vinyl}-cyclohexyl)-3-phénylpropyl]-diméthylamine
148. la diméthyl-(phényl-{4-[2-(3-phényl-[1,2,4]oxadiazol-5-yl)-vinyl]-cyclohexyl}-méthyl)-amine
149. la [(4-{2-[3-(4-chlorobenzyl)-[1,2,4]oxadiazol-5-yl]-vinyl}-cyclohexyl)-phénylméthyl]-diméthylamine (diastéréo-isomère polaire)
150. la [(4-{2-[3-(4-chlorobenzyl)-[1,2,4]oxadiazol-5-yl]-vinyl}-cyclohexyl)-phénylméthyl]-diméthylamine (diastéréo-isomère non polaire)
151. la [(4-chlorophényl)-(4-{2-[3-(2,4-difluorophényl)-[1,2,4]oxadiazol-5-yl]-vinyl}-cyclohexyl)-méthyl]-diméthylamine
152. la [(4-chlorophényl)-(4-{2-[3-(4-méthoxybenzyl)-[1,2,4]oxadiazol-5-yl]-vinyl}-cyclohexyl)-méthyl]-diméthylamine
153. la [(4-{2-[3-(4-chlorobenzyl)-[1,2,4]oxadiazol-5-yl]-vinyl}-cyclohexyl)-(4-chlorophényl)-méthyl]-diméthylamine
154. la [(4-{2-[3-(4-méthoxybenzyl)-[1,2,4]oxadiazol-5-yl]-vinyl}-cyclohexyl)-thiophén-2-ylméthyl]-diméthylamine
155. la [(4-{2-[3-(4-chlorobenzyl)-[1,2,4]oxadiazol-5-yl]-vinyl}-cyclohexyl)-thiophén-2-ylméthyl]-diméthylamine
156. la ((3-fluorophényl)-{4-[2-(3-phényl-[1,2,4]oxadiazol-5-yl)-vinyl]-cyclohexyl}-méthyl)-diméthylamine
157. la [(3-fluorophényl)-(4-{2-[3-(4-méthoxybenzyl)-[1,2,4]oxadiazol-5-yl]-vinyl}-cyclohexyl)-méthyl]-diméthylamine
158. la ((4-fluorophényl)-{4-[2-(3-p-toluyl[1,2,4]oxadiazol-5-yl)-vinyl]-cyclohexyl}-méthyl)-diméthylamine
159. la [(4-{2-[3-(3,4-diméthoxyphényl)-[1,2,4]oxadiazol-5-yl]-vinyl}-cyclohexyl)-(4-fluorophényl)-méthyl]-diméthylamine
160. la diméthyl-(3-phényl-1-{4-[2-(3-p-toluyl-[1,2,4]oxadiazol-5-yl)-vinyl]-cyclohexyl}-propyl)-amine
161. la [1-(4-{2-[3-(3,4-diméthoxyphényl)-[1,2,4]oxadiazol-5-yl]-vinyl}-cyclohexyl)-3-phénylpropyl]-diméthylamine
162. la diméthyl-(phényl-{4-[2-(3-p-toluyl-[1,2,4]oxadiazol-5-yl)-vinyl]-cyclohexyl}-méthyl)-amine
163. la [(4-{2-[3-(3,4-diméthoxy-phényl)-[1,2,4]oxadiazol-5-yl}-vinyl]-cyclohexyl)-phénylméthyl]-diméthylamine
164. la ((4-chlorophényl)-{4-[2-(3-p-toluyl-[1,2,4]oxadiazol-5-yl)-vinyl]-cyclohexyl}-méthyl)-diméthylamine
165. la [(4-chlorophényl)-(4-{2-[3-(3,4-diméthoxyphényl)-[1,2,4]oxadiazol-5-yl]-vinyl}-cyclohexyl)-méthyl]-diméthylamine
166. la diméthyl-(thiophén-2-yl-{4-[2-(3-p-toluyl-[1,2,4]oxadiazol-5-yl)-vinyl]-cyclohexyl}-méthyl)-amine
167. la [(4-{2-[3-(3,4-diméthoxyphényl)-[1,2,4]oxadiazol-5-yl]-vinyl}-cyclohexyl)-thiophén-2-ylméthyl]-diméthylamine
168. la ((3-fluorophényl)-{4-[2-(3-p-toluyl-[1,2,4]oxadiazol-5-yl)-vinyl]-cyclohexyl}-méthyl)-diméthylamine
169. la [(4-{2-[3-(3,4-diméthoxyphényl)-[1,2,4]oxadiazol-5-yl]-vinyl}-cyclohexyl)-(3-fluorophényl)-méthyl]-diméthylamine
170. la [(4-{2-[3-(3-chlorophényl)-[1,2,4]oxadiazol-5-yl]-vinyl}-cyclohexyl)-(4-fluorophényl)-méthyl]-diméthylamine
171. la [(4-{2-[3-(4-tert-butylphényl)-[1,2,4]oxadiazol-5-yl]-vinyl}-cyclohexyl)-(4-fluorophényl)-méthyl]-diméthylamine
172. la [(4-{2-[3-(4-tert-butylphényl)-[1,2,4]oxadiazol-5-yl]-vinyl}-cyclohexyl)-(4-fluorophényl)-méthyl]-diméthylamine
173. la ((4-fluorophényl)-{4-[2-(3-m-toluyl-[1,2,4]oxadiazol-5-yl)-vinyl]-cyclohexyl}-méthyl)-diméthylamine
174. la [1-(4-{2-[3-(3-chlorophényl)-[1,2,4]oxadiazol-5-yl]-vinyl}-cyclohexyl)-3-phénylpropyl]-diméthylamine
175. la [1-(4-{2-[3-(4-tert-butylphényl)-[1,2,4]oxadiazol-5-yl]-vinyl}-cyclohexyl)-3-phénylpropyl]-diméthylamine (diastéréo-isomère polaire)
176. la [1-(4-{2-[3-(4-tert-butylphényl)-[1,2,4]oxadiazol-5-yl]-vinyl}-cyclohexyl)-3-phénylpropyl]-diméthylamine (diastéréo-isomère non polaire)
177. la diméthyl-(3-phényl-1-{4-[2-(3-m-toluyl-[1,2,4]oxadiazol-5-yl)-vinyl]-cyclohexyl}-propyl)-amine (diastéréo-isomère polaire)
178. la diméthyl-(3-phényl-1-{4-[2-(3-m-toluyl-[1,2,4]oxadiazol-5-yl)-vinyl]-cyclohexyl}-propyl)-amine (diastéréo-isomère non polaire)
179. la [(4-{2-[3-(3-chlorophényl)-[1,2,4]oxadiazol-5-yl]-vinyl}-cyclohexyl)-phénylméthyl]-diméthylamine
180. la [(4-{2-[3-(4-tert-butylphényl)-[1,2,4]oxadiazol-5-yl]-vinyl}-cyclohexyl)-phénylméthyl]-diméthylamine (diastéréo-isomère polaire)
181. la [(4-{2-[3-(4-tert-butylphényl)-[1,2,4]oxadiazol-5-yl]-vinyl}-cyclohexyl)-phénylméthyl]-diméthylamine (diastéréo-isomère non polaire)
182. la diméthyl-(phényl-{4-[2-(3-m-toluyl-[1,2,4]oxadiazol-5-yl)-vinyl]-cyclohexyl}-méthyl)-amine (diastéréo-isomère polaire)
183. la diméthyl-(phényl-{4-[2-(3-m-toluyl-[1,2,4]oxadiazol-5-yl)-vinyl]-cyclohexyl}-méthyl)-amine (diastéréo-isomère non polaire)
184. la [(4-{2-[3-(4-chlorophényl)-[1,2,4]oxadiazol-5-yl]-vinyl}-cyclohexyl)-phénylméthyl]-diméthylamine (diastéréo-isomère polaire)
185. la [(4-{2-(3-(4-chlorophényl)-[1,2,4]oxadiazol-5-yl]-vinyl}-cyclohexyl)-phénylméthyl]-diméthylamine (diastéréo-isomère non polaire)
186. la [(4-chlorophényl)-(4-{2-[3-(3-chlorophényl)-[1,2,4]oxadiazol-5-yl]-vinyl}-cyclohexyl)-méthyl]-diméthylamine
187. la [(4-{2-[3-(4-tert-butylphényl)-[1,2,4]oxadiazol-5-yl]-vinyl}-cyclohexyl)-(4-chlorophényl)-méthyl]-diméthylamine (diastéréo-isomère polaire)
188. la [(4-{2-[3-(4-tert-butylphényl)-[1,2,4]oxadiazol-5-yl]-vinyl}-cyclohexyl)-(4-chlorophényl)-méthyl]-diméthylamine (diastéréo-isomère non polaire)
189. la ((4-chlorophényl)-{4-[2-(3-m-toluyl-[1,2,4]oxadiazol-5-yl)-vinyl]-cyclohexyl}-méthyl)-diméthylamine (diastéréo-isomère polaire)
190. la ((4-chlorophényl)-{4-[2-(3-m-toluyl-[1,2,4]oxadiazol-5-yl)-vinyl]-cyclohexyl}-méthyl)-diméthylamine (diastéréo-isomère non polaire)
191. la [(4-chlorophényl)-(4-{2-[3-(4-chlorophényl)-[1,2,4]oxadiazol-5-yl]-vinyl}-cyclohexyl)-méthyl]-diméthylamine
192. la [(4-{2-[3-(3-chlorophényl)-[1,2,4]oxadiazol-5-yl]-vinyl}-cyclohexyl)-thiophén-2-ylméthyl]-diméthylamine
193. la [(4-{2-[3-(4-tert-butylphényl)-[1,2,4]oxadiazol-5-yl]-vinyl}-cyclohexyl)-thiophén-2-ylméthyl]-diméthylamine (diastéréo-isomère polaire)
194. la [(4-{2-[3-(4-tert-butylphényl)-[1,2,4]oxadiazol-5-yl]-vinyl}-cyclohexyl)-thiophén-2-ylméthyl]-diméthylamine (diastéréo-isomère non polaire)
195. la diméthyl-(thiophén-2-yl-{4-[2-(3-m-toluyl-[1,2,4]oxadiazol-5-yl)-vinyl]-cyclohexyl}-méthyl)-amine
196. la [(4-{2-[3-(4-chlorophényl)-[1,2,4]oxadiazol-5-yl]-vinyl}-cyclohexyl)-thiophén-2-ylméthyl]-diméthylamine
197. la [(4-{2-[3-(4-chlorophényl)-[1,2,4]oxadiazol-5-yl]-vinyl}-cyclohexyl)-(3-fluorophényl)-méthyl]-diméthylamine
198. la [(4-{2-[3-(4-tert-butylphényl)-[1,2,4]oxadiazol-5-yl]-vinyl}-cyclohexyl)-(3-fluorophényl)-méthyl]-diméthylamine (diastéréo-isomère polaire)
199. la [(4-{2-[3-(4-tert-butylphényl)-[1,2,4]oxadiazol-5-yl]-vinyl}-cyclohexyl)-(3-fluorophényl)-méthyl]-diméthylamine (diastéréo-isomère non polaire)
201. la ((3-fluorophényl)-{4-[2-(3-m-toluyl-[1,2,4]oxadiazol-5-yl)-vinyl]-cyclohexyl}-méthyl)-diméthylamine
202. la [(4-{2-[3-(4-chlorophényl)-[1,2,4]oxadiazol-5-yl]-vinyl}-cyclohexyl)-(3-fluorophényl)-méthyl]-diméthylamine
203. la [(4-{2-[3-(2,3-dichlorophényl)-[1,2,4]oxadiazol-5-yl]-vinyl}-cyclohexyl)-(4-fluorophényl)-méthyl]-diméthylamine
204. la [(4-fluorophényl)-(4-{2-[3-(4-trifluorométhoxyphényl)-[1,2,4]oxadiazol-5-yl]-vinyl}-cyclohexyl)-méthyl]-diméthylamine
205. la [(4-{2-[3-(3,4-diméthoxybenzyl)-[1,2,4]oxadiazol-5-yl]-vinyl}-cyclohexyl)-(4-fluorophényl)-méthyl]-diméthylamine
206. la [(4-{2-[3-(1,5-diméthyl-1H-pyrrol-2-yl)-[1,2,4]oxadiazol-5-yl]-vinyl}-cyclohexyl)-(4-fluorophényl)-méthyl]-diméthylamine
207. la [1-(4-{2-[3-(2,3-dichlorophényl)-[1,2,4]oxadiazol-5-yl]-vinyl}-cyclohexyl)-3-phénylpropyl]-diméthylamine
208. la diméthyl-[3-phényl-1-(4-{2-[3-(4-trifluorométhoxyphényl)-[1,2,4]oxadiazol-5-yl]-vinyl}-cyclohexyl)-propyl]-amine (diastéréo-isomère polaire)
209. la diméthyl-[3-phényl-1-(4-{2-[3-(4-trifluorométhoxyphényl)-[1,2,4]oxadiazol-5-yl]-vinyl}-cyclohexyl)-propyl]-amine (diastéréo-isomère non polaire)
210. la [1-(4-{2-[3-(3,4-diméthoxybenzyl)-[1,2,4]oxadiazol-5-yl]-vinyl}-cyclohexyl)-3-phénylpropyl]-diméthylamine (diastéréo-isomère polaire)
211. la [1-(4-{2-[3-(3,4-diméthoxybenzyl)-[1,2,4]oxadiazol-5-yl]-vinyl}-cyclohexyl)-3-phénylpropyl]-diméthylamine (diastéréo-isomère non polaire)
212. la [1-(4-{2-[3-(1,5-diméthyl-1H-pyrrol-2-yl)-[1,2,4]oxadiazol-5-yl]-vinyl}-cyclohexyl)-3-phénylpropyl]-diméthylamine
213. la [(4-{2-[3-(2,3-dichlorophényl)-[1,2,4]oxadiazol-5-yl]-vinyl}-cyclohexyl)-phénylméthyl]-diméthylamine
214. la [(4-{2-[3-(3,4-diméthoxybenzyl)-[1,2,4]oxadiazol-5-yl]-vinyl}-cyclohexyl)-phénylméthyl]-diméthylamine (diastéréo-isomère polaire)
215. la [(4-{2-[3-(3,4-diméthoxybenzyl)-[1,2,4]oxadiazol-5-yl]-vinyl}-cyclohexyl)-phénylméthyl]-diméthylamine (diastéréo-isomère non polaire)
217. la [(4-{2-[3-(1,5-diméthyl-1H-pyrrol-2-yl)-[1,2,4]oxadiazol-5-yl]-vinyl}-cyclohexyl)-phénylméthyl]-diméthylamine
218. la [(4-chlorophényl)-(4-{2-[3-(2,3-dichlorophényl)-[1,2,4]oxadiazol-5-yl]-vinyl}-cyclohexyl)-méthyl]-diméthylamine
219. la [(4-chlorophényl)-(4-{2-[3-(4-trifluorométhoxyphényl)-[1,2,4]oxadiazol-5-yl]-vinyl}-cyclohexyl)-méthyl]-diméthylamine
220. la [(4-chlorophényl)-(4-{2-[3-(3,4-diméthoxybenzyl)-[1,2,4]oxadiazol-5-yl]-vinyl}-cyclohexyl)-méthyl]-diméthylamine
221. la [(4-chlorophényl)-(4-{2-[3-(1,5-diméthyl-1H-pyrrol-2-yl)-[1,2,4]oxadiazol-5-yl]-vinyl}-cyclohexyl)-méthyl]-diméthylamine
222. la [(4-{2-[3-(2,3-dichlorophényl)-[1,2,4]oxadiazol-5-yl]-vinyl}-cyclohexyl)-thiophén-2-ylméthyl]-diméthylamine
223. la diméthyl-[thiophén-2-yl-(4-{2-[3-(4-trifluorométhoxyphényl)-[1,2,4]oxadiazol-5-yl]-vinyl}-cyclohexyl)-méthyl]-amine
224. la [(4-{2-[3-(1,5-diméthyl-1H-pyrrol-2-yl)-[1,2,4]oxadiazol-5-yl]-vinyl}-cyclohexyl)-thiophén-2-ylméthyl]-diméthylamine
225. la [(4-{2-[3-(2,3-dichlorophényl)-[1,2,4]oxadiazol-5-yl]-vinyl}-cyclohexyl)-(3-fluorophényl)-méthyl]-diméthylamine
226. la [(3-fluorophényl)-(4-{2-[3-(4-trifluorométhoxyphényl)-[1,2,4]oxadiazol-5-yl]-vinyl}-cyclohexyl)-méthyl]-diméthylamine (diastéréo-isomère polaire)
227. la [(3-fluorophényl)-(4-{2-[3-(4-trifluorométhoxyphényl)-[1,2,4]oxadiazol-5-yl]-vinyl}-cyclohexyl)-méthyl]-diméthylamine (diastéréo-isomère non polaire)
228. la [(4-{2-[3-(3,4-diméthoxybenzyl)-[1,2,4]oxadiazol-5-yl]-vinyl}-cyclohexyl)-(3-fluorophényl)-méthyl]-diméthylamine (diastéréo-isomère polaire)
229. la [(4-{2-[3-(3,4-diméthoxybenzyl)-[1,2,4]oxadiazol-5-yl]-vinyl}-cyclohexyl)-(3-fluorophényl)-méthyl]-diméthylamine (diastéréo-isomère non polaire)
231. la [(4-{2-[3-(1,5-diméthyl-1H-pyrrol-2-yl)-[1,2,4]oxadiazol-5-yl]-vinyl}-cyclohexyl)-(3-fluorophényl)-méthyl]-diméthylamine
232. la {(3-fluorophényl)-[4-(3-phényl-[1,2,4]oxadiazol-5-ylméthyl)-cyclohexyl]-méthyl}-diméthylamine
233. la {(4-fluorophényl)-[4-(3-phényl-[1,2,4]oxadiazol-5-ylméthyl)-cyclohexyl]-méthyl}-diméthylamine
234. la diméthyl-{phényl-[4-(3-phényl-[1,2,4]oxadiazol-5-ylméthyl)-cyclohexyl]-méthyl}-amine
235. la [{4-[3-(2,4-difluorophényl)-[1,2,4]oxadiazol-5-ylméthyl]-cyclohexyl}-(3-fluorophényl)-méthyl]-diméthylamine
236. la [{4-[3-(2,4-difluorophényl)-[1,2,4]oxadiazol-5-ylméthyl]-cyclohexyl}-(4-fluorophényl)-méthyl]-diméthylamine
237. la ((3-fluorophényl)-{4-[3-(4-méthoxybenzyl)-[1,2,4]oxadiazol-5-ylméthyl]-cyclohexyl}-méthyl)-diméthylamine
238. la ((4-fluorophényl)-{4-[3-(4-méthoxybenzyl)-[1,2,4]oxadiazol-5-ylméthyl]-cyclohexyl}-méthyl)-diméthylamine
239. la ({4-[3-(4-méthoxybenzyl)-[1,2,4]oxadiazol-5-ylméthyl]-cyclohexyl}-phénylméthyl)-diméthylamine
240. la [f4-[3-(4-chlorobenzyl)-[1,2,4]oxadiazol-5-ylméthyl]-cyclohexyl}-(3-fluorophényl)-méthyl]-diméthylamine
241. la [14-[3-(4-chlorobenzyl)-[1,2,4]oxadiazol-5-ylméthyl]-cyclohexyl}-(4-fluorophényl)-méthyl]-diméthylamine
242. la [14-[3-(3-chlorophényl)-[1,2,4]oxadiazol-5-ylméthyl]-cyclohexyl}-(4-fluorophényl)-méthyl]-diméthylamine
243. la ({4-[3-(3-chlorophényl)-[1,2,4]oxadiazol-5-ylméthyl]-cyclohexyl}-phénylméthyl)-diméthylamine
244. la [{4-[3-(2,3-dichlorophényl)-[1,2,4]oxadiazol-5-ylméthyl]-cyclohexyl}-(3-fluorophényl)-méthyl]-diméthylamine
245. la [{4-[3-(2,3-dichlorophényl)-[1,2,4]oxadiazol-5-ylméthyl]-cyclohexyl}-(4-fluorophényl)-méthyl]-diméthylamine
246. la ({4-[3-(2,3-dichlorophényl)-[1,2,4]oxadiazol-5-ylméthyl]cyclohexyl}-phénylméthyl)-diméthylamine
247. la {(3-fluorophényl)-[4-(3-p-toluyl-[1,2,4]oxadiazol-5-ylméthyl)-cyclohexyl]-méthyl}-diméthylamine
248. la {(4-fluorophényl)-[4-(3-p-toluyl-[1,2,4]oxadiazol-5-ylméthyl)-cyclohexyl]-méthyl}-diméthylamine
249. la diméthyl-{phényl-[4-(3-p-toluyl-[1,2,4]oxadiazol-5-ylméthyl)-cyclohexyl]-méthyl}-amine
250. la [{4-[3-(3,4-diméthoxyphényl)-[1,2,4]oxadiazol-5-ylméthyl]-cyclohexyl}-(3-fluorophényl)-méthyl]-diméthylamine
251. la [{4-[3-(3,4-diméthoxyphényl)-[1,2,4]oxadiazol-5-ylméthyl]-cyclohexyl}-(4-fluorophényl)-méthyl]-diméthylamine
252. la ({4-[3-(3,4-diméthoxyphényl)-[1,2,4]oxadiazol-5-ylméthyl]-cyclohexyl}-phénylméthyl)-diméthylamine
253. la [{4-[3-(4-tert-butylphényl)-[1,2,4]oxadiazol-5-ylméthyl]-cyclohexyl}-(3-fluorophényl)-méthyl]-diméthylamine
254. la [{4-[3-(4-tert-butylphényl)-[1,2,4]oxadiazol-5-ylméthyl]-cyclohexyl}-(4-fluorophényl)-méthyl]-diméthylamine
255. la ({4-[3-(4-tert-butylphényl)-(1,2,4]oxadiazol-5-ylméthyl]-cyclohexyl}-phénylméthyl)-diméthylamine
256. la {(3-fluorophényl)-[4-(3-m-toluyl-[1,2,4]oxadiazol-5-ylméthyl)-cyclohexyl]-méthyl}-diméthylamine
257. la {(4-fluorophényl)-[4-(3-m-toluyl-[1,2,4]oxadiazol-5-ylméthyl)-cyclohexyl]-méthyl}-diméthylamine
258. la diméthyl-{phényl-[4-(3-m-toluyl-[1,2,4]oxadiazol-5-ylméthyl)-cyclohexyl]-méthyl}-amine
259. la [{4-[3-(4-chlorophényl)-[1,2,4]oxadiazol-5-ylméthyl]-cyclohexyl}-(3-fluorophényl)-méthyl]-diméthylamine
260. la ({4-[3-(4-chlorophényl)-[1,2,4]oxadiazol-5-ylméthyl]-cyclohexyl}-phénylméthyl)-diméthylamine
261. la ((3-fluorophényl)-{4-[3-(4-trifluorométhoxyphényl)-[1,2,4]oxadiazol-5-ylméthyl]-cyclohexyl}-méthyl)-diméthylamine
262. la ((4-fluorophényl)-{4-[3-(4-trifluorométhoxyphényl)-[1,2,4]oxadiazol-5-ylméthyl]-cyclohexyl}-méthyl)-diméthylamine
263. la diméthyl-(phényl-{4-[3-(4-trifluorométhoxyphényl)-[1,2,4]oxadiazol-5-ylméthyl]-cyclohexyl}-méthyl)-amine
264. la ((4-fluorophényl)-{4-[2-(3-phényl-[1,2,4]oxadiazol-5-yl)-éthyl]-cyclohexyl}-méthyl)-diméthylamine
265. la diméthyl-(phényl-{4-[2-(3-phényl-[1,2,4]oxadiazol-5-yl)-éthyl]-cyclohexyl}-méthyl)-amine
266. la diméthyl-(3-phényl-1-{4-[2-(3-phényl-[1,2,4]oxadiazol-5-yl)-éthyl]-cyclohexyl}-propyl)-amine
267. la ((3-fluorophényl)-{4-[2-(3-phényl-[1,2,4]oxadiazol-5-yl)-éthyl]-cyclohexyl}-méthyl)-diméthylamine
268. la [(4-fluorophényl)-(4-{2-[3-(4-méthoxybenzyl)-[1,2,4]oxadiazol-5-yl]-éthyl}-cyclohexyl)-méthyl]-diméthylamine
269. la [(4-{2-[3-(4-méthoxybenzyl)-[1,2,4]oxadiazol-5-yl]-éthyl}-cyclohexyl)-phénylméthyl]-diméthylamine
270. la [1-(4-{2-[3-(4-méthoxybenzyl)-[1,2,4]oxadiazol-5-yl]-éthyl}-cyclohexyl)-3-phénylpropyl]-diméthylamine
271. la [(3-fluorophényl)-(4-{2-[3-(4-méthoxybenzyl)-[1,2,4]oxadiazol-5-yl]-éthyl}-cyclohexyl)-méthyl]-diméthylamine
272. la [(4-{2-[3-(4-chlorobenzyl)-[1,2,4]oxadiazol-5-yl]-éthyl}-cyclohexyl)-(4-fluorophényl)-méthyl]-diméthylamine
273. la [(4-{2-[3-(4-chlorobenzyl)-[1,2,4]oxadiazol-5-yl]-éthyl}-cyclohexyl)-phénylméthyl]-diméthylamine
274. la [1-(4-{2-[3-(4-chlorobenzyl)-[1,2,4]oxadiazol-5-yl]-éthyl}-cyclohexyl)-3-phénylpropyl]-diméthylamine
275. la [(4-{2-[3-(4-chlorobenzyl)-[1,2,4]oxadiazol-5-yl]-éthyl}-cyclohexyl)-(3-fluorophényl)-méthyl]-diméthylamine
276. la [(4-{2-[3-(3-chlorophényl)-[1,2,4]oxadiazol-5-yl]-éthyl}-cyclohexyl)-(4-fluorophényl)-méthyl]-diméthylamine
277. la [(4-{2-[3-(3-chlorophényl)-[1,2,4]oxadiazol-5-yl]-éthyl}-cyclohexyl)-phénylméthyl]-diméthylamine
278. la [1-(4-{2-[3-(3-chlorophényl)-[1,2,4]oxadiazol-5-yl]-éthyl}-cyclohexyl)-3-phénylpropyl]-diméthylamine
279. la [(4-{2-[3-(3-chlorophényl)-[1,2,4]oxadiazol-5-yl]-éthyl}-cyclohexyl)-(3-fluorophényl)-méthyl]-diméthylamine
280. la [(4-{2-[3-(2,4-difluorophényl)-[1,2,4]oxadiazol-5-yl]-éthyl}-cyclohexyl)-(4-fluorophényl)-méthyl]-diméthylamine
281. la [(4-{2-[3-(2,4-difluorophényl)-[1,2,4]oxadiazol-5-yl]-éthyl}-cyclohexyl)-phénylméthyl]-diméthylamine
282. la [(4-{2-[3-(2,4-difluorophényl)-[1,2,4]oxadiazol-5-yl]-éthyl}-cyclohexyl)-(3-fluorophényl)-méthyl]-diméthylamine
283. la [(4-{2-[3-(3,4-diméthoxyphényl)-[1,2,4]oxadiazol-5-yl]-éthyl}-cyclohexyl)-(4-fluorophényl)-méthyl]-diméthylamine
284. la [(4-{2-[3-(3,4-diméthoxyphényl)-[1,2,4]oxadiazol-5-yl]-éthyl}-cyclohexyl)-phénylméthyl]-diméthylamine
285. la [1-(4-{2-[3-(3,4-diméthoxyphényl)-[1,2,4]oxadiazol-5-yl]-éthyl}-cyclohexyl)-3-phénylpropyl]-diméthylamine
286. la [(4-{2-[3-(3,4-diméthoxyphényl)-[1,2,4]oxadiazol-5-yl]-éthyl}-cyclohexyl)-(3-fluorophényl)-méthyl]-diméthylamine
287. la ((4-fluorophényl)-{4-[2-(3-p-toluyl-[1,2,4]oxadiazol-5-yl)-éthyl]-cyclohexyl}-méthyl)-diméthylamine
288. la diméthyl-(phényl-{4-[2-(3-p-toluyl-[1,2,4]oxadiazol-5-yl)-éthyl]-cyclohexyl}-méthyl)-amine
289. la diméthyl-(3-phényl-1-{4-[2-(3-p-toluyl-[1,2,4]oxadiazol-5-yl)-éthyl]-cyclohexyl}-propyl)-amine
290. la ((3-fluorophényl)-{4-[2-(3-p-toluyl-[1,2,4]oxadiazol-5-yl)-éthyl]-cyclohexyl}-méthyl)-diméthylamine
291. la [(4-{2-[3-(4-tert-butylphényl)-[1,2,4]oxadiazol-5-yl]-éthyl}-cyclohexyl)-(4-fluorophényl)-méthyl]-diméthylamine
292. la [(4-{2-[3-(4-tert-butylphényl)-[1,2,4]oxadiazol-5-yl]-éthyl}-cyclohexyl)-phénylméthyl]-diméthylamine
293. la [1-(4-{2-[3-(4-tert-butylphényl)-[1,2,4]oxadiazol-5-yl]-éthyl}-cyclohexyl)-3-phénylpropyl]-diméthylamine
294. la [(4-{2-[3-(4-tert-butylphényl)-[1,2,4]oxadiazol-5-yl]-éthyl}-cyclohexyl)-(3-fluorophényl)-méthyl]-diméthylamine
295. la ((4-fluorophényl)-{4-[2-(3-m-toluyl-[1,2,4]oxadiazol-5-yl)-éthyl]-cyclohexyl}-méthyl)-diméthylamine
296. la diméthyl-(phényl-{4-[2-(3-m-toluyl-[1,2,4]oxadiazol-5-yl)-éthyl]-cyclohexyl}-méthyl)-amine
297. la diméthyl-(3-phényl-1-{4-[2-(3-m-toluyl-[1,2,4]oxadiazol-5-yl)-éthyl]-cyclohexyl}-propyl)-amine
298. la ((3-fluorophényl)-{4-[2-(3-m-toluyl-[1,2,4]oxadiazol-5-yl)-éthyl]-cyclohexyl}-méthyl)-diméthylamine
299. la [(4-{2-[3-(4-chlorophényl)-[1,2,4]oxadiazol-5-yl]-éthyl}-cyclohexyl)-(4-fluorophényl)-méthyl]-diméthylamine
300. la [(4-{2-[3-(4-chlorophényl)-[1,2,4]oxadiazol-5-yl]-éthyl}-cyclohexyl)-phénylméthyl]-diméthylamine
301. la [1-(4-{2-[3-(4-chlorophényl)-[1,2,4]oxadiazol-5-yl]-éthyl}-cyclohexyl)-3-phénylpropyl]-diméthylamine
302. la [(4-{2-[3-(4-chlorophényl)-[1,2,4]oxadiazol-5-yl]-éthyl}-cyclohexyl)-(3-fluorophényl)-méthyl]-diméthylamine
303. la [(4-{2-[3-(2,3-dichlorophényl)-[1,2,4]oxadiazol-5-yl]-éthyl}-cyclohexyl)-(4-fluorophényl)-méthyl]-diméthylamine
304. la [(4-{2-[3-(2,3-dichlorophényl)-[1,2,4]oxadiazol-5-yl]-éthyl}-cyclohexyl)-phénylméthyl]-diméthylamine
305. la [1-(4-{2-[3-(2,3-dichlorophényl)-[1,2,4]oxadiazol-5-yl]-éthyl}-cyclohexyl)-3-phénylpropyl]-diméthylamine
306. la [(4-{2-[3-(2,3-dichlorophényl)-[1,2,4]oxadiazol-5-yl]-éthyl}-cyclohexyl)-(3-fluorophényl)-méthyl]-diméthylamine
307. la [(4-fluorophényl)-(4-{2-[3-(4-trifluorométhoxyphényl)-[1,2,4]oxadiazol-5-yl]-éthyl}-cyclohexyl)-méthyl]-diméthylamine
308. la diméthyl-[phényl-(4-{2-[3-(4-trifluorométhoxyphényl)-[1,2,4]oxadiazol-5-yl]-éthyl}-cyclohexyl)-méthyl]-amine
309. la diméthyl-[3-phényl-1-(4-{2-[3-(4-trifluorométhoxyphényl)-[1,2,4]oxadiazol-5-yl]-éthyl}-cyclohexyl)-propyl]-amine
310. la [(3-fluorophényl)-(4-{2-[3-(4-trifluorométhoxyphényl)-[1,2,4]oxadiazol-5-yl]-éthyl}-cyclohexyl)-méthyl]-diméthylamine
311. la (4-((3-(4-fluorobenzyl)-1,2,4-oxadiazol-5-yl)méthyl)cyclohexyl)(4-fluorophényl)-N,N-diméthylméthanamine
312. la (4-((3-(4-fluorobenzyl)-1,2,4-oxadiazol-5-yl)méthyl)cyclohexyl)-N,N-diméthyl(phényl)méthanamine
313. la (4-((3-(3-chlorobenzyl)-1,2,4-oxadiazol-5-yl)méthyl)cyclohexyl)(3-fluorophényl)-N,N-diméthylméthanamine
314. la (4-((3-(3-chlorobenzyl)-1,2,4-oxadiazol-5-yl)méthyl)cyclohexyl)(4-fluorophényl)-N,N-diméthylméthanamine
315. la (4-((3-(3-chlorobenzyl)-1,2,4-oxadiazol-5-yl)méthyl)cyclohexyl)-N,N-diméthyl(phényl)méthanamine
316. la (4-((3-(3-bromophényl)-1,2,4-oxadiazol-5-yl)méthyl)cyclohexyl)(3-fluorophényl)-N,N-diméthylméthanamine
317. la (4-((3-(3-bromophényl)-1,2,4-oxadiazol-5-yl)méthyl)cyclohexyl)(4-fluorophényl)-N,N-diméthylméthanamine
318. la (4-((3-(3-bromophényl)-1,2,4-oxadiazol-5-yl)méthyl)cyclohexyl)-N,N-diméthyl(phényl)méthanamine
319. la (4-((3-(4-bromobenzyl)-1,2,4-oxadiazol-5-yl)méthyl)cyclohexyl)(3-fluorophényl)-N,N-diméthylméthanamine
320. la (4-((3-(4-bromobenzyl)-1,2,4-oxadiazol-5-yl)méthyl)cyclohexyl)(4-fluorophényl)-N,N-diméthylméthanamine
321. la (4-((3-(4-bromobenzyl)-1,2,4-oxadiazol-5-yl)méthyl)cyclohexyl)-N,N-diméthyl(phényl)méthanamine
322. la (3-fluorophényl)-N,N-diméthyl(4-((3-(thiophén-2-ylméthyl)-1,2,4-oxadiazol-5-yl)méthyl)cyclohexyl)méthanamine
323. la (4-fluorophényl)-N,N-diméthyl(4-((3-(thiophén-2-ylméthyl)-1,2,4-oxadiazol-5-yl)méthyl)cyclohexyl)méthanamine
324. la N,N-diméthyl(phényl)(4-((3-(thiophén-2-ylméthyl)-1,2,4-oxadiazol-5-yl)méthyl)cyclohexyl)méthanamine
325. la (4-((3-benzyl-1,2,4-oxadiazol-5-yl)méthyl)cyclohexyl)(3-fluorophényl)-N,N-diméthylméthanamine
326. la (4-((3-benzyl-1,2,4-oxadiazol-5-yl)méthyl)cyclohexyl)(4-fluorophényl)-N,N-diméthylméthanamine
327. la (4-((3-benzyl-1,2,4-oxadiazol-5-yl)méthyl)cyclohexyl)-N,N-diméthyl(phényl)méthanamine
328. la (3-fluorophényl)-N,N-diméthyl(4-((3-phénéthyl-1,2,4-oxadiazol-5-yl)méthyl)cyclohexyl)méthanamine
329. la (4-fluorophényl)-N,N-diméthyl(4-((3-phénéthyl-1,2,4-oxadiazol-5-yl)méthyl)cyclohexyl)méthanamine
330. la N,N-diméthyl(4-((3-phénéthyl-1,2,4-oxadiazol-5-yl)méthyl)cyclohexyl)(phényl)méthanamine
331. la (4-((3-((1H-indol-3-yl)méthyl)-1,2,4-oxadiazol-5-yl)méthyl)cyclohexyl)(3-fluorophényl)-N,N-diméthylméthanamine.

9. Procédé pour la préparation d'un dérivé d'oxadiazole selon la revendication 1, des amidoximes de formule générale D étant transformées dans un milieu réactionnel avec addition d'une base, par exemple NaH, avec des esters tant saturés qu'insaturés de formule générale A en composés selon l'invention.

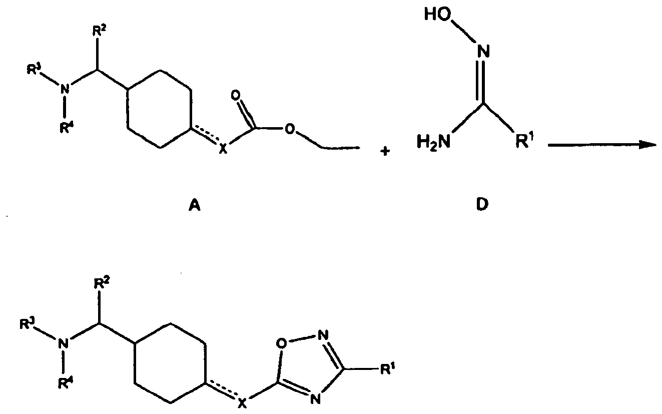

10. Médicament contenant au moins un dérivé substitué d'oxadiazole selon la revendication 1, le cas échéant sous forme du racémate ; des énantiomères, diastéréo-isomères, mélanges des énantiomères ou diastéréo-isomères ou d'un seul énantiomère ou diastéréo-isomère ; des bases et/ou des sels d'acides physiologiquement acceptables, ainsi que le cas échéant des additifs et/ou des adjuvants appropriés et/ou le cas échéant d'autres substances actives.

11. Utilisation d'un dérivé substitué d'oxadiazole selon la revendication 1, le cas échéant sous forme du racémate ; des énantiomères, diastéréo-isomères, mélanges des énantiomères ou diastéréo-isomères ou d'un seul énantiomère ou diastéréo-isomère ; des bases et/ou des sels d'acides physiologiquement acceptables pour la préparation d'un médicament destiné au traitement de la douleur, en particulier de la douleur aiguë, neuropathique ou chronique.

12. Utilisation d'un dérivé substitué d'oxadiazole selon la revendication 1, le cas échéant sous forme du racémate ; des énantiomères, diastéréo-isomères, mélanges des énantiomères ou diastéréo-isomères ou d'un seul énantiomère ou diastéréo-isomère ; des bases et/ou des sels d'acides physiologiquement acceptables pour la préparation d'un médicament destiné au traitement des dépressions, de l'incontinence urinaire, de la diarrhée, du prurit, de l'abus d'alcool et de l'abus de drogues, de la dépendance aux médicaments, du manque d'énergie et/ou à l'anxiolyse.
